# FASCICULE DE BREVET EUROPEEN

(11) **EP 0 912 613 B1**
(45) Date de publication et mention de la délivrance du brevet: **25.09.2002**
(21) Numéro de dépôt: 97934596.4
(22) Date de dépôt: 18.07.1997
(51) Int. Cl.: C08B 37/00, C07H 15/04, C07H 19/01, A61K 31/715

(54) **POLYSACCHARIDES SYNTHETIQUES, PROCEDE POUR LEUR PREPARATION ET COMPOSITIONS PHARMACEUTIQUES LES CONTENANT**
SYNTHETISCHE POLYSACCHARIDE, VERFAHREN ZU DEREN HERSTELLUNG UND DIESE ENTHALTENDE, PHARMAZEUTISCHE ZUSAMMENSETZUNGEN
SYNTHETIC POLYSACCHARIDES, PREPARATION METHOD THEREFOR AND PHARMACEUTICAL COMPOSITIONS CONTAINING SAME

(30) Priorité: 19.07.1996 FR 9609116
(43) Date de publication de la demande: 06.05.1999
(73) Titulaire: SANOFI-SYNTHELABO, 75635 Paris Cédex 13 (FR); Akzo Nobel N.V., 6824 BM Arnhem (NL)
(72) Inventeur: DRIGUEZ, Pierre, Alexandre, 31400 Toulouse (FR); DUCHAUSSOY, Philippe, 31300 Toulouse (FR); HERBERT, Jean-Marc, 31170 Tournefeuille (FR); PETITOU, Maurice, 75645 Paris Cedex 13 (FR); VAN BOECKEL, Constant, NL-5345 LX Oss (NL); GROOTENHUIS, Peter, NL-5344 HS Oss (NL); DREEF-TROMP, Cornélia, SN Wijcken (NL); BASTEN, Johannes, 6654 AV Afferden (NL)
(74) Mandataire: Bernasconi, Jean Raymond
(86) Numéro de dépôt international: FR9701344
(87) Numéro de publication internationale: WO98003554

(56) Documents cités:
- WO-A-97/47659

## Description

La présente invention concerne de nouveaux polysaccharides de synthèse possédant les activités pharmacologiques anticoagulante et antithrombotique de l'héparine.

L'héparine appartient à la famille des glycosaminoglycanes (GAGs), qui sont des polysaccharides sulfatés naturels hétérogènes.

Les préparations d'héparine sont des mélanges de chaînes comprenant un nombre d'unités monosaccharidiques allant de 10 jusqu'à 100 et plus. À cette hétérogénéité de taille s'ajoute une hétérogénéité de structure. au niveau de la nature des monosaccharides constitutifs, mais également au niveau des substituants qu'ils portent (L. Rodén in : « The Biochemistry of Glycoproteins and Glycosaminoglycans », Ed. by Lennarz W.J., Plenum Press, New York and London, 267-371, 1980).

Chaque famille de GAGs naturels possède en général un éventail d'activités pharmacologiques. Toutes sont réunies dans les préparations que l'on peut obtenir à partir de produits naturels. Ainsi, par exemple, les héparines et les héparanes sulfate possèdent une activité antithrombotique qui est liée à l'action simultanée sur plusieurs facteurs de la coagulation.

L'héparine catalyse, notamment *via* l'antithrombine III (AT III), l'inhibition de deux enzymes qui interviennent dans la cascade de la coagulation du sang à savoir, le facteur Xa et le facteur IIa (ou thrombine). Les préparations d'héparines de bas poids moléculaire (HBPM), contiennent des chaînes formées de 4 à 30 monosaccharides et ont la propriété d'agir plus sélectivement sur le facteur Xa que sur la thrombine.

Certains oligosaccharides de synthèse notamment ceux décrits dans EP 84999 ont la propriété d'inhiber sélectivement, *via* l'antithrombine III, le facteur Xa sans aucune activité sur la thrombine.

Il est connu que l'inhibition du facteur Xa nécessite une fixation de l'héparine sur l'AT III *via* le domaine de liaison à l'antithrombine (DLA), et que l'inhibition du facteur IIa (thrombine) nécessite une fixation à l'AT III, via le DLA, ainsi qu'à la thrombine *via* un domaine de liaison moins bien défini (DLT).

Les oligosaccharides de synthèse correspondant au domaine DLA de l'héparine sont connus et manifestent une activité antithrombotique dans la thrombose veineuse. Ces composés sont décrits dans EP 529715, EP 621282 et dans le brevet canadien 2.040.905.

L'efficacité de ces oligosaccharides dans la prévention de la thrombose artérielle est néanmoins gênée par leur incapacité à inhiber la thrombine.

Une synthèse de glycosaminoglycanes de type héparine capables d'inhiber la thrombine via l'activateur de l'AT III présente de grandes difficultés et, en fait, elle n'a jamais été réalisée.

Dans le but de retrouver l'activité de produits inhibiteurs de thrombine et du facteur Xa, il a été proposé de relier deux oligosaccharides de petite taille (un DLA et un DLT) par une entité (« spacer ») n'intervenant pas dans l'activité biologique.

Il a maintenant été trouvé que de nouveaux dérivés pofysaccharidiques peuvent être synthétisés de façon relativement simple et sont biologiquement actifs. Ils sont en particulier anticoagulants et antithrombotiques. De plus, du fait de l'obtention par synthèse de ces polysaccharides, il est possible de modifier sélectivement leur structure, et en particulier d'éliminer des substituants sulfates non désirés intervenant dans l'interaction avec certaines protéines. Ainsi, on peut obtenir des polysaccharides qui sont de puissants agents antithrombotiques et anticoagulants et qui, de plus, peuvent échapper *in vivo* à l'action de protéines telles que le facteur plaquettaire 4 (FP4), qui neutralisent' l'effet de l'héparine en particulier sur la thrombine.

Ainsi, il a été trouvé de façon surprenante que des polysaccharides sulfatés et alkylés peuvent être de puissants antithrombotiques et des anticoagulants selon la disposition des groupes alkyles et des groupes sulfates portés par le squelette glucidique.

De façon plus générale, il a été trouvé que par réalisation de séquences polysaccharidiques il est possible de moduler avec précision les activités de type GAGs pour obtenir des produits très actifs présentant les propriétés de l'héparine.

Ainsi, selon un de ses aspects, la présente invention concerne un nouveau polysaccharide de synthèse, comprenant un domaine de liaison à l'antithrombine III constitué par un enchaînement de cinq monosaccharides portant au total deux fonctions' acide carboxylique et au moins quatre groupes sulfates, ce domaine étant lié directement à son extrémité non réductrice par un domaine de liaison à la thrombine comprenant un enchaînement de 10 à 25 unités monosaccharidiques choisies parmi des hexoses, des pentoses ou des sucres désoxy dont tous les groupes hydroxyles sont indépendamment éthérifiés par un groupe (C₁-C₆)alkyle ou estétiftés sous forme sulfate, ainsi que ses sels, notamment ceux pharmaceutiquement acceptables.

De préférence, l'invention se rapporte à un polysaccharide tel que défini ci-dessus, caractérisé en ce que tous ses groupes hydroxyles sont méthylés ou estérifiés sous forme sulfate et ses sels, notamment ceux pharmaceutiquement acceptables.

Les produits de la présente invention sont notamment des polysaccharides représentés par la formule suivante 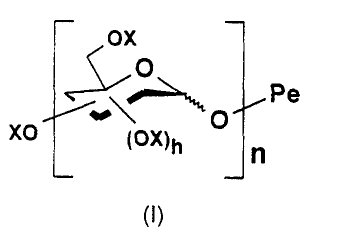 dans laquelle
- le trait ondulé désigne une liaison située soit au-dessous soit au-dessus du plan du cycle pyranosique, 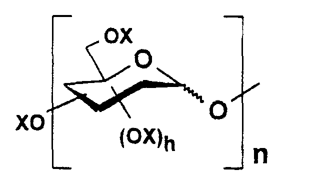 désigne un polysaccharide Po, contenant n unités monosaccharidiques identiques ou différentes, lié par son carbone anomère à Pe, 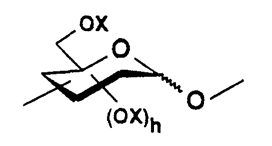 est une représentation schématique d'une unité monosaccharidique à structure pyranosique choisie parmi les hexoses, les pentoses et les sucres desoxy correspondants, cette unité étant liée par son carbone anomère à une autre unité monosaccharidique, et les groupes hydroxy de cette unité étant substitués par des groupes -X identiques ou différents, les groupes X étant choisis parmi les groupes (C₁-C₆)alkyle et les groupes sulfo,
- n est un nombre entier de 10 à 25,
- Pe représente un pentasaccharide de structure : 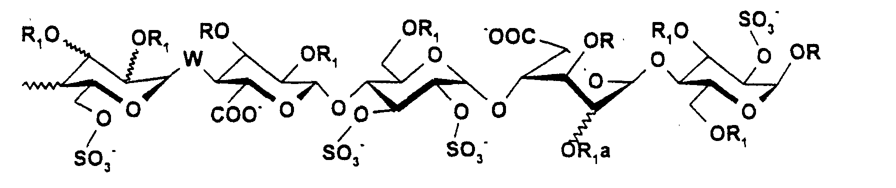
dans lequel
- R₁ représente un groupe (C₁-C₆)alkyle ou un groupe sulfo,
- R₁a représente R₁ ou constitue avec l'atome d'oxygène auquel il est lié et l'atome de carbone porteur de la fonction carboxylique sur le même cycle un groupe

   C―CH₂―O,
- R représente un groupe (C₁-C₆)alkyle,
- W représente un atome d'oxygène ou un groupe méthylène,
ou un de leurs sels, notamment pharmaceutiquement acceptable.

On notera que de façon générale dans la présente description qu'un trait ondulé désigne une liaison située soit au-dessous soit au-dessus du plan du cycle pyranosique.

Les monosaccharides contenus dans Po peuvent être identiques ou différents les uns des autres, les liaisons interglycosidiques peuvent être du type α ou β.

Ces monosaccharides sont avantageusement choisis parmi les hexoses D ou L allose, altrose, glucose, mannose, galose, idose, galactose, talose (dans ce cas h = 2) ou parmi les pentoses D ou L ribose, arabinose, xylose, lyxose (dans ce cas h = 2). D'autres monosaccharides tels que par exemple les sucres désoxy peuvent également être utilisés (h = 1 et/ou -CH₂OX = CH₃).

Lorsque dans les pentasaccharides Pe, l'unité W représente un atome d'oxygène et R₁a est tel que défini pour R₁, ces pentasaccharides constituent des composés connus et décrits notamment dans les brevets EP 300099, EP 529715, EP 621282 et

EP 649854 ainsi que dans la littérature. Ils sont obtenus à partir de synthons également décrits dans la littérature selon C. Van Boeckel, M. Petitou, Angew. Chem. Int. Ed. Engl., 1993, 32, 1671-1690.

Lorsque dans les pentasaccharides Pe, R₁a est différent de R₁ et/ou W représente un atome de carbone, ces pentasaccharides sont préparés à l'aide de nouveaux synthons qui constituent un aspect ultérieur de l'invention.

Lorsque dans les pentasaccharides Pe, l'unité de type acide L-iduronique est remplacée par une unité dont la conformation est verrouillée par un pont, ces pentasaccharides sont préparés à l'aide de nouveaux synthons qui constituent un aspect ultérieur de l'invention.

Ainsi selon un autre de ses aspects, la présente invention concerne des intermédiaires nouveaux utiles pour la préparation des composés (I).

La partie polysaccharidique Po peut être constituée de 10 à 25 unités monosaccharidiques alkylées et di- ou trisulfatées.

La partie polysaccharidique Po peut être constituée de 10 à 25 unités monosaccharidiques alkylées et mono- ou disulfatées.

La partie polysaccharidique Po peut être constituée de 10 à 25 unités monosaccharidiques alkylées non chargées et/ou partiellement chargées et/ou totalement chargées.

Les unités chargées ou non chargées peuvent être dispersées tout au long de la chaîne ou elles peuvent au contraire être groupées en domaines saccharidiques chargés ou non chargés.

Les liaisons peuvent être 1,2 ; 1,3 ; 1,4 ; 1,5 ; 1,6 ; et du type α ou β.

Dans la présente description, il a été choisi de représenter les conformations ¹C₄ pour l'acide-L-iduronique, ⁴C₁ pour l'acide D-glucuronique, mais il est notoire que, d'une façon générale, la conformation en solution des unités monosaccharides est fluctuante.

Ainsi, l'acide L-iduronique peut être de conformation ¹C₄ ²S₀ ou ⁴C₁.

Des composés préférés selon l'invention sont ceux de formule (LA) 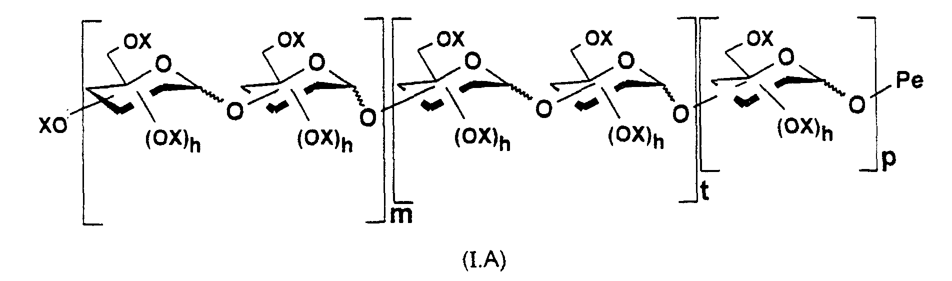 dans lesquels 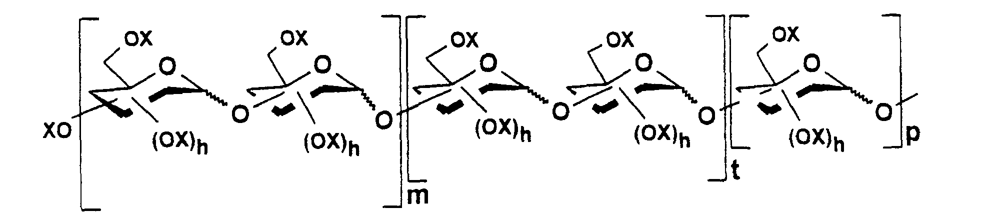 désigne une famille particulière de polysaccharides Po, liés par leur carbone anomère à Pe tel que défini pour (I), 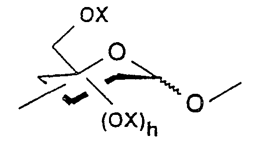 est tel que défini pour (I),
- les OX sont tel que défini pour (I) et, pour un même monosaccharide, peuvent être identiques ou différents,
- les monosaccharides contenus dans []ₘ forment un disaccharide répété m fois, les monosaccharides contenus dans []ₜ forment un disaccharide répété t fois,
- m varie de 1 à 8, t varie de 0 à 5 et p varie de 0 à 1 étant entendu que 5 ≤ m + t ≤ 12.
et leurs sels, notamment pharmaceutiquement acceptables.

Des composés avantageux sont les sels dont l'anion répond à la formule (1.1): 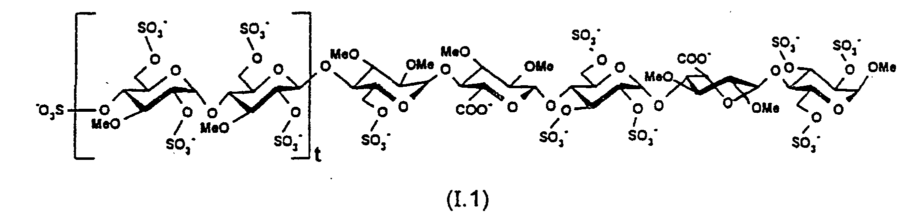 dans laquelle t représente 5, 6 ou 7, et le cation est un cation monovalent pharmaceutiquement acceptable, ainsi que les acides correspondants.

Sont également avantageux les sels dont l'anion répond à la formule (I.2) : 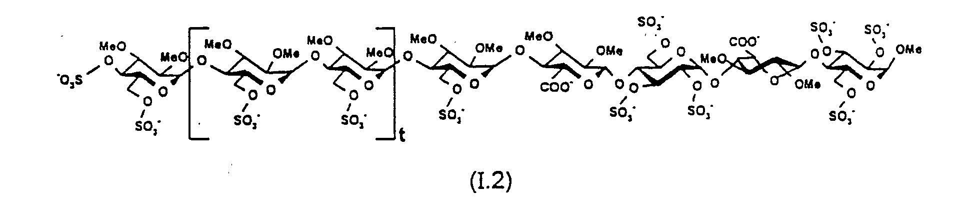 dans laquelle t représente 5, 6 ou 7 et le cation est un cation monovalent pharmaceutiquement acceptable, ainsi que les acides correspondants.

Sont particulièrement avantageux les sels dont l'anion a pour formule (1.3) : 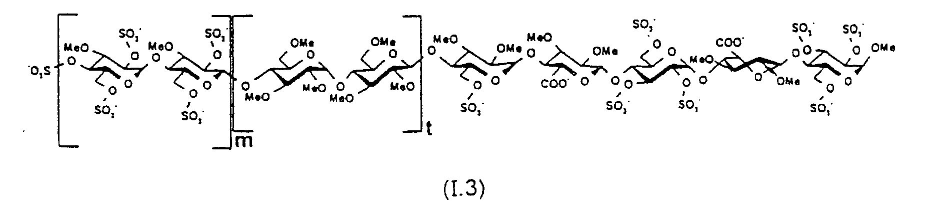 dans laquelle m représente 1,2 ou 3 et t représente 2, 3, 4 ou 5, et le cation est un cation monovalent pharmaceutiquement acceptable, ainsi que les acides correspondants.

D'autres composés préférés selon l'invention sont ceux de formule (II.A) : 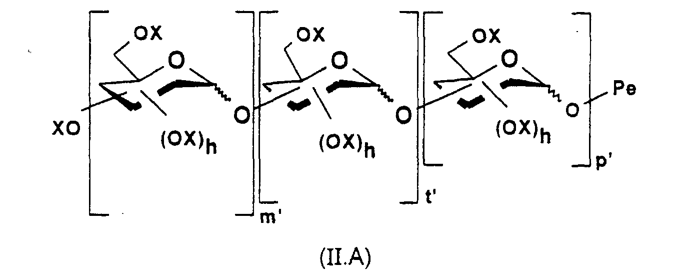 dans lesquels 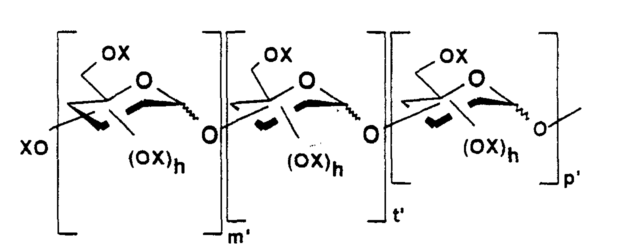 désigne une famille particulière de polysaccharides Po, liés par leur carbone anomère à Pe tel que défini pour (I), 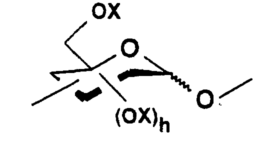 est tel que défini pour (I),
- les OX sont tel que défini pour (I) et, pour un même monosaccharide, peuvent être identiques ou différents,
- le monosaccharide contenu dans [ ]_{m'} est répété m' fois, le monosaccharide contenu dans [ ]_{t'} est répété t' fois, le monosaccharide contenu dans [ ]_{p'} est répété p' fois,
- m' varie de 1 à 5, t' varie de 0 à 24 et p' varie de 0 à 24 étant entendu que 10 ≤ m' + t' + p' ≤ 25.
et leurs sels, notamment pharmaceutiquement acceptables.

Les sels préférés de l'invention sont ceux dont le cation est choisi parmi les cations des métaux alcalins et plus préférablement encore ceux dont le cation est Na⁺ ou K⁺.

Particulièrement préférés sont les polysaccharides suivants
- Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)]₄-*O*-(2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium
- Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)]₅-*O*-(2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium
- Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)]₆-*O*-(2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium
- Méthyl *O*-(2,3-di-*O*-méthyl-4,6-di-*O*-sulfo-α-D-glucopyranosyl)-(1→4)-[*O*-(2,3-di-*O*-méthyl-6-*O*-sulfo-α-D-glucopyranosyl)-(1→4)-]₁₁-*O*-(acide 2,3-di-*O*-méthyl-β-D-glucopyranosyluronique)-(1→4)-*O*-(2,3,6-tri-*O*-sulfo-α-D-glucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium.
- Méthyl *O*-(2,3-di-*O*-méthyl-4,6-di-*O*-sulfo-α-D-glucopyranosyl)-(1→4)-[*O*-(2,3-di-*O*-méthyl-6-*O*-sulfo-α-D-glucopyranosyl)-(1→4)-]₁₃-*O*-(acide 2,3-di-*O*-méthyl-β-D-glucopyranosyluronique)-(1→4)-*O*-(2,3,6-tri-*O*-sulfo-α-D-glucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium
- Méthyl *O*-(2,3-di-*O*-méthyl-4,6-di-*O*-sulfo-α-D-glucopyranosyl)-(1→4)-[*O*-(2,3-di-*O*-méthyl-6-*O*-sulfo-α-D-glucopyranosyl)-(1→4)-]₁₅-*O*-(acide 2,3-di-*O*-méthyl-β-D-giucopyranosyluronique)-(1→4)-*O*-(2,3,6-tri-*O*-sulfo-α-D-giucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium
- Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)]₂-[*O*-(2,3,6-tri-*O*-méthyl-α-D-glucopyranosyl)-(1→4)-*O*-(2,3,6-tri-*O*-méthyl-β-D-glucopyranosyl)-(1→4)]₂-*O*-(2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-(acide 2,3-di-*O*-méthyl-α-L-idopyranosyluronique)-(1→4)-2,3,6-tri-*O*-sulfo-α-D-glucopyranoside, sel de sodium
- Méthyl *O*-(3-*O*-méthyl-2,4,6-tri-*O*-sulfo-α-D-glucopyranosyl)-(1→4)-*O*-(3-*O*-méthyl-2,6-di-*O-*sulfo-β-D-glucopyranosyl)-(1→4)-[*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)]₂-[*O*-(2,3,6-tri-*O*-méthyl-α-D-glucopyranosyl)-(1→4)-O-(2,3,6-tri-*O*-méthyl-β-D-glucopyranosyl)-(1→4)]₃-O-(2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-(acide 2,3-di-*O*-méthyl-α-L-idopyranosyluronique)-(1→4)-2,3,6-tri-*O*-sulfo-α-D-glucopyranoside, sel de sodium
- Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)-[*O*-(2,3,6-tri-*O*-méthyl-α-D-glucopyranosyl)-(1→4)-*O*-(2,3,6-tri-*O*-méthyl-β-D-glucopyranosyl)-(1→4)]₄-*O*-(2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-O-sulfo-α-D-glucopyranosyl)-(1→4)-(acide 2,3-di-*O*-méthyl-α-L-idopyranosyluronique)-(1→4)-2,3,6-tri-*O*-sulfo-α-D-glucopyranoside, sel de sodium
- Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)-[*O*-(2,3,6-tri-*O*-méthyl-α-D-glucopyranosyl)-(1→4)-*O*-(2,3,6-tri-*O*-méthyl-β-D-glucopyranosyl)-(1→4)]₃-*O*-(2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(23,6-tri-*O*-sulfo-α-D-glucopyranosyl)-(1→4)-(acide 2,3-di-*O*-méthyl-α-L-idopyranosyluronique)-(1→4)-2,3,6-tri-*O*-sulfo-α-D-glucopyranoside, sel de sodium
- Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[*O*-(2,3,6-tri-*O*-méthyl-α-D-glucopyranosyl)-(1→4)-*O*-(2,3,6-tri-*O*-méthyl-β-D-glucopyranosyl)-(1→4)]₄-*O*-(2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-(acide 2,3-di-*O*-méthyl-α-L-idopyranosyluronique)-(1→4)-2,3,6-tri-*O*-sulfo-α-D-glucopyranoside, sel de sodium
- Méthyl *O*-(3-*O*-méthyl-2,4,6-tri-*O*-sulfo-α-D-glucopyranosyl)-(1→4)-O-(3-O-méthyl-2,6-di-*O*-sulfo-β-D-glucopyranosyl)-(1→4)-[*O*-(2,3,6-tri-*O*-méthyl-α-D-glucopyranosyl)-(1→4)-*O*-(2,3,6-tri-*O*-méthyl-β-D-glucopyranosyl)-(1→4)]₅-*O*-(2,3-di-*O*-méthyl-6-*O*-sulfo-α-D-glucopyranosyl)-(1→4)-*O*-(acide 2,3-di-*O*-méthy|-β-D-glucopyranosyluronique)-(1→4)-*O*-(2,3,6-tri-*O*-sulfo-α-D-glucopyranosyl)-(1→4)-(acide 2,3-di-*O*-méthyl-α-L-idopyranosyluronique)-(1→4)-2,3,6-tri-*O*-sulfo-α-D-glucopyranoside, sel de sodium.

La présente invention concerne également un procédé pour la préparation des composés de formule (I) caractérisé en ce que : dans une première étape, un précurseur complètement protégé du polysaccharide (I) désiré, contenant un précurseur protégé du domaine Pe (ce domaine étant montré dans le SCHÉMA 1) prolongé à son extrémité non réductrice par un précurseur protégé du polysaccharide sulfaté Po est synthétisé puis, dans une seconde étape, les groupes chargés négativement sont introduits et/ou démasqués.

### SCHÉMA 1 - Structure du pentasaccharide Pe lorsque W = O ou C (les lettres « DEFGH » sont utilisées dans le texte pour désigner les monosaccharides concernés)

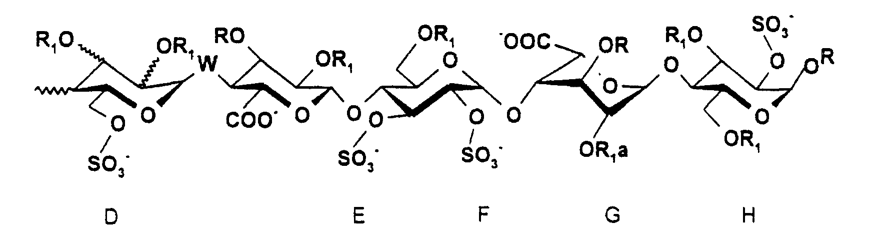

Dans une première approche, on peut utiliser le précurseur totalement protégé de la partie tétrasaccharidique EFGH du pentasaccharide. On ajoute ensuite un polysaccharide Po qui contient à son extrémité terminale réductrice l'unité D manquante de Pe pour obtenir après couplage, l'intégrité du DLA qui est ainsi restaurée.

Dans une autre approche, on peut utiliser le précurseur totalement protégé de la partie disaccharidique GH du pentasaccharide. On ajoute ensuite un polysaccharide Po précurseur du DLT qui contient à son extrémité terminale réductrice l'unité DEF manquante de Pe pour obtenir après couplage, l'intégrité du DLA qui est ainsi restaurée.

La synthèse de ces précurseurs de Pe est réalisée comme indiqué précédemment à partir de synthons décrits dans la littérature ou faisant partie de la présente invention. La synthèse de la partie polysaccharidique précurseur de Po est réalisée selon des réactions bien connues de l'homme de l'art, en utilisant les méthodes de la synthèse d'oligosaccharides (G.J. Boons, Tetrahedron, 1996, 52, 1095-1121) ou un oligosaccharide lorsqu'un oligosaccharide donneur de liaison glycosidique est couplé avec un oligosaccharide accepteur de liaison glycosidique pour conduire à un autre oligosaccharide dont la taille est égale à la somme des tailles des deux espèces réactives.

Cette séquence est répétée jusqu'à l'obtention du composé de formule (I) désiré. La nature et le profil de la charge du composé final désiré déterminent la nature des entités chimiques utilisées dans les différentes étapes de la synthèse, selon les règles bien connues de l'homme de l'art.

Une méthode préférée pour la préparation des précurseurs de Po selon la présente invention est montrée sur le SCHÉMA 2 ci-après.

### SCHÉMA 2 - Synthèse du précurseur protégé du DLT

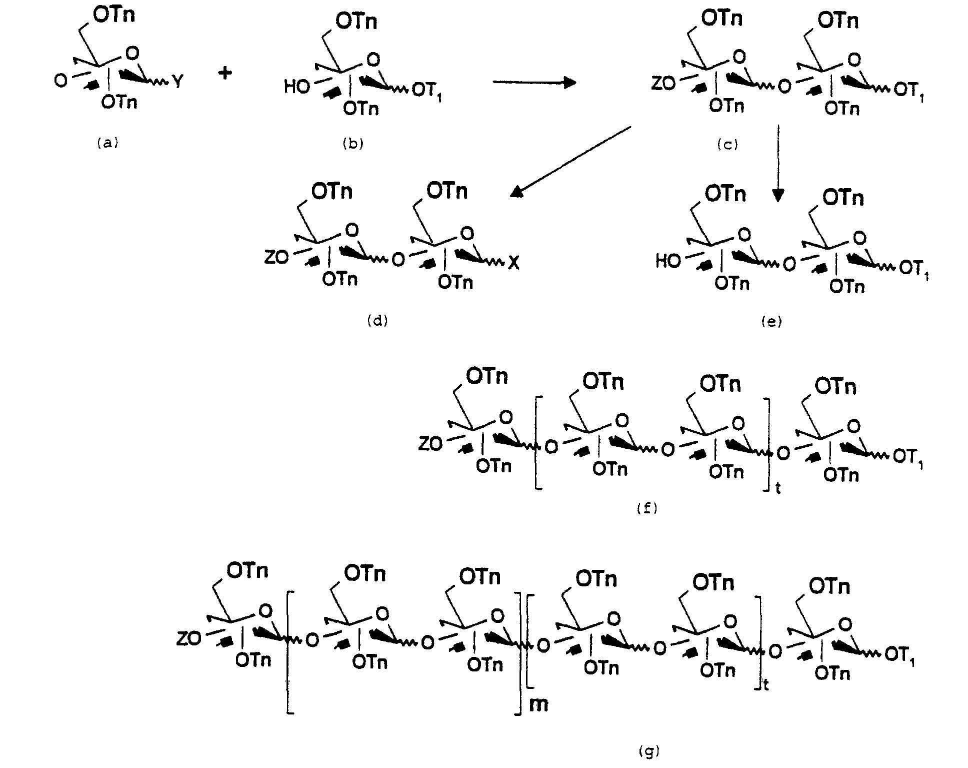

Par temporaire, on entend un substituant conservé pendant un nombre limité d'étapes, par semi-permanent un substituant conservé pendant un plus grand nombre d'étapes et par permanent, un substituant conservé jusqu'à la fin de la synthèse ; les substituants permanents sont éliminés au cours de la dernière étape. Certains groupes permanents peuvent faire partie de la molécule finale.

Dans le SCHÉMA 2, (a) représente un monosaccharide donneur de liaison glycosidique dans lequel Z est un groupe protecteur temporaire d'une fonction hydroxyle, et Y est un activateur du carbone anomérique, Tn identiques ou différents, sont des substituants temporaires, semi-permanents ou permanents de toutes les autres fonctions hydroxyles.

Le composé (b) qui possède un groupe hydroxyle non substitué, représente un monosaccharide accepteur de liaison glycosidique dans lequel Tn identiques ou différents sont des substituants temporaires semi-permanents ou permanents des groupes hydroxyles. T₁ est un groupement protecteur temporaire semi-permanent ou permanent de la position anomérique. Il est éliminé lorsque l'on veut activer le carbone anomérique.

Dans le but d'obtenir les composés de l'invention, le donneur de liaison glycosidique (a) et l'accepteur de liaison glycosidique (b) réagissent ensemble pour donner le disaccharide (c).

Le disaccharide (c) obtenu ci-dessus est converti spécifiquement en un disaccharide donneur de liaison glycosidique (d) par élimination de T₁ et introduction de et/ou en un accepteur de liaison glycosidique (e) par élimination de Z.

Ensuite, le donneur de liaison glycosidique (d) et l'accepteur de liaison glycosidique (e) réagissent ensemble pour donner le tétrasaccharide (f) dans lequel t représente 1,

La répétition de cet enchaînement de réactions donne un oligo- ou un polysaccharide (f) dans lequel test supérieur à 1.

Il est également possible, en utilisant le procédé représenté dans le SCHÉMA 2, d'obtenir une grande variété d'oligo- ou de polysaccharides complètement protégés comme (g) dans lequel les oligosaccharides [ ]ₘ et [ ]ₜ sont des précurseurs complètement protégés de domaines différemment chargés des composés de l'invention.

Dans l'étape suivante du procédé, les composés comme (f) et (g), sont convertis en donneurs de liaison glycosidique, et couplés à l'unité terminale non réductrice de précurseurs complètement protégés de Pe.

Comme il a été précédemment mentionné, l'oligosaccharide de l'unité terminale non réductrice d'un polysaccharide donneur de liaison glycosidique (g) peut constituer une partie de Pe, dans le cas où (g) est couplé à l'unité terminale non réductrice d'un oligosaccharide complètement protégé qui est le précurseur du reste de la structure de Pe.

Les composés de l'invention sont obtenus à partir de leurs précurseurs polysaccharidiques complètement protégés en utilisant l'enchaînement suivant de réactions :
- les fonctions alcool devant être transformées en un groupe O-sulfo, et les acides carboxyliques sont déprotégés par l'élimination des Tn utilisés pour les protéger durant l'élaboration du squelette puis,
- les groupes sulfo sont ensuite introduits.

Les composés de l'invention peuvent naturellement être préparés en utilisant différentes stratégies connues par l'homme de l'art de la synthèse des oligosaccharides.

Le procédé décrit ci-dessus est le procédé préféré de l'invention. Toutefois, les composés de formule (I) peuvent être préparés par d'autres méthodes bien connues de la chimie des sucres décrites par exemple dans Monosaccharides, Their chemistry and their rotes in natural products, P.M. Collins et R.J. Ferrier, J. Wiley & sons, 1995 et dans G.J. Boons, Tetrahedron, 1996, 52, 1095-1121.

Le précurseur de la partie du pentasaccharide Pe lorsque W représente un atome d'oxygène et R₁a est R₁ est préparé selon les méthodes de la synthèse des oligosaccharides et particulièrement selon les méthodes décrites dans les brevets EP 84999, EP 301618, EP 454220 et EP 529715 et dans les demandes de brevets EP 9304769 et EP 94202470. Lorsque la protection complète est réalisée, il est possible, en utilisant les groupes protecteurs appropriés d'obtenir un groupe hydroxyle libre sur la position 4 de l'unité terminale non réductrice (D). Le précurseur totalement protégé de Pe est alors couplé à cette position utilisant les méthodes connues de la synthèse d'oligosaccharides.

Le pentasaccharide Pe dans lequel W représente un atome de carbone et R₁a est R₁ de formule : 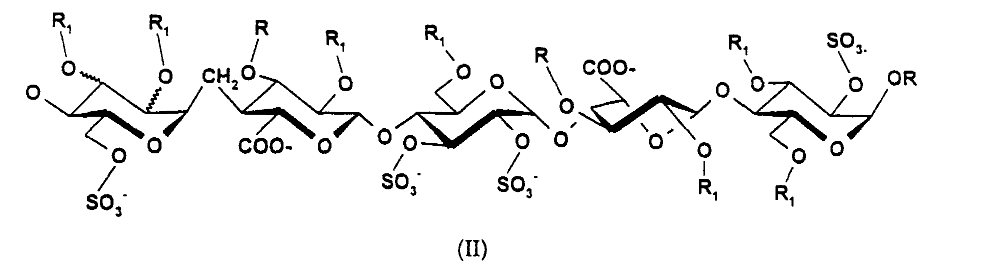 dans laquelle R et R₁ sont tels que définis pour (I) est obtenu à partir du synthon de formule : 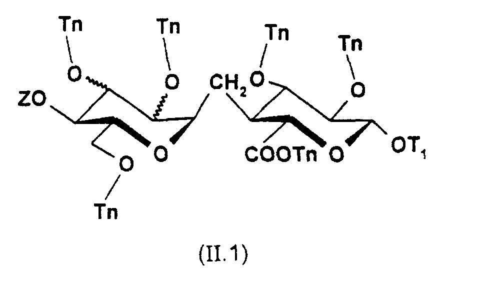 dans laquelle T₁, Tₙ identiques ou différents représentent un substituant temporaire, semi-permanent ou permanent, Z est un groupe protecteur d'une fonction hydroxyle lui-même obtenu par une synthèse réalisée au moyen d'une réaction radicalaire entre un monosaccharide générateur de radicaux libres et un monosaccharide comportant une double liaison, le C-disaccharide ainsi obtenu étant alors converti en synthon (II.1) selon les méthodes classiques décrites précédemment selon C. Van Boeckel, M. Petitou.

Le synthon de formule (II. 1 ) particulièrement utile à la synthèse des composés (II) est de formule :

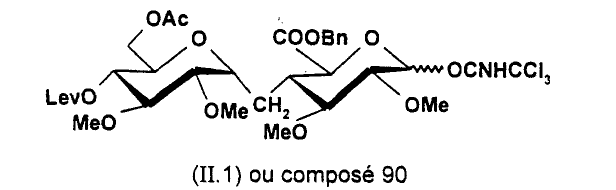

Ce synthon est préparé selon le schéma réactionnel décrit dans le SCHÉMA 22 ci-après.

Le pentasaccharide Pe dans lequel figure un substituant R₁a qui constitue une unité d'acide L-iduronique de configuration verrouillée de formule : 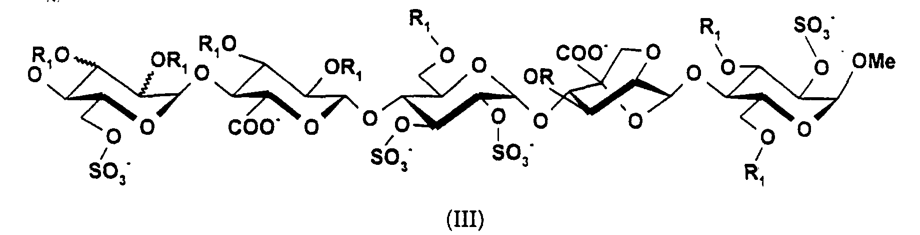 dans laquelle R et R₁ sont tels que définis pour (I) et W représente un atome d'oxygène sont obtenus à partir du synthon de formule : 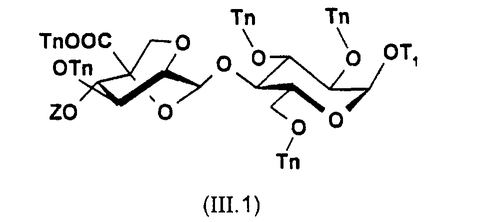 dans laquelle T₁, Tₙ identiques ou différents représentent un substituant temporaire, semi-permanent ou permanent, Z est un groupe protecteur d'une fonction hydroxyle lui-même obtenu par une synthèse réalisée selon les méthodes décrites dans la littérature M. K. Gurjar et al., Tetrahedron letters, 1995, 36, 11, 1937-1940, 1933-1936 et 1994, 35, 14, 2241-2244.

Le synthon de formule (III. 1) particulièrement utile à la synthèse des composés (III) est de formule : 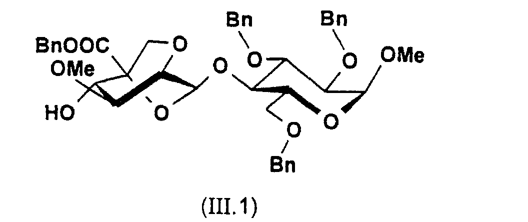 Ce synthon est préparé selon le schéma réactionnel décrit dans le SCHÉMA 34 ci-après.

Les intermédiaires (II.1) et (III.1) sont des intermédiaires nouveaux particulièrement utiles à la préparation des composés (I) selon l'invention.

Les pentasaccharides Pe peuvent donc être obtenus à partir de ces synthons disaccharidiques (II.1) ou (III.1), de la façon décrite dans la publication de C.A.A Van Boeckel et M. Petitou, Angew. Chem. Int. Ed. Engl. citée ci-dessus.

Par groupements semi-permanents utilisés ci-dessus, on entend les groupements éliminables en premier lieu après les réactions de glycosylation lorsque le squelette glucidique comporte le nombre de motifs désirés, sans enlèvement ou altération des autres groupes présents, permettant alors l'introduction de groupements fonctionnels souhaités aux positions qu'ils occupent.

Les groupements permanents sont des groupements capables de maintenir la protection des fonctions OH durant l'introduction de groupements fonctionnels à la place des groupements semi-permanents.

Ces groupements sont choisis parmi ceux compatibles avec les groupes fonctionnels introduits après élimination des groupes semi-permanents. Il s'agit, en outre, de groupements inertes vis-à-vis des réactions effectuées pour la mise en place de ces groupes fonctionnels et qui sont éliminables sans que ces groupements fonctionnels ne soient altérés.

Selon l'invention, les groupes permanents sont de façon préférée les groupes alkyle en C₁-C₆.

Comme exemple de groupe semi-permanent et/ou temporaire on peut citer les groupes benzyle et acétyle, lévulinyle, p-méthoxybenzyle, etc.

Les substituants en position 3 des motifs uroniques du composé cible peuvent être déjà présents dans les synthons de départ, ainsi que le substituant R₁.

Les groupes protecteurs utilisés dans le procédé de préparations des composés (I) sont ceux couramment utilisés dans la chimie des sucres par exemple dans Protective Groups in Organic Synthesis, TW Greene, John Wiley & sons, New-York, 1981.

Les groupes protecteurs sont avantageusement choisis par exemple parmi les groupes acétyles, halogénométhyles, benzoyles, lévulinyles, benzyles, benzyles substitués, trityles éventuellement substitués, tétrahydropyranyles, allyles, pentenyles, tert-butyldiméthylsilyles (tBDMS) ou triméthylsilyléthyles (...).

Les groupes activateurs sont ceux classiquement utilisés en chimie des sucres selon par exemple G.J. Boons, Tetrahedron, 1996, 52, 1095-1121. Ces groupes activateurs sont choisis par exemple parmi les imidates, les thioglycosides, les penténylglycosides, les xanthates, les phosphites ou les halogénures.

Le procédé décrit ci-dessus permet d'obtenir les composés de l'invention sous forme de sels. Pour obtenir les acides correspondants, les composés de l'invention sous forme de sels, sont mis en contact avec une résine échangeuse de cations sous forme acide.

Les composés de l'invention sous forme d'acides peuvent être ensuite neutralisés par une base pour obtenir un sel souhaité.

Pour la préparation des sels des composés de formule (I), on peut utiliser toute base minérale ou organique, donnant avec les composés de formule (I), des sels pharmaceutiquement acceptables.

On utilise de manière préférentielle comme base l'hydroxyde de sodium, de potassium, de calcium ou de magnésium. Les sels de sodium et de calcium des composés de formule (I), sont les sels préférés.

Dans l'étape (a) du procédé, les groupes protecteurs utilisés sont ceux habituellement utilisés par l'homme du métier en chimie des sucres par exemple selon EP 84999 ou encore selon Protective Groups in Organic Synthesis, TW Greene, J. Wiley & sons, 1995.

Les composés (I) ainsi obtenus peuvent être éventuellement salifiés.

Les composés de la formule (I) ci-dessus comprennent également ceux dans lesquels un ou plusieurs atomes d'hydrogène ou de carbone ont été remplacés par leur isotope radioactif par exemple le tritium ou le carbone-14. De tels composés marqués sont utiles dans les travaux de recherche, de métabolisme ou de pharmacocinétique, dans les essais biochimiques en tant que ligands.

Les composés selon l'invention ont fait l'objet d'études biochimiques et pharmacologiques qui ont montré qu'ils possèdent des propriétés très intéressantes. Les composés de la présente invention qui se lient sélectivement à l'AT III avec une affinité égale ou supérieure à celle de l'héparine, possèdent les propriétés anticoagulantes et antithrombotiques de l'héparine.

L'activité antithrombotique globale des produits de formule (I) a été évaluée par voie intraveineuse ou sous cutanée chez le rat, dans un modèle de stase veineuse et induction par la thromboplastine, selon la méthode décrite par J. Reyers *et al*. dans Thrombosis Research, 1980, *18*, 669-674 ainsi que dans un modèle de thrombose artérielle constitué d'un shunt implanté entre l'artère carotide et la veine jugulaire de rats tel que décrit par Umetsu *et al.* Thromb. Haemost., 1978, 39, 74-83. Dans ces deux modèles expérimentaux, la DE₅₀ des composés de l'invention est au moins du même ordre ou inférieure à celle des autres héparinoïdes de synthèse déjà connus (DE₅₀ comprises entre 5 et 500 µg/kg). Les composés de l'invention présentent donc une spécificité d'action et une activité anticoagulante et antithrombotique particulièrement intéressantes.

Grâce à leur activité biochimique et pharmaceutique, les composés de la présente invention sont des médicaments très intéressants. Leur toxicité est parfaitement compatible avec cette utilisation. Ils sont également très stables et sont donc particulièrement appropriés pour constituer le principe actif de spécialités pharmaceutiques.

De plus, les composés de l'invention ne sont pas neutralisés par de fortes doses de protéines cationiques plaquettaires telles que le facteur 4 plaquettaire (FP4) relargués lors de l'activation de celles-ci durant le processus de thrombose. Les composés de l'invention sont donc tout particulièrement intéressants pour le traitement et la prévention des thromboses d'origine artérielle ou veineuse.

Ils peuvent être utilisés dans diverses pathologies consécutives à une modification de l'hémostasie du système de la coagulation apparaissant en particulier lors des troubles du système cardio-vasculaire et cérébro-vasculaire comme les troubles thrombo-emboliques associés à l'arthérosclérose et au diabète tels l'angine instable, l'attaque cérébrale, la resténose après angioplastie, l'endartérectomie, la pose de prothèses endovasculaires ; ou les troubles thromboemboliques associés à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires de pontages aorto-coronariens. Ces produits peuvent par ailleurs être utilisés pour le traitement ou la prévention de pathologies thrombo-emboliques d'origine veineuse telles les embolies pulmonaires. Ils peuvent être utilisés ou pour prévenir ou pour traiter les complications thrombotiques apparaissant lors d'interventions chirurgicales ou conjointement à d'autres pathologies telles que cancer, infections bactériennes ou virales. Dans le cas de leur utilisation lors de la pose de prothèses, les composés de la présente invention peuvent recouvrir des prothèses et les rendre ainsi hémocompatibles. En particulier, ils peuvent être fixés à des prothèses intravasculaires (stents). Dans ce cas, ils peuvent éventuellement être modifiés chimiquement par introduction à l'extrémité non réductrice ou réductrice d'un bras approprié, comme décrit selon EP 649 854.

Les composés de la présente invention peuvent également être utilisés comme adjuvant lors d'endartérectomie réalisée avec des ballonets poreux.

Les composés de l'invention sont très stables et sont donc ainsi particulièrement appropriés pour constituer le principe actif de médicaments.

Selon un autre de ses aspects, la présente invention a donc pour objet une composition pharmaceutique contenant, en tant que principe actif, un polysaccharide de synthèse tel que défini ci-dessus.

L'invention concerne de préférence, des compositions pharmaceutiques contenant comme principe actif, un composé de formule (I), (I.1), (I.2), (I.3) ou l'un de ses sels pharmaceutiquement acceptables, éventuellement en association avec un ou plusieurs excipients inertes et appropriés.

Dans chaque unité de dosage le principe actif est présent dans les quantités adaptées aux doses journalières envisagées. En général, chaque unité de dosage est convenablement ajustée selon le dosage et le type d'administration prévu, par exemple comprimés, gélules et similaires, sachets, ampoules, sirops et similaires, gouttes, patch transdermique ou transmuqueux de façon à ce qu'une telle unité de dosage contienne de 0,1 à 100 mg de principe actif, de préférence 0,5 à 50 mg.

Les composés selon l'invention peuvent également être utilisés en association avec un autre principe actif utile pour la thérapeutique souhaitée tels que par exemple des antithrombotiques, des anticoagulants, des antiagrégants plaquettaires tels que par exemple le dipyridamole, l'aspirine, la ticlopidine, le clopidogrel ou des antagonistes du complexe de la glycoproteine IIb/IIIa.

Les compositions pharmaceutiques sont formulées pour l'administration aux mammifères, y compris l'homme, pour le traitement des maladies susdites.

Les compositions pharmaceutiques ainsi obtenues sont présentées avantageusement sous des formes diverses, telles que par exemple, des solutions injectables ou buvables, dragées, comprimés ou gélules. Les solutions injectables sont les formes pharmaceutiques préférées. Les compositions pharmaceutiques de la présente invention sont notamment utiles pour le traitement à titre préventif ou curatif, des troubles de la paroi vasculaire, tels que l'athérosclérose, les états d'hypercoagulabilité observés par exemple à la suite d'opérations chirurgicales,de développement tumoraux ou de dérèglements de la coagulation, induits par des activateurs bactériens, viraux, ou enzymatiques. La posologie peut largement varier en fonction de l'âge, du poids et de l'état de santé du patient, de la nature et de la sévérité de l'affection, ainsi que de la voie d'administration. Cette posologie comprend l'administration d'une ou plusieurs doses d'environ 0,1 mg à 100 mg par jour, de préférence d'environ 0,5 à 50 mg par jour, par voie intramusculaire ou sous cutanée, en administrations continues ou à intervalles réguliers.

La présente invention a donc également pour objet les compositions pharmaceutiques qui contiennent à titre de principe actif un des composés ci-dessus éventuellement en association avec un autre principe actif. Ces compositions sont réalisées de façon à pouvoir être administrées par la voie digestive ou parentérale.

Dans les compositions pharmaceutiques de la présente invention pour l'administration orale, sublinguale, sous-cutanée, intramusculaire, intraveineuse, transdermique, transmuqueux, locale ou rectale, l'ingrédient actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Pour une administration transmuqueuse le principe actif peut être formulé en présence d'un promoteur tel qu'un sel biliaire, d'un polymère hydrophile tel que par exemple l'hydroxypropylcellulose, l'hydroxypropylméthylcellulose, l'hydroxyéthylcellulose, l'éthylcellulose, la carboxyméthylcellulose, le dextran, la polyvinylpyrrolidone, les pectines, les amidons, la gélatine, la caséine, les acides acryliques, les esters acryliques et leurs copolymères, les polymères ou copolymères de vinyle, les alcools vinyliques, les alcoxypolymères, les polymères d'oxyde de polyéthylène, les polyéthers ou leur mélange.

Le principe actif peut être formulé également sous forme de microcapsules, éventuellement avec un ou plusieurs supports ou additifs.

Le principe actif peut être également présenté sous forme de complexe avec une cyclodextrine, par exemple α, β ou γ cyclodextrine, 2-hydroxypropyl-β-cyclodextrine ou méthyl-β-cyclodextrine.

Le principe actif peut également être libéré par un ballonnet le contenant ou par un extenseur endovasculaire introduit dans les vaisseaux sanguins. L'efficacité pharmacologique du principe actif n'est ainsi pas affectée.

L'administration par voie sous-cutanée est la voie préférée.

Les méthodes, les préparations et les schémas suivants illustrent la synthèse des différents intermédiaires utiles à l'obtention des polysaccharides selon l'invention.

Les exemples ci-après illustrent également l'invention sans toutefois la limiter,

Les abréviations suivantes sont utilisées :
TBDMS : *tert*-butyldiméthylsilyle ; Lev : lévulinyie ; Bn : benzyle ; Bz : benzoyle ; CCM: chromatographie sur couche mince ; Olm : trichloroacétimidyle ; LSIMS : est le sigle anglais de *Liquid Secondary Ion Mass Spectrometry ;* ESIMS : est le sigle anglais de *Electron Spray Ionisation Mass Spectrometry ;* TMS : triméthylsilyle ; TSP : triméthylsilyle tétradeuterio propionate de sodium ; Tf : triflate MS : tamis moléculaire ; All: allyle ; PMB : *p*-méthoxybenzyle ; SE : triméthylsilyléthyle.; Im : imidate Dowex®, Sephadex®, Chelex®, Toyopearl® sont des marques déposées.

Dans les méthodes, les préparations et dans les exemples décrits ci-après, des modes opératoires généraux concernant le couplage catalytique des imidates, le clivage des esters lévuliniques, le couplage catalytique des thiogiycosides, la saponification, la méthylation et la déprotection sélective du groupe p-méthoxybenzyle, la déprotection et la sulfatation des oligo- et des poiysaccharides par hydrogénolyse dès esters ou des éthers benzyliques, la saponification des esters ou encore les sulfatations peuvent être réalisés en appliquant les méthodes générales ci-après aux intermédiaires appropriés.

### MÉTHODES GÉNÉRALES

### MÉTHODE 1 - Couplage aux imidates catalysé par le triflate de tert-butyldiméthylsilyle

Une solution de triflate de *tert*-butyldiméthylsilyle dans du dichlorométhane (1M, 0,2 mol/mol d'imidate) est ajoutée, sous argon à - 20° C, à une solution de l'imidate et de l'accepteur de glycosyle dans le dichiorométhane (17,5 mL/mmol) en présence de tamis moléculaire 4 Å. Après 10-20 minutes (CCM), on ajoute de l'hydrogénocarbonate de sodium solide. On filtre la solution, on lave à l'eau, on sèche et on évapore à sec.

### MÉTHODE 2 - Coupure du groupe lévulinique

On dissout le composé à déprotéger dans un mélange éthanol/toluène 2 : 1 (42 mL/mmol) et on ajoute l'acétate d'hydrazine (5 mol/mol). On laisse agiter pendant 15-30 minutes (CCM) et on concentre.

### MÉTHODE 3 - Couplage aux thioglycosides catalysé par N-iodosuccinimide/triflate d'argent

On dissout dans un ballon en verre inactinique le thioglycoside et l'accepteur de glycosyle dans le toluène anhydre (18 mL/mmol de thioglycoside) en présence de tamis moléculaire 4 Å. On agite 1 heure à température ambiante. On refroidit à 0° C et on ajoute du *N*-iodosuccinimide (3 mol/mol de thioglycoside) puis du triflate d'argent (0,28 mol/mol de thioglycoside). Après 10-15 minutes (CCM) on ajoute de l'hydrogénocarbonate de sodium solide. Après filtration, on lave la solution avec une solution aqueuse de thiosulfate de sodium 1M, de l'eau, on sèche et on évapore.

### MÉTHODE 4 - Saponification, méthylation et déprotection sélective du groupe p-méthoxybenzyle

Saponification des esters. On dissout le composé à saponifier dans un mélange de dichlorométhane/méthanol 1 : 1 (4 mUmmol). On ajoute du méthanolate de sodium, on agite 20 minutes, et on neutralise à l'aide d'une résine Dowex® 50 H⁺. On concentre et on utilise ce composé à l'étape suivante sans purification.

Méthylation. De l'hydrure de sodium est ajouté par petites quantités, à 0° C, à un mélange du brut précèdent et de l'iodure de méthyle dans du *N*,*N*-diméthylformamide (7 mL/mmol). Après réaction complète, on verse le mélange dans de l'eau, et on extrait à l'acétate d'éthyle. On lave les phases organiques à l'eau, on sèche et on évapore à sec.

Coupure du *p*-méthoxybenzyle. Le composé brut précédent est dissous dans un mélange acétonitrile/eau 9 : 1 (20 mL/mmol). À 0° C on ajoute du nitrate de cérium et d'ammonium (0,5 mol/mol). On agite le mélange réactionnel pendant 2 heures (contrôle par CCM), on ajoute une solution saturée d'hydrogénocarbonate de sodium, on extrait à l'acétate d'éthyle, on sèche et on évapore.

### MÉTHODE 5 - Déprotection et sulfatation des oligo- et polysaccharides

Hydrogénolyse des benzyl éthers et benzyl esters. On laisse agiter pendant 6-12 heures (CCM) une solution du composé dans l'acide acétique glacial sous une atmosphère d'hydrogène (40 bars) en présence de catalyseur Pd/C 5 % (2 fois la masse du composé). Après filtration on engage le produit directement dans l'étape suivante.

Saponification des esters. On ajoute une solution aqueuse d'hydroxyde de sodium 5M (en quantité telle que la concentration d'hydroxyde de sodium soit de 0,5 M en fin d'addition) à une solution d'un ester dans le méthanol (150 mL/mmol). Après 2-5 heures on introduit de l'eau et on passe au travers d'une colonne de gel Sephadex® G-25 (1,6 x 115 cm) éluée par l'eau. On concentre, on passe au travers d'une colonne Dowex® 50 H⁺(2 mL) et on lyophilise. À ce stade on vérifie par ¹H RMN que tous les groupes protecteurs ont été enlevés. Si cela est nécessaire on soumet de nouveau le produit à l'hydrogénation et/ou la saponification.

Sulfatation. On ajoute du complexe triéthylamine/trioxyde de soufre (5 mol/mol fonction hydroxyle) à une solution dans le diméthylformamide (5 mg/mL) du composé à sulfater. Après un jour à 55° C on dépose la solution au sommet d'une colonne de Sephadex® G-25 (1,6 x 115 cm) éluée par du chlorure de sodium 0,2 M. On concentre les fractions contenant le produit et on dessale en utilisant la même colonne éluée par l'eau. Le composé final est obtenu après lyophilisation.

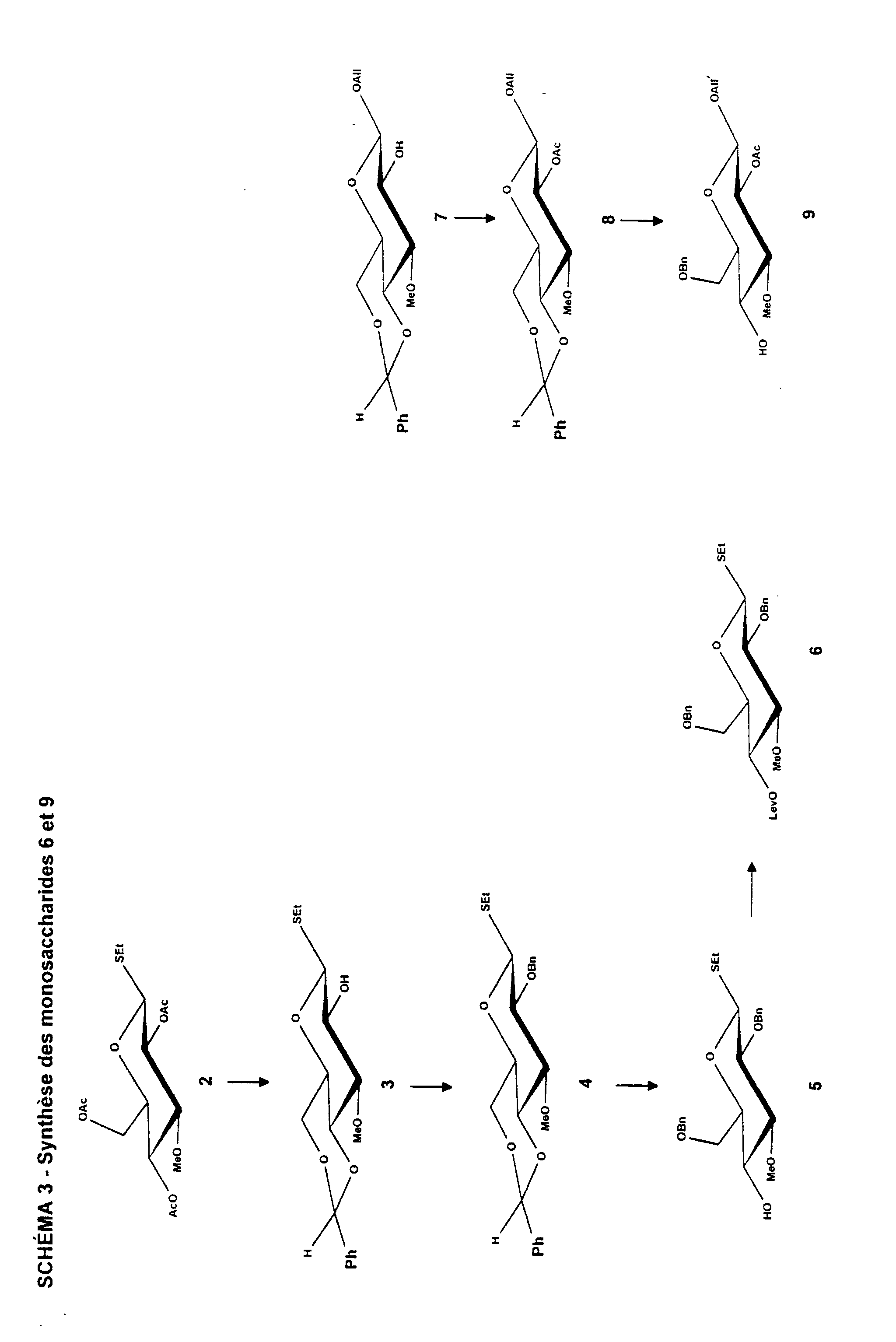

### PRÉPARATION 1

### Éthyl 2,4,6-tri-O-acétyl-3-O-méthyl-1-thio-β-D-glucopyranoside (2)

On dissout du 1,2,4,6-tétra-*O*-acétyl-3-*O*-méthyl-β-D-glucopyranose **1** (69 g, 0,19 mmol), (B. Helferich et *al*, J. prakt. Chem., 132, 321 [1932]) dans le toluène (580 mL). On ajoute de l'éthanethiol (28 mL, 0,38 mmol), puis goutte à goutte une solution de diéthyléthérate de trifluoroborane (1M dans du toluène, 190 mL). On laisse sous agitation pendant 1,5 heure (CCM), ont introduit de l'hydrogénocarbonate de sodium solide, on filtre, on lave à l'eau, on sèche et on concentre. Une chromatographie sur colonne de silice (cyclohexane/acétate d'éthyle 3 : 1) donne 2 (37 g, 54 %). [α]_{D} - 26 (c = 1, dichlorométhane). ¹H RMN (CDCl₃). δ 5,05-4,96 (m, 2H, H-2, H-4), 4,39, (d, 1H, J = 9,5 Hz, H-1), 4,18-4,12 (m, 2H, H-6, H-6'), 3,60 (m, 1H, H-5), 3,50 (dd, 1H, J = 9,3 Hz, H-3), 3,41 (s, 3H, OCH₃), 2,65-2,53 (m, 2H, SC*H*_{*2*}CH₃), 2,12, 2,11, 2,09 (3s, 9H, 3 Ac), 1,25 (t, 1H, SCH₂C*H*₃).

### PRÉPARATION 2

### Éthyl 4,6-O-benzylidène-3-O-méthyl-1-thio-β-D-glucopyranoside (3)

On dissout le composé **2** (37 g, 0,1 mmol) dans un mélange de méthanol et de dichlorométhane 1 : 2 (1,5 L). On ajoute une solution 2M de méthanolate de sodium (150 mL). Après 0,5 heure à température ambiante on neutralise avec de la résine Dowex® 50 (H⁺), filtre, et on concentre.

On dissout le composé brut précédent dans l'acétonitrile anhydre (1 L) et ajoute de l'α, α-diméthoxytoluène (30 mL, 0,2 mol) et de l'acide camphorsulfonique (2,3 g, 10 mmol). On laisse sous agitation pendant 1,5 heure (CCM), on additionne de la triéthylamine (1,4 mL) et on concentre. Le résidu obtenu est précipité dans l'éther éthylique et donne **3** (27 g, 81 %). [α]_{D} - 60 (c = 1,63, dichlorométhane). ¹H RMN (CDCl₃) δ 7,51-7,34 (m, 5H, Ph), 5,55 (s, 1H, C₆H₅C*H*), 4,56 (d, 1H, J = 9,2 Hz, H-1), 2,75 (m, 2H, SC*H*₂CH₃), 1,32 (t, 3H, SCH₂C*H*₃).
Anal. calculée pour C₁₆H₂₂O₅S (326,41) : C, 58,58 ; H, 6,79 ; S, 9,82. Trouvé : C, 58,99 ; H, 6,74 ; S, 9,75.

### PRÉPARATION 3

### Éthyl 2-O-benzyl-4,6-O-benzylidène-3-O-méthyl-1-thio-β-D-glucopyranoside (4)

On ajoute, à 0° C, de l'hydrure de sodium (2,00 g, 83,3 mmol) à une solution de **3** (23 g, 71,0 mmol) et de bromure de benzyle (11 mL, 93,0 mmol) dans le *N,N*-diméthylformamide (200 mL). On laisse sous agitation pendant 2 heures (CCM), on additionne du méthanol, et on verse le mélange réactionnel dans l'eau. On extrait avec de l'acétate d'éthyle, on lave avec de l'eau, on sèche et on concentre. On précipite dans l'éther éthylique pour obtenir **4** (18,8 g, 63 %). pf 123 C. [α]_{D} - 35 (c = 0,63, dichlorométhane). ¹H RMN (CDCl₃) δ 7,50-7,25 (m, 10H, 2Ph), 5,55 (s, 1H, C₆H₅C*H*), 4,54 (d, 1H, J = 9,7 Hz, H-1), 4,34 (m, 1H, H-6), 3,75 (t, 1H, J = 10,2 Hz, H-6'), 3,65 (s, 3H, OCH₃₎, 3,60-3,33 (m, 4H, H-5, H-4, H-3, H-2), 2,75 (m, 2H, SC*H*₂CH₃), 1,32 (t, 3H, SCH₂C*H*₃)*.*
Anal. calculée pour C₂₃H₂₈O₅S (416,54) : C, 66,32 ; H, 6,78 ; S, 7,70. Trouvé : C, 66,25 ; H, 7,28 ; S, 7,54.

### PRÉPARATION 4

### Éthyl 2,6-di-O-benzyl-3-O-méthyl-7-thio-β-D-glucopyranoside (5)

On ajoute, sous argon, une solution d'anhydride trifluoroacétique (0,65 ml, 4,50 mmol) dans de l'acide trifluoroacétique (16 mL, 0,21 mol) à une solution de **4** (28,8 g, 69,0 mmol) et de triéthylsilane (33 mL, 0,21 mol) dans du dichlorométhane (120 mL). On laisse sous agitation pendant 2 heures, on dilue avec de l'acétate d'éthyle et on additionne une solution aqueuse 1M d'hydroxyde de sodium jusqu'à pH 9. On extrait avec de l'acétate d'éthyle, on lave à l'eau, on sèche et on évapore à sec. On purifie sur colonne de silice (cyclohexane/acétate d'éthyle 3 : 1 puis 2 : 1) pour obtenir **5** (17,4 g, 60 %). [α]_{D} - 47 (c = 1, dichlorométhane). ¹H RMN (CDCl₃) δ 7,45-7,25 (m, 10H, 2Ph), 4,47 (d, 1H, J = 9,3 Hz, H-1), 3,66 (s, 3H, OCH₃), 3,61-3,40 (m, 2H, H-4 et H-5), 3,36-3,19 (m, 2H, H-2 et H-3), 2,73 (m, 2H, SC*H*₂CH₃), 1,31 (t, 3H, SCH₂C*H*₃).
Anal. calculée pour C₂₃H₃₀O₅S (418,55) : C, 66,00 ; H, 7,22 ; S, 7,66. Trouvé : C, 65,62; H,7,28; S, 7,21.

### PRÉPARATION 5

### Éthyl 2, 6-di-O-benzyl-4-O-lévulinyl-3-O-méthyl-1-thio-β-D-glucopyranoside (6)

On dissout le composé **5** (17,3 g, 41,4 mmol) dans du dioxane anhydre (400 mL). On ajoute de l'acide lévulinique (9,60 g, 83,0 mmol), du chlorhydrate de 1-(3-diméthylaminopropyl)-3-éthylcarbodiimide (16 g, 86 mmol) et de la 4-diméthylaminopyridine (1 g, 8,3 mmol). On laisse sous agitation pendant 4 heures, on extrait avec de l'acétate d'éthyle, on lave successivement avec une solution aqueuse à 5 % d'hydrogénosulfate de potassium, à l'eau, avec une solution aqueuse saturée d'hydrogénocarbonate de sodium, à l'eau, on sèche et on concentre. On purifie sur colonne de silice (toluène/acétate d'éthyle 6 : 1) pour obtenir **6** pur (19,9 g, 93 %). [α]_{D} - 5 (c = 1,46, dichlorométhane). LSIMS, mode positif : m/z thioglycerol + NaCl, 539 (M+Na)⁺; thioglycerol + KF, 555 (M+K)⁺. ¹H RMN (CDCl₃) δ 7,40-7,20 (m, 10H, 2 Ph), 4,92 (m, 1H, H-4), 2,8-2,4 (m, 6H, SC*H*₂CH₃ et O(C:O)C*H*₂C*H*₂(C:O)CH₃), 2,16 (s, 3H, O(C:O)CH₂CH₂(C:O)C*H*₃), 1,32 (t, 1H, J = 7,3 Hz, SCH₂C*H*₃).
Anal. calculée pour C₂₈H₃₆O₇S (516,65) : C, 65,09 ; H, 7,02 ; S, 6,21. Trouvé : C, 65,30 ; H, 7,03 ; S, 5,75.

### PRÉPARATION 6

### Allyl 4,6-O-benzylidène-3-O-méthyl-α,β-D-glucopyranoside (7)

On additionne de l'acide trifluorométhanesulfonique (1,10 mL, 0,012 mol) à une suspension de 3-*O*-méthyl-glucose commercial (135 g, 0,7 mol) dans de l'alcool allylique (1 L). On chauffe à 120° C pendant 2 heures. On neutralise par addition de triéthylamine (2 mL) et on évapore à sec.
Au composé brut précédent dissous dans du *N,N*-diméthylformamide (2 L), on ajoute de l'α,α-diméthoxytoluène (136 mL, 0,9 mol) et de l'acide camphorsulfonique (25 g, 0,13 mmol). On chauffe à 80° C pendant 1 heure sous vide. On neutralise par addition de triéthylamine (21 mL) et on extrait avec de l'acétate d'éthyle, on lave à l'eau, on sèche et on concentre pour obtenir un solide, mélange α/β = 3/2, (144 g, 57 %). On recristallise dans de l'éthanol pour obtenir **7**-α pur (60 g, 26 %). Une chromatographie d'une partie des eaux-mères sur colonne de silice (cyclohexane/acétate d'éthyle 3 : 1) donne **7**-β pur (7,6 g), **7**-α/β (6,8 g) et **7**-α pur (1,4 g).
Composé **7**-β: [α]_{D} - 43 (c = 1, dichlorométhane). pf : 131 C. ¹H RMN (CDCl₃) δ 7,50-7,26 (m, 5H, Ph), 6,01-5,90 (m, 1H, OCH₂(C*H* :CH₂)), 5,55 (s, 1H, C₆H₅C*H*), 5,38-5,32 (m, 2H, OCH₂(CH :C*H*₂)), 4,47 (d, 1H, J = 7,5 Hz, H-1), 4,42-4,32 (m, 2H, H-6' et OC*H*₂(CH :CH₂)), 4,21-4,15 (m, 1H, OC*H*₂(CH :CH₂)), 3,80 (dd, 1H, J = 10,2 Hz, H-6), 3,67(s, 3H, OCH₃)
Anal. calculée pour C₁₇H₂₂O₆ (322,36) : C, 63,34 ; H, 6,88. Trouvé : C, 63,23 ; H, 7,12.

### PRÉPARATION 7

### Allyl 2-O-acétyl-4,6-O-benzylidène-3-O-méthyl-β-D-glucopyranoside (8)

On dissout **7** (11,5 g, 35,7 mmol) dans du dichlorométhane (100 mL) et on ajoute de l'anhydride acétique (4,0 mL, 42,8 mmol), de la triéthylamine (6,40 mL, 46,4 mmol) et de la 4-diméthylaminopyridine (440 mg, 3,60 mmol). On laisse sous agitation pendant 2 heures (CCM), on lave successivement avec une solution aqueuse à 5 % d'hydrogénosulfate de potassium, à l'eau, avec une solution aqueuse saturée d'hydrogénocarbonate de sodium, à l'eau, on sèche et on évapore jusqu'à l'obtention d'un solide **8** (12,3 g, 95 %). pf : 115 C. [α]_{D}. 68 (c = 1, dichlorométhane). LSIMS, mode positif : m/z thioglycerol + NaCl, 387 (M+Na)⁺ ; thioglycerol + KF, 403 (M+K)⁺. ¹H RMN (CDCl₃) δ 7,51-7,34 (m, 5H, Ph), 5,98-5,78 (m, 1H, OCH₂(C*H*:CH₂)), 5,56 (s, 1H, C₆H₅C*H*), 5,32-5,17 (m, 2H, OCH₂(CH :C*H*₂)), 4,99 (dd, J = 8 Hz, 1H, H-2), 4,55 (d, J = 7,9 Hz, 1H, H-1), 4,39-4,29 (m, 2H, H-6 et OC*H*₂(CH :CH₂)), 4,14-4,04 (m, 1H, OC*H*₂(CH :CH₂)) 3,82 (t, J = 10,2 Hz, 1H, H-6'), 3,60 (s, 3H, OCH₃), 2,12 (s, 3H, Ac). Anal. calculée pour C₁₉H₂₄O₇ (366,39) : C, 62,63 ; H, 6,64. Trouvé : C, 62,63 ; H, 6,64.

### PRÉPARATION 8

### Allyl 2-O-acétyl-6-O-benzyl-3-O-méthyl-β-D-glucopyranoside (9)

On additionne, à 0° C, à une solution de **8** (12,0 g, 33,3 mmol) et de triéthylsilane (21,3 mL, 133 mmol) dans du dichlorométhane anhydre (50 mL) une solution d'anhydride trifluoroacétique (306 µL, 2,10 mmol) dans l'acide trifluoroacétique (10 mL). On laisse sous agitation pendant 4 heures (CCM), on dilue avec de l'acétate d'éthyle et on ajoute une solution aqueuse 1M d'hydroxyde de sodium jusqu'à pH 9. On extrait avec de l'acétate d'éthyle, on lave à l'eau, on sèche et on concentre. On purifie sur colonne de silice (cyclohexane/acétone 8 : 5) pour donner **9** pur (10 g, 82 %). [α]_{D}. 40 (c = 1,06, dichlorométhane). ¹H RMN (CDCl₃) δ 7,35-7,28 (m, 5H, Ph), 5,87-5,79 (m, 1H, OCH₂(C*H* :CH₂)), 5,28-5,14 (m, 2H, OCH₂(CH :C*H*₂)), 4,43 (d, 1H, J = 7,9 Hz, H-1), 4,41-4,28 (m, 1H, OC*H*₂(CH :CH₂)), 4,10-4,02 (m, 1H, OC*H*₂(CH :CH₂), 3,77-3,75 (m, 2H, H-6 et H-6'), 3,51 (s, 3H, OCH₃), 3,30 (dd, 1H, J = 8,9 Hz, H-3), 2,8 (d, 1H, OH).
Anal. calculée pour C₁₉H₂₆O₇ (366,39) : C, 62,28 ; H, 7,15. Trouvé : C, 61,73 : H, 7,19.

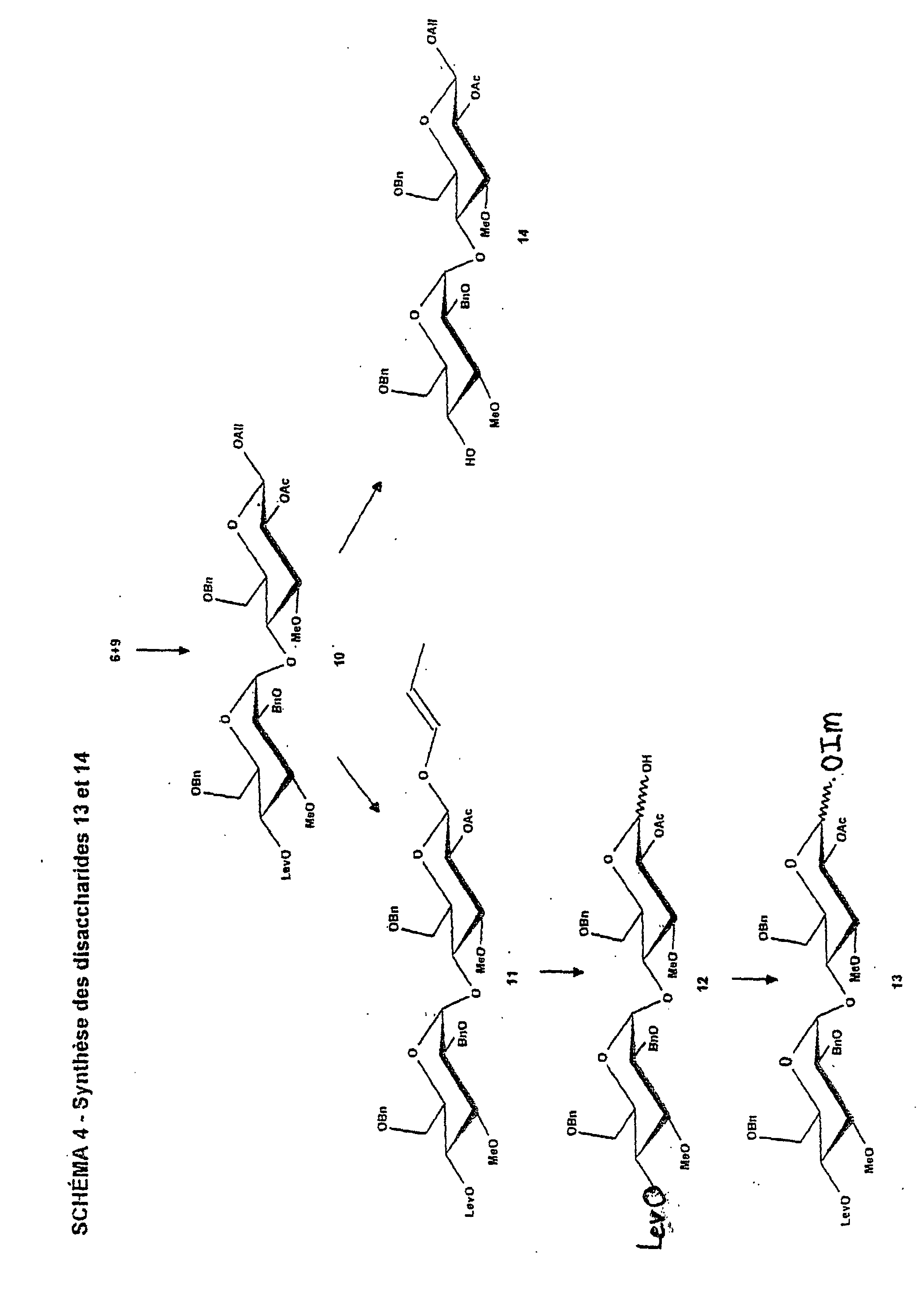

### PRÉPARATION 9

### Allyl 2-O-acétyl-6-O-benzyl-3-O-méthyl-4-O-(2,6-di-O-benzyl-4-O-lévu-linyl-3-O-méthyl-α-D-glucopyranosyl)-β-D-glucopyranoside (10)

On dissout le thioglycoside **6** (17,4 g, 33,7 mmol) et l'accepteur de glycosyle **9** (10,3 g, 28,1 mmol) dans le dichloroéthane (150 mL). On ajoute du tamis moléculaire 4 Å et on laisse sous agitation pendant 1 heure. À - 20° C et sous une atmosphère d'argon, on additionne une solution de *N*-iodosuccinimide (8,30 g, 33,7 mmol) et d'acide trifluorométhanesulfonique (0,30 mL, 3,30 mmol) dans un mélange de dichloroéthane et d'éther éthylique (415 mL, 1 : 1). On laisse sous agitation pendant 10 minutes (CCM), on ajoute de l'hydrogénocarbonate de sodium, on filtre et on lave successivement avec une solution aqueuse 1M de thiosulfate de sodium, à l'eau, avec une solution aqueuse saturée d'hydrogénocarbonate de sodium, à l'eau, on sèche et on concentre. On purifie sur colonne de silice (dichlorométhane/acétate d'éthyle 11:1) pour donner le disaccharide **10-α** pur (11,7 g, 52 %). [α]_{D} + 38 (c = 1,01, dichlorométhane) .¹H RMN (CDCl₃) δ 7,35-7,23 (m, 15H, 3 Ph), 5,90-5,80 (m, 1H, OCH₂(CH :CH₂)), 5,47 (d, 1H, J = 3,6 Hz, H-1'), 5,27-5,14 (m, 2H, OCH₂(CH :C*H*₂)), 5,05-4,90 (m, 2H, H-4' et H-2), 4,42 (d, 1H, J = 7,6 Hz, H-1), 4,38-4,32 (m, 1H, OC*H*₂(CH :CH₂)), 4,15-4,0 (m, 1H, OC*H*₂(CH :CH₂)) 3,90 (dd, 1H, J = 8,8 Hz, H-4), 3,54, 3,34 (2 s, 6H, 2 OCH₃), 2,75-2,40 (m, 4H, O(C:O)C*H*₂C*H*₂(C:O)CH₃), 2,16, 2,10 (2 s, 6H, Ac et O(C:O)CH₂CH₂(C:O)C*H*₃).
Anal. calculée pour C₄₅H₅₆O₁₄ (820,94) : C, 65,84 ; H, 6,88. Trouvé : C, 65,74 ; H, 6,90.

### PRÉPARATION 10

### Prop-1'-ényl2-O-acétyl-6-O-benzyl-3-O-méthyl-4-O-(2, 6-di-O-benzyl-4-O-lévulinyl-3-O-méthyl-α-D-glucopyranosyl)-β-D-glucopyranoside (11)

On ajoute de l'hexafluorophosphate de 1,5-cyclooctadiène-bis[méthyldiphényl-phosphine]-iridium (5,80 mg, 0,70 µmol) à une solution de **10** (1,36 g, 1,66 mmol) dans du tétrahydrofurane sans peroxydes (4,30 mL). On dégaze la solution, on met sous atmosphère d'argon et on introduit de l'hydrogène. On laisse sous agitation pendant 10 minutes (CCM), on évapore. On reprend avec du dichlorométhane, on lave avec une solution aqueuse saturée d'hydrogénocarbonate de sodium, à l'eau, on sèche et on concentre. On purifie sur colonne de silice (toluène/acétate d'éthyle 3 :1) pour obtenir **11** pur (1,04 g, 76 %). [α]_{D} + 47 (c = 1,1, dichlorométhane). LSIMS, mode positif : m/z thioglycerol + NaCl, 951 (M + Na)⁺ ; thioglycerol + KF, 967 (M + K)⁺. ¹H RMN (CDCl₃) δ 7,34-7,23 (m, 15H, 3 Ph), 6,21-6,16 (m, 1H, O(CH :CH)CH₃), 5,45 (d, 1H, J = 3,5 Hz, H-1'), 5,13-4,97 (m, 3H, H-4', H-2 et O(CH :C*H*)CH₃), 4,6 (d, 1H, J = 7,55 Hz, H-1), 3,96 (dd, 1H, J = 8,9 Hz, H-4'), 3,54, 3,34 (2 s, 6H, 2 OCH₃), 2,74-2,36 (m, 4H, O(C:O)C*H*₂C*H*₂(C:O)CH₃), 2,15, 2,08 (2s, 6H, Ac et O(C:O)CH₂CH₂(C:O)C*H*₃) 1,56-1,51 (dd, 3H, O(CH :CH)C*H*₃).
Anal. calculée pour C₄₅H₅₆O₁₄ (820,94) : C, 65,84 ; H, 6,88 ; Trouvé : C, 66,21 ; H, 6,92.

### PRÉPARATION 11

### 2-O-Acétyl-6-O-benzyl-3-O-méthyl-4-O-(2,6-di-O-benzyl-4-O-lévulinyl-3-O-méthyl-a-D-glucopyranosyl)-α,β-D-glucopyranose (12)

On ajoute goutte à goutte une solution de chlorure mercurique (3,9 g, 14,3 mmol) dans un mélange d'acétone et d'eau (26 mL, 5 : 1) à une solution de **11** (7,8 g, 9,53 mmol) et d'oxyde mercurique dans le même solvant (80 mL). On laisse sous agitation pendant 1 heure, on filtre et on concentre. On extrait avec du dichlorométhane, on lave avec une solution aqueuse saturée d'iodure de potassium, à l'eau, on sèche et on concentre. On purifie sur colonne de silice (dichlorométhane/acétone 10 : 1 puis 4:1) pour obtenir **12** (6,70 g, 90 %). [α]_{D+} 92 (c = 1,37, dichlorométhane). CCM, R_{F} 0,31, dichlorométhane/acétone : 14 :1. ¹H RMN (CDCl₃) δ 7,37-7,24 (m, 15H, 3 Ph), 5,46 (d, 1H, J = 3,5 Hz, H-1'), 5,37 (d, J = 3,6 Hz, H-1α), 4,58 (d, J = 8 Hz, H-1β), 3,54, 3,39, 3,36 (3s, 6H, 2 OCH₃), 2,75-2,4 (m, 4H, O(C:O)C*H*₂C*H*₂(C:O)CH₃), 2,16, 2,15 (2s, 6H, Ac et O(C:O)CH₂CH₂(C:O)C*H*₃).
Anal. calculée pour C₄₂H₅₂O₁₄ (780,83) ; C, 64,60 ; H, 6,71 ; Trouvé : C, 65,09 ; H, 6,82.

### PRÉPARATION 12

### 2-O-Acétyl-6-O-benzyl-3-O-méthyl-4-O-(2,6-di-O-benzyl-4-O-lévulinyl-3-O-méthyl-α-D-glucopyranosyl)-α,β-D-glucopyranose trichloroacétimidate (13)

On dissout le composé **12** (5,00 g, 6,4 mmol) dans du dichlorométhane (50 mL) et on ajoute sous argon du trichloroacétonitrile (3,9 mL, 38,8 mmol) et du carbonate de potassium (1,6 g, 11,6 mmol). On laisse sous agitation pendant 16 heures (CCM) et on filtre. On purifie sur colonne de silice (dichlorométhane/acétone 8 : 1 puis 4:1) pour donner un mélange (α/β = 60/40) d'imidates **13** (5,22 g, 87 %). CCM, R_{F} 0,66 et 0,51, dichlorométhane/acétone 20:1. ¹H RMN (CDCl₃) δ 8,62-8,59 (2s, 1H, N :H-α et β), 7,37-7,23 (m, 15H, 3 Ph), 6,51 (d, J = 3,7 Hz, H-1α), 5,81 (d, J = 7,1 Hz, H-1β), 5,50 (d, 1H, J = 3,5 Hz, H-1'), 3,55, 3,41, 3,37 (3s, 9H, 3 OCH₃), 2,75-2,40 (m, 4H, O(C:O)C*H*₂C*H*₂(C:O)CH₃), 2,16, 2,07, 2,04 (3s, 6H, Ac et (C:O)CH₂CH₂(C:O)C*H*₃).
Anal. calculée pour C₄₄H₅₂Cl₃NO₁₄ (925,26) : C, 57,12 ; H, 5,66 ; N, 1,51. Trouvé : C, 57,31 ; H, 5,87 ; N, 1,55.

### PRÉPARATION 13

### Allyl 2-O-acétyl-6-O-benzyl-3-O-méthyl-4-O-(2,6-di-O-benzyl-3-O-méthyl-α-D-glucopyranosyl)-β-D-glucopyranoside (14)

Le composé **10** (3,11 g, 3,80 mmol) est traité selon la MÉTHODE 2 pour donner **14** (2,70 g, 97 %). [α]_{D} +25 (c = 1,7, dichlorométhane). LSIMS, mode positif : m/z thioglycerol + NaCl, 745 (M+Na)⁺ ; thioglycerol + KF, 761 (M+K)⁺. ¹H RMN (CDCl₃) δ 7,33-7,20 (m, 15H, 3 Ph), 5,87-5,78 (m, 1H, OCH₂(C*H* :CH₂)), 5,50 (d, 1H, *J* = 3,5 Hz, H-1'), 5,30-5,17 (m, 2H, OCH₂(CH :C*H*₂)), 5,02 (dd, 1H, H-2), 4,43 (d, 1H, *J* = 7,6 Hz, H-1), 4,34-4,28 (m, 1H, OC*H*₂(CH :CH₂)), 4,12-4,02 (m, 1H, OC*H*₂(CH :CH₂)), 3,63, 3,36 (2s, 6H, 2 OCH₃), 2,10 (s, 3H, Ac).
Anal. calculée pour C₄₀H₅₀O₁₂ (722,84) : C, 66,47 ; H, 6,97. Trouvé : C, 66,31 ; H, 7,24.

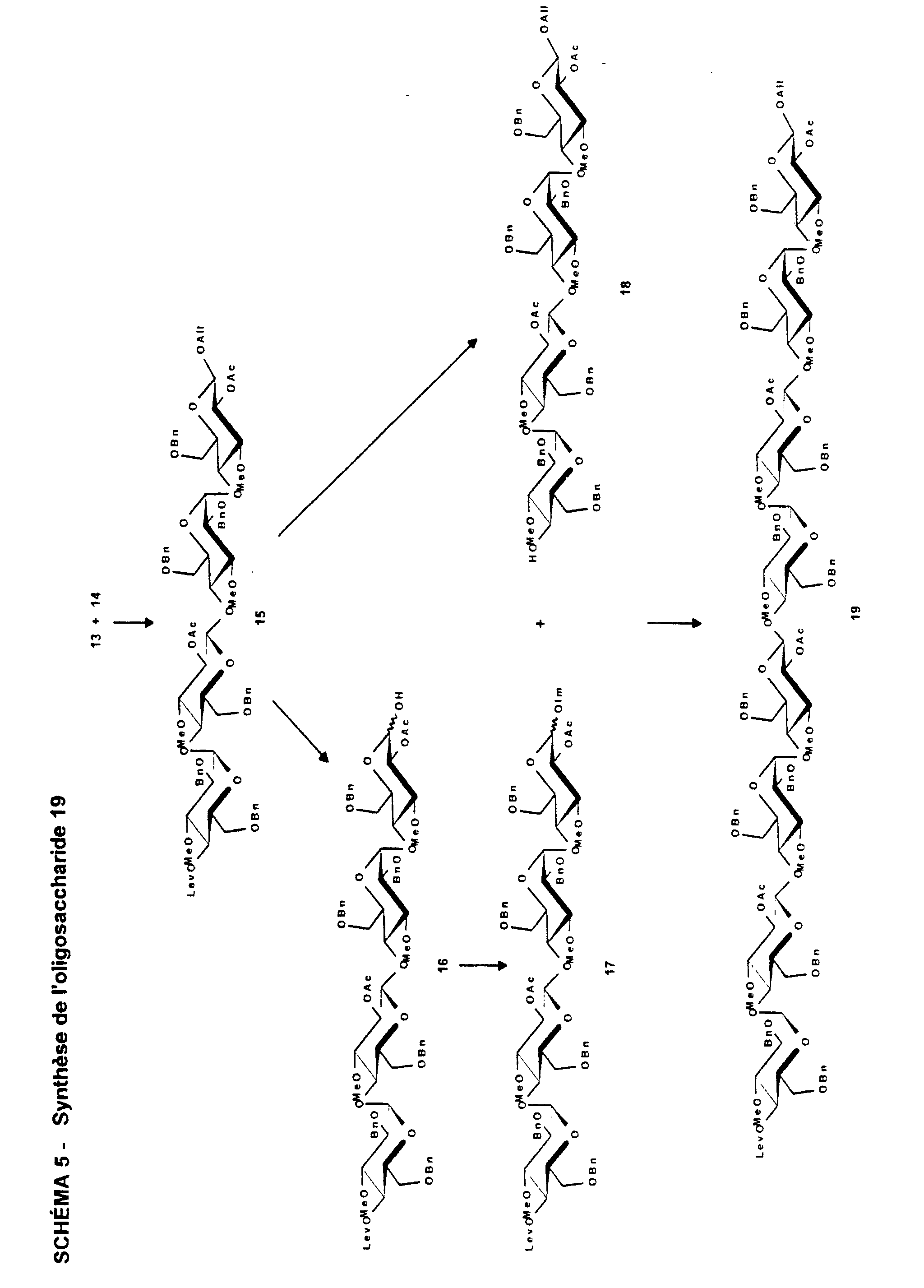

### PRÉPARATION 14

### Allyl O-(2,6-di-O-benzyl-4-O-lévulinyl-3-O-méthyl-α-D-glucopyranosyl)-(1→4)-O-(2-O-acétyl-6-O-benzyl-3-O-méthyl-β-D-glucopyranosyl)-(1→4)-O-(2,6-di-O-benzyl-3-O-méthyl-α-D-glucopyranosyl)-(1→4)-2-O-acétyl-6-O-benzyl-3-O-méthyl-β-D-glucopyranoside (15)

Un mélange de l'imidate **13** (4,22 g, 4,56 mmol) et de l'accepteur de glycosyle **14** (2,63 g, 3,64 mmol) est traité selon la MÉTHODE 1. On purifie sur colonne de silice (toluène/éther éthylique 3 : 2 puis 1:1) pour donner le tétrasaccharide **15** (4,31 g, 80 %). [α]_{D} + 52 (c = 0,66, dichlorométhane). ¹H RMN (CDCl₃) δ 7,35-7,23 (m, 30H, 6 Ph), 5,83-5,79 (m, 1H, OCH₂(C*H* :CH₂)), 5,47 (d, 2H, J = 3,5 Hz, H-1''' et H-1'), 5,25-5,14 (m, 2H, OCH₂(CH :C*H*₂)), 4,38 (d, 1H, J = 7,7 Hz, H-1"), 4,30 (d, 1H, J = 8 Hz, H-1), 4,32-4,25 (m, 1H, OC*H*₂(CH :CH₂)), 4,08-4,02 (m, 1H, OC*H*₂(CH :CH₂)), 3,56, 3,53, 3,34, 3,27 (4s, 12H, 4 OCH₃), 2,78-2,40 (m, 4H, O(C:O)C*H*₂C*H*₂(C:O)CH₃), 2,15, 2,09, 1,85 (3s, 9H, 2 Ac et O(C:O)CH₂CH₂(C:O)C*H*₃).
Anal. calculée pour C₈₂H₁₀₀O₂₅ (1485,7) : C, 66,29 ; H, 6,78. Trouvé : C, 66,10 ; H, 6,79.

### PRÉPARATION 15

### O-(2,6-Di-O-benzyl-4-O-lévulinyl-3-O-méthyl-α-D-glucopyranosyl)-(1→4)-O-(2-O-acétyl-6-O-benzyl-3-O-méthyl-β-D-glucopyranosyl)-(1→4)-O-(2,6-di-O-benzyl-3-O-méthyl-α-D-glucopyranosyl)-(1→4)-2-O-acétyl-6-O-benzyl-3-O-méthyl-α,β-D-glucopyranose (16)

Le composé **15 (2,30** g, 1,54 mmol) est traité comme pour la PRÉPARATION 10. Après 10 minutes, on ajoute au mélange réactionnel une solution de *N*-bromosuccinimide (0,30 g, 1,70 mmol) dans le dichlorométhane (15 mL) et de l'eau (5,50 mL). On laisse sous agitation pendant 5 minutes (CCM). On dilue avec du dichlorométhane, on lave avec une solution aqueuse saturée d'hydrogénosulfate de sodium, avec de l'eau, on sèche et on concentre. On purifie sur colonne de silice (toluène/acétate d'éthyle 3 : 2) pour donner **16** pur (1,57 g, 71 % sur les deux étapes). [α]_{D} + 69 (c = 0,87, dichlorométhane). ¹H RMN (CDCl₃) δ 7,38-7,20 (m, 30H, 6Ph), 5,47 (d, 1H, J = 3,5 Hz, H-1''' et H-1'), 5,36 (d, 1H, J = 3,5 Hz, H-1α), 4,55 (d, 1H, J = 8 Hz, H-1), 4,36 (d, 1H, J = 8 Hz, H-1"), 3,56, 3,54, 3,39, 3,36, 3,28 (5s, 9H, 3 OCH₃), 2,75-2,35 (m, 4H, O(C:O)C*H*₂C*H*₂(C:O)CH₃), 2,16, 2,13, 2,12, 1,86 (4s, 9H, 2 Ac et O(C:O)CH₂CH₂(C:O)C*H*₃).

### PRÉPARATION 16

### O-(2,6-Di-O-benzyl-4-O-lévulinyl-3-O-méthyl-α-D-glucopyranosyl)-(1→4)-O-(2-O-acétyl-6-O-benzyl-3-O-méthyl-β-D-glucopyranosyl)-(1→4)-O-(2,6-di-O-benzyl-3-O-méthyl-α-D-glucopyranosyl)-(1→4)-2-O-acétyl-6-O-benzyl-3-O-méthyl-α,β-D-glucopyranose trichloroacétimidate (17)

On laisse agiter pendant 16 heures, à température ambiante, un mélange de **16** (1,5 g, 1,04 mmol), de trichloroacétonitrile (0,63 mL, 6,22 mmol) et de carbonate de potassium (0,26 g, 1,87 mmol) dans le dichlorométhane (15 mL). On filtre la solution et on concentre. On purifie sur colonne de silice (toluène/acétone + 1 ‰ de triéthylamine 4 : 1) pour donner **17** (1,47 g, 89,6 %). CCM, R_{F} 0,5, toluène/acétone 7 : 2.

### PRÉPARATION 17

### Allyl O-(2,6-di-O-benzyl-3-O-méthyl-α-D-glucopyranosyl)-(1→4)-O-(2-O-acétyl-6-O-benzyl-3-O-méthyl-β-D-glucopyranosyl)-(1→4)-O-(2,6-di-O-benzyl-3-O-méthyl-α-D-glucopyranosyl)-(1→4)-2-O-ecétyl-6-O-benzyl-3-O-méthyl-β-D-glucopyranoside (18)

La délevulination de **15** (1,3 g, 0,87 mmol) est traitée selon la MÉTHODE 2 pour donner **18** (1,05 g, 86 %). [α]_{D} + 40 (c = 0,6, dichlorométhane). ¹H RMN (CDCl₃) δ 7,36-7,23 (m, 30H, 6 Ph), 5,83-5,78 (m, 1H, OCH₂(C*H* :CH₂)), 5,50 (d, 1H, J = 3,5 Hz, H-1"'), 5,47 (d, 1H, J = 3,5 Hz, H-1'), 5,25-5,21 (dd, 1H, J = 1,6 Hz, J = 17 Hz, OCH₂(CH :C*H*₂)), 5,16-5,13 (dd, 1H, J = 1,4 Hz, J = 10 Hz, OCH₂(CH :C*H*₂)), 4,38 (d, 1H, J = 6,5 Hz, H-1"), 4,31 (d, 1H, J = 6,5 Hz, H-1), 4,08-4,02 (m, 1H, OC*H*₂(CH :CH₂)), 3,59 (m, 1H, H-4"'), 3,67, 3,53, 3,39, 3,29 (4s, 12H, 4 OCH₃), 2,09, 1,86 (2s, 6H, 2 Ac).

### PRÉPARATION 18

### Allyl O-(2, 6-di-O-benzyl-4-O-lévulinyl-3-O-méthyl-α-D-glucopyranosyl)-(1→4)-[O-(2-O-acétyl-6-O-benzyl-3-O-méthyl-β-D-glucopyranosyl)-(1→4)-O-(2,6-di-O-benzyl-3-O-méthyl-α-D-glucopyranosyl)-(1→4)]₃-2-O-acétyl-6-O-benzyl-3-O-méthyl-β-D-glucopyranoside (19)

On traite un mélange de **18** (842 mg, 0,53 mmol) et de **17** (1,17 g, 0,74 mmol) selon la MÉTHODE 1. On purifie sur colonne Toyopearl® HW-50 (110 × 3,2 cm ; dichlorométhane/éthanol 1 : 1) pour donner **19** (1,44 g, 85 %). [α]_{D} + 57 (c = 1,01, dichlorométhane). ¹H RMN (CDCl₃) δ 7,35-7,20 (m, 60H, 12 Ph), 5,83-5,78 (m, 1H, OCH₂(C*H* :CH₂)), 5,24-5,21 (dd, 1H, OCH₂(CH :C*H*₂)), 5,16-5,13 (dd, 1H, OCH₂(CH :CH₂)), 3,59, 3,56, 3,51, 3,47, 3,33, 3,26 (6s, 24H, 8 OCH₃), 2,75-2,35 (m, 4H, O(C:O)C*H*₂C*H*₂(C:O)CH₃), 2,15, 2,09, 1,85, 1,84 (4s, 15H, 4 Ac et O(C:O)CH₂CH₂(C:O)C*H*₃);
δ des principaux protons anomériques : 5,48 ; 4,37 ; 4,29 ; 4,23 ppm.
Anal. calculée pour C₁₅₆O₄₇H₁₈₈ (2815,51) : C, 66,56 ; H, 6,73. Trouvé : C, 66,22 ; H; 6,75

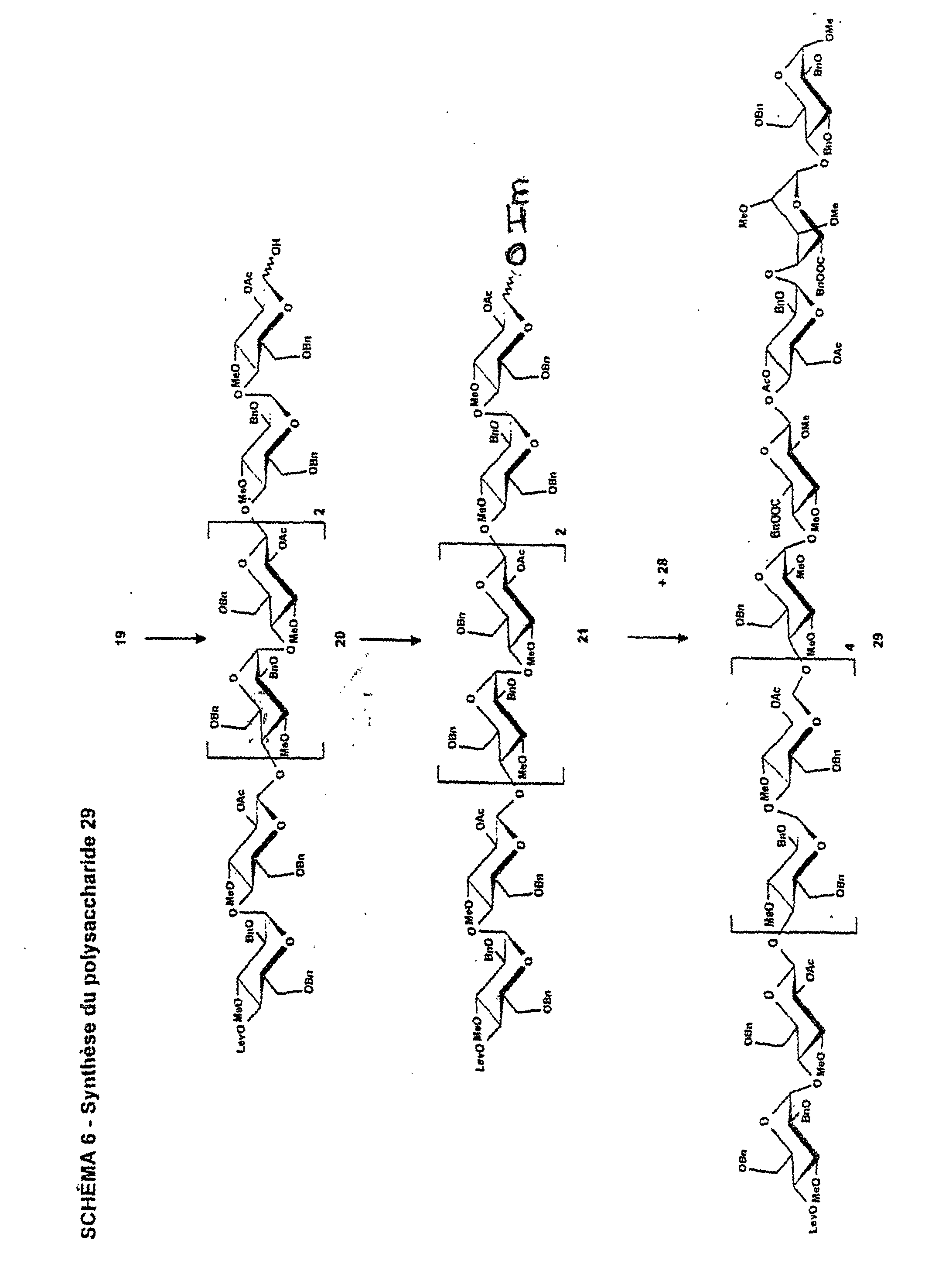

### PRÉPARATION 19

### O-(2,6-Di-O-benzyl-4-O-lévulinyl-3-O-méthyl-α-D-glucopyranosyl)-(1→4)-[O-(2-O-acétyl-6-O-benzyl-3-O-méthyl-β-D-glucopyranosyl)-(1→4)-O-(2,6-di-O-benzyl-3-O-méthyl-α-D-glucopyranosyl)-(1→4)]₃-2-O-acétyl-6-O-benzyl-3-O-méthyl-α,β-D-glucopyranose (20)

On traite le composé **19** (720 mg, 0,25 mmol) comme dans la PRÉPARATION 15. On purifie sur colonne de silice (toluène/acétate d'éthyle 3 : 2 puis 4 : 3) pour obtenir **20** (555 mg, 78 %). [α]_{D} + 70 (c = 0,94, dichlorométhane). CCM, R_{F} 0,43, toluène/acétate d'éthyle 1 : 1

### PRÉPARATION 20

### O-(2,6-Di-O-benzyl-4-O-lévulinyl-3-O-méthyl-α-D-glucopyranosyl)-(1→4)-[O-(2-O-acétyl-6-O-benzyl-3-O-méthyl-β-D-glucopyranosyl)-(1→4)-O-(2,6-di-O-benzyl-3-O-méthyl-α-D-glucopyranosyl)-(1→4)]₃-2-O-acétyl-6-O-benzyl-3-O-méthyl-α,β-D-glucopyranose trichloroacétimidate (21)

On traite le composé **20** (540 mg, 0,195 mmol) comme dans la PRÉPARATION 16. On purifie sur colonne de silice (toluène/acétate d'éthyle + 1 ‰ de triéthylamine 3 : 2) pour donner le mélange (α/β = 27/73) des imidates **21** (455 mg, 80 %).CCM, R_{F} 0,48, toluène/acétate d'éthyle 3 : 2. ¹H RMN (CDCl₃) δ 8,60, 8,59 (2s, 1H, N :Hα et β), 7,35-7,21 (m, 60H, 12 Ph), 2,75-2,40 (m, 4H, O(C:O)C*H*₂C*H*₂(C:O)CH₃), 2,16, 2,06, 2,04, 1,85, 1,84 (5s, 15H, 4 Ac et O(C:O)CH₂CH₂(C:O)C*H*₃). ¹H RMN (CDCl₃) δ des principaux protons anomériques : 6,50 ; 5,79 ; 5,51 ; 5,48 ; 4,29 ; 4,25 ppm.

### PRÉPARATION 21

### Phényl 2,4,6-tri-O-acétyl-3-O-méthyl-1-thio-α-D-glucopyranoside (22)

On dissout le 1,2,4,6-tétra-*O*-acétyl-3-*O*-méthyl-β-D-glucopyranose **1** (5,23 g, 14,4 mmol) dans le toluène (45 mL). On ajoute le thiophénol (3,0 mL, 28,8 mmol) et goutte à goutte du diéthyléthérate de trifluoroborane (1,77 mL, 14,4 mmol) puis on chauffe à 50° C pendant 0,5 heure. On dilue avec du dichlorométhane, on lave avec une solution aqueuse saturée d'hydrogénocarbonate de sodium, à l'eau, on sèche, on concentre. On purifie sur colonne de silice (cyclohexane/acétate d'éthyle 5 : 2) pour donner **22**-α (1,00 g, 17 %) et **22**-β (2,71 g, 46 %).
**22**-α. R_{F} 0,44, cyclohexane/acétate d'éthyle 3:2. [α]_{D} + 230 (c = 1, dichlorométhane). ESIMS, mode positif : m/z + NaCl, 435 (M+Na)⁺ ; + KF, 451 (M+K)⁺. ¹H RMN (CDCl₃) δ 7,46-7,27 (m, 5H, Ph), 5,89 (d, 1H, J = 5,6 Hz, H-1), 5,05-4,97 (m, 2H, H-2 et H-4), 4,49-4,42 (m, 1H, H-5), 4,25-4,18 (m, 1H, H-6), 4,05-4,00 (m, 1H, H-6'), 3,66 (dd, 1H, J = 9,5 Hz, H-3), 3,51 (s, 3H, OCH₃), 2,16, 2,12, 2,00 (3s, 9H, 3 Ac).
Anal. calculée pour C₁₉H₂₄O₈S (412,46) : C, 55,33 ; H, 5,87 ; S, 7,77. Trouvé : C, 55,25 ; H, 5,90 ; S, 7,75.

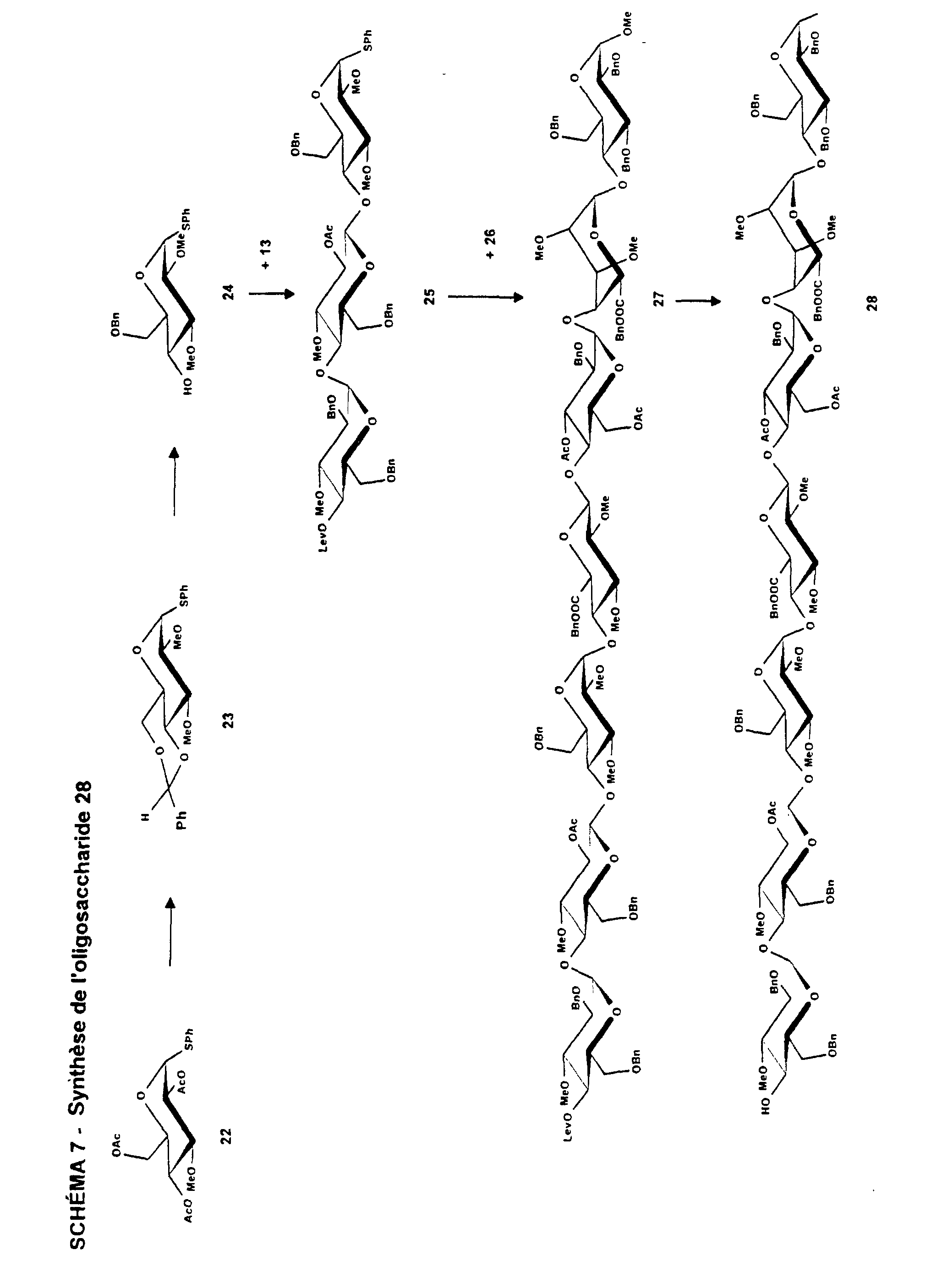

### PRÉPARATION 22

### Phényl 4,6-O-benzylidène-2,3-di-O-méthyl-1-thio-α-D-glucopyranoside (23)

On dissout le composé **22** (970 mg, 2,35 mmol) dans un mélange de méthanol et de dichlorométhane 2 : 1 (18 mL). On ajoute une solution 2M de méthanolate de sodium (150 mL). Après 0,5 heure à température ambiante, on neutralise avec de la résine Dowex® 50 (H⁺), filtre, et on concentre.
Au brut réactionnel précédent dans l'acétonitrile (22 mL), on ajoute de l'α,α-diméthoxytoluène (0,7 mL, 4,0 mmol) et de l'acide camphorsulfonique (51 mg, 0,22 mmol). On laisse sous agitation pendant 1 heure, on neutralise par addition de triéthylamine (0,50 mL) et on concentre.
On ajoute, à 0° C, de l'hydrure de sodium (73,0 mg, 2,80 mmol) à une solution du brut précédent et d'iodure de méthyle (163 µL, 4,0 mmol) dans le *N,N*-diméthylformamide (9 mL). On laisse sous agitation pendant 1 heure et on ajoute du méthanol. On extrait avec de l'acétate d'éthyle, on lave avec de l'eau, on sèche et on concentre jusqu'à obtention de **23** sous forme solide (840 mg, 94 %). pf : 178 C. [α]_{D} + 330 (c = 1, dichlorométhane). ESIMS, mode positif : m/z + NaCl, 411.4 (M+Na)⁺ ; + KF, 427.4 (M+K)⁺. ¹H RMN (CDCl₃) δ 7,50-7,24 (m, 10H, 2 Ph), 5,71 (d, 1H, J = 3,4 Hz, H-1), 5,52 (s, 1H, C₆H₅C*H*), 3,62 (s, 3H, OCH₃), 3,55 (s, 3H, OCH₃).
Anal. calculée pour C₂₁H₂₄O₅S (388,48) : C, 64,92 ; H, 6,23 ; S, 8,25. Trouvé : C, 64,87 ; H, 6,17 ; S, 7,85.

### PRÉPARATION 23

### Phényl 6-O-benzyl-2,3-di-O-méthyl-1-thio-α-D-glucopyranoside (24)

On traite le composé **23** (792 mg, 0,47 mmol) comme dans la PRÉPARATION 4. On purifie sur colonne de silice (cyclohexane/acétate d'éthyle 7 : 2 puis 2:1) pour donner **24** (318 mg, 80 %). [α]_{D} + 243 (c = 1, dichlorométhane). ESIMS, mode positif : m/z + NaCl, 413 (M+Na)⁺ ; + KF, 429 (M+K)⁺. ¹H RMN (CDCl₃) δ 7,52-7,22 (m, 10H, 2 Ph), 5,71 (d, 1H, J = 5,3 Hz, H-1), 3,64 et 3,49 (2s, 6H, 2 OCH₃), 3,36 (dd, 1H, H-3).
Anal. calculée pour C₂₁H₂₆O₅S (390,50) : C, 64,59 ; H, 6,71 ; S, 8,21. Trouvé : C, 64,05 ; H, 6,88 ; S, 7,74.

### PRÉPARATION 24

### Phényl O-(2, 6-di-O-benzyl-4-O-lévulinyl-3-O-méthyl-α-D-glucopyranosyl)-(1→4)-O-(2-O-acétyl-6-O-benzyl-3-O-méthyl-β-D-glucopyranosyl)-(1→4)-6-O-benzyl-2,3-di-O-méthyl-1-thio-α-D-glucopyranoside (25)

On traite un mélange de **13** (436 mg, 0,47 mmol) et de **24** (153 mg, 0,39 mmol) selon la MÉTHODE 1. On purifie sur colonne (Sephadex® LH20, éthanol/dichlorométhane 1:1) pour donner **25** pur (309 mg, 68 %). [α]_{D} + 144 (c = 1, dichlorométhane). ESIMS, mode positif : m/z + NaCl, 1175 (M+Na)⁺ ; +KF, 1191 (M+K)⁺. ¹H RMN (CDCl₃) δ 7,51-7,21 (m, 25H, 5 Ph), 5,73 (d, 1H, J = 5,2 Hz, H-1), 5,48 (d, 1H, J = 3,5 Hz, H-1"), 4,46 (d, 1H, J = 8 Hz, H-1'), 3,7, 3,54, 3,5, 3,31 (4s, 12H, 4 OCH₃), 2,70-2,41 (m, 4,H, O(C:O)C*H*₂C*H*₂(C:O)CH₃), 2,16, 2,01 (2s, 6H, 1 Ac et O(C:O)CH₂CH₂(C:O)C*H*₃).
Anal. calculée pour C₆₃H₇₆O₁₈S : C, 65,61 ; H, 6,64 ; S, 2,78. Trouvé : C, 65,02 ; H, 6,60 ; S, 2,72.

### PRÉPARATION 25

### Méthyl O-(2,6-di-O-benzyl-4-O-lévulinyl-3-O-méthyl-α-D-glucopyranosyl)-(1→4)-O-(2-O-acétyl-6-O-benzyl-3-O-méthyl-β-D-glucopyranosyl)-(1→4)-O-(6-O-benzyl-2,3-di-O-méthyl-α-D-glucopyranosyl)-(1→4)-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,3,6-tri-O-benzyl-α-D-glucopyranoside (27)

On ajoute, à - 25° C et sous argon, une solution de *N*-iodosuccinimide (92 mg, 0,38 mmol) et d'acide triflique (37,5 µL, 0,38 mmol) dans une solution de 1,2-dichloroéthane et d'éther éthylique 1 :1 (22 mL) à un mélange de **25** (451 mg (0,39 mmol) et de **26** (434 mg, 0,31 mmol), (P. Westerduin, *et al*. BioOrg. Med. Chem., 1994, 2, 1267) dans du 1,2-dichloroéthane (7,5 ml) en présence de tamis moléculaire 4Å (400 mg ). Après 30 minutes, on ajoute de l'hydrogénocarbonate de sodium solide. On filtre la solution, on lave avec une solution de thiosulfate de sodium, à l'eau, on sèche et on évapore. On purifie sur colonne Sephadex® LH-20 (dichlorométhane/éthanol 1:1) puis sur colonne de silice (cyclohexane/acétate d'éthyle 1 : 1 puis 2 : 3) pour donner **27** pur (487 mg, 64 %). [α]_{D} + 63 (c = 0,54, dichlorométhane). CCM, R_{F} 0,28, cyclohexane/acétate d'éthyle 2:1. ESIMS, mode positif : m/z + NaCl, 2454 (M+Na)⁺ ; + KF, 2469 (M+K)⁺. ¹H RMN (CDCl₃) δ 7,38-7,2 (m, 50H, 10 Ph), 3,56, 3,52, 3,48, 3,46, 3,44, 3,42, 3,39, 3,30, 3,17, (9s, 27H, 9 OCH₃), 2,75-2,4 (m, 4H, O(C:O) C*H*₂C*H*₂C :O)CH₃), 2,15, 1,98, 1,97, 1,87, (4s, 12H, 3 Ac et O(C:O)CH₂CH₂(C:O)C*H*₃) ; principaux protons anomériques : 5,57 ; 5,47 ; 5,30 ; 5,18 ; 4,57 ; 4,29; 4,08.

### PRÉPARATION 26

### Méthyl O-(2,6-di-O-benzyl-3-O-méthyl-α-D-glycopyranosyl)-(1→4)-O-(2-O-acétyl-6-O-benzyl-3-O-méthyl-β-D-glucopyranosyl)-(1→4)-O-(6-O-benzyl-2,3-di-O-méthyl-α-D-glucopyranosyl)-(1→4)-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyl-uronate)-(1→4)-2,3,6-tri-O-benzyl-α-D-glucopyranoside (28)

On réalise la délévulinisation de **27** (498 mg, 0,2 mmol) suivant la MÉTHODE 2 pour donner **28** (402 mg, **84** %). [α]_{D} + 64 (c = 1, CH₂Cl₂). ESIMS, mode positif : m/z 2352,9 (M+NH₄)⁺. ¹H RMN (CDCl₃) δ 7,38-7,20 (m, 50H, 10 Ph), 3,67, 3,52, 3,49, 3,46, 3,44, 3,41, 3,40, 3,28, 3,17 (9s, 27H, 9 Ac), 2,65 (d, 1H, J = 2,14 Hz, OH), 1,98, 1,96, 1,87 (3s, 9H, 3 Ac) ; principaux protons anomériques : 5,55 ; 5,49 ; 5,30 ; 5,18 ; 4,56 ; 4,31 ; 4,08.
Anal. calculée pour C₁₂₇H₁₅₂O₄₁ (2334,48) : C, 65,34 ; H, 6,56. Trouvé : C, 65,40 ; H, 6,62.

### PRÉPARATION 27

### Méthyl O-(2,6-di-O-benzyl-4-O-lévulinyl-3-O-méthyl-α-D-glucopyranosyl)-(1→4)-O-(2-O-acétyl-6-O-benzyl-3-O-méthyl-β-D-glucopyranosyl)-(1→4)-[O-(2,6-di-O-benzyl-3-O-méthyl-α-D-glucopyranosyl)-(1→4)-O-(2-O-acétyl-6-O-benzyl-3-O-méthyl-β-D-glycopyranosyl)-(1→4)]₄-O-(6-O-benzyl-2,3-di-O-méthyl-α-D-glucopyranosyl)-(1→4)-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,3,6-tri-O-benzyl-α-D-glucopyranoside (29)

On traite un mélange de **21** (340 mg, 1,16 mmol) et de **28** (256 mg, 1,09 mmol) suivant la MÉTHODE 1. On purifie le résidu sur colonne Toyopearl® HW-40 (3,2 x 70 cm, dichlorométhane/éthanol 1 : 1) pour donner le 15-mer **29** pur (421 mg, 76 %). [α]_{D} + 65 (c = 1, dichlorométhane). ESIMS, mode positif : m/z + KF, 2584,3 (M+2K)²⁺ ; 1736,5 (M+3K)³⁺. ¹H RMN (CDCl₃) δ 7,35-7,18 (m, 105H, 21 Ph), 2,75-2,4 (m, 4H, O(C:O)C*H*₂C*H*₂(C:O)CH₃), 2,15, 1,97, 1,95, 1,87, 1,84, 1,83 (6s, 24H, 7 Ac, et O(C:O)CH₂CH₂(C:O)C*H*₃); principaux protons anomériques : 5,55 ; 5,48 ; 5,30 ; 5,18 ; 4,56 ; 4,29 ; 4,22 ; 4,08.

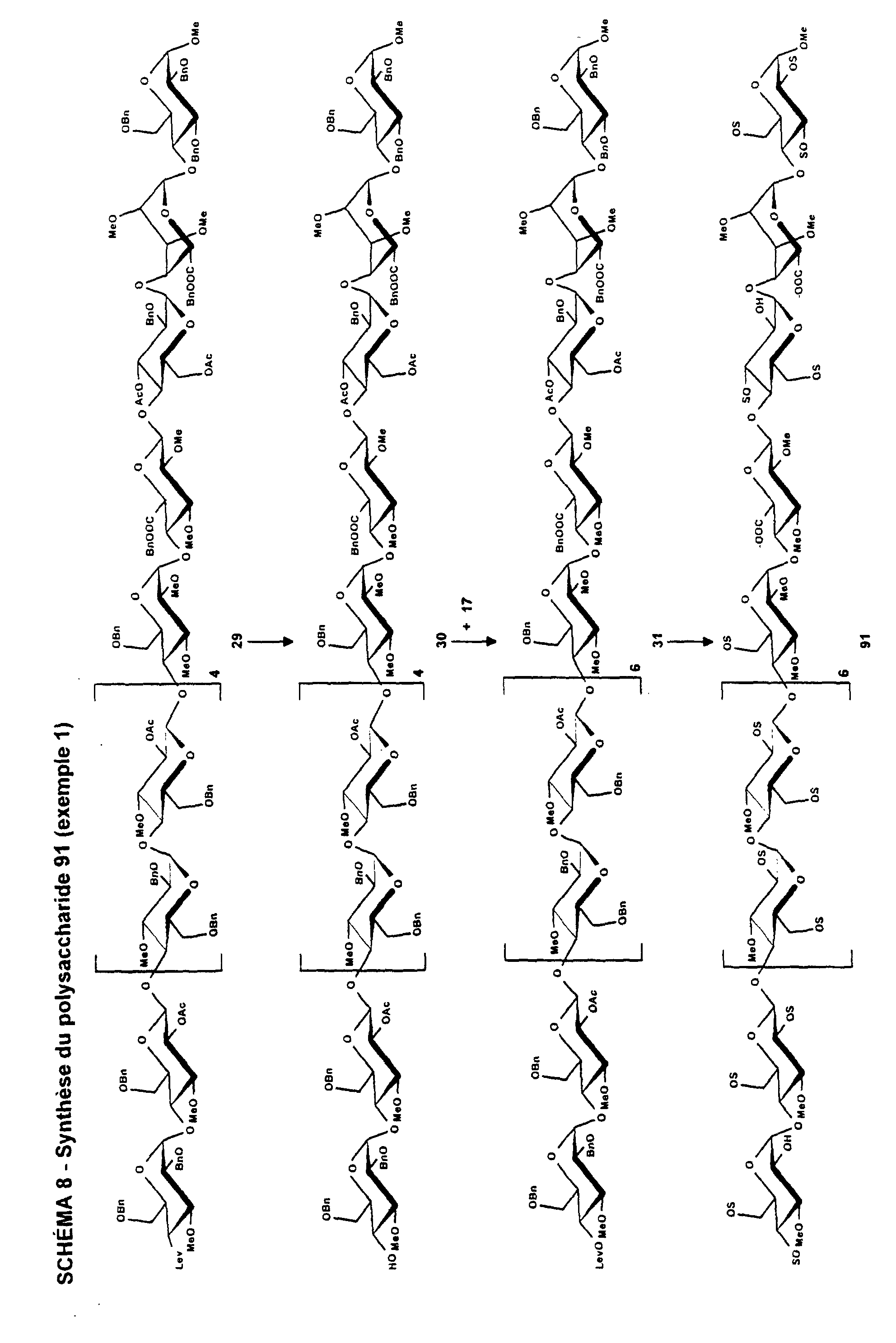

### PRÉPARATION 28

### Méthyl O-(2,6-di-O-benzyl-3-O-méthyl-α-D-glucopyranosyl)-(1→4)-O-(2-O-acétyl-6-O-benzyl-3-O-méthyl-β-D-glucopyranosyl)-(1→4)-[O-(2,6-di-O-benzyl-3-O-méthyl-α-D-glucopyranosyl)-(1→4)-O-(2-O-acétyl-6-O-benzyl-3-O-méthyl-β-D-glycopyranosyl)-(1→4)]₄-O-(6-O-benzyl-2,3-di-O-méthyl-α-D-glucopyranosyl)-(1→4)-O-(benzyl 2,3-di-O-méthyl-β-D-glucopyranosyluro-nate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,3,6-tri-O-benzyl-α-D-glucopyranaside (30)

On traite le composé **29** (342 mg, 0,067 mmol) selon la MÉTHODE 2 pour donner **30** (253 mg, 75 %). [α]_{D} + 59 (c = 0,92, dichlorométhane). ESIMS, mode positif : m/z + KF, 2535,6 (M+2K)²⁺. ¹H RMN (CDCl₃) δ des principaux protons anomériques : 5,55 ; 5,50 ; 5,48 ; 5,30 ; 4,56 ; 4,30 ; 4,22 ; 4,08.
Anal. calculée pour C₂₇₅H₃₂₈O₈₅ (4993,37) : C, 65,57 ; H, 6,60. Trouvé : C, 65,09 ; H, 6,57.

### PRÉPARATION 29

### Méthyl O-(2,6-di-O-benzyl-4-O-lévulinyl-3-O-méthyl-α-D-glucopyranosyl)-(1→4)-O-(2-O-acétyl-6-O-benzyl-3-O-méthyl-β-D-glucopyranosyl)-(1→4)-[O-(2,6-di-O-benzyl-3-O-méthyl-α-D-glucopyranosyl)-(1→4)-O-(2-O-acétyl-6-O-benzyl-3-O-méthyl-β-D-glycopyranosyl)-(1→4)]₆-O-(6-O-benzyl-2,3-di-O-méthyl-α-D-glucopyranosyl)-(1→4)-O-(benzyl 2,3-di-O-méthyl-β-D-glucopyranosyluronate)-(1→4)-O-(3,6-di-O-acétyl-2-O-benzyl-α-D-glucopyranosyl)-(1→4)-(benzyl 2,3-di-O-méthyl-α-L-idopyranosyluronate)-(1→4)-2,3,6-tri-O-benzyl-α-D-glucopyranoside (31)

On traite un mélange de **17** (32,7 mg, 20,6 mmol) et de **30** (80,7 mg, 16,3 mmol) selon la MÉTHODE 1. On purifie sur colonne Toyopearl® HW-40 (dichlorométhane/éthanol 1 : 1) pour donner le 19-mer **31** (60 mg, 59 %). [α]_{D} + 61 (c = 0,82, dichlorométhane). ESIMS, mode positif : m/z + NaCl, 2162,4 (M+3Na)³⁺ ; + KF, 2178,5 (M+3K)³⁺.
¹H RMN (CDCl₃) δ des principaux protons anomériques : 5,55 ; 5,48 ; 5,30 ; 5,17 ; 4,56 ; 4,28 ; 4,22 ; 4,08.

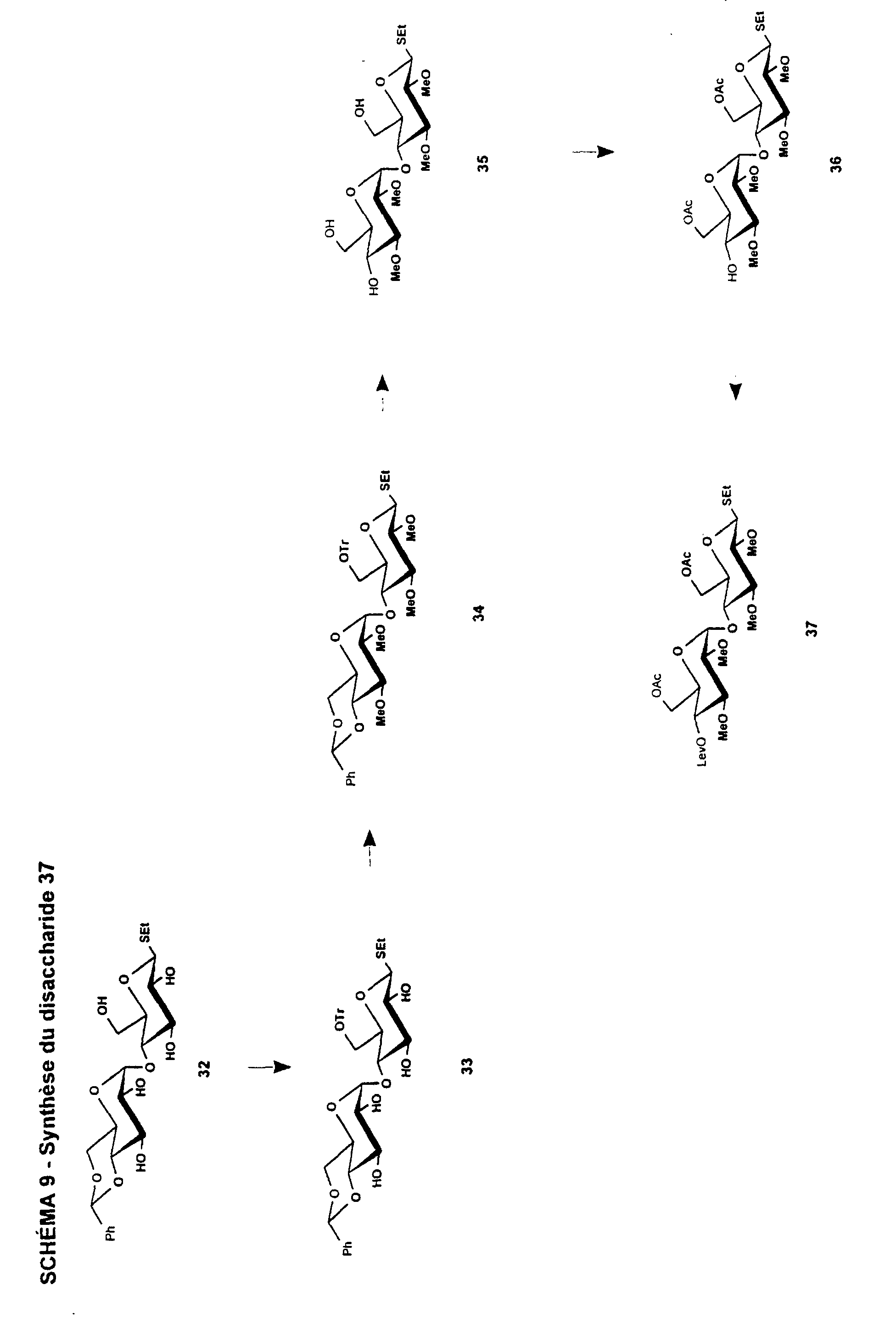

### PRÉPARATION 30

### Éthyl O-(4,6-O-benzylidène-α-D-glucopyranosyl)-(1→4)-6-O-trityl-1-thio-β-D-glucopyranoside (33)

À une suspension de **32** (50,0 g, 0,105 mol) (J. Westman et M. Nilsson, J. Carbohydr. Chem., 1995, *14(7)*, 949-960) dans du dichlorométhane (620 mL) sous argon, on ajoute de la triéthylamine (35 mL, 0,252 mol), du chlorure de trityle (29,3 g, 0,105 mol), et du 4-diméthylaminopyridine (1,28 g, 10 mmol). On porte le mélange à reflux pendant 2 heures (CCM), on le laisse refroidir à température ambiante, on le dilue avec du dichlorométhane (500 mL), puis on le lave successivement avec une solution aqueuse froide à 10 % d'hydrogénosulfate de potassium, de l'eau, et une solution saturée de chlorure de sodium. On sèche, on concentre, et on filtre sur colonne de silice (toluène/acétone 65 : 35 puis 50 : 50) pour obtenir le produit brut **33,** suffisamment pur pour être utilisé dans l'étape suivante. Un échantillon analytique est chromatographié. [α]_{D} + 53 (*c* = 0,74, dichlorométhane). ESIMS, mode négatif : m/z 715 (M-H)⁻. ¹H RMN (CD₂Cl₂) δ 7,52-7,25 (m, 20H, 4Ph), 5,42 (s, C₆H₅C*H*), 4,97 (d, J = 3,5 Hz, H-1'), 4,40 (d, J = 9,6 Hz, H-1), 3,82 (t, J = 9,3 Hz, H-3'), 3,70, 3,68 (m, 2H, H-3, H-4), 3,60 (dd, J ≈ 2,0, 11,0 Hz, H-6a), 3,55 (td, J = 5,2, 9,7, 9,7 Hz, H-5'), 3,49-3,45 (m, 3H, H-2, H-2', H-5), 3,38 (dd, J = 10,5 Hz, H-6'a), 3,33 (dd, H-6'b), 3,30-3,27 (m, 2H, H-4', H-6b), 2,90-2,77 (m, 2 H, SC*H*₂CH₃), 1,40-1,37 (t, 3H, CH₂C*H*₃).
Anal. calculée pour C₄₀H₄₄O₁₀S : C, 67,02 ; H, 6,19 ; S, 4,47. Trouvé : C, 66,83 ; H, 6,19; S, 4,19.

### PRÉPARATION 31

### Éthyl O-(4,6-O-benzylidène-2,3-di-O-méthyl-α-D-glucopyranosyl)-(1→4)-2,3-di-O-méthyl-6-O-trityl-1-thio-β-D-glucopyranoside (34)

On ajoute goutte à goutte sous argon de l'iodure de méthyle (34 mL, 0,536 mol) à une solution du composé **33** (64,1 g) dans du *N*,*N*-diméthylformamide (600 mL). On refroidit à 0° C, et on ajoute lentement de l'hydrure de sodium (13,5 g, 0,536 mol). On agite la suspension pendant 2 heures à température ambiante, puis on refroidit à 0° C, et on ajoute goutte à goutte du méthanol (35 mL) et après 2 heures d'agitation, on dilue le mélange dans de l'acétate d'éthyle (500 mL) et de l'eau (600 mL). On extrait la phase aqueuse avec de l'acétate d'éthyle, on lave les phases organiques avec de l'eau, on sèche, et on concentre. Le résidu **34** est suffisamment pur pour l'étape suivante. Un échantillon analytique est purifié sur colonne de silice (cyclohexane/acétone 70 : 30). [α]_{D} + 45 (c = 0,83, dichlorométhane). ESIMS, mode positif : m/z, + NaCl, 795 (M+Na)⁺ ; + KF, 811 (M+K)⁺. ¹H RMN (CDCl₃) δ 7,52-7,19 (m, 20H, 4Ph), 5,51 (d, J = 3,3 Hz, H-1'), 5,43 (s, C₆H₅C*H*), 4,45 (d, J = 9,8 Hz, H-1), 3,60, 3,59, 3,51, 3,49 (4s, 12H, 40CH₃), 2,86 (q, 2H, J = 7,5 Hz, SC*H*₂CH₃), 1,40 (t, 3H, SCH₂C*H*₃).
Anal. calculée pour C₄₄H₅₂O₁₀S : C, 68,37 ; H, 6,78 ; S, 4,15. Trouvé : C, 68,28 ; H, 6,98 ; S, 4,09.

### PRÉPARATION 32

### Éthyl O-(2,3-di-O-méthyl-α-D-glucopyranosyl)-(1→4)-2,3-di-O-méthyl-1-thio-β-D-glucopyranoside (35)

On chauffe une suspension de produit brut **34** (67,4 g) à 80° C pendant 2 heures dans une solution aqueuse à 60 % d'acide acétique (470 mL). On refroidit, on filtre et on concentre le mélange réactionnel. On traite le résidu avec du méthanolate de sodium (940 mg) dans du méthanol (200 mL) pendant 1 heure. On neutralise ensuite la solution avec de la résine Dowex® 50WX4 (H+), puis on filtre, on concentre, et on purifie sur colonne de silice (toluène/acétone 60 : 40) pour obtenir **35** (27,9 g, 60 % en trois étapes). [α]_{D} + 26 (c = 1,07, dichlorométhane). ESIMS, mode positif : m/z, + NaCl, 465 (M+Na)⁺ ; + KF, 481 (M+K)⁺. ¹H RMN (CDCl₃) δ 5,62 (d, J = 3,9 Hz, H-1'), 4,35 (d, J = 9,8 Hz, H-1), 3,64, 3,64, 3,59, 3,58 (4s, 12H, 40CH₃), 1,29 (t, 3H, J = 7,4 Hz, SCH₂C*H*₃).
Anal. calculée pour C₁₈H₃₄O₁₀S. H₂O : C, 46,94 ; H, 7,87 ; S, 6,96. Trouvé : C, 47,19 ; H, 7,72 ; S, 6,70.

### PRÉPARATION 33

### Éthyl O-(6-O-acétyl-2,3-di-O-méthyl-α-D-glucopyranosyl)-(1→4)-6-O-acétyl-2,3-di-O-méthyl-1-thio-β-D-glucopyranoside (36)

On porte à reflux une solution de triol **35** (5,86 g, 13,2 mmol) et de *N*-acétylimidazole (3,21 g, 29,1 mmol) dans du 1,2-dichloroéthane (120 mL) pendant 16 heures. On rajoute une portion de *N*-acétylimidazole (440 mg, 3,96 mmol) et on agite pendant 4 heures. On laisse revenir à température ambiante, puis on ajoute du méthanol (2 mL). On agite à nouveau 1 heure, puis on dilue le mélange avec du dichlorométhane (1 L), et on lave successivement avec une solution aqueuse 1M froide d'acide chlorhydrique, de l'eau froide, une solution aqueuse saturée d'hydrogénocarbonate de sodium, de l'eau, on sèche et on concentre. On chromatographie le résidu (toluène/acétone 3,5 : 1) pour obtenir le diacétate **36** (3,97 g, 57 %). [α]_{D} + 33 (*c* = 1,90, dichlorométhane). ESIMS, mode positif : m/z, + NaCl, 549 (M+Na)⁺ ; + KF, 565 (M+K)⁺. ¹H RMN (CDCl₃) δ 5,51 (d, J = 3,9 Hz, H-1'), 4,34 (d, J = 9,8 Hz, H-1), 3,64, 3,63, 3,59, 3,56 (4s, 12H, 4OCH₃), 2,11, 2,06 (2s, 6H, 2Ac), 1,31 (t, 3H, J = 7,4 Hz, SCH₂C*H*₃).
Anal. calculée pour C₂₂H₃₈O₁₂: C, 50,17 ; H, 7,27 ; S, 6,09. Trouvé : C, 50,15 ; H, 7,49 ; S, 5,89.

### PRÉPARATION 34

### Éthyl O-(6-O-acétyl-4-O-lévulinyl-2,3-di-O-méthyl-α-D-glucopyranosyl)-(1→4)-6-O-acétyl-2,3-di-O-méthyl-1-thio-β-D-glucopyranoside (37)

On ajoute à une solution de diacétate **36** (19,4 g, 36,8 mmol) dans du dioxane (400 mL), sous argon, de l'acide lévulinique (7,53 mL, 73,5 mmol), du chlorhydrate de 1-(3-diméthylaminopropyl)-3-éthylcarbodiimide (14,1 g, 73,5 mmol) et du 4-diméthylaminopyridine (900 mg, 7,35 mmol). On agite le mélange pendant 3,5 heures, on dilue avec du dichlorométhane (1,5 L), puis on lave successivement avec de l'eau, avec une solution aqueuse à 10 % d'hydrogénosulfate de potassium, à l'eau, avec une solution aqueuse à 2 % d'hydrogénocarbonate de sodium, à l'eau, puis on sèche et on concentre. On chromatographie le résidu (dichlorométhane/acétone 97 : 3 puis 79 : 21) pour obtenir le dérivé **37** (21,8 g, 95 %). [α]_{D} + 40 (*c* = 0,72, dichlorométhane). ESIMS, mode positif : m/z, + NaCl, 647 (M+Na)⁺ ; + KF, 663 (M+K)⁺. ¹H RMN (CDCl₃) δ 5,56 (d, J = 3,9 Hz, H-1), 4,35 (d, J = 9,8 Hz, H-1), 3,64, 3,60, 3,58, 3,55 (4s, 12H, 4OCH₃), 2,76-2,71 (m, 4H, O(C:O)C*H*₂C*H*₂(C:O)CH₃), 2,19, 2,08, 2,07 (3s, 9H, 2Ac et O(C:O)CH₂CH₂(C:O)C*H*₃), 1,31 (t, 3H, J = 7,4 Hz, SCH₂C*H*₃).
Anal. calculée pour C₂₇H₄₄O₁₄S : C, 51,91 ; H, 7,10 ; S, 5,13. Trouvé : C, 51,88 ; H, 7,05 ; S, 4,96.

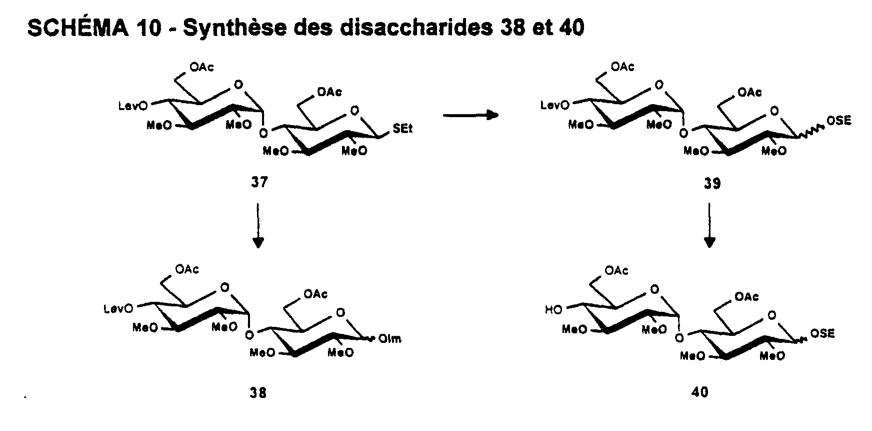

### PRÉPARATION 35

### O-(6-O-Acétyl-4-O-lévulinyl-2,3-di-O-méthyl-α-D-glucopyranosyl)-(1→4)-6-O-acétyl-2,3-di-O-méthyl-α,β-D-glucopyranose trichloroacétimidate (38)

On ajoute à une solution de thioglycoside **37** (9,53 g, 15,3 mmol) dans un mélange dichlorométhane/éther éthylique 1 : 1 (180 mL), de l'eau (1,4 mL, 76,3 mmol), du N-iodosuccinimide (6,84 g, 30,5 mmol) et du triflate d'argent (0,51 g, 1,98 mmol). Après 15 minutes (CCM), on ajoute une solution aqueuse saturée d'hydrogénocarbonate de sodium (5 mL) et on dilue le mélange réactionnel dans du dichlorométhane (1,5 L), on le lave avec de l'eau, une solution aqueuse 1M de thiosulfate de sodium, une solution aqueuse à 2 % d'hydrogénocarbonate de sodium. On sèche, on concentre, et on purifie le résidu sur colonne de silice (acétate d'éthyle/cyclohexane 80 : 40 puis 100: 0) pour donner un solide qui est utilisé sans caractérisation dans l'étape suivante. On traite une solution du composé précèdent (7,88 g, 13,6 mmol) dans du dichlorométhane (120 mL), sous argon avec du carbonate de césium (7,08 g 21,7 mmol) et du trichloroacétonitrile (6,81 mL, 67,9 mmol). Après 40 minutes (CCM), on filtre le mélange, on le concentre et on le purifie (toluène/acétone 85 : 15) pour obtenir l'imidate **38** (9,16 g, 83 % en deux étapes). [α]_{D} + 118 (c = 1,00, dichlorométhane). ESIMS, mode positif : m/z, + NaCl, 746 (M+Na)⁺ ; 741 (M+NH₄)⁺. ¹H RMN (CDCl₃) δ 8,66, 8,65 (2s, 1H, α et β N :H), 6,52 (d, J = 3,6 Hz, H-1α), 5,70 (d, J = 7,5 Hz, H-1β), 5,58 (d, J = 3,7 Hz, H-1 ), 2,78-2,57 (m, 4H, O(C:O)C*H*₂C*H*₂(C:O)CH₃), 2,18, 2,07, 2,06 (3s, 9H, 2Ac et O(C:O)CH₂CH₂(C:O)C*H*₃).
Anal. calculée pour C₂₇H₄₀Cl₃NO₁₅. 0,5 H₂O : C, 44,18 ; H, 5,63 ; N, 1,98. Trouvé : C, 44,14; H, 5,61 ; N, 1,97.

### PRÉPARATION 36

### 2-(Triméthylsilyl)éthyl O-(6-O-acétyl-4-O-lévulinyl-2,3-di-O-méthyl-α-D-glucopyranosyl)-(1→4)-6-O-acétyl-2,3-di-O-méthyl-α,β-D-glucopyranoside (39)

On traite le thioglycoside **37** (10,6 g, 16,94 mmol) selon la MÉTHODE 3 avec du 2-(triméthylsilyl)éthanol (4,8 mL, 33,90 mmol), dans un mélange dichlorométhane/éther éthylique 1 : 2 (105 mL). On purifie le résidu obtenu par chromatographie (acétone/dichlorométhane 1:1) pour obtenir le composé **39** (9,80 g, 85 %) sous forme d'un mélange d'anomères (α/β 65 :35). ESIMS, mode positif : m/z, + NaCI, 703 (M+Na)⁺. ¹H RMN (CDCl₃) δ 5,58 (d, J = 3,9 Hz, H-1'), 4,94 (d, J = 3,5 Hz, H-1α), 4,26 (d, J = 7,7 Hz, H-1β), 2,76-2,56 (m, 4H, O(C:O)C*H*₂C*H*₂(C:O)CH₃), 2,17, 2,08, 2,05 (3s, 9H, 2Ac et O(C:O)CH₂CH₂(C:O)C*H*₃), 1,18-0,88 (m, 2H, OCH₂C*H*₂Si(CH₃)₃), 0,02 (s, 9H, CH₂CH₂Si(C*H*₃)₃).
Anal. calculée pour C₃₀H₅₂O₁₅Si : C, 52,92 ; H, 7,69. Trouvé : C, 53,29 ; H, 7,75.

### PRÉPARATION 37

### 2-(Triméthylsilyl)éthyl O-(6-O-acétyl-2,3-di-O-méthyl-α-D-glucopyranosyl) -(1→4)-6-O-acétyl-2,3-di-O-méthyl-α,β-D-glucopyranoside (40)

On traite le composé **39** (9,41 g, 13,82 mmol) selon la MÉTHODE 2 avec de l'acétate d'hydrazine (10 mol/mol) dans un mélange toluène/éthanol 1 : 2 (21 mL/mmol). On chromatographie le résidu (acétone/toluène 60 : 40) pour obtenir **40**α (4,81 g, 60 %), ainsi qu'un mélange **40**α/β (3,06 g, 37 %). **40**α : [α]_{D} + 132 (*c* = 0,61, dichlorométhane). ESIMS, mode positif : m/z, + NaCl, 605 (M+Na)⁺ ; + KF, 621 (M+K)⁺. ¹H RMN(CDCl₃) δ 5,55 (d, J = 3,8 Hz, H-1'), 4,95 (d, J = 3,6 Hz, H-1), 2,10, 2,08 (2s, 6H, 2Ac), 1,16-0,89 (m, 2H, OCH₂C*H*₂Si(CH₃)₃), 0,02 (s, 9H, OCH₂CH₂Si(C*H*₃)₃).
Anal. calculée pour C₂₅H₄₆O₁₃Si : C, 51,53 ; H, 7,96. Trouvé : C, 51,37 ; H, 8,06.

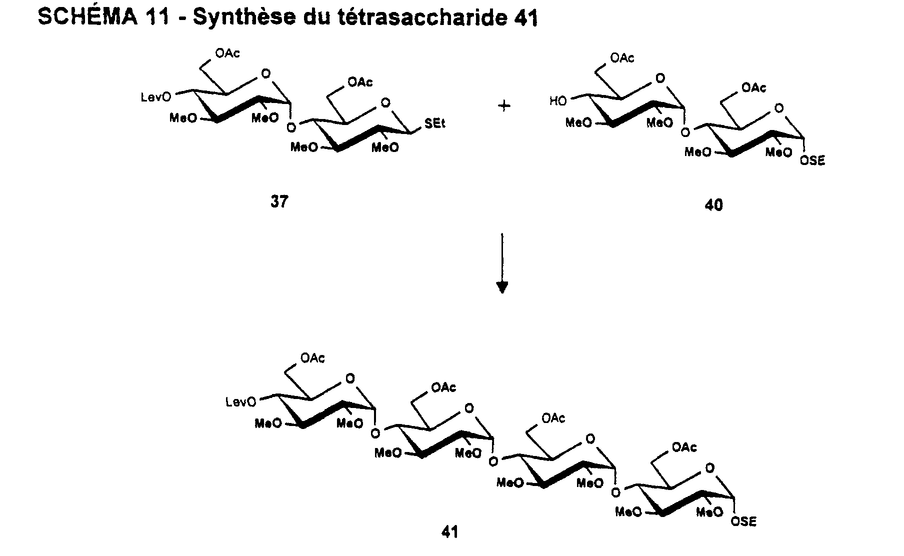

### PRÉPARATION 38

### 2-(Triméthylsilyl)éthyl O-(6-O-acétyl-4-O-lévulinyl-2,3-di-O-méihyl-α-D-glucopyranosyl)-(1→4)-[O-(6-O-acétyl-2,3-di-O-méthyl-α-D-glucopyranosyl)-(1→4)-]₂-6-O-acétyl-2,3-di-O-méfhyl-α-D-glucopyranoside (41)

On traite le thioglycoside **37** (4,21 g, 6,74 mmol) et l'accepteur glycosyle **40** (3,57 g, 6,13 mmol) selon la MÉTHODE 3 dans un mélange dichlorométhane/éther éthylique 1 : 2 (105 mL). On purifie le résidu par chromatographie sur silice (acétone/cyclohexane 3 : 1 puis 9 : 1) pour obtenir **41** (4,81 g, 69 %). [α]_{D} + 143 (*c* = 0,56, dichlorométhane ). ESIMS, mode positif : m/z, + NaCl, 1167 (M+Na)⁺ ; + KF, 1183 (M+K)⁺. ¹H RMN (CDCl₃) δ 5,57 (d, J = 3,9 Hz, H-1'''), 5,44, 5,41 (2d, J = 3,8 Hz, H-1'', H-1'), 4,96 (d, J = 3,6 Hz, H-1), 2,78-2,58 (m, 4H, O(C:O)C*H*₂C*H*₂(C:O)CH₃), 2,18, 2,12, 2,12, 2,09, 2,06 (5s, 15H, 4Ac et O(C:O)CH₂CH₂(C:O)C*H*₃), 1,21-0,97 (m, 2H, OCH₂C*H*₂Si(CH₃)₃), 0,03 (s, 9H, OCH₂CH₂Si(C*H*₃)₃).
Anal. calculée pour C₅₀H₈₄O₂₇Si: C, 52,44 ; H, 7,39. Trouvé : C, 52,29 ; H, 7,46.

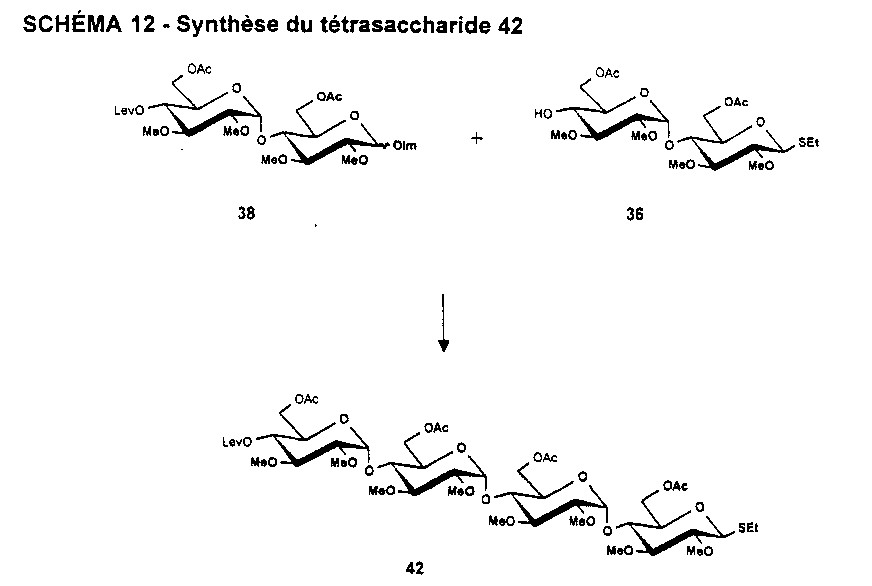

### PRÉPARATION 39

### Éthyl O-(6-O-acétyl-4-O-lévulinyl-2,3-di-O-méthyl-α-D-glucopyranosyl)-(1→4)-[O-(6-O-acétyl-2,3-di-O-méthyl-α-D-glucopyranosyl)-(1→4)-]₂-6-O-acétyl-2,3-di-O-méthyl-1-thio-β-D-glucopyranoside (42)

On traite une solution d'imidate **38** (1,10 g, 1,52 mmol) et d'accepteur de glycosyle **36** (806 mg, 1,38 mmol) dans un mélange dichlorométhane/éther éthylique 1 : 2 (22 mL) selon la MÉTHODE 1. On purifie par chromatographie sur silice (acétate d'éthyle/cyclohexane 2,5 : 1 puis 3:1) pour obtenir **42** (1,12 g, 71 %). [α]_{D} + 95 (*c* = 1,00, dichlorométhane). ESIMS, mode positif: m/z, + NaCI, 1111 (M+Na)⁺ ; + KF, 1127 (M+K)⁺. ¹H RMN(CDCl₃) δ 5,55 (d, J = 3,9 Hz, H-1'''), 5,39, 5,37 (2d, J = 3,8 et 3,9 Hz, H-1", H-1'), 4,34 (d, J = 9,7 Hz, H-1), 2,84-2,51 (m, 6H, SC*H*₂CH₃, O(C:O)C*H*₂C*H*₂(C:O)CH₃), 2,17, 2,10, 2,09, 2,08, 2,04 (5s, 15H, 4Ac et O(C:O)CH₂CH₂(C:O)C*H*₃), 1,30 (t, 3H, J = 7,4 Hz, SCH₂C*H*₃).
Anal. calculée pour C₄₇H₇₆O₂₆S : C, 51,83 ; H, 7,03 ; S, 2,94. Trouvé : C, 51,66 ; H, 7,02 ; S, 2,94.

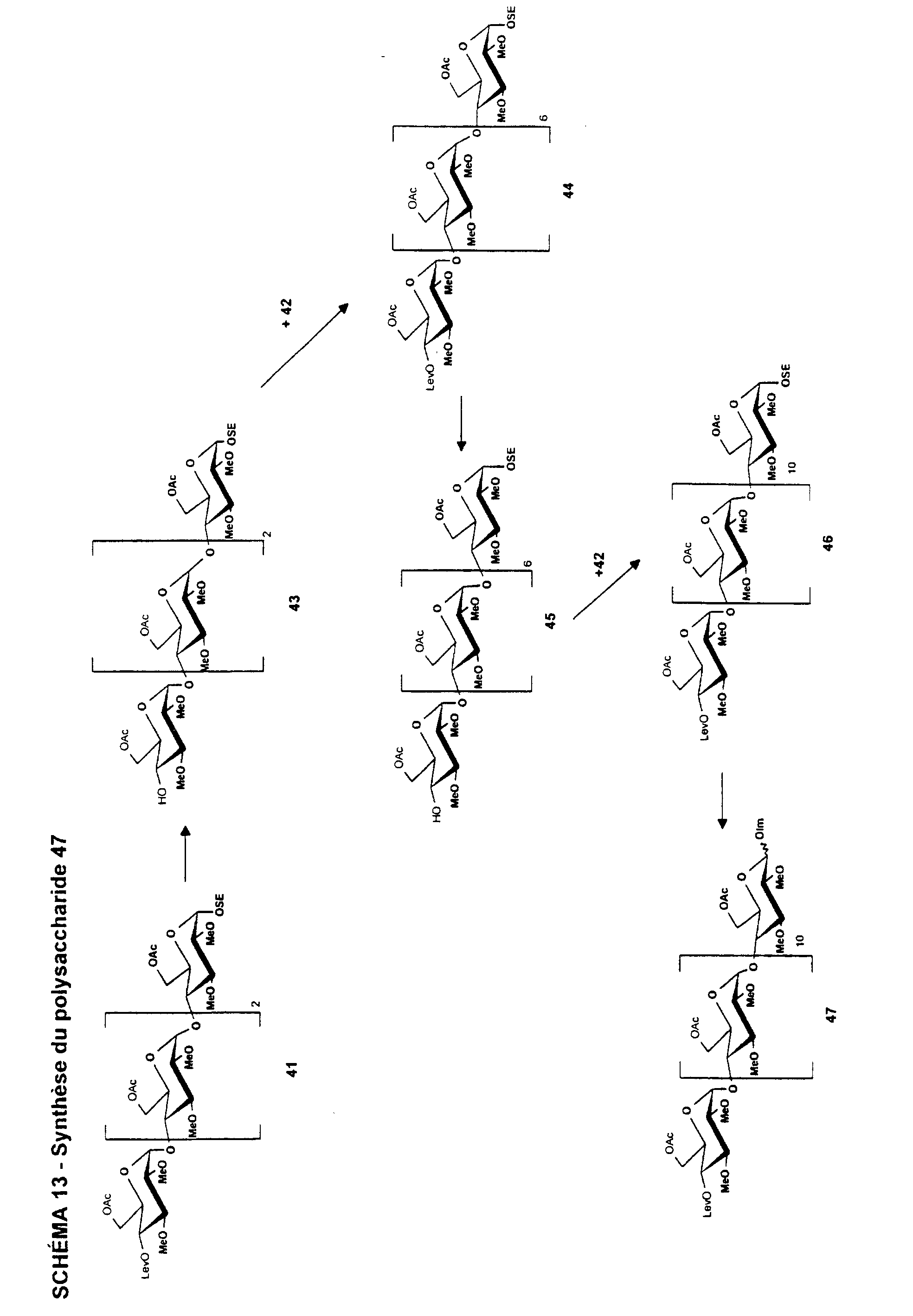

### PRÉPARATION 40

### 2-(Triméthylsilyl)éthyl [O-(6-O-acétyl-2,3-di-O-méthyl-α-D-glucopyranosyl)-(1→4)-]₃-6-O-acétyl-2,3-di-O-méthyl-α-D-glucopyranoside (43)

On fait réagir le composé **41** (4,71 g, 4,11 mmol) comme pour la PRÉPARATION 37 afin d'obtenir, après colonne de chromatographie (cyclohexane/acétone 3 : 2), le dérivé **43** (4,11 g, 95 %). [α]_{D} + 154 (*c* = 0,63, dichlorométhane). ESIMS, mode positif : m/z, + NaCl, 1069 (M+Na)⁺ ; + KF, 1085 (M+K)⁺. ¹H RMN(CDCl₃) δ 5,46, 5,46, 5,41 (2d, 3H, J = 3,9 Hz, H-1', H-1", H-1"'), 4,95 (d, J = 3,5 Hz, H-1), 2,81 (d, J = 4,4 Hz, OH), 2,11, 2,09, 2,08 (3s, 12H, 4Ac), 1,19-0,97 (m, 2H, OCH₂C*H*₂Si(CH₃)₃), 0,03 (s, 9H, OCH₂CH₂Si(C*H*₃)₃).
Anal. calculée pour C₄₅H₇₈O₂₅Si : C, 51,61 ; H, 7,51. Trouvé : C, 51,39 ; H, 7,54.

### PRÉPARATION 41

### 2-(Triméthylsilyl)éthyl O-(6-O-acétyl-4-O-lévulinyl-2,3-di-O-méthyl-α-D-glucopyranosyl)-(1→4)-[O-(6-O-acétyl-2,3-di-O-méthyl-α-D-glucopyranosyl)-(1→4)-]₆-6-O-acétyl-2,3-di-O-méthyl-α-D-glucopyranoside (44)

On traite le thioglycoside **42** (3,86 g, 3,54 mmol) et l'accepteur glycosyle **43** (3,60 g, 3,44 mmol) selon la PRÉPARATION 38. On chromatographie (dichlorométhane/acétone 7 : 2 puis 2:1), pour obtenir **44** (5,71 g, 80 %). [α]_{D} + 161 (*c* = 0,65, dichlorométhane). ESIMS, mode positif : masse monoisotopique = 2072,8, masse chimique = 2074,2, masse expérimentale = 2074 ± 1 u.m.a.. ¹H RMN (CDCl₃) δ 5,54 (d, J = 3,8 Hz, H-1 unité NR), 5,47-5,40 (m, 6H, H-1), 4,95 (d, J = 3,7 Hz, H-1 unité R), 2,84-2,51 (m, 4H, O(C:O)C*H*₂C*H*₂(C:O)CH₃), 2,17, 2,13, 2,12, 2,11, 2,11, 2,08, 2,05 (7s, 27H, 8Ac et O(C:O)CH₂CH₂(C:O)C*H*₃), 1,18-0,97 (m, 2H, OCH₂C*H*₂Si(CH₃)₃), 0,03 (s, 9H, OCH₂CH₂Si(C*H*₃)₃).
Anal. calculée pour C₉₀H₁₄₈O₅₁Si : C, 52,12 ; H, 7,19. Trouvé : C, 51,98 ; H, 7,25.

### PRÉPARATION 42

### 2-(Triméthylsilyl)éthyl [O-(6-O-acétyl-2,3-di-O-méthyl-α-D-glucopyranosyl)-(1→4)-]₇-6-O-acétyl-2,3-di-O-méthyl-α-D-glucopyranoside (45)

On ajoute, à 0° C, une solution 1 M d'hydrate d'hydrazine dans un mélange acide acétique/pyridine 3 : 2 (7,3 mL) à une solution du composé **44** (3,00 g, 1,45 mmol) dans la pyridine (5 mL). Après 20 minutes d'agitation, on évapore le mélange réactionnel, on dilue dans du dichlorométhane (400 mL), on lave avec une solution aqueuse à 10 % d'hydrogénosulfate de potassium, de l'eau, une solution aqueuse à 2 % d'hydrogénocarbonate de sodium, et de l'eau. On sèche, on concentre, et on chromatographie le résidu pour obtenir **45** (2,43 g, 85 %). [α]_{D} + 167 (c = 0,57, dichlorométhane). ESIMS, mode positif : masse monoisotopique = 1974,8, masse chimique = 1976,1, masse expérimentale = 1975,4 ± 2 u.m.a.. ¹H RMN (CDCl₃) δ 5,47-5,40 (m, 7H, H-1), 4,95 (d, J = 3,7 Hz, H-1 unité R), 2,80 (d, J = 4,4 Hz, OH), 2,13, 2,11, 2,10, 2,08, 2,07 (5s, 24H, 8Ac), 1,18-0,97 (m, 2H, OCH₂C*H*₂Si(CH₃)₃), 0,03 (s, 9H, OCH₂CH₂Si(C*H*₃)₃).
Anal. calculée pour C₈₅H₁₄₂O₄₉Si : C, 51,66 ; H, 7.24. Trouvé : C, 51,32 ; H, 7,26.

### PRÉPARATION 43

### 2-(Triméthylsilyl)éthyl O-(6-O-acétyl-4-O-lévulinyl-2,3-di-O-méthyl-α-D-glucopyranosyl)-(1→4)-[O-(6-O-acétyl-2,3-di-O-méthyl-α-D-glucopyranosyl)-(1→4)-]₁₀-6-O-acétyl-2,3-di-O-méthyl-α-D-glucopyranoside (46)

On traite le composé **42** (1,35 g, 1,24 mmol) et le composé **45** (2,38 g, 1,20 mmol) selon la PRÉPARATION 38. On chromatographie le résidu (cyclohexane/acétone 4 : 3) pour obtenir **46** (2,56 g, 71 %). [α]_{D} + 166 (*c* = 0,88, dichlorométhane). ESIMS, mode positif : masse monoisotopique = 3001,3, masse chimique = 3003,2, masse expérimentale = 3004 ± 1 u.m.a.. ¹H RMN (CDCl₃) δ 5,54 (d, J = 3,8 Hz, H-1 unité NR), 5,47-5,40 (m, 10H, H-1), 4,95 (d, J = 3,7 Hz, H-1 unité R), 2,81-2,51 (m, 4H, O(C:O)C*H*₂C*H*₂(C:O)CH₃), 2,17, 2,13, 2,12, 2,11, 2,11, 2,08, 2,05 (7s, 39H, 12Ac et O(C:O)CH₂CH₂(C:O)C*H*₃), 1,17-0,96 (m, 2H, OCH₂C*H*₂Si(CH₃)₃), 0,03 (s, 9 H, OCH₂CH₂Si(C*H*₃)₃).
Anal. calculée pour C₁₃₀H₂₁₂O₇₅Si : C, 51,99 ; H, 7,12. Trouvé : C, 51,63 ; H, 7,12.

### PRÉPARATION 44

### (6-O-Acétyl-4-O-lévulinyl-2,3-di-O-méthyl-α-D-glucopyranosyl)-(1→4)-[O-(6-O-acétyl-2,3-di-O-méthyl-α-D-glucopyranosyl)-(1→4)-]₁₀-6-O-acétyl-2,3-di-O-méthyl-α,β-D-glucopyranose trichloroacétimidate (47)

(a) On agite pendant 1,5 heure (CCM) une solution de glycoside **46** (400 mg, 0,133 mmol) dans un mélange d'acide trifluoroacétique/dichlorométhane 2 : 1 (2 mL). On dilue dans un mélange toluène/acétate de *n*-propyle 2 : 1 (12 mL), on concentre et on co-évapore avec du toluène (5 x 10 mL). On purifie le résidu (acétone/cyclohexane 4 : 3) pour obtenir un solide (364 mg).
(b) On dissout le solide précédemment obtenu dans du dichlorométhane (2,5 mL). On ajoute du carbonate de césium (65 mg, 0,200 mmol) et du trichloroacétonitrile (63 mL, 0,620 mmol), on agite le mélange pendant 2,5 heures, puis on filtre *(Célite),* on concentre et on purifie sur colonne de silice (cyclohexane/acétone/triéthylamine 50 : 50 : 0,1) pour obtenir l'imidate **47** (348 mg, 86 %). [α]_{D} + 185 (c = 0,91, dichlorométhane). ESIMS, mode positif : masse monoisotopique = 3044,1, masse chimique = 3047,3, masse expérimentale = 3046,9 ± 0,2 u.m.a.. ¹H RMN (CD₂Cl₂) δ 8,61, 8,58 (2s, 1H, α et β N :H), 6,35 (d, J = 3,7 Hz, H-1α unité R), 5,59 (d, J = 7,5 Hz, H-1β unité R), 5,38 (d, J = 3,8 Hz, H-1 unité NR), 5,32-5,25 (m, 10H, H-1), 2,64-2,40 (m, 4H, O(C:O)C*H*₂C*H*₂(C:O)CH₃), 2,02, 1,96, 1,95, 1,94, 1,93, 1,89 (6s, 39H, 12Ac et O(C:O)CH₂CH₂(C:O)C*H*₃).
   Anal. calculée pour C₁₂₇H₂₀₀Cl₃NO₂₅: C, 50,06 ; H, 6,61 ; N, 0,46. Trouvé : C, 49,93 ; H, 6,52 ; N, 0,42.

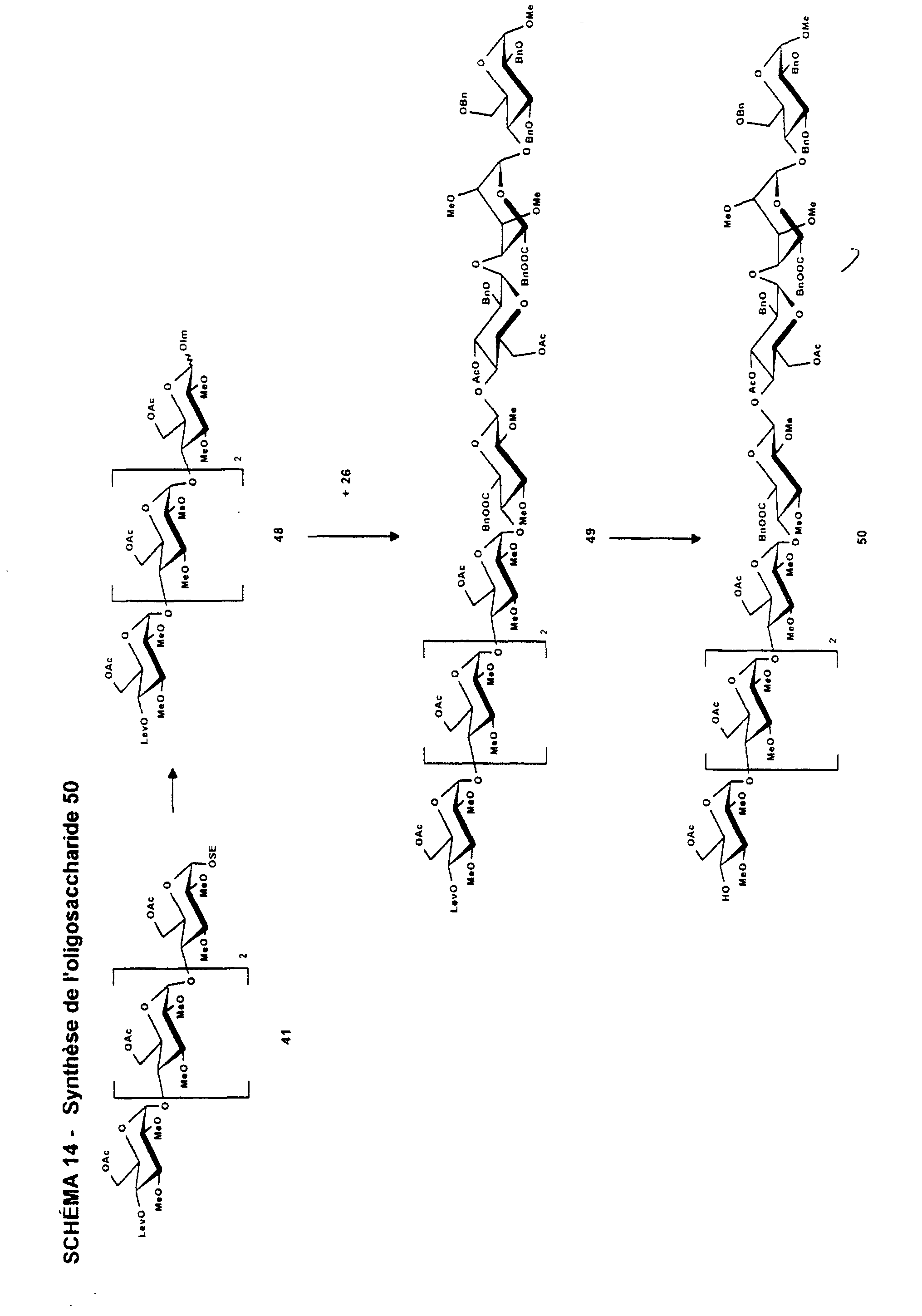

### PRÉPARATION 45

### (6-O-Acétyl-4-O-lévulinyl-2,3-di-O-méthyl-α-D-glucopyranosyl)-(1→4)-[O-(6-O-acétyl-2,3-di-O-méthyl-α-D-glucopyranosyl)-(1→4)-]₂-6-O-acétyl-2,3-di-O-méthyl-α,β-D-glucopyranose trichloroacétimidate (48)

On traite le composé **41** (200 mg, 0,174 mmol) selon la PRÉPARATION 44. On purifie le mélange réactionnel sur colonne de silice (toluène/acétone 3 : 2) pour obtenir l'imidate **48** (230 mg, 77 %). ESIMS, mode positif : m/z, + NaCl, 1210 (M+Na)⁺ ; + KF. 1226 (M+K)⁺. ¹H RMN (CDCl₃) δ 8,66-8,64 (2s, 1 H, α et β N :H), 6,51 (d, J = 3,6 Hz, H-1α), 5,71 (d, J = 7,5 Hz, H-1β), 2,90-2,52 (m, 4H, O(C:O)C*H*₂C*H*₂(C:O)CH₃), 2,17, 2,11, 2,11, 2,09, 2,05 (5s, 4Ac et O(C:O)CH₂CH₂(C:O)C*H*₃).

### PRÉPARATION 46

### Méthyl O-(6-O-acétyl-4-O-lévulinyl-2,3-di-O-méthyl-α-D-glucopyranosyl)-(1→4)-[O-(6-O-acétyl-2,3-di-O-méthyl-α-D-glucopyranosyl)-(1→4)-]₃-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,3,6-tri-O-benzyl-α-D-glucopyranoside (49)

On traite l'imidate **48** (73 mg, 0,061 mmol) et l'accepteur de glycosyle **26** (82 mg, 0,059 mmol) selon la PRÉPARATION 39. On purifie le composé avec une colonne de chromatographie Séphadex® LH-20 (dichlorométhane/éthanol 1:1), puis sur une colonne de silice (toluène/acétone 3:1) pour donner le dérivé **49** (98 mg, 69 %).
[α]_{D} + 95 *(c* = 1,01, dichlorométhane). ESIMS, mode positif : masse monoisotopique = 2414,97, masse chimique = 2416,54, masse expérimentale = 2416,2. ¹H RMN (CDCl₃) δ 7,43-7,20 (m, 30H, 6Ph), 5,55 (d, J = 3,9 Hz, H-1 unité NR), 5,50 (d, J = 3,9 Hz, H-1 unité NR-3), 5,44, 5,38 (2d, J = 3,7 et 3,9 Hz, H-1 unité NR-1, unité NR-2), 5,29 (d, J = 6,8 Hz, H-1 unité R-1), 5,17 (d, J = 3,5 Hz, H-1 unité R-2), 4,56 (d, J = 3,7 Hz, H-1 unité R), 4,10 (d, J = 7,9 Hz, H-1 unité R-3), 2,81-2,50 (m, 4H, O(C:O)C*H*₂C*H*₂(C:O)CH₃), 2,17, 2,15, 2,11, 2,09, 2,05, 2,00 (7s, 21H, 6Ac et O(C:O)CH₂CH₂(C:O)C*H*₃).
Anal. calculée pour C₁₂₀H₁₅₈O_{51:} C, 59,63 ; H, 6,59. Trouvé : C, 59,23 ; H, 6,58.

### PRÉPARATION 47

### Méthyl [O-(6-O-acétyl-2,3-di-O-méthyl-α-D-glucopyranosyl)-(1→4)-]₄-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,3,6-tri-O-benzyl-α-D-glucopyranoside (50)

On fait réagir l'octasaccharide **49** (120 mg, 0,050 mmol) comme pour la PRÉPARATION 37. On purifie le résidu sur colonne de silice (toluène/acétone 3:1) pour obtenir le composé **50** (95 mg, 83 %). [α]_{D} + 80 (*c* = 0,62, dichlorométhane). ESIMS, mode positif : masse monoisotopique = 2316,9, masse chimique = 2318,4, masse expérimentale = 2318,2 ± 0,4 u.m.a.. ¹H RMN (CDCl₃) δ 7,42-7,12 (m, 30H, 6Ph), 5,50, 5,46, 5,43, 5,40 (4d, J = 3,9, 3,9, 3,7, 3,7 Hz, H-1 unité NR, unité NR-1, unité NR-2, unité NR-3), 2,14, 2,10, 2,09, 2,08, 2,00, 1,88 (6s, 18H, 6Ac).
Anal. calculée pour C₁₁₅H₁₅₂O_{49:} C, 59,57 ; H, 6,60. Trouvé : C, 59,49 ; H, 6,61.

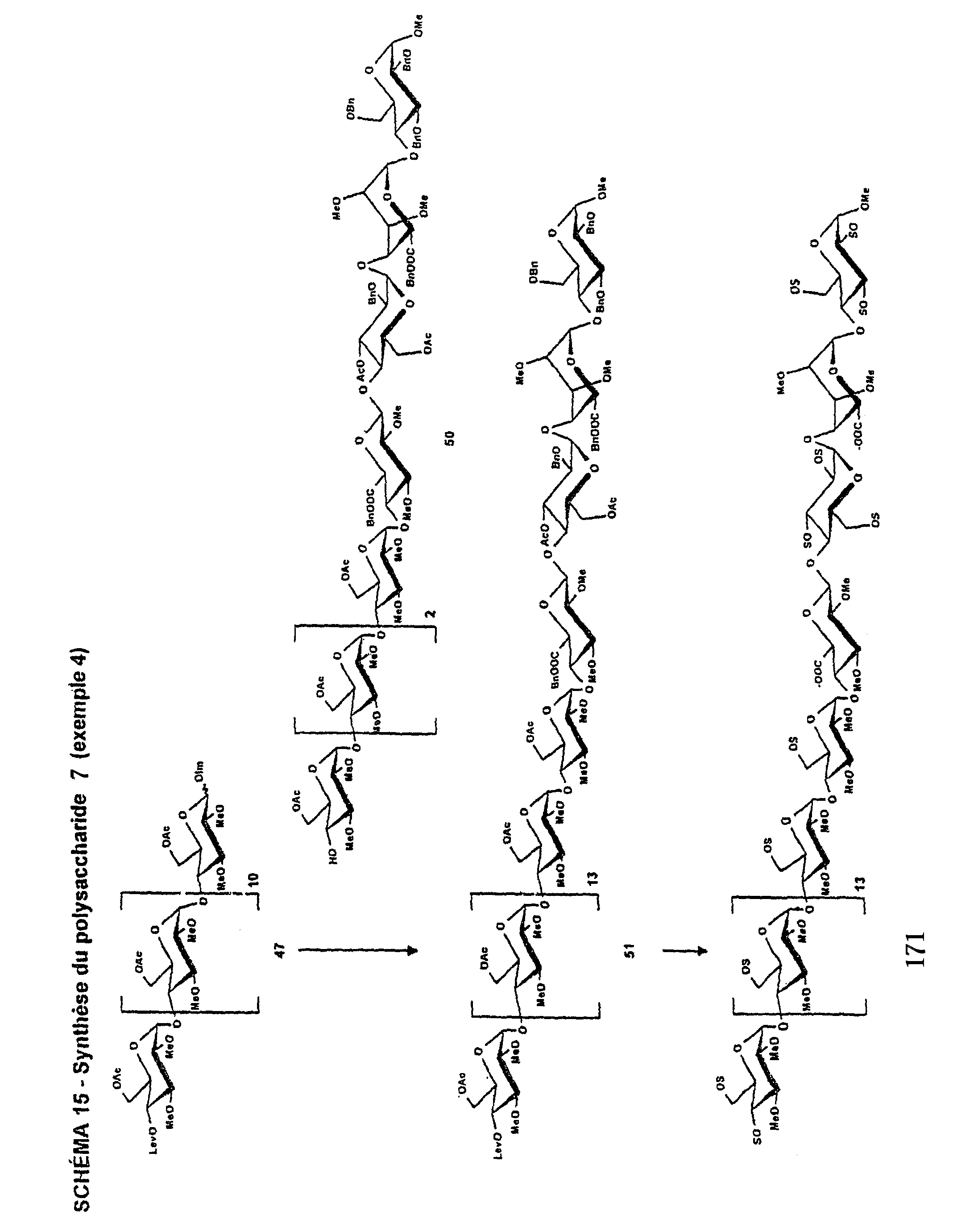

### PRÉPARATION 48

### Méthyl O-(6-O-acétyl-4-O-lévulinyl-2,3-di-O-méthyl-α-D-glucopyranosyl)-(1→4)-[O-(6-O-acétyl-2,3-di-O-méthyl-α-D-glucopyranosyl)-(1→4)-]₁₅-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,3,6-tri-O-benzyl-α-D-glucopyranoside (51)

On traite l'imidate **47** (84 mg, 0,027 mmol) avec l'accepteur de glycosyle **50** (62 mg, 0,027 mmol) selon la PRÉPARATION 39. On purifie d'abord le résidu sur une colonne Toyopearl® HW-40 puis sur une colonne de silice (cyclohexane/acétone 1 : 1) afin d'obtenir le dérivé **51** (71 mg, 51 % non optimisé). [α]_{D} + 136 (c = 0,95, dichlorométhane). ESIMS, mode positif : masse monoisotopique = 5200,11, masse chimique = 5203,39, masse expérimentale = 5203,5. ¹H RMN (CDCl₃) δ 7,42-7,18 (m, 30H, 6Ph), 5,54 (d, J = 3,8 Hz, H-1 unité NR), 5,51-5,40 (m, 15 H-1), 5,30 (d, J = 6,8 Hz, H-1 unité R-1), 5,17 (d, J = 3,5 Hz, H-1 unité R-2), 4,56 (d, J = 3,7 Hz, H-1 unité R), 4,09 (d, J = 7,9 Hz, H-1 unité R-3), 2,85-2,53 (m, 4H, O(C:O)C*H*₂C*H*₂(C:O)CH₃), 2,17, 2,16, 2,13, 2,11, 2,08, 2,05, 2,00, 1,88 (8s, 57H, 18Ac et O(C:O)CH₂CH₂(C:O)C*H*₃).
Anal. calculée pour C₂₄₀H₃₅₀O₁₂₃.4H₂O : C, 54,64 ; H, 6,84. Trouvé : C, 54,51 ; H, 6,79.

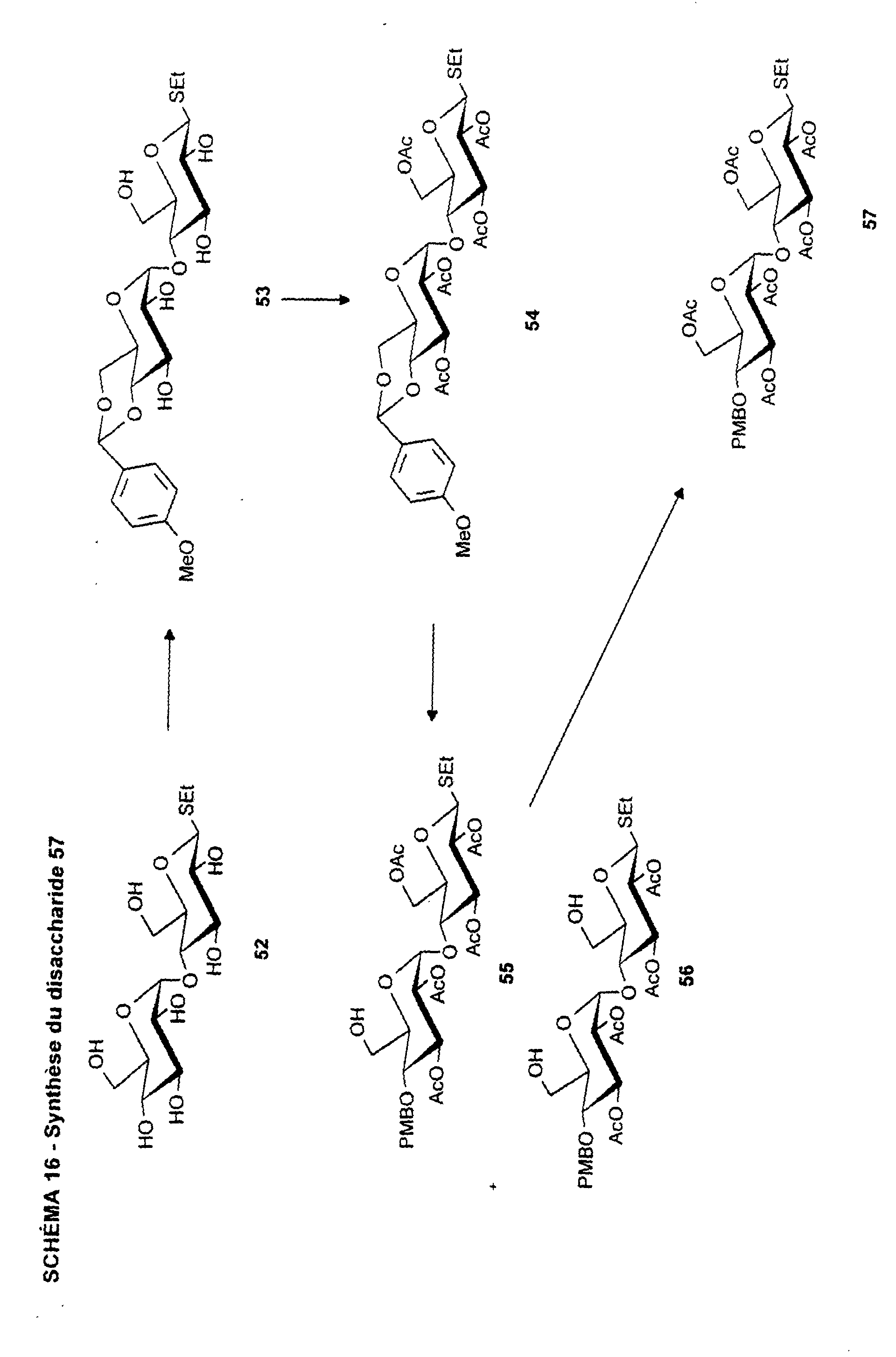

### PRÉPARATION 49

### Éthyl O-(4,6-O-p-méthoxybenzylidène-α-D-glucopyranosyl)-(1→4)-1-thio-β-D-glucopyranoside (53)

On ajoute, à + 5° C, sous argon, de l'anisaldéhydediméthylacetal (35,8 mL, 0,21 mol) et de l'acide camphorsulfonique (4,44 g, 19,1 mmol) à une solution du composé **52** (73,89 g, 0,19 mol) (W.E. Dick Jr et J.E. Hodge, Methods in Carbohydrate Chemistry, 7, 1976, 15-18) dans un mélange acétonitrile/*N*,*N*-diméthylformamide 3,5 : 1 (990 mL). Après 1,5 heure d'agitation à température ambiante, on neutralise par addition de triéthylamine (2,96 mL, 21,0 eq). On concentre et on purifie le sirop par colonne sur silice (dichlorométhane/méthanol 100 : 0 puis 50 : 50) pour obtenir **53** (58,6 g, 61 % non optimisé). [α]_{D} + 47 (*c* = 1,03, méthanol). ESIMS, mode négatif : m/z, 503 (M-H)⁻. ¹H RMN (CD₃OD) δ 7,41, 6,89 (2d, 4H, CH₃OC₆*H*₄), 5,51 (s, C*H*C₆H₄), 5,20 (d, J = 3,6 Hz, H-1'), 4,39 (d, J = 7,1 Hz, H-1), 3,78 (s, 3H, C*H*₃OC₆H₄), 2,75 (q, 2H, J = 7,0 Hz, SC*H*₂CH₃), 1,29 (t, 3H, SCH₂C*H*₃).
Anal. calculée pour C₂₂H₃₂O₁₁S : C, 52,37 ; H, 6,39 ; S, 6,35. Trouvé : C, 52,15 ; H, 6,61 ; S, 5,84.

### PRÉPARATION 50

### Éthyl O-(2,3-di-O-acétyl-4,6-p-méthoxybenzylidène-α-D-glucopyranosyl)-(1→4)-2,3,6-tri-O-acétyl-1-thio-β-D-glucopyranoside (54)

On ajoute goutte à goutte et à 0° C de la triéthylamine (65 mL, 0,47 mol) et de l'anhydride acétique (89 mL, 0,94 mol) à une suspension de **53** (47,22 g, 93,6 mmol) dans du dichlorométhane (450 mL). On additionne ensuite de la 4-diméthylaminopyridine (5,71 g, 46,8 mmol) et on laisse agiter 1,5 heure à température ambiante. On arrête la réaction par addition de méthanol (45 mL, 1,12 mol) puis on lave successivement avec une solution aqueuse froide à 10 % d'hydrogénosulfate de potassium, de l'eau, une solution saturée d'hydrogénocarbonate de sodium, et de l'eau. On sèche, on concentre, et on cristallise (cyclohexane/acétate d'éthyle) pour obtenir **54** (64,9 g, 97 %). [α]_{D} + 21 (*c* = 1,00, dichlorométhane ). pf 213-215 C. ESIMS, mode positif : m/z, + NaCl, 737 (M+Na)⁺, + KF, 753 (M+K)⁺. ¹H RMN (CDCl₃) δ 7,34, 6,86 (2d, 4H, CH₃OC₆*H*₄), 5,43 (s, C*H*C₆H₄), 5,34 (d, J =4,1 Hz, H-1'), 4,54 (d, J = 9,9 Hz, H-1), 3,78 (s, 3H, C*H*₃OC₆H₄), 2,74-2,60 (m, 2H, SC*H*₂CH₃), 2,10, 2,06, 2,03, 2,01 (4s, 15H, 5Ac), 1,26 (t, 3H, SCH₂C*H*₃).
Anal. calculée pour C₃₂H₄₂O₁₆S : C, 53,78 ; H, 5,92 ; S, 4,49. Trouvé : C, 53,74 ; H, 6,08 ; S, 4,40.

### PRÉPARATION 51

### Éthyl O-(2, 3-di-O-acétyl-4-p-méthoxybenzyl-α-D-glucopyranosyl)-(1→4)-2,3,6-tri-O-acétyl-1-thio-β-D-glucopyranoside (55), et éthyl O-(2,3-di-O-acétyl-4-p-méthoxybenzyl-α-D-glucopyranosyl)-(1→4)-2,3-di-O-acétyl-1-thio-β-D-glucopyranoside (56)

On agite une suspension de **54** (25,0 g, 35,0 mmol), de complexe borane-triméthylamine (20,4 g, 0,28 mol) et de tamis moléculaire (33 g, 4Å) pendant 1 heure sous argon dans du toluène (810 mL). On refroidit à 0° C et on ajoute lentement du chlorure d'aluminium (14,0 g, 0,11 mol). On agite pendant 25 minutes (CCM), on verse le milieu réactionnel dans une solution aqueuse froide à 20 % d'hydrogénosulfate de potassium et on agite 1 heure à 0° C, puis on filtre *(Célite).* On lave la phase organique à l'eau, avec une solution aqueuse à 2 % d'nydrogénocarbonate de sodium, à l'eau, on sèche et on concentre. On purifie le résidu sur colonne de silice pour obtenir **55** (7,37 g, 29 % non optimisé), et **56** (1,36 g, 6 %). Un échantillon analytique de **55** est cristallisé dans un mélange cyclohexane/acétate d'éthyle. [α]_{D} + 30 (*c* = 1,00, dichlorométhane). pf 151-153 C. ESIMS, mode positif : m/z, + NaCl, 739 (M+Na)⁺, + KF, 755 (M+K)⁺. ¹H RMN (CDCl₃) δ 7,19, 6,87 (2d, 4H, CH₃OC₆*H*₄), 5,36 (d, J = 3,5 Hz, H-1'), 4,54 (s, 2H, C₆H₄C*H*₂), 4,53 (d, J = 9,0 Hz, H-1), 2,70-2,65 (m, 2H, SCH₂C*H*₃).
Anal. calculée pour C₃₂H₄₂O₁₆S : C, 53,62 ; H, 6,19 ; S, 4,47. Trouvé : C, 53,57 ; H, 6,21 ; S, 4 43.

Composé **56** : [α]_{D} + 19 (c = 1,11, dichlorométhane). ESIMS, mode positif : m/z, + NaCl, 697 (M+Na)⁺, + KF, 713 (M+K)⁺. ¹H RMN (CDCl₃) δ 7,17, 6,84 (2d, 4H, CH₃OC₆*H*₄), 5,36 (d, J = 4,5 Hz, H-1'), 4,54 (d, J = 10,1 Hz, H-1), 4,49 (s, 2H, C₆H₄C*H*₂), 2,69-2,64 (m, 2H, SC*H*₂CH₃), 2,02, 2,01, 2,00, 1,96 (4s, 12H, 4Ac), 1,25 (t, 3H, SCH₂C*H*₃).
Anal. calculée pour C₃₀H₄₂O₁₅S : C, 53,40 ; H, 6,27 ; S, 4,75. Trouvé : C, 53,29 ; H, 6,39 ; S, 4,53.

### PRÉPARATION 52

### Éthyl O-(2,3-tri-O-acétyl-4-p-méthoxybenzyl-α-D-glucopyranosyl)-(1→4)-2,3,6-tri-O-acétyl-1-thio-β-D-glucopyranoside (57)

On ajoute, à 0° C, de l'anhydride acétique (1,47 mL, 15,5 mmol) à un mélange de **55** (5,6 g, 7,77 mmol), de triéthylamine (1,19 mL, 8,54 mmol), et de 4-diméthylaminopyridine (190 mg, 1,55 mmol) dans du dichlorométhane (40 mL). Après 40 minutes d'agitation (CCM) à température ambiante, on dilue avec du dichlorométhane (50 mL) et on lave avec une solution aqueuse froide à 10 % d'hydrogénosulfate de potassium, de l'eau, une solution saturée d'hydrogénocarbonate de sodium, et de l'eau. On sèche, on concentre, et on purifie sur colonne de silice (acétate d'éthyle/cyclohexane 35 : 65) pour obtenir **57** (5,66 g, 96 %). [α]_{D} + 44 (*c* = 1,03, dichlorométhane). ESIMS, mode positif : m/z, + NaCI, 781 (M+Na)⁺, + KF, 797 (M+K)⁺. ¹H RMN (CDCl₃) δ 7,15, 6,85 (2d, 4H, CH₃OC₆*H*₄), 5,30 (d, J = 4,2 Hz, H-1'), 4,53 (d, J = 10,0 Hz, H-1), 2,69-2,64 (m, 2H, SC*H*₂CH₃), 2,09, 2,07, 2,04, 2,01, 2,00, 1,98 (6s, 18H, 6Ac), 1,25 (t, 3H, SCH₂C*H*₃).
Anal. calculée pour C₃₄H₄₆O₁₇S : C, 53,82 ; H, 6,11; S, 4,22. Trouvé : C, 53,77 ; H, 6,24 ; S, 4,09.

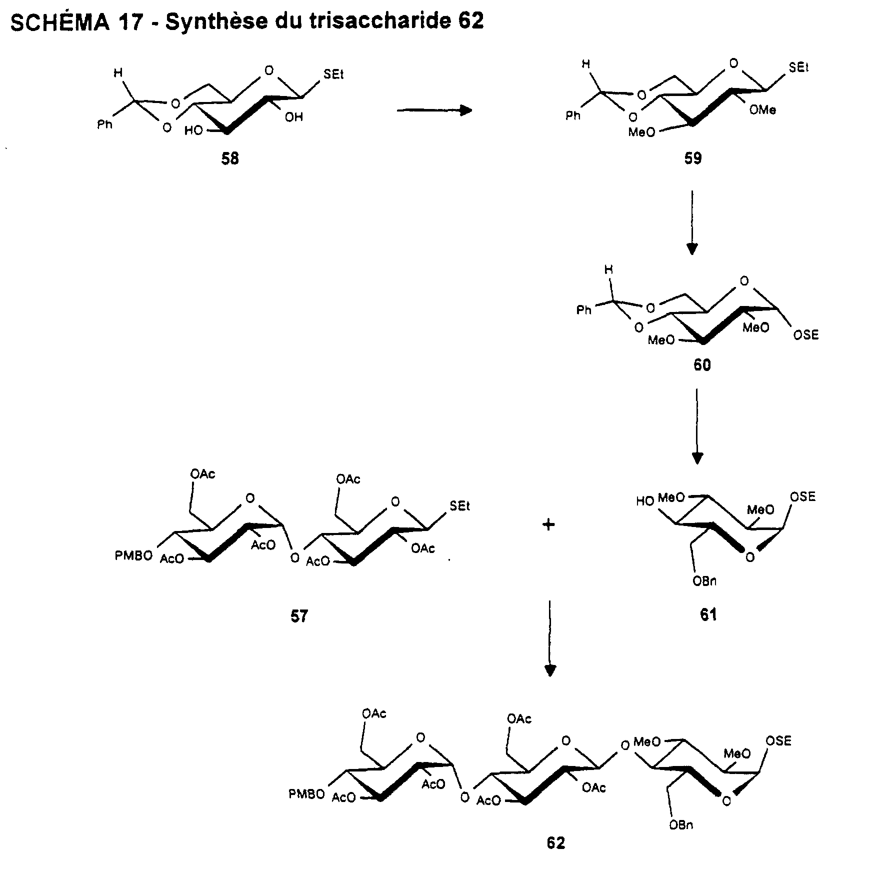

### PRÉPARATION 53

### Éthyl 4,6-O-benzylidène-2,3-di-O-méthyl-1-thio-β-D-glucopyranoside (59)

On ajoute, à 0° C, de l'hydrure de sodium (500 mg, 19,9 mmol) au mélange du composé **58** (2,59 g, 8,29 mmol), (A.F. Bochkov et *al*, lzv. Akad. Nauk SSSR, Ser. Khim. (1968), (1), 179) et d'iodure de méthyle (1,70 mL, 19,9 mmol) dans du *N,N*-diméthylformamide (25 mL), et on laisse revenir la température à 20° C. On laisse sous agitation pendant 30 minutes (CCM), puis on additionne du méthanol. On verse le mélange dans de l'eau et on extrait avec de l'acétate d'éthyle. On lave successivement avec une solution aqueuse 1M de thiosulfate de sodium, de l'eau, on sèche et on concentre. On triture le résidu dans l'éther éthylique pour obtenir **59** (0,42 g, 15 %) ; la purification sur colonne de silice (toluènelacétone 12 : 1) des eaux-mères suivi d'une cristallisation permet d'obtenir une fraction supplémentaire de **59** (1,6 g, rendement global : 71 %). pf : 108 C. [α]_{D} - 78 (*c* = 1,00, dichlorométhane). LSIMS, mode positif : m/z thioglycerol + NaCl, 363 (M+Na)⁺ ; thioglycerol + KF, 379 (M+K)⁺. ¹H RMN (CDCl₃) δ 7,51-7,33 (m, 5H, Ph), 5,52 (s, 1H, C₆H₅C*H*), 4,45 (d, 1H, J = 9,8 Hz, H-1), 3,64 (s, 3H, OCH₃), 3,62 (s, 3H, OCH₃), 2,78-2,68 (m, 2H, SC*H*₂CH₃), 1,31 (t, 3H, J = 2,7 Hz, SCH₂C*H*₃).
Anal. calculée pour C₁₇H₂₄O₅S (340,44) : C, 59,98 ; H, 7,11 ; S, 9,42. Trouvé : C, 59,91 ; H, 7,15; S, 8,96.

### PRÉPARATION 54

### 2-(Triméthylsilyl)éthyl 4,6-O-benzylidène-2,3-di-O-méthyl-α-D-glucopyranoside (60)

On dissout le composé **59** (23,0 g, 67,5 mmol) et le 2-(triméthylsilyl)éthanol (19,4 mL, 135 mmol) dans un mélange d'éther éthylique et de dichlorométhane 2 : 1 (345 mL) et on ajoute du tamis moléculaire 4 Å (11 g). On laisse agiter 1 heure à 25° C, on ajoute du *N*-iodosuccinimide (49,7 g, 220 mmol), puis, à 0° C, du triflate d'argent (2,20 g, 8,78 mmol). On laisse agiter pendant 20 minutes (CCM) puis on ajoute de l'hydrogénocarbonate de sodium solide. On dilue avec du dichlorométhane, on filtre sur *Célite,* on lave successivement avec une solution aqueuse 1M de thiosulfate de sodium, de l'eau, on sèche et on évapore à sec. On purifie sur une colonne de silice (cyclohexane/acétate d'éthyle 15 : 1 puis 5 : 1 ) pour obtenir **60** β (4,20 g, 15 %) et **60** α (8,40 g, 31 %).
composé **60α.** [α]_{D} + 96 (*c* = 0,4, dichlorométhane). ESIMS, mode positif : m/z, 419 (M+Na)⁺ ; 435 (M+K)⁺. ¹H RMN (CDCl₃) δ 7,52-7,35 (m, 5H, Ph), 5,54 (s, 1H, C₆H₅C*H*), 4,98 (d, 1H, J = 3,7 Hz, H-1), 3,64 (s, 3H, OCH₃), 3,62 (s, 3H, OCH₃), 1,24-0,96 (m, 2H, OCH₂C*H*₂Si(CH₃)₃), 0,00 (s, 9H, OCH₂CH₂Si(C*H*₃)₃).
Anal. calculée pour C₂₀H₃₂O₆S (396,56) : C, 60,58 ; H, 8,13. Trouvé : C, 60,26 ; H, 8,39.

### PRÉPARATION 55

### 2-(Triméthylsilyl)éthyl 6-O-benzyl-2,3-di-O-méthyl-α-D-glucopyranoside (61)

On dissout le composé **60** (21,1 g, 53,3 mmol) dans le dichlorométhane (154 mL). À température ambiante, on ajoute du triéthylsilane (34 mL, 213 mmol), puis on additionne goutte à goutte le mélange acide trifluoroacétique (16,3 mL, 213 mmol) et anhydride trifluoroacétique (0,49 mL, 3,47 mmol). On laisse agiter 2 heures et on ajoute une solution aqueuse 1M d'hydroxyde de sodium jusqu'à pH basique. Après décantation, on extrait la phase aqueuse à l'acétate d'éthyle puis réunir les phases organiques, on sèche et on concentre. On purifie le résidu sur colonne de silice (dichlorométhane/acétone 14 : 1 puis 12 : 1) pour obtenir **61** (12,5 g, 59 %). [α]_{D} + 100 (*c* = 1,45, dichlorométhane). ¹H RMN δ 7,40-7,20 (m, 5H, Ph), 4,98 (d, 1H, J = 3,5 Hz, H-1), 3,62 (s, 3H, OCH₃), 3,49 (s, 3H, OCH₃), 1,14-0,91 (m, 2H, CH₂C*H*₂Si(CH₃)₃), 0,00 (s, 9H, CH₂CH₂Si(C*H*₃)₃).
Anal. calculée pour C₂₀H₃₀O₆Si (398,58) : C, 60,27 ; H, 8,60. Trouvé : C, 60,18 ; H, 8,81.

### PRÉPARATION 56

### 2-(Triméthylsilyl)éthyl O-(2,3,6-tri-O-acétyl-4-O-p-méthoxybenzyl-α-D-glucopyranosyl)-(1→4)-O-(2,3,6-tri-O-acétyl-β-D-glucopyranosyl)-(1→4)-6-O-benzyl-2,3-di-O-méthyl-α-D-glucopyranoside (62)

On traite un mélange du thioglycoside **57** (19,1 g, 25,1 mmol) et de l'accepteur de glycosyle **61** (7,5 g, 18,7 mmol) selon la MÉTHODE 3 pour obtenir, après purification sur colonne de silice (dichlorométhane/acétone 20 : 1 puis 10 : 1), **62** (19,7 g, 95 %). [α]_{D} + 90 (*c* = 1,15, dichlorométhane).
Anal. calculée pour C₅₂H₇₄O₂₃Si (1095,24) : C, 57,03 ; H, 6,81. Trouvé : C, 57,38 ; H, 6,85.

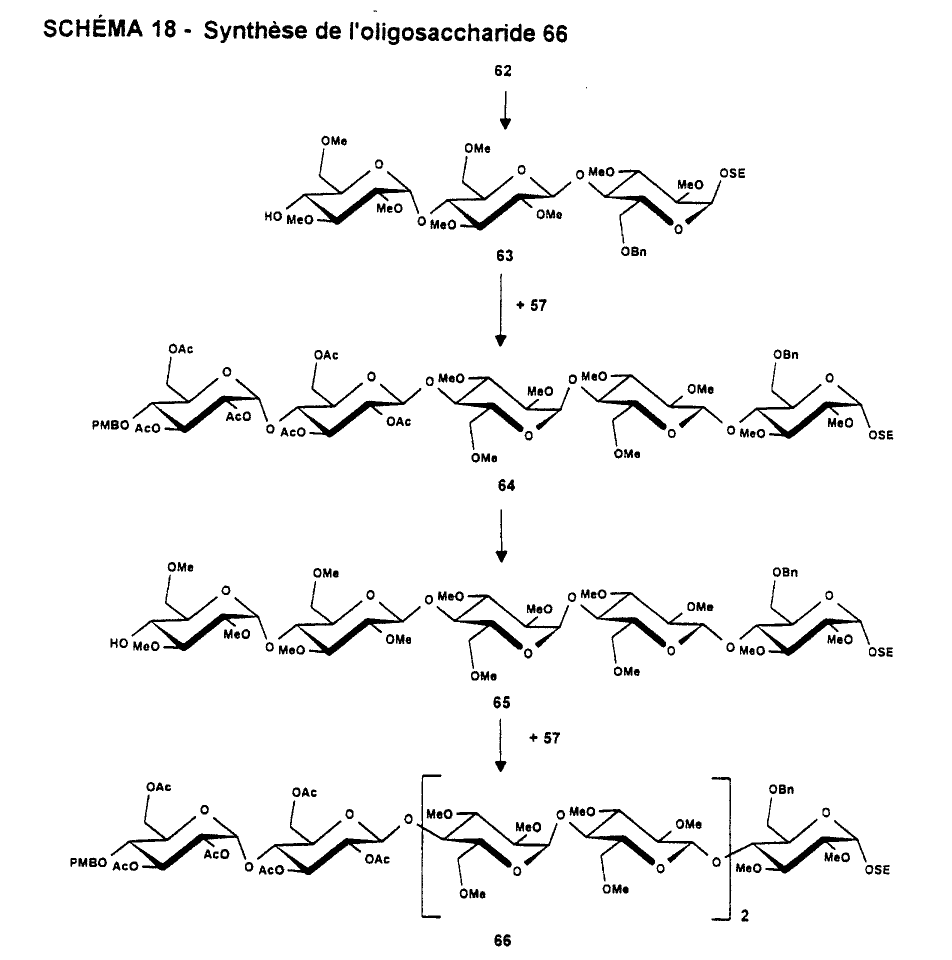

### PRÉPARATION 57

### 2-(Triméthylsilyl)éthyl O-(2,3,6-tri-O-méthyl-α-D-glucopyranosyl)-(1→4)-O-(2,3,6-tri-O-méthyl-β-D-glucopyranosyl)-(1→4)-6-O-benzyl-2,3-di-O-méthyl-α-D-glucopyranoside (63)

On traite le composé **62** (19,7 g, 18,0 mmol) selon la MÉTHODE 4 pour obtenir, après purification sur colonne de silice (toluène/acétone 3 : 1 puis 2 : 1), le composé **63** (11,2 g, 79 % sur les trois étapes). [α]_{D} + 95 (c = 1,15, dichlorométhane). ESIMS, mode positif : m/z + NaCl, 829 (M+Na)⁺ ; + KF, 845 (M+K)⁺. ¹H RMN (CDCl₃) δ 7,34-7,25 (m, 5H, Ph), 5,60 (d, 1H, J = 3,8 Hz, H-1"), 4,96 (d, 1H, J = 3,8 Hz, H-1), 4,29 (d, 1H, J = 8,0 Hz, H-1'), 1,08-0,91 (m, 2H, CH₂C*H*₂Si(CH₃)₃), 0,00 (s, 9H, CH₂CH₂Si(C*H*₃)₃).

### PRÉPARATION 58

### 2-(Triméthylsilyl)éthyl O-(2,3,6-tri-O-acétyl-4-O-p-méthoxybenzyl-α-D-glucopyranosyl)-(1→4)-O-(2,3,6-tri-O-acétyl-β-D-glucopyranosyl)-(1→4)-O-(2,3,6-tri-O-méthyl-α-D-glucopyranosyl)-(1→4)-O-(2,3,6-tri-O-méthyl-β-D-glucopyranosyl)-(1→4)-6-O-benzyl-2,3-di-O-méthyl-α-D-glucopyranoside (64)

On traite un mélange du thioglycoside **57** (6,87 g, 9,10 mmol) et d'accepteur de glycosyle **63** (6,67 g, 8,30 mmol) selon la MÉTHODE 3, et on purifie le résidu sur colonne de silice (toluène/acétone 2 : 5) pour obtenir **64** (10,7 g, 86 %). [α]_{D} + 90 (c = 0,83, dichlorométhane). ESIMS, mode positif : m/z + NaCI, 1525 (M+Na)⁺ ; + KF, 1541 (M+K)⁺. ¹H RMN (CDCl₃) δ 7,33-7,25 (m, 5H, Ph), 7,15-6,84 (m, 4H, C₆*H*₄OCH₃), 5,56 (d, 1H, J = 3,9 Hz, H-1"), 5,29 (d, 1H, J = 4,0 Hz, H-1""), 4,97 (d, 1H, J = 3,7 Hz, H-1), 4,71 (d, 1H, J = 8,1 Hz, H-1"'), 4,27 (d, 1H, J = 7,9 Hz, H-1'), 1,08-0,91 (m, 2H, CH₂C*H*₂Si(CH₃)₃), 0,00 (s, 9H, CH₂CH₂Si(C*H*₃)₃).
Anal. calculée pour C₇₀H₁₀₆O₃₃Si (1503,69) : C, 55,91 ; H, 7,11. Trouvé : C, 56,05 ; H, 7,24.

### PRÉPARATION 59

### 2-(Triméthylsilyl)éthyl [O-(2,3,6-tri-O-méthyl-α-D-glucopyranosyl)-(1→4)-O-(2,3,6-tri-O-méthyl-β-D-glucopyranosyl)-(1→4)]₂-6-O-benzyl-2,3-di-O-méthyl-α-D-glucopyranoside (65)

On traite le composé **64** (10,7 g, 7,2 mmol) selon la MÉTHODE 4, et on purifie le résidu sur colonne de silice (toluène/acétone 2 : 1 puis 6 : 5) pour obtenir **65** (6,50 g, 74 % sur les trois étapes). [α]_{D} + 102 (c = 0,68, dichlorométhane). ¹H RMN (CDCl₃) δ 7,33-7,25 (m, 5H, Ph), 5,65 (d, 1H, J = 3,8 Hz, H-1""), 5,62 (d, 1H, J = 3,8 Hz, H-1"), 4,98 (d, 1H, J = 3,7 Hz, H-1), 4,31 (d, 1H, J = 8,1 Hz, H-1"'), 4,29 (d, 1H, J = 7,9 Hz, H-1'), 1,08-0,91 (m, 2H, CH₂C*H*₂Si(CH₃)₃), 0,00 (s, 9H, CH₂CH₂Si(C*H*₃)₃).
Anal. calculée pour C₅₆H₉₈O₂₆Si (1215,48) : C, 55,34 ; H, 8,13. Trouvé : C, 55,31 ; H, 8,19.

### PRÉPARATION 60

### 2-(Triméthylsilyl)éthyl O-(2,3,6-tri-O-acétyl-4-O-p-méthoxybenzyl-α-D-glucopyranosyl)-(1→4)-O-(2,3,6-tri-O-acétyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₂-6-O-benzyl-2,3-di-O-méthyl-α-D-glucopyranoside (66)

On traite un mélange du thioglycoside **57** (4,03 g, 5,31 mmol) et d'accepteur de glycosyle **65** (5,78 g, 4,75 mmol) selon la MÉTHODE 3, et on purifie le résidu sur colonne de silice (toluène/acétone 2 : 5) pour obtenir **66** (8,63 g, 95 %). [α]_{D} + 94 (c = 0,74_{,} dichlorométhane). ESIMS, mode positif : masse monoisotopique = 1910,83, masse chimique = 1912,14, masse expérimentale = 1911,61 ± 0,12 u.m.a.. ¹H RMN (CDCl₃) δ 7,33-7,25 (m, 5H, Ph), 7,15-6,83 (m, 4H, C₆*H*₄OCH₃), 5,62 (d, 1H, J = 3,8 Hz, H-1 unité R-2), 5,60 (d, 1H, J = 3,8 Hz, H-1 unité NR-2), 5,30 (d, 1H, J = 4,0 Hz, H-1 unité NR), 4,98 (d, 1H, J = 3,7 Hz, H-1 unité R), 4,71 (d, 1H, J = 7,9 Hz, H-1 unité NR-1), 4,30 (d, 1H, J = 8,1 Hz, H-1 unité C), 4,29 (d, 1H, J = 8,4 Hz, H-1 unité R-1), 2,08 (s, 3H, Ac), 2,07 (s, 3H, Ac), 2,03 (s, 3H, Ac), 1,99 (s, 3H, Ac), 1,98 (s, 3H, Ac), 1,96 (s, 3H, Ac), 1,08-0,91 (m, 2H, CH₂C*H*₂Si(CH₃)₃), 0,00 (s, 9H, CH₂CH₂Si(C*H*₃)₃).
Anal. calculée pour C₈₈H₁₃₈O₄₃Si (1912, 14) : C, 55,28 ; H, 7,27. Trouvé : C, 55,61 ; H, 7,35.

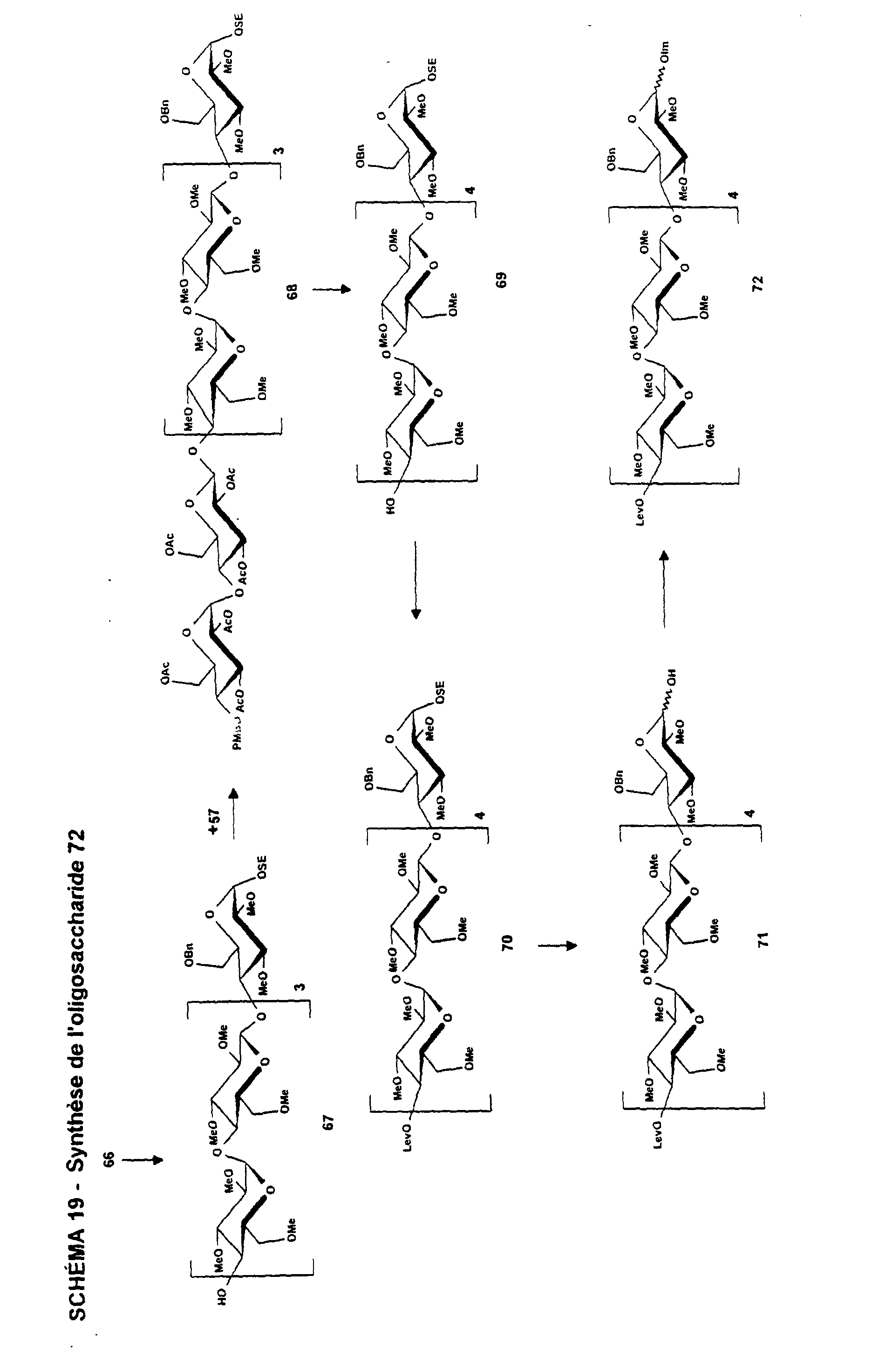

### PRÉPARATION 61

### 2-(Triméthylsilyl)éthyl[O-(2,3,6-tri-O-méthyl-α-D-glucopyranosyl)-(1→4)-O-(2,3,6-tri-O-méthyl-β-D-glucopyranosyl)-(1→4)]₃-6-O-benzyl-2,3-di-O-méthyl-α-D-glucopyranoside (67)

On traite le composé **66** (8,63 g, 4,50 mmol) selon la MÉTHODE 4 et on purifie le résidu sur colonne de silice (toluène/acétone 3 : 2 puis 4 : 3) pour obtenir **67** (5,67 g, 77 % sur les trois étapes). [α]_{D} + 102 (c = 0,70, dichlorométhane). LSIMS, mode positif : m/z thioglycerol + NaCl, 1645,9 (M+Na)⁺. ¹H RMN (CDCl₃) δ 7,33-7,32 (m, 5H, Ph), 5,65 (d, 2H, J = 3,8 Hz, H-1 unité NR et H-1 unité NR-2), 5,62 (d, 1H, J = 3,8 Hz, H-1 unité R-2), 4,98 (d, 1H, J = 3,7 Hz, H-1 unité R), 4,31 (d, 1H, J = 8,1 Hz, H-1 unité NR-1), 4,30 (d, 1H, J = 7,9 Hz, H-1 unité C), 4,29 (d, 1H, J = 7,9 Hz, H-1 unité R-1), 1,08-0,91 (m, 2H, CH₂C*H*₂Si(CH₃)₃), 0,00 (s, 9H, CH₂CH₂Si(C*H*₃)₃).
Anal. calculée pour C₇₄H₁₃₀O₃₆Si (1912,14) : C, 54,73 ; H, 8,07. Trouvé : C, 54,61 ; H, 8,07.

### PRÉPARATION 62

### 2-(Triméthylsilyl)éthyl O-(2,3,6-tri-O-acétyl-4-O-p-méthoxybenzyl-α-D-glucopyranosyl)-(1→4)-O-(2,3,6-tri-O-acétyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-6-O-benzyl-2,3-di-O-méthyl-α-D-glucopyranoside (68)

On traite un mélange du thioglycoside **57** (1,47 g, 1,95 mmol) et d'accepteur de glycosyle **67** (2,89 g, 1,77 mmol) selon la MÉTHODE 3. On filtre le résidu sur colonne de silice (cyctohexane/acétone 3 : 2) pour obtenir **68** (3,71 g, 92 %). Un échantillon analytique est purifié sur colonne de silice (toluène/acétone 3 : 2). [α]_{D} + 99 (c = 0,58, dichiorométhane); ESIMS, mode positif : masse monoisotopique = 2319,03 ; masse chimique = 2320,59 ; masse expérimentale = 2319,03 ± 0,12 u.m.a.. ¹H RMN (CDCl₃) δ 7,33-7,32 (m, 5H, Ph), 7,15-6,83 (m, 4H, C₆*H*₄OCH₃), 5,62 (d, 1H, J = 3,8 Hz, H-1 unité R-2), 5,60 (d, 2H, J = 3,8 Hz, H-1 unité NR-2 et unité C), 5,29 (d, 1H, J = 4,0 Hz, H-1 unité NR), 4,97 (d, 1H, J = 3,7 Hz, H-1 unité R), 4,70 (d, 1H, J = 7,9 Hz, H-1 unité NR-1), 4,30 (d, 2H, J = 8,1 Hz, H-1 unité NR-3 et unité R-3), 4,29 (d, 1H, J = 7,9 Hz, H-1 unité R-1), 2,08 (s, 3H, Ac), 2,06 (s, 3H, Ac), 2,02 (s, 3H, Ac), 1,99 (s, 3H, Ac), 1,98 (s, 3H, Ac), 1,96 (s, 3H, Ac), 1,08-0,91 (m, 2H, CH₂C*H*₂Si(CH₃)₃), 0,00 (s, 9H, CH₂CH₂Si(C*H*₃)₃).
Anal. calculée pour C₁₀₆H₁₇₀O₅₃Si (2320,59) : C, 54,86 ; H, 7,38. Trouvé : C, 54,76 ; H, 7,45.

### PRÉPARATION 63

### 2-(Triméthylsilyl)éthyl [O-(2,3,6-tri-O-méthyl-α-D-glucopyranosyl)-(1→4)-O-(2,3,6-tri-O-méthyl-β-D-glucopyranosyl)-(1→4)]₄-6-O-benzyl-2,3-di-O-méthyl-α-D-glucopyranoside (69)

On traite le composé **68** (3,61 g, 1,55 mmol) selon la MÉTHODE 4, puis on purifie le résidu sur colonne de silice (toluène/acétone 4 : 5) pour obtenir **69** (2,22 g, 70 % sur les trois étapes). [α]_{D} + 103 (*c* = 0,80, dichiorométhane). ESIMS, mode positif : masse monoisotopique = 2031,01 ; masse chimique = 2032,38 ; masse expérimentale = 2032,38 u.m.a.. ¹H RMN (CDCl₃) 87,33-7,32 (m, 5H, Ph), 5,65 (d, 1H, J = 3,8 Hz, H-1 unité NR), 5,64 (d, 1H, J = 3,8 Hz, H-1 unité NR-2), 5,62 (d, 2H, J = 3,8 Hz, H-1 unité C et unité R-2), 4,97 (d, 1H, J = 3,7 Hz, H-1 unité R), 4,30 (d, 1H, J = 7,9 Hz, H-1 unité NR-1), 4,29 (d, 3H, J = 7,9 Hz, H-1 unité R-1, unité R-3 et unité NR-3), 1,08-0,91 (m, 2H, CH₂C*H*₂Si(CH₃)₃), 0,00 (s, 9H, CH₂CH₂Si(C*H*₃)₃).
Anal. calculée pour C₉₂H₁₆₂O₄₆Si (2032,38) : C, 54,37 ; H, 8,03. Trouvé : C, 54,51 ; H, 8,04.

### PRÉPARATION 64

### 2-(Triméthylsilyl)éthyl O-(4-O-lévulinyl-2,3,6-tri-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-6-O-benzyl-2,3-di-O-méthyl-α-D-glucopyranoside (70)

On dissout le composé **69** (855 mg, 0,42 mmol) dans le dichlorométhane (8 mL) et on ajoute à 25° C de la triéthylamine (83 µL, 0,59 mmol), de la 4-diméthylaminopyridine (5,14 mg, 0,04 mmol) et de l'anhydride lévulinique (117 mg, 0,55 mmol). On laisse sous agitation pendant 2 heures (CCM), puis on ajoute du dichlorométhane, on lave avec une solution aqueuse à 10 % d'hydrogénosulfate de potassium, puis à l'eau, on sèche et on évapore à sec. Ce composé peut être utilisé brut à l'étape suivante sans procéder à une purification. Un échantillon analytique est purifié sur colonne de silice (cyclohexane/acétone 5 : 4) pour donner **70** pur. [α]_{D} + 101 (*c* = 0,89, dichlorométhane). ESIMS, mode positif : masse monoisotopique = 2129,05 ; masse chimique = 2130,48 ; masse expérimentale = 2130,00 u.m.a.. ¹H RMN (CDCl₃) δ 7,33-7,32 (m, 5H, Ph), 5,65 (d, 3H, J = 3,8 Hz, H-1 unité NR, unité NR-2 et unité C), 5,63 (d, 2H, J = 3,8 Hz, H-1 unité R-2), 5,02 (t, 1H, J = 10,1 Hz, H-4 unité NR), 4,98 (d, 1H, J = 3,7 Hz, H-1 unité R), 4,32 (d, 1H, J = 7,9 Hz, H-1 unité NR-1), 4,30 (d, 2H, J = 7,9 Hz, H-1 unité NR-3 et unité R-3), 4,29 (d, 1H, J = 7,9 Hz, H-1 unité R-1), 2,80-2,50 (m, 4H, O(C:O)C*H*₂C*H*₂(C:O)CH₃), 2,18 (s, 3H, O(C:O)CH₂CH₂(C:O)C*H*₃), 1,08-0,91 (m, 2H, CH₂C*H*₂Si(CH₃)₃), 0,00 (s, 9H, CH₂CH₂Si(C*H*₃)₃).
Anal. calculée pour C₉₇H₁₆₈O₄₈Si (2032,38) : C, 54,69 ; H, 7,95. Trouvé : C, 54,55 ; H, 8,07.

### PRÉPARATION 65

### O-(4-O-Lévulinyl-2,3,6-tri-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopy-ranosyl)-(1→4)-O-(2,3,6-tri-O-méthyl-β-D-glucopyranosyl)-(1→4)]₃-6-O-benzyl-2,3-di-O-méthyl-α,β-D-glucopyranose (71)

On traite le composé **70** (876 mg, 0,41 mmol) comme pour la PRÉPARATION 44(a). On purifie le résidu sur colonne de silice pour obtenir un mélange d'isomères (α/β = 60/40) de **71** (600 mg, 50 % sur les deux étapes). [α]_{D} + 89 (c = 0,74, dichlorométhane). ESIMS, mode positif : masse monoisotopique = 2028,97 ; masse chimique = 2030,24 ; masse expérimentale = 2030,19 ± 0,09 u.m.a.. ¹H RMN (CDCl₃) δ 7,33-7,32 (m, 5H, Ph), 5,65 (d, 3H, J = 3,8 Hz, H-1 unité NR, unité NR-2 et unité C), 5,63 (d, 1H, J = 3,8 Hz, H-1 unité R-2), 5,33 (d, 1H, J = 3,2 Hz, H-1α unité R), 5,02 (t, 1H, J = 10,1 Hz, H-4 unité NR), 4,59 (d, 1H, J = 5,3 Hz, H-1β unité R), 4,32 (d, 1H, J = 7,9 Hz, H-1 unité NR-1), 4,30 (d, 2H, J = 7,9 Hz, H-1 unité NR-3 et unité R-3), 4,29 (d, 1H, J = 7,9 Hz, H-1 unité R-1), 2,80-2,50 (m, 4H, O(C:O)C*H*₂C*H*₂(C:O)CH₃), 2,18 (s, 3H, O(C:O)CH₂CH₂(C:O)C*H*₃).

### PRÉPARATION 66

### O-(4-O-Lévulinyl-2,3,6-tri-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopy-ranosyl)-(1→4)-O-(2,3,6-tri-O-méthyl-β-D-glucopyranosyl)-(1→4)]₃-6-O-benzyl-2,3-di-O-méthyl-α,β-D-glucopyranose trichloroacétimidate (72)

À une solution de **71** (593 mg, 0,29 mmol) dans le dichlorométhane (7 mL), on ajoute du carbonate de potassium (72,2 mg, 0,52 mmol) et du trichloroacétonitrife (176 µL, 1,75 mmol). On laisse sous agitation pendant 16 heures, on filtre et on évapore. On filtre sur gel de silice (cyclohexane/acétone + 1%o de triéthylamine 5 : 4) pour obtenir le mélange des anomères (α/β = 47/53) de l'imidate **72** (414 mg, 46 %). [α]_{D} + 86 (c = 0,84, dichlorométhane). ¹H RMN (CDCl₃) δ 7,33-7,32 (m, 5H, Ph), 6,50 (d, 1H, J = 3,5 Hz, H-1α unité R), 5,65 (d, 1H, J = 8,2 Hz, H-1β unité R), 5,65 (d, 1H, J = 3,8 Hz, H-1 unité NR), 5,64 (d, 2H, J = 3,8 Hz, H-1 unité NR-2 et unité C), 5,61 (d, 1H, J = 3,8 Hz, H-1 unité R-2), 5,02 (t, 1H, J = 10,1 Hz, H-4 unité NR), 4,37 (d, 1H, J = 7,9 Hz, H-1 unité R-1), 4,30 (d, 3H, J = 7,9 Hz, H-1 unité NR-1, unité NR-3 et unité R-3), 2,80-2,50 (m, 4H, O(C:O)C*H*₂C*H*₂(C:O)CH₃), 2,18 (s, 3H, O(C:O)CH₂CH₂(C:O)C*H*₃).

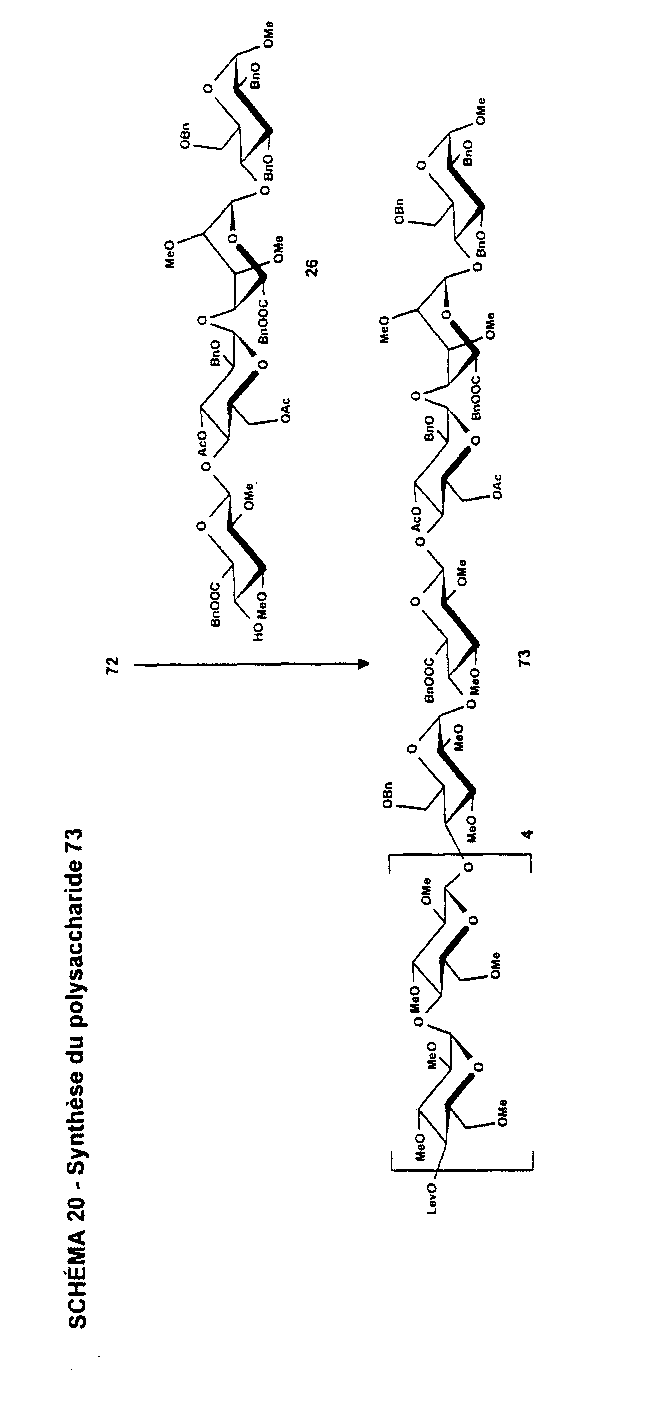

### PRÉPARATION 67

### Méthyl O-(4-O-lévulinyl-2,3,6-tri-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(6-O-benzyl-2,3-di-O-méthyl-α-D-glucopyranosyl)-(1→4)-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,3,6-tri-O-benzyl-α-D-glucopyranoside (73)

On dissout l'accepteur de glycosyle **26** (220 mg, 0,16 mmol) et l'imidate **72** (344 mg, 0,16 mmol) dans un mélange dichlorométhane/éther éthylique 1 : 2 (5 mL). On ajoute du tamis moléculaire 4 Å (750 mg/mmol) et on laisse sous agitation, à 25° C, pendant 1 heure. On refroidit le mélange à - 25° C et on ajoute un solution 1M de triflate de *tert*-butyldiméthylsilyle dans du dichlorométhane (0,20 mol/mol d'imidate). On laisse sous agitation pendant 15 minutes puis on ajoute de l'hydrogénocarbonate de sodium solide, on filtre et on concentre. On dépose sur colonne Toyopearl® HW-50 (dichlorométhane/éthanol 1 : 1) et on remet en réaction la fraction contenant l'accepteur de glycosyle, puis on la traite comme précédemment. On fait des purifications successives sur colonne de silice (toluène/acétone 5 : 4 puis 1:1) pour obtenir une fraction **73**α/β = 7/3 (107 mg) et une fraction **73** α/β = 9/1 (201 mg) avec un rendement global de 57 % (308 mg). ¹H RMN (CDCl₃) δ 7,33-7,25 (m, 35H, 7Ph), 5.65 (d, 3H, J = 3,5 Hz, H-1 unité A, unité C et unité E), 5,57 (d, 1H, J = 3,9 Hz, H-1 unité G), 5,52 (d, 1H, J = 3,3 Hz, H-1 unité I), 5,29 (d, 1H, J = 6,8 Hz, H-1 unité L), 5,17 (d, 1H, J = 3,5 Hz, H-1 unité K), 4,56 (d, 1H, J = 3,5 Hz, H-1 unité M), 4,31 (d, 3H, J = 7,9 Hz, H-1 unité B, unité D et unité F), 4,27 (d, 1H, J = 8,0 Hz, H-1 unité H), 4,08 (d, 1H, J = 8,0 Hz, H-1 unité J), 2,80-2,50 (m, 4H, O(C:O)C*H*₂C*H*₂(C:O)CH₃), 2,18 (s, 3H, O(C:O)CH₂CH₂(C:O)C*H*₃), 1,97 (s, 3H, Ac), 1,81 (s, 3H, Ac).

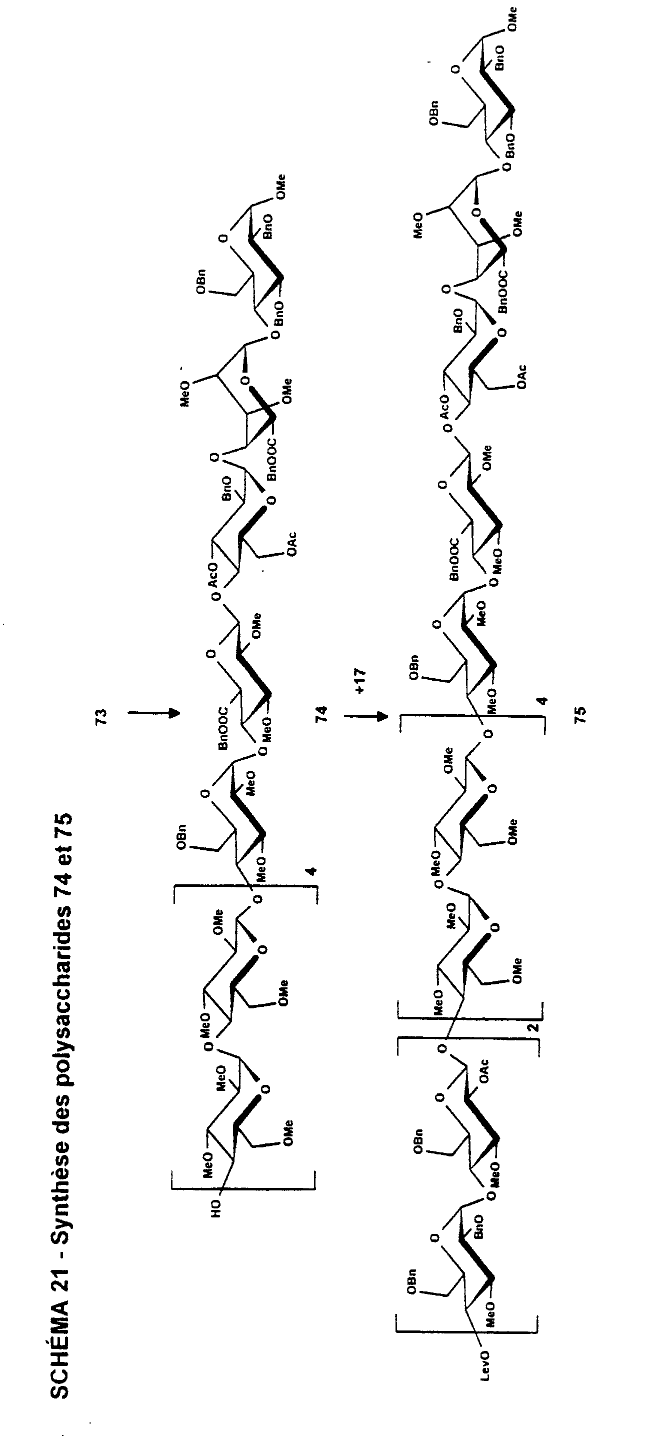

### PRÉPARATION 68

### Méthyl [O-(2,3,6-tri-O-méthyl-α-D-glucopyranosyl)-(1→4)-O-(2,3,6-tri-O-méthyl-β-D-glucopyranosyl)-(1→4)]₄-O-(6-O-benzyl-2,3-di-O-méthyl-α-D-glucopyranosyl)-(1→4)-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,3,6-tri-O-benzyl-α-D-glucopyranoside (74)

On traite le composé **73** (130 mg, 38,2 µmol) selon la MÉTHODE 2, et on purifie le brut sur une colonne de silice pour obtenir **74** (118 mg, 93 %). [α]_{D} + 78 (c = 0,48, dichlorométhane) ; ESIMS, mode positif: masse monoisotopique = 3301,49 ; masse chimique = 3303,65 ; masse expérimentale = 3302,40 u.m.a.. ¹H RMN (CDCl₃) δ 7,33-7,25 (m, 35H, 7Ph), 5,64 (d, 3H, J = 3,5 Hz, H-1 unité A, unité C et unité E), 5,57 (d, 1H, J = 3,9 Hz, H-1 unité G), 5,52 (d, 1H, J = 3,3 Hz, H-1 unité I), 5,29 (d, 1H, J = 6,8 Hz, H-1 unité L), 5,17 (d, 1H, J = 3,5 Hz, H-1 unité K), 4,56 (d, 1H, J = 3,5 Hz, H-1 unité M), 4,31 (d, 3H, J = 7,9 Hz, H-1 unité B, unité D et unité F), 4,27 (d, 1H, J = 8,0 Hz, H-1 unité H), 4,08 (d, 1H, J = 8,0 Hz, H-1 unité J), 1,97 (s, 3H, Ac), 1,81 (s, 3H, Ac).

### PRÉPARATION 69

### Méthyl O-(2,6-di-O-benzyl-4-O-lévulinyl-3-O-méthyl-α-D-glucopyranosyl)-(1→4)-O-(2-O-acétyl-6-O-benzyl-3-O-méthyl-β-D-glucopyranosyl)-(1→4)-O-(2,6-di-O-benzyl-3-O-méthyl-α-D-glucopyranosyl)-(1→4)-O-(2-O-acétyl-6-O-benzyl-3-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₄-O-(6-O-benzyl-2,3-di-O-méthyl-α-D-glucopyranosyl)-(1→4)-O-(benzyl 2,3-di-O-méthyl-β-D-glucopyranosyluronate)-(1→4)-O-(3,6-di-O-acétyl-2-O-benzyl-α-D-glucopyranosyl)-(1→4)-O-(benzyl 2,3-di-O-méthyl-α-L-idopyranosyluronate)-(1→4)-2,3,6-tri-O-benzyl-α-D-glucopyranoside (75)

On dissout l'accepteur de glycosyle **74** (112 mg, 33,9 µmol) et l'imidate **17** (59,1 mg, 37,2 µmol) (voir PRÉPARATION 16) dans du toluène (2 mL). On ajoute du tamis moléculaire 4 Å (42 mg) et on laisse sous agitation, à 25° C, pendant 1 heure. On refroidit le mélange à - 20° C et on ajoute une solution 1M de triflate de *tert*-butyldiméthylsilyl dans du toluène (0,20 mol/mol d'imidate). On laisse sous agitation pendant 15 minutes puis on ajoute de l'hydrogénocarbonate de sodium solide, on filtre et on concentre. On dépose sur une colonne Toyopearl® HW-50 (dichtorométhane/éthanol 1 : 1), on remet en réaction la fraction contenant l'accepteur et le 17-mer et on la traite comme précédemment. On purifie successivement sur colonne Toyopearl® HW-50 et sur colonne de silice pour obtenir **75** (71 mg, 44 %). [α]_{D} + 80 (*c* = 0,26, dichlorométhane). ESIMS, mode positif : masse monoisotopique = 4728,10 ; masse chimique = 4731,26 ; masse expérimentale = 4731,27 ± 0,39 u.m.a.. ¹H RMN (CDCl₃) δ des principaux protons anomériques : 5,64 ; 5,57 ; 5,52 ; 5,48 ; 5,47 ; 5,29 ; 5,17 ; 4,56 ; 4,50 ; 4,29 ; 4,27 ; 4,08 ppm.

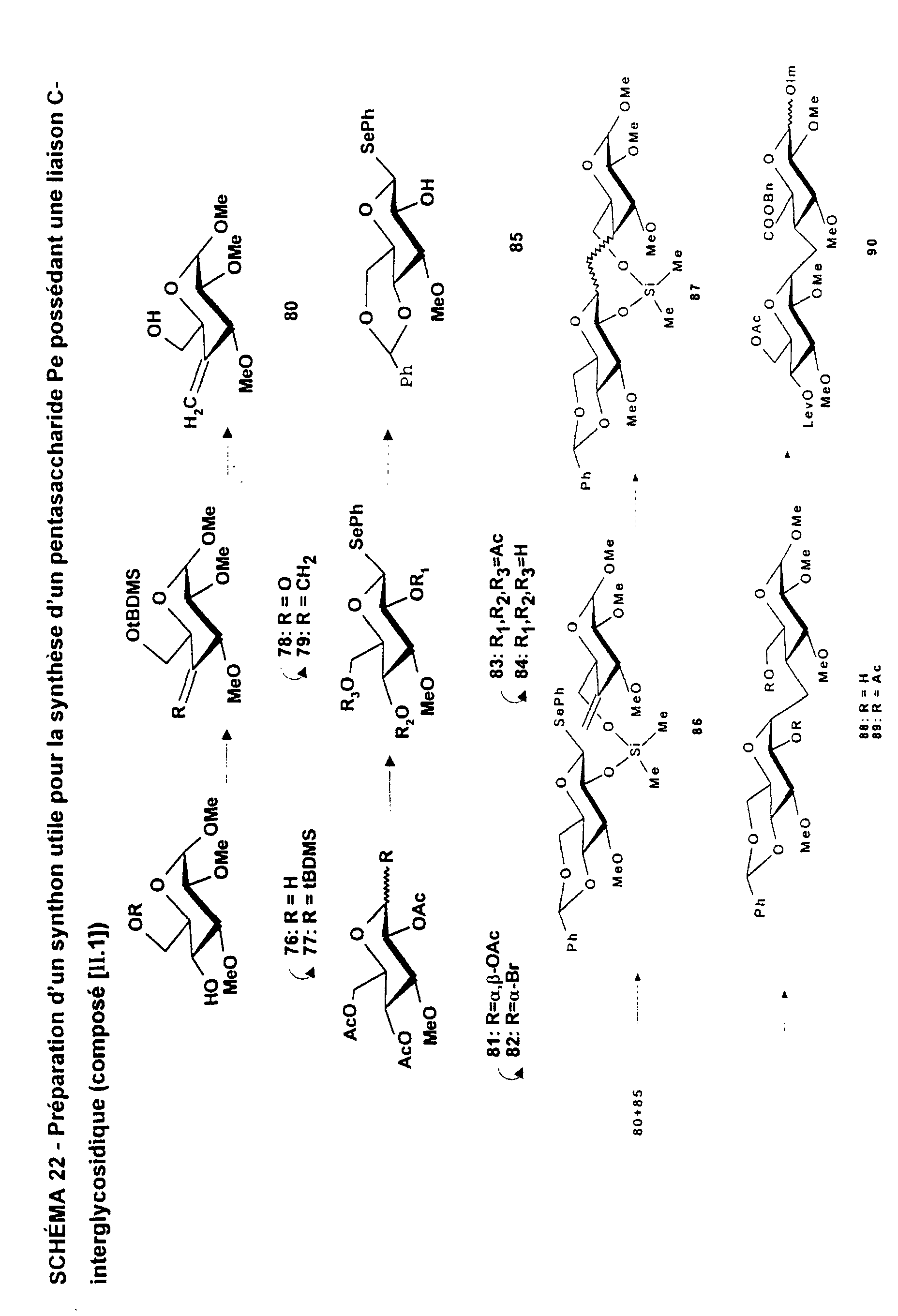

### PRÉPARATION 70

### Méthyl 2,3-di-O-méthyl-6-O-tert-butyldiméthylsilyl-α-D-glucopyranoside (77)

On ajoute sous argon du chlorure de tert-butyldiméthylsilyle (14,0 g, 92,9 mmol), de la triéthylamine (15 mL, 108 mmol), et du 4-diméthylaminopyridine (260 mg, 2,13 mmol) à une solution de **76** (D. Trimmell, W. M. Doane, C. R. Russel, C. E. Rist, *Carbohydr.*

*Res.,* (1969) **11,** 497) (15,84 g, 71,3 mmol) dans du dichlorométhane (300 mL). Après 15 heures d'agitation à température ambiante, on dilue la solution avec du dichlorométhane, on lave avec une solution aqueuse saturée d'hydrogénocarbonate de sodium, on sèche (sulfate de magnésium), on filtre, et on concentre. On purifie le résidu par chromatographie flash (cyclohexane/acétate d'éthyle 1,3 : 1) pour obtenir **77** (22,79 g, 95 %) sous forme de sirop incolore. [α]_{D} + 87 (*c* = 1,2, chloroforme).

### PRÉPARATION 71

### Méthyl 2,3-di-O-méthyl-6-O-tert-butyldiméthylsilyl-α-D-xylo-4-hexulo-pyranoside (78)

On ajoute sous argon et à - 70° C, une solution de diméthylsulfoxyde (8,7 mL, 123 mmol) dans du dichlorométhane (20 mL) à une solution de chlorure d'oxalyle (5,4 mL, 61,9 mmol) dans du dichlorométhane (120 mL). Après 15 minutes, on ajoute goutte à goutte une solution de **77** (18,78 g, 55,8 mmol) dans du dichlorométhane.

Après 15 minutes d'agitation magnétique, on ajoute de la triéthylamine (37 mL, 265 mmol), et après 15 minutes, on laisse revenir à température ambiante. On ajoute de l'eau (150 mL), et on extrait la phase aqueuse avec du dichlorométhane (150 mL).

On joint les phases organiques, on lave par une solution aqueuse saturée de chlorure de sodium, on sèche (sulfate de magnésium), et on concentre. On purifie le résidu par chromatographie flash (cyclohexane/acétate d'éthyle 9 : 1 puis 4 : 1) pour obtenir **78** (17,3 g, 93 %) sous forme d'une huile incolore. [α]_{D} + 98 (*c* = 1,0, chloroforme).

### PRÉPARATION 72

### Méthyl 4-désoxy-2,3-di-O-méthyl-4-C-méthylène-6-O-tert-butyldiméthyl-silyl-α-D-xylo-hexopyranoside (79)

On ajoute goutte à goutte et sous argon une solution 1,6 M de *n*-butyllithium dans du n-hexane (75 mL) à une suspension de bromure de méthyltriphénylphosphonium (43,3 g, 127 mmol) dans du tétrahydrofurane (250 mL). Après 30 minutes à température ambiante, le mélange est refroidi à - 70° C. On ajoute ensuite une solution de **78** (13,97 g, 41,8 mmol) dans du tétrahydrofurane (60 mL). Après 30 minutes à -70° C on laisse revenir à température ambiante. Après 1 heure, on ajoute une solution aqueuse saturée de chlorure d'ammonium (300 mL) et on extrait la phase aqueuse à l'éther. On sèche la phase organique (sulfate de magnésium), on filtre, et on concentre. On purifie le résidu par chromatographie flash (cyclohexane/ acétate d'éthyle 7 : 1 puis 4 : 1) pour obtenir **79** (8,30 g, 60 %) sous forme d'une huile incolore.
[α]_{D} + 151 (*c* = 1,3, chloroforme).

### PRÉPARATION 73

### Méthyl 4-désoxy-2,3-di-O-méthyl-4-C-méthylène-α-D-xylo-hexopyranoside (80)

On ajoute de l'acide camphorsulfonique (pH 1) à une solution de **79** (8,50 g, 25,6 mmol) dans un mélange dichlorométhane-méthanol 5 : 1 (250 mL). Après disparition complète du produit de départ (CCM cyclohexane/acétate d'éthyle 1 : 1), on neutralise la solution par addition de triéthylamine. Après concentration, le résidu est purifié par chromatographie flash (cyclohexane/ acétate d'éthyle 1 : 1 puis 1 : 2) pour donner **80** (5,32 g, 95 %) sous forme d'un sirop incolore. [α]_{D} + 239 (c = 1,0, chloroforme).

### PRÉPARATION 74

### Phényl 2,4,6-tri-O-acétyl-3-O-méthyl-1-séléno-β-D-glucopyranoside (83)

MÉTHODE 1 : On ajoute du sélénophénol (5,7 mL, 53,7 mmol) à une solution sous argon de **81** (E.L. Hirst, E. Percival, Methods Carbohydrate Chem., (1963) 2, 145) (mélange d'anomères 1 : 1 ; 13,05 g, 36,0 mmol) dans du dichlorométhane (120 mL). On refroidit le milieu réactionnel à 0° C et on ajoute goutte à goutte une solution à 48 % de diéthylétherate de trifluoroborane (8,8 mL, 71,9 mmol). Après 3 heures à température ambiante, on dilue le mélange réactionnel avec du dichlorométhane (100 mL), on lave avec une solution aqueuse saturée d'hydrogénocarbonate de sodium, on sèche (sulfate de magnésium), on filtre, et on concentre. On engage directement le composé brut 83 (8,17 g, 68 % à partir de **81**) dans ta réaction de désacétylation.

MÉTHODE 2 : On ajoute à 0° C et sous argon du borohydrure de sodium (510 mg, 14,6 mmol) à une suspension de diphényldisélénide (2,27 g, 7,27 mmol) dans de l'éthanol. On ajoute une nouvelle portion de diéthylétherate de trifluoroborane si le milieu réactionnel n'a pas perdu sa couleur jaune d'origine en 15 minutes. On transfère cette solution sous argon à une solution de **82** (A.K. Sen, K.K. Sakar, N. Banerji, *J. Carbohydr. Chem.,* (1988) **7,** 645) (4,22 g, 11,0 mmol) dans du dichlorométhane (25 mL). Après 3 heures de reflux, on laisse refroidir à température ambiante, on filtre le bromure de sodium, et on concentre. On dissout le résidu dans du dichlorométhane (100 mL) et on lave par une solution aqueuse 1M d'hydroxyde de sodium (50 mL) et une solution aqueuse saturée de chlorure d'ammonium (50 mL). On extrait les phases aqueuses au dichlorométhane (20 mL), on sèche les phases organiques (sulfate de magnésium), on filtre et on concentre. On purifie le résidu par chromatographie flash (cyclohexane/acétate d'éthyle 1,7 : 1) puis on cristallise dans l'acétate d'éthyle pour obtenir **83** (4,35g, 86 %). pf 101-102 C. [α]_{D} - 20 (c = 1,0, chloroforme).

### PRÉPARATION 75

### Phényl 3-O-méthyl-1-séléno-β-D-glucopyranoside (84)

On dissout le composé brut **83** obtenu à partir de **82** (32,3 g, 84,3 mmol) dans du méthanol (500 mL), et on introduit lentement du sodium (1,2 g). Après 1 heure, on neutralise la solution par addition de résine IR-120 (H⁺), on filtre et on concentre. On purifie le résidu par chromatographie flash (cyclohexane/acétate d'éthyle/acétone 1,5 : 1 : 1) pour obtenir **84** sous forme d'un sirop (22,7 g, 81 % depuis **82).** [α]_{D} - 58 (*c* = 1,0, méthanol).

### PRÉPARATION 76

### Phényl 4,6-O-benzylidène-3-O-méthyl-1-séléno-β-D-glucopyranoside (85)

On ajoute sous argon de l'acide *p*-toluènesulfonique (45 mg) et du benzaldéhydediméthylacetal (5,4 mL, 36,0 mmol) à une solution de triol **84** (7,65 g, 23,0 mmol) dans de l'acétonitrile (150 mL). Après 2 heures d'agitation à température ambiante, on ajoute du carbonate de potassium (1,5 g). Après 30 minutes, on filtre la solution, puis on concentre. On purifie le résidu par chromatographie flash (cyclohexane/acétate d'éthyle 3,5 : 1) pour obtenir **85** (8,55g, 88 %) sous forme de cristaux blancs. pf 123-124°C (cyclohexane/acétate d'éthyle). [α]_{D} - 38 (*c* = 1,0, chloroforme).

### PRÉPARATION 77

### Phényl 4,6-O-benzylidène-3-O-méthyl-2-O-(méthyl 4-désoxy-6-O-diméthylsilyl-2,3-di-O-méthyl-4-C-méthyiène-α-D-xylo-hexopyranoside)-1-séléno-β-D-glucopyranoside (86)

On ajoute, sous argon et à - 70° C, une solution 1,6 M de *n*-butyllithium (7,0 mL, 11,2 mmol) à une solution de **85** (4,30 g, 10,2 mmol) dans du tétrahydrofurane (30 mL) placée dans un appareil de Schlenck. Après 10 minutes, on ajoute du dichlorodiméthylsilane (5,0 mL, 41,2 mmol), et le milieu réactionnel est réchauffé à température ambiante. Après 3 heures, on concentre et on ajoute une solution de **80** (2,10 g, 9,62 mmol) et d'imidazole (985 mg, 14,4 mmol) dans du tétrahydrofurane (20 mL). Après 30 minutes à température ambiante, on concentre la solution, on additionne de l'eau (50 mL), et on extrait au dichlorométhane. On sèche la phase organique (sulfate de magnésium), on filtre, et on concentre. Un échantillon analytique de **86** est purifié par chromatographie flash (toluène/acétone 25 : 1 contenant 0,5 % de triéthylamine). Un sirop incolore est obtenu avec un rendement de 90 %.
¹H RMN (400M Hz, C₆D₆) d 7,77-6,99 (m, 10H, aromatiques), 5,51 (m, 1H, C :C*H*₂), 5,24 (m, 1H, C :C*H*₂), 5,16 (s, 1H, C*H*Ph), 4,85 (d, 1H, J = 3,7 Hz, H-1), 4,81 (d, 1H, J = 9,8 Hz, H-1'), 4,45 (m, 1H, H-5), 4,38 (dd, 1H, J = 10,8 Hz, 4,8 Hz, H-6a), 4,33 (dd, 1H, J = 6,2 Hz, H-6b), 4,20 (m, 1H, J = 9.2 Hz, H-3), 4,05 (dd, 1H, J = 10,3 Hz, 4,9 Hz, H-6a'), 3,87 (dd, 1H, J = 8,1 Hz, H-2'), 3,52, 3,38, 3,31 et 3,27 (s, 3H, OC*H*₃), 3,37 (t, 1H, J = 10,3 Hz, H-6b'), 3,35 (t, 1H, H-4'), 3,32 (dd, 1H, H-2), 3,22 (dd, 1H, J = 9,3 Hz, H-3'), 3,05 (ddd, 1H, J = 9,3 Hz, H-5'), 0,38 et 0,37 (s, 3H, Si(C*H*₃*)*₂). MS (m/z) : 714 (M+NH₄)⁺

### PRÉPARATION 78

### Réaction de cyclisation radicalaire (formation de 87) et coupure du téther (88)

On ajoute, sur une période de 8 heures, une solution d'hydrure de tributylétain (6,1 mL, 22,7 mmol) et de 2,2'-azobisisobutyronitrile (200 mg, 1,22 mmol) dans du toluène dégazé (14 mL), à une solution de **86** brut dans du toluène (850 mL), issu de **85** (10,2 mmol) et de **80** (9,62 mmol).
Après la cyclisation radicalaire, on concentre et on dissout le résidu dans du tétrahydrofurane. On ajoute un excès (20 équivalents) d'acide fluorhydrique (à 40 % dans l'eau). Après totale désilylation (CCM, toluène/acétone 4 : 1) on neutralise la solution par addition d'hydrogénocarbonate de sodium solide, on filtre et on concentre. Le composé majoritaire **88** peut être purifié par cristallisation. pf 105 C. [α]_{D} + 119 (*c =* 1,1, chloroforme). ¹³C RMN (62,896M Hz, CDCl₃) d 137,29 (C aromatiques quarternaires), 128,88-125,96 (C aromatiques), 101,11 (CHPh), 97,64 (C-1), 83,52 (C-2), 82,76, 81,95, 80,84, 72,01, 71,92 et 64,31 (C-3, C-5, C-2', C-3', C-4', C-5'), 75,19 (C-1'), 69,41 (C-6'), 62,69 (C-6), 60,93, 60,70, 58,31 et 55,20 (OCH₃), 38,80 (C-4), 25,58 (C méthylène).
Anal. calculée pour C₂₄H₃₆O₁₀,H₂O (502,558) : C, 57.36 ; H, 7,62. Trouvé : C, 57,31 ; H, 7,54.

### PRÉPARATION 79

### Méthyl 6-O-acétyl-4-C-(2-O-acétyl-4,6-O-benzylidène-3-O-méthyl-α-D-glucopyranosylméthyl)-4-désoxy-2,3-di-O-méthyl-α-D-glucopyranoside (89)

Le composé **88** est acétylé quantitativement dans un mélange d'anhydride acétique/pyridine 1 : 1, en présence d'une quantité catalytique de 4-diméthylaminopyridine. Le produit est obtenu après concentration et chromatographie. [α]_{D} + 87 (*c* = 1,0, chloroforme). ¹H RMN (500M Hz, CDCl₃) : voir table 1. ¹³C RMN (62,896M Hz, CDCl₃) d 170,81, 169,80 (C:O), 137,18 (C quarternaires aromatiques), 128,92-125,95 (C aromatiques), 101,31 (CHPh), 97,51 (C-1), 83,22 (C-2), 81,94, 69,25 et 63,83 (C-5, C-4', C-5'), 81,81 (C-3), 78,78 (C-3'), 72,74 (C-2'), 71,96 (C-1'), 69,49 (C-6'), 64,17 (C-6), 60,64, 59,82, 58,30 et 55,19 (OCH₃), 39,00 (C-4), 26,47 (C méthylène), 20,91, 20,76 (OCOCH₃). Spectre de masse (m/z) : 586 (M+NH₄)⁺, 569 (M+H)⁺, 554 (M-OMe+NH₃)⁺, 537 (M-OMe)⁺.
Anal. calculée pour C₂₈H₄₀O₁₂,H₂O (586,632) : C, 57,33 ; H, 7,22. Trouvé : C 57,28 ; H 7,07.
La dernière partie de la synthèse consiste en la transformation de **89** en imidate **90.** Pour ce faire, le benzylidène est ouvert en utilisant le cyanoborohydrure de sodium et l'acide chlorhydrique. Le groupement hydroxyle ainsi libéré est temporairement protégé sous forme d'éther *p*-méthoxybenzyle. Après désacétylation, la fonction alcool primaire est protégée par introduction sélective d'éther de *tert*-butyldiméthylsilyle, et le composé ainsi obtenu est méthylé. Une oxydation dans les conditions de Jones conduit à l'acide uronique qui est benzylé. L'éther p-méthoxy-benzylique est alors coupé puis un ester lévulinique est introduit à cette position. Un système utilisant un mélange acide sulfurique/acide acétique/anhydride acétique conduit à l'acétolyse du groupement méthyle anomère ainsi que de l'éther benzylique de la position 6', et fournit un mélange de deux acétates anomères. La désacétylation sélective de la position 1 est réalisée en utilisant de l'hydrazine dans le diméthylformamide, et le mélange d'anomères, dissous dans du dichlorométhane est transformé en **90** par le trichloroacétonitrile en présence de 1,8-diazabicyclo[5.4.0]undec-7-ène.

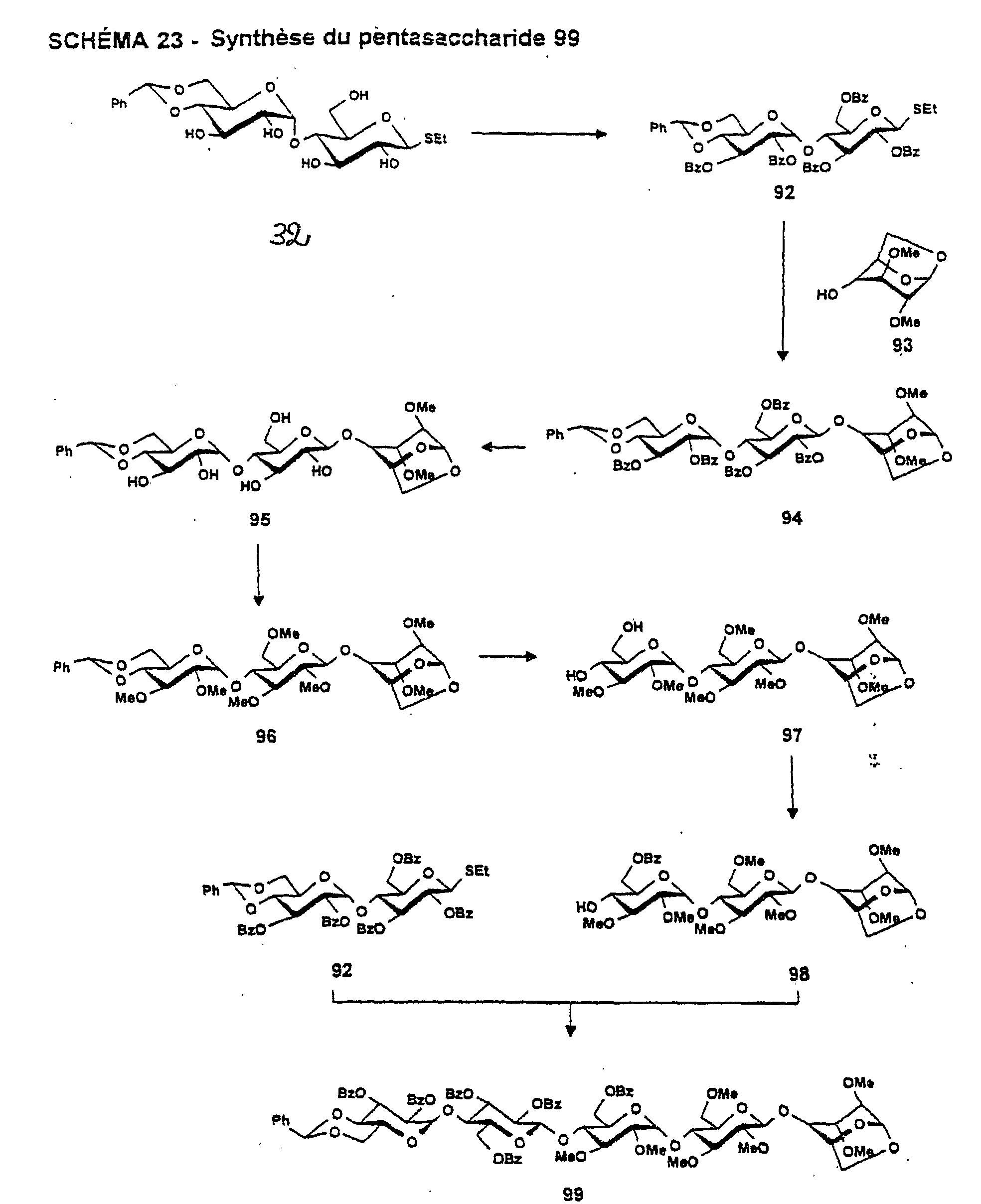

### PRÉPARATION 80

### Éthyl O-(2,3-di-O-benzoyl-4,6-O-benzylidène-α-D-glucopyranosyl)-(1→4)-2,3,6-tri-O-benzoyl-1-thio-β-D-glucopyranose (92)

À une solution refroidie (0° C) du composé 32 (16,7 g, 35,2 mmol) (J. Westman and M. Nilsson, J. Carbohydr. Chem., 1995, *14* (7), 949-960) dans la pyridine (202 ml); on ajoute pendant 20 minutes goutte à goutte du chlorure de benzoyle (24,5 ml, 211 mmol). On agite pendant 20 heures le mélange réactionnel à température ambiante ; la CCM révèle une conversion de 50 % environ. Le mélange est dilué avec de l'eau et du dichlorométhane. Après extraction, la phase organique est lavée avec une solution d'hydrogénocarbonate de sodium à 10 %, de l'eau, séchée sur sulfate de magnésium et concentrée. Le résidu est à nouveau traité avec du chlorure de benzoyle selon le mode opératoire décrit ci-dessus. Le produit brut est purifié par une chromatographie sur colonne de gel de silice pour fournir 22 g du composé **92.**
CCM : Rf = 0,80, gel de silice, toluène/éthanol 9/1 v/v

### PRÉPARATION 81

### O-(2,3-Di-O-benzoyl-4,6-O-benzylidène-α-D-glucopyranosyl)-(1→4)-O-(2,3,6-tri-O-benzoyl-β-D-glucopyranosyl)-(1→4)-1,6-anhydro-2,3-di-O-méthyl-β-D-glucopyranose (94)

Un mélange de thioglycoside **92** (1,05 g, 1,05 mmol), du composé **93** (200 mg, 1,05 mmol) (Jeanioz *et al.,* J. Org. Chem. 1961, 26, 3939-3944) et d'un tamis moléculaire 4Å en poudre (1,1 g) dans le toluène (18 ml) est agité sous atmosphère d'azote pendant 15 minutes. Le mélange est ensuite refroidi à - 20° C et une solution fraîchement préparée de N-iodosuccinimide (1,11 mmol) et d'acide trifluorométhanesulfonique (0,125 mmol) dans le dichlorométhane/dioxane 1/1 v/v (6 ml) y est introduite. Après 10 minutes, le mélange réactionnel rouge est filtré, dilué avec du dichlorométhane, extrait, successivement lavé avec une solution de thiosulfate de sodium à 10 %, une solution d'hydrogénocarbonate de sodium à 10 % et de l'eau, séché sur sulfate de magnésium puis concentré sous vide. La purification du résidu est effectuée par chromatographie sur une colonne de gel de silice afin d'obtenir 1,25 g du composé **94.**
CCM : Rf = 0,55, gel de silice, heptane/acétate d'éthyle 4/6 v/v

### PRÉPARATION 82

### O-(4,6-O-Benzylidène-α-D-glucopyranosyl)-(1→4)-O-(β-D-glucopyranosyl)-(1→4)-1,6-anhydro-2,3-di-O-méthyl-β-D-glucopyranose (95)

À une solution du composé **94** (1,24 g, 1,11 mmol) dans du méthanol/dioxane 1/1 v/v (7 ml), on ajoute du tert-butylate de potassium (environ 50 mg). On agite pendant une heure, puis on ajoute à nouveau 50 mg de tert-butylate de potassium ; le mélange est ensuite agité pendant encore 60 minutes. Le mélange réactionnel est neutralisé avec une résine Dowex® 50WX8 H⁺, filtré et concentré sous vide. Après une chromatographie sur colonne de gel de silice, 665 mg du composé **95** sont isolés sous forme d'huile.
CCM : Rf = 0,50, gel de silice, dichlorométhane/méthanol 85/15 v/v

### PRÉPARATION 83

### O-(4,6-O-Benzylidène-2,3-di-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (96)

À une solution refroidie (5° C) du composé **95** (660 mg, 1,1 mmol) dans du tétrahydrofurane sec (8 ml), on ajoute de l'hydrure de sodium (387 mg, 9,65 mmol) sous atmosphère d'azote. On ajoute goutte à goutte de l'iodure de méthyle (0,51 ml, 8,22 mmol) et le mélange est agité pendant 20 heures à température ambiante. L'excès d'hydrure de sodium est détruit avec du méthanol et le mélange est versé dans 50 ml d'eau glacée. Après extraction avec de l'acétate d'éthyle (3 fois 20 ml), la phase organique est lavée avec une solution de chlorure de sodium séchée sur sulfate de magnésium et concentrée pour donner 690 mg du composé **96** pur.
CCM : Rf = 0,25, gel de silice, dichlorométhane/méthanol 95/5 v/v

### PRÉPARATION 84

### O-(2,3-Di-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (97)

Le composé **96** pur (690 mg, 1,03 mmol) est dissous dans de l'acide acétique à 80 % (7,3 ml) et agité pendant 20 heures à 40° C. Le mélange est concentré sous vide et co-évaporé avec du toluène. Une chromatographie sur colonne de gel de silice dans du dichlorométhane/acétate d'éthyle/méthanol 8/1/1 permet d'obtenir 569 mg du composé **97.**
CCM : Rf = 0,40, gel de silice, dichlorométhane/méthanol 9/1 v/v

### PRÉPARATION 85

### O-(6-O-Benzoyl-2,3-di-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (98)

À une solution du composé **97** (560 mg, 0,96 mmol) dans du dichlorométhane, on ajoute du 1-benzoyloxy-1*H*-benzotriazole (227 mg, 1,05 mmol) et de la triéthylamine (1,15 mmol) puis le mélange est agité pendant 20 heures à température ambiante. Le mélange réactionnel est dilué avec du dichlorométhane, lavé avec une solution d'hydrogénocarbonate de sodium à 10 % et de l'eau. La phase organique est séchée sur sulfate de magnésium, filtrée et évaporée à sec. Le produit est purifié par chromatographie sur colonne de gel de silice afin d'obtenir 600 mg du composé 98.
CCM : Rf = 0,50, gel de silice, dichlorométhane/méthanol 9/1 v/v

### PRÉPARATION 86

### O-(2,3-Di-O-benzoyl-4,6-O-benzylidène-α-D-glucopyranosyl)-(1→4)-O-(2,3,6-tri-O-benzoyl-β-D-glucopyranosyl)-(1→4)-O-(6-O-benzoyl-2,3-di-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (99)

Le composé **98** est transformé en composé **99** selon le mode opératoire décrit pour la préparation du composé **94.** La réaction de couplage est réalisée à 5° C.
CCM : Rf = 0,50, gel de silice, heptane/acétate d'éthyle 2/8 v/v

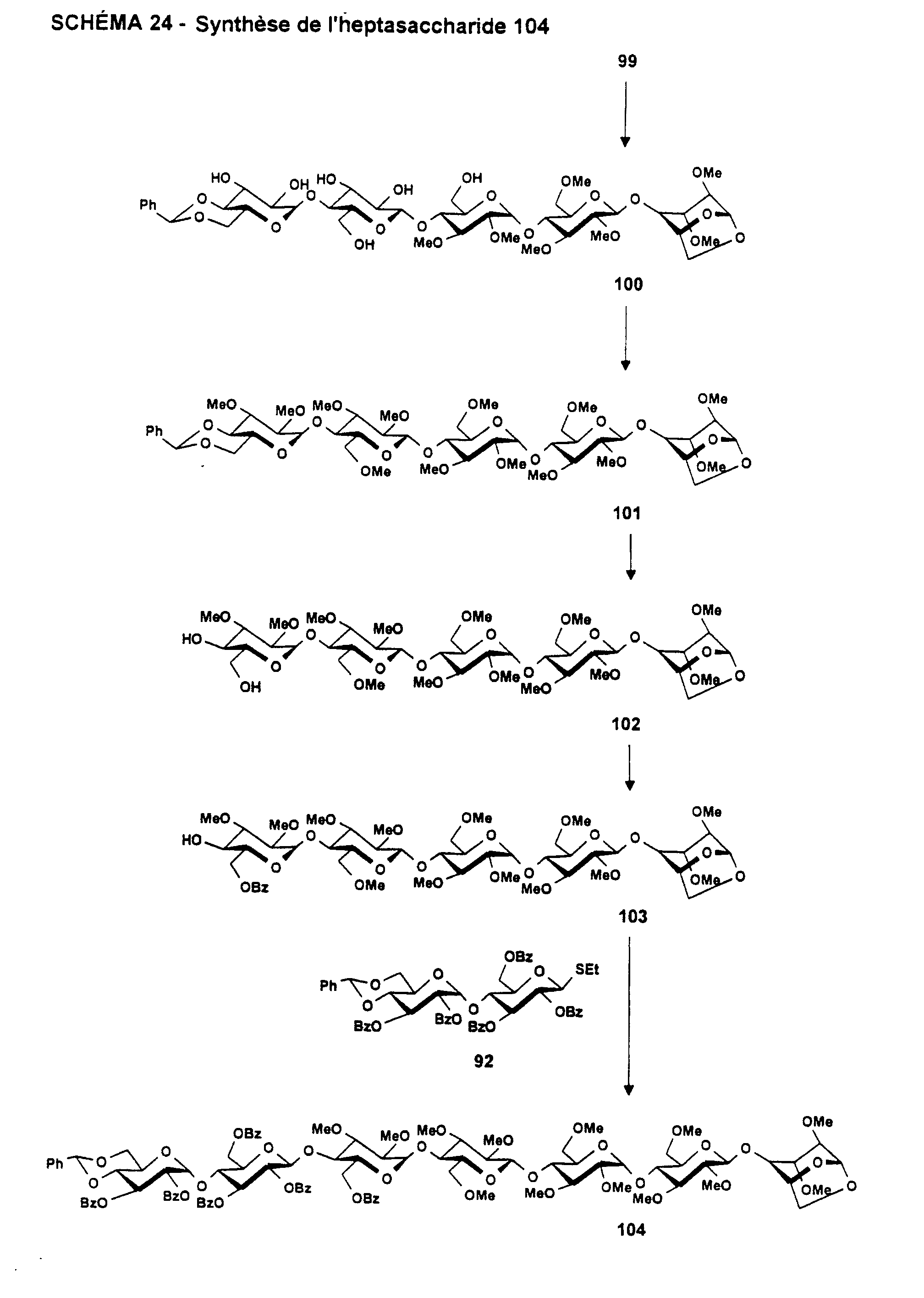

### PRÉPARATION 87

### O-(4,6-O-Benzylidène-α-D-glucopyranosyl)-(1→4)-O-(β-D-glucopyranosyl)-(1→4)-O-(2,3-di-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (100)

Le composé **99** est transformé en composé **100** selon le même mode opératoire que celui décrit pour la préparation du composé **95.**
CCM : Rf = 0,35, gel de silice, dichlorométhane/méthanol/ 9/1 v/v

### PRÉPARATION 88

### O-(4,6-O-Benzylidène-2,3-di-O-méthyl-α-D-glucopyranosyl)-(1→4)-O-(2,3,6-tri-O-méthyl-β-D-glucopyranosyl)-(1→4)-O-(2,3,6-tri-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (101)

Le composé **100** est transformé en composé **101** selon le même mode opératoire que celui décrit pour la préparation du composé **96**.
CCM : Rf = 0,50, gel de silice, dichlorométhane/méthanol/ 9/1 v/v

### PRÉPARATION 89

### O-(2,3-Di-O-méthyl-α-D-glucopyranosyl)-(1→4)-O-(2,3,6-tri-O-méthyl-β-D-glucopyranosyl)-(1→4)-O-(2,3,6-tri-O-méthyl-α-D-glucopyranosyl)-(1→4)-O-(2,3,6-tri-O-méthyl-β-D-glucopyranosyl)-(1→4)-1,6-anhydro-2,3-di-O-méfhyl-β-D-glucopyranose (102)

Le composé **101** est transformé en composé **102** selon le même mode opératoire que celui décrit pour la préparation du composé **97.**
CCM : Rf = 0,35, gel de silice, dichlorométhane/méthanol/ 9/1 v/v

### PRÉPARATION 90

### O-(6-O-Benzoyl-2,3-di-O-méthyl-α-D-glucopyranosyl)-(1→4)-O-(2,3,6-tri-O-méthyl-β-D-glucopyranosyl)-(1→4)-O-(2,3,6-tri-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (103)

Le composé **102** est transformé en composé **103** selon le même mode opératoire que celui décrit pour la préparation du composé **98.**
CCM : Rf = 0,40, gel de silice, toluène/acétate d'éthyle/éthanol 7,0/1,5/1,5 v/v/v

### PRÉPARATION 91

### O-(2,3-Di-O-benzoyl-4,6-O-benzylidène-α-D-glucopyranosyl)-(1→4)-O-(2,3,6-tri-O-benzoyl-β-D-glucopyranosyl)-(1→4)-O-(6-O-benzoyl-2,3-di-O-méthyl-α-D glucopyranosyl)-(1→4)-O-(2,3,6-tri-O-méthyl-β-D-glucopyranosyl)-(1→4)-O-(2,3,6-tri-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (104)

La réaction de couplage du composé **103** avec le disaccharide **2** est réalisée selon le mode opératoire décrit pour la préparation du composé **99.**
CCM : Rf = 0,40, gel de silice, toluène/acétate d'éthyle/éthanol 7,0/1,5/1,5 v/v/v

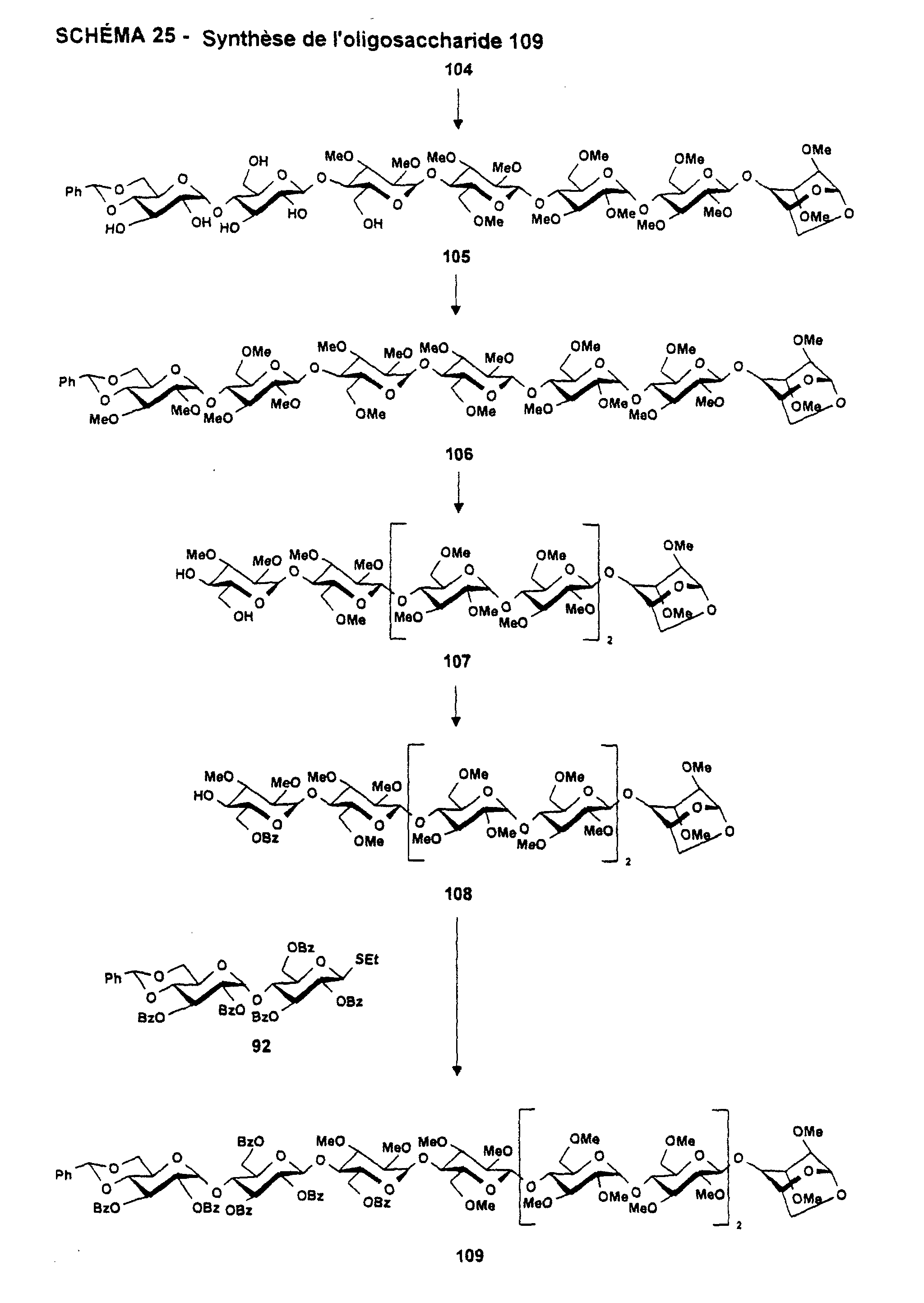

### PRÉPARATION 92

### O-(4,6-O-Benzylidène-α-D-glucopyranosyl)-(1→4)-O-(β-D-glucopyranosyl)-(1→4)-O-(2,3-di-O-méthyl-α-D-glucopyranosyl)-(1→4)-O-(2,3,6-tri-O-méthyl-β-D-glucopyranosyl)-(1→4)-O-(2,3,6-tri-O-méthyl-α-D-glucopyranosyl)-(1→4)-O-(2,3,6-tri-O-méthyl-β-D-glucopyranosyl)-(1→4)-1,6-anhydro-2,3-di-O-méthyl-β-D-glucopyranose (105)

Le composé **104** est transformé en composé **105** selon le même mode opératoire que celui décrit pour la préparation du composé **95**.
CCM : Rf = 0,60, gel de silice, dichlorométhane/méthanol 9/1 v/v

### PRÉPARATION 93

### O-(4,6-O-Benzylidène-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₂-1,6-anhydro-2,3-di-O-méthyl-β-D-glucopyranose (106)

Le composé **105** est transformé en composé **106** selon le même mode opératoire que celui décrit pour la préparation du composé **96**.
CCM : Rf = 0,70, gel de silice, dichlorométhane/méthanol 9/1 v/v

### PRÉPARATION 94

### O-(2,3-Di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,8-tri-O-méthyl-β-D-glucopyranosyl)-(1→4)]₂-1,6-anhydro-2,3-di-O-méthyl-β-D-glucopyranose (107)

Une solution du composé **106** (5,05 g, 2,0 mmol) dans de l'acide acétique à 80 % (50 ml) est agitée pendant 20 heures à 40° C. Le mélange est concentré sous vide et co-évaporé avec du toluène. Le résidu est dissous dans de l'acétate d'éthyle et extrait avec de l'eau. La phase aqueuse est extraite avec du dichlorométhane et la phase organique est séchée sur sulfate de magnésium, filtrée et évaporée à sec pour donner 2,68 g du composé **107.**
CCM : Rf = 0,50, gel de silice, dichlorométhane/méthanol 9/1 v/v

### PRÉPARATION 95

### O-(6-O-Benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₂-1,6-anhydro-2,3-di-O-méthyl-β-D-glucopyranose (108)

Le composé **107** est transformé en composé **108** selon le même mode opératoire que celui décrit pour la préparation du composé **98**.
CCM : Rf = 0,80, gel de silice, toluène/acétate d'éthyle/éthanol 7,0/1,5/1,5 v/v/v

### PRÉPARATION 96

### O-(2,3-Di-O-benzoyl-4,6-O-benzylidène-α-D-glucopyranosyl)-(1→4)-O-(2,3,6-tri-O-benzoyl-β-D-glucopyranosyl)-(1,4)-O-(6-O-benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₂-1,6-anhydro-2,3-di-O-méthyl-β-D-glucopyranose (109)

Un mélange du thioglycoside **92** (1,97 g, 2,0 mmol, 3,5 eq), d'heptasaccharide **108** (0,86 g, 0,57 mmol) et d'un tamis moléculaire 4Å en poudre dans le toluène (22 ml) est agité sous atmosphère d'azote pendant 15 minutes. On ajoute alors goutte à goutte, à température ambiante, une solution fraîchement préparée contenant de la N-iodosuccinimide (496 mg, 2,2 mmol) et de l'acide trifluorométhanesulfonique (0,808 mmol) dans du dichlorométhane/dioxane 1/1 v/v (12 ml). Après 10 minutes, le mélange réactionnel est filtré, dilué avec du dichlorométhane, extrait, lavé avec une solution de thiosulfate de sodium à 10 % et une solution d'hydrogénocarbonate de sodium à 10 %, séché sur sulfate de magnésium et concentré sous vide. Le produit brut est purifié par chromatographie sur une colonne de gel de silice pour fournir 1,09 g du composé **109**.
CCM : Rf = 0,80, gel de silice, toluène/acétate d'éthyle/éthanol 6/2/2 v/v/v

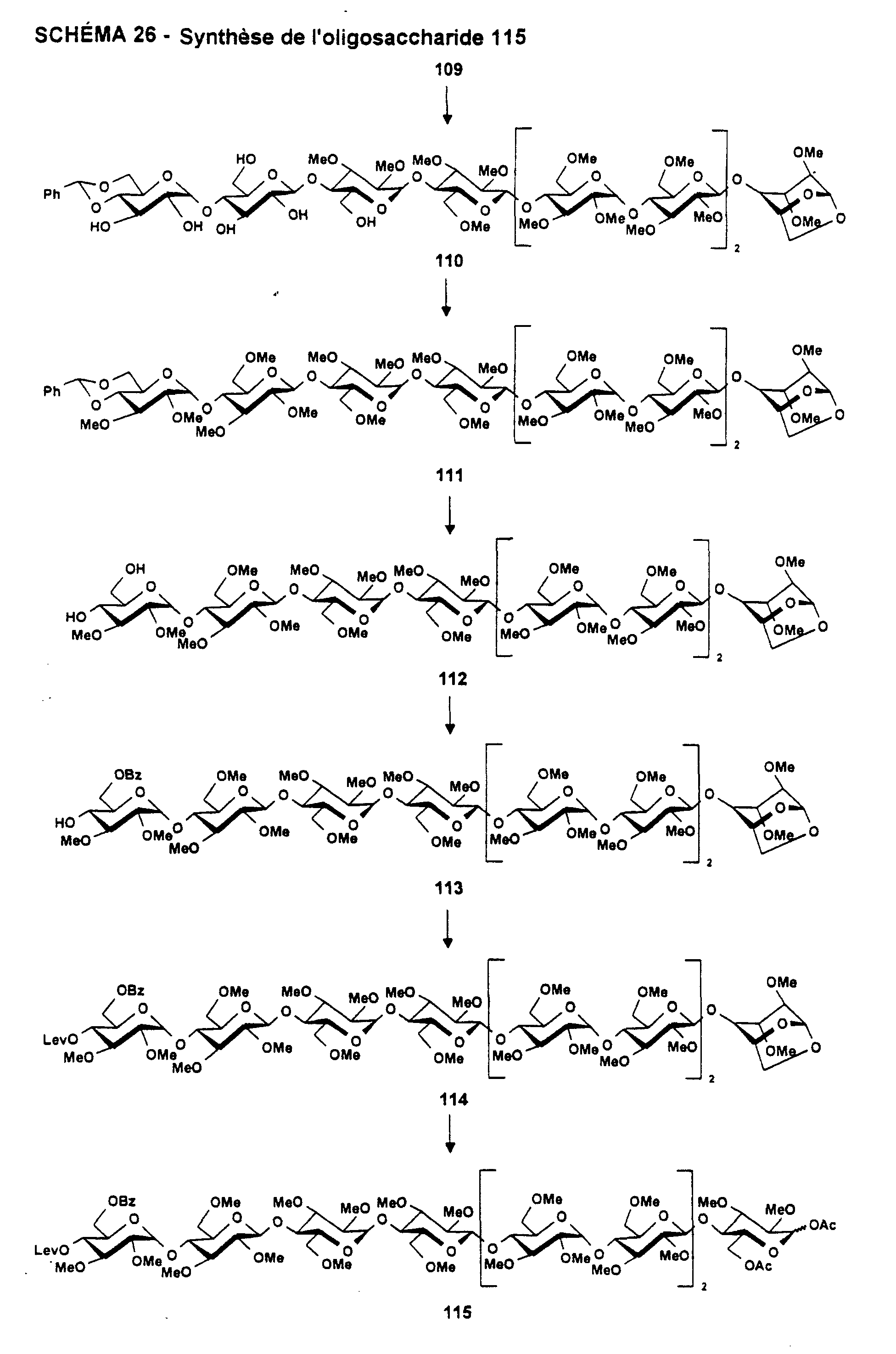

### PRÉPARATION 97

### O-(4,6-O-Benzylidène-α-D-glucopyranosyl)-(1→4)-O-(β-D-glucopyranosyl)-(1→4)-O-(2,3-di-O-méthyl-α-D-glucopyranosyl)-(1→4)-O-(2,3,6-tri-O-méthyl-β-D-glucopyranosyl)-[(1-4)-O-(2,3,6-tri-O-méthyl-α-D-glucopyranosyl)-(1→4)-O-(2,3,6-tri-O-méthyl-β-D-glucopyranosyl)]₂-(1→4)-1,6-anhydro-2,3-di-O-méthyl-β-D-glucopyranose (110)

Le composé **109** est transformé en composé **110** selon le même mode opératoire que celui décrit pour la préparation du composé **95**.
CCM : Rf = 0,25, gel de silice, toluène/acétate d'éthyle/éthanol 5,0/2,5/2,5 v/v/v

### PRÉPARATION 98

### O-(4,6-O-Benzylidène-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-anhydro-2,3-di-O-méthyl-β-D-glucopyranose (111)

Le composé **110** est transformé en composé **111** selon le même mode opératoire que celui décrit pour la préparation du composé **96.**
CCM : Rf = 0,50, gel de silice, toluène/acétate d'éthyle/éthanol 6/2/2 v/v/v

### PRÉPARATION 99

### O-(2,3-Di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-anhydro-2,3-di-O-méthyl-β-D-glucopyranose (112)

Le composé **111** est transformé en composé **112** selon le même mode opératoire que celui décrit pour la préparation du composé **97.**
CCM : Rf = 0,20, gel de silice, toluène/acétate d'éthyte/éthanol 6/2/2 v/v/v

### PRÉPARATION 100

### O-(6-O-Benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-anhydro-2,3-di-O-méthyl-β-D-glucopyranose (113)

Le composé **112** est transformé en composé **113** selon le même mode opératoire que celui décrit pour la préparation du composé **98**.
CCM : Rf = 0,20, gel de silice, toluène/acétate d'éthyle/éthanol 6/2/2 v/v/v

### PRÉPARATION 101

### O-(6-O-Benzoyl-4-O-lévulinyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-anhydro-2,3-di-O-méthyl-β-D-glucopyranose (114)

À une solution du composé **113** (320 mg, 0,167 mmol) dans du dioxane (1 ml), on ajoute du chlorhydrate de 1-(3-diméthylaminopropyl)-3-éthylcarbodiimide (48 mg, 0.25 mmol), de l'acide lévulinique (29 mg, 0,25 mmol) et de la diméthylaminopyridine (4 mg, 0,033 mmol). Le mélange réactionnel est agité pendant 3 heures à température ambiante sous atmosphère d'azote. On ajoute alors du dichlorométhane et de l'eau et après extraction, la phase organique est lavée à l'eau, séchée sur sulfate de magnésium, filtrée et concentrée. Le produit brut est purifié par chromatographie sur une colonne de gel de silice pour donner 312 mg du composé **114**.
CCM : Rf = 0,50, gel de silice, toluène/acétate d'éthyle/éthanol 6/2/2 v/v/v

### PRÉPARATION 102

### O-(6-O-Benzoyl-4-O-lévulinyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-1,6-di-O-acétyl-2,3-di-O-méthyl-α,β-D-glucopyranose (115)

Une solution du composé **114** (312 mg, 0,155 mmol) dans un mélange d'anhydride acétique (2,25 ml), d'acide acétique (50 µl) et d'acide trifluoroacétique (0,14 ml) est agitée pendant 4 heures à température ambiante. Après l'ajout de toluène (10 ml), le mélange est concentré et co-évaporé avec du toluène (3 fois 10 ml). Après chromatographie sur une colonne de gel de silice, 324 mg du composé **115** sont isolés.
CCM : Rf = 0,65, gel de silice, toluène/acétate d'éthyle/éthanol 6/2/2 v/v/v

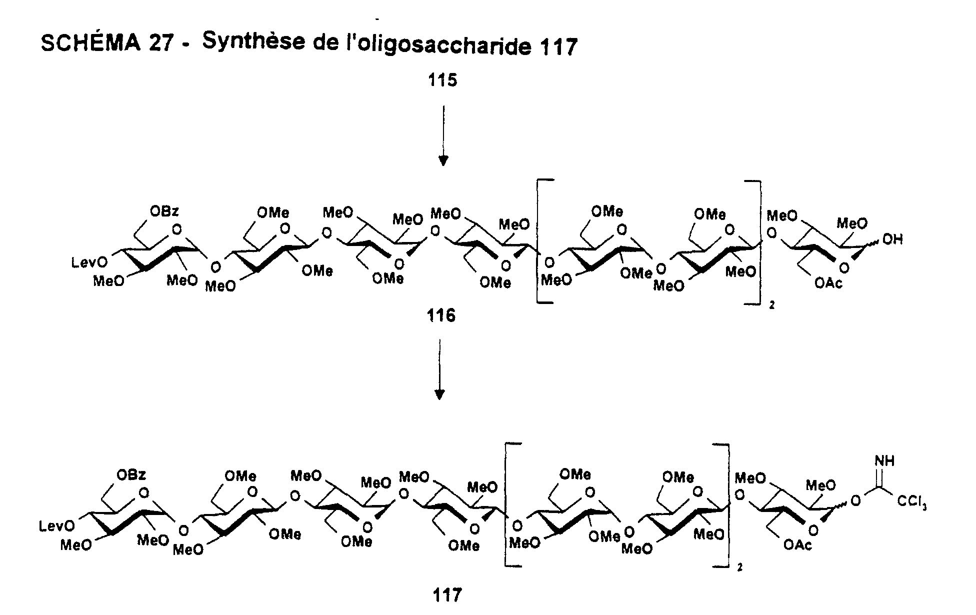

### PRÉPARATION 103

### O-(6-O-Benzoyl-4-O-lévulinyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-6-O-acétyl-2,3-di-O-méthyl-α,β-D-glucopyranose (116)

Une solution du composé **115** (324 mg, 0,153 mmol) et de morpholine (22,3 µl, 0,256 mmol) dans du toluène (2 ml) est agitée pendant 4 heures à 35° C. Ensuite, on ajoute à nouveau de la morpholine (22,3 µl) et le mélange réactionnel est agité pendant 20 heures à 35° C. Le mélange est refroidi rapidement avec de l'eau. Après extraction avec du dichlorométhane, la phase organique est successivement lavée avec de l'acide chlorhydrique 0,1N et de l'eau, séchée et évaporée jusqu'à sec. Après chromatographie sur une colonne de gel de silice, 280 mg du composé **116** sont isolés.
CCM : Rf = 0,45, gel de silice, toluène/acétate d'éthyle/éthanol 6/2/2 v/v/v

### PRÉPARATION 104

### O-(6-O-Benzoyl-4-O-lévulinyl-2,3-dl-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-6-O-acétyl-2,3-di-O-méthyl-α,β-D-glucopyranose trichloroacétimidate (117)

Du trichloroacétonitrile (39 µl, 0,39 mmol) et du carbonate de césium (4,7 mg) sont ajoutés à une solution du composé **116** (138 mg, 0,066 mmol) dans du dichlorométhane (1,5 ml). Après agitation pendant 2 heures, le mélange est filtré, concentré et le résidu est soumis à une chromatographie sur une colonne de gel de silice pour donner 152 mg de l'imidate **117.**
CCM : Rf = 0,35, gel de silice, toluène/acétate d'éthyle/éthanol 8/1/1 v/v/v

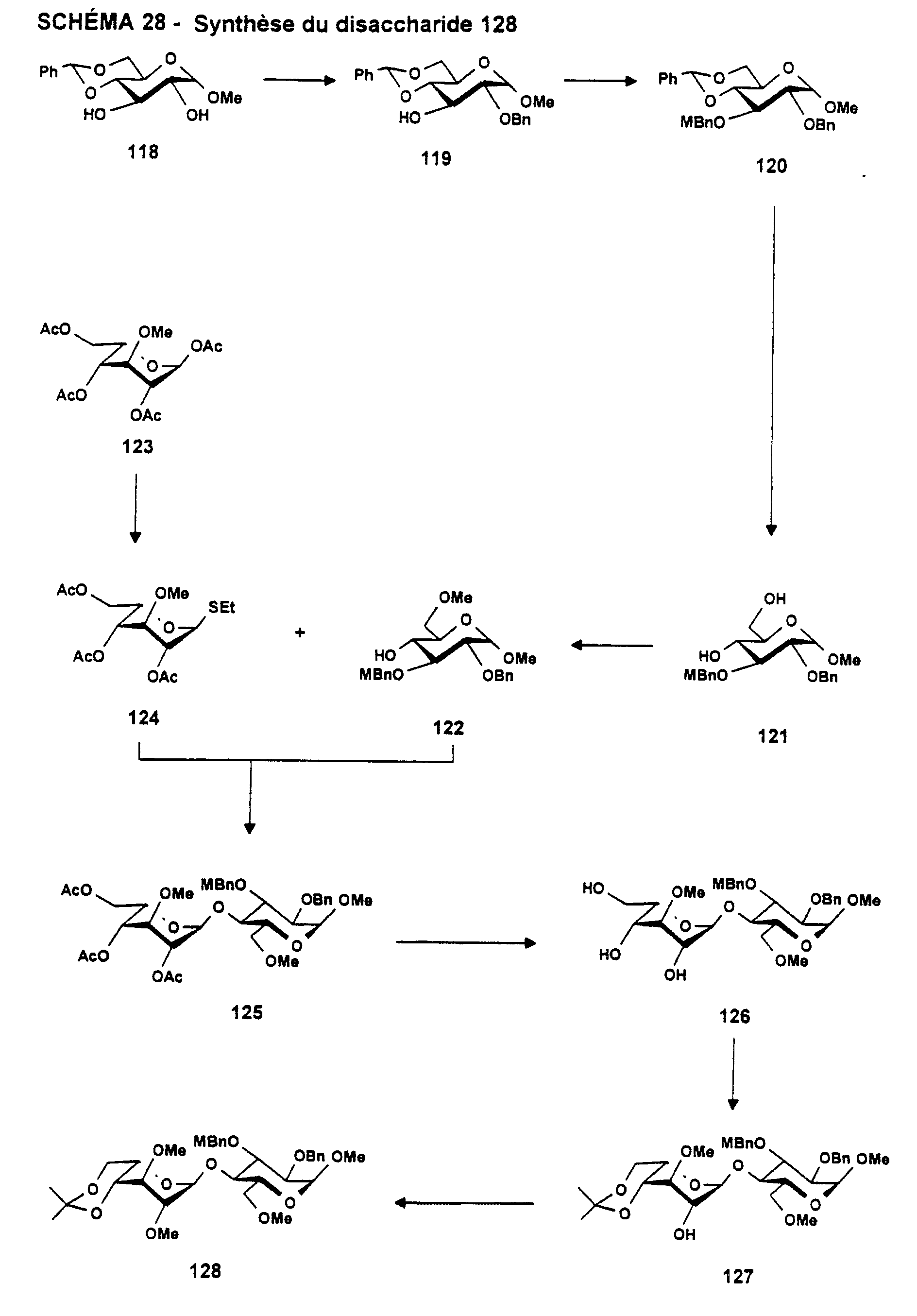

### PRÉPARATION 105

### Méthyl 2-O-benzyl-4,6-O-benzylidène-α-D-glucopyranoside (119)

Le composé **118** (60 g) (commercialement disponible) est dissous dans du diméthylformamide (858 ml) avec du bromure de benzyle (50,5 ml). Après refroidissement à 10° C, une solution aqueuse d'hydroxyde de sodium à 20 % est ajoutée goutte à goutte. Après agitation pendant 1 heure, la température est montée à 20° C et le mélange est agité pendant 20 heures supplémentaires. La solution est ensuite versée dans un mélange d'eau glacée et de toluène et extraite. La phase organique est concentrée et le produit brut est purifié par cristallisation pour donner 30,0 g du composé **119**.
CCM : Rf = 0,60, gel de silice, toluène/acétate d'éthyle 7/3 v/v

### PRÉPARATION 106

### Méthyl 2-O-benzyl-4, 6-O-benzylidène-3-O-p-méthoxybenzyl-α-D-glucopyranoside (120)

Le composé **119** (26,4 g) est dissous dans du diméthylformamide (211 ml) et refroidi à 5° C. De l'hydrure de sodium (2,5 g) est ajouté sous atmosphère d'azote. Puis on ajoute goutte à goutte du chlorure de 4-méthoxybenzyle (13,3 g) et le mélange est agité pendant 1 heure à température ambiante. Le mélange est dilué avec de l'acétate d'éthyle, lavé 2 fois avec de l'eau et concentré pour donner 40,7 g du composé **120** pur.
CCM : Rf = 0,80, gel de silice, toluène/acétate d'éthyle 7/3 v/v

### PRÉPARATION 107

### Méthyl 2-O-benzyl-3-O-p-méthoxybenzyl-α-D-glucopyranoside (121)

Le composé **120** (34,9 g) est dissous dans de l'acide acétique aqueux à 60 % et agité pendant 4 heures à 60° C. Le mélange est dilué avec du toluène et concentré. La purification par chromatographie sur une colonne de gel de silice permet d'obtenir 26,4 g du composé **121.**
CCM : Rf = 0,07, gel de silice, toluène/acétate d'éthyle 7/3 v/v

### PRÉPARATION 108

### Méthyl 2-O-benzyl-3-O-p-méthoxybenzyl-6-O-méthyl-α-D-glucopyranoside (122)

Le composé **121** (26,4 g) est dissous dans du dichlorométhane (263 ml) sous atmosphère d'azote. On ajoute à température ambiante du triméthyloxoniumtétrafluoroborate (11,6 g) et du 2,6-di-tert-butyl-4-méthylpyridine (17,4 g). Après 4 heures, le mélange est versé sur de l'eau glacée et extrait avec du dichlorométhane. La phase organique est lavée avec de l'hydrogénocarbonate de sodium et concentrée. La purification du produit brut par une chromatographie sur colonne de gel de silice permet d'obtenir 18,5 g du composé **122**.
CCM : Rf = 0,25, gel de silice, toluène/acétate d'éthyle 7/3 v/v

### PRÉPARATION 109

### Éthyl 2,4,6-tri-O-acétyl-3-O-méthyl-1-thio-α-L-idopyranose (124)

Le composé **123** (1,2,4,6-tétra-O-acétyl-3-O-méthyl-α-L-idopyranose) (Jaurand *et al.* Bio. Med. Chem. Lett. 1992, 2, 897-900) (48,4 g) est dissous dans du toluène (175 ml). Sous atmosphère d'azote, on ajoute de l'éthanethiol (20 ml) et de l'éthérate de trifluorure de bore dans le toluène, (134 ml). Après agitation pendant 1 heure, on ajoute de l'hydrogénocarbonate de sodium aqueux (400 ml) et le mélange est agité pendant encore une heure. Le mélange est ensuite versé dans de l'acétate d'éthyle. La phase organique est lavée deux fois à l'eau et concentrée. La purification par chromatographie sur colonne de gel de silice permet d'obtenir 29,6 g du composé **124**.
CCM : Rf = 0,45, gel de silice, toluène/acétate d'éthyle 6/4 v/v

### PRÉPARATION 110

### Méthyl O-(2,4,6-tri-O-acétyl-3-O-méthyl-α-L-idopyranosyl)-(1→4)-2-O-benzyl-3-O-p-méthoxybenzyl-6-O-méthyl-α-D-glucopyranoside (125)

Le composé **122** (17,5 g) et le composé **124** (28,2 g) sont dissous dans le toluène (525 ml) sous atmosphère d'azote. Après l'ajout d'un tamis moléculaire 4Å, la réaction est refroidie à - 20° C. Une solution de 0,1M de N-iodosuccinimide (17,4 g) fraîchement préparée et d'acide trifluorométhanesulfonique (1,38 ml) dans le dioxane/dichlorométhane 1/1 v/v est ajoutée goutte à goutte sous un flux continu d'azote. Après 10 minutes, le mélange réactionnel rouge est filtré et lavé successivement avec du thiosulfate de sodium aqueux et de l'hydrogénocarbonate de sodium aqueux. La phase organique est concentrée sous vide et 30,0 g du composé **125** sont isolés.
CCM : Rf = 0,45, gel de silice, dichlorométhane/acétate d'éthyle 8/2 v/v

### PRÉPARATION 111

### Méthyl O-(3-O-méthyl-α-L-idopyranosyl)-(1→4)-2-O-benzyl-3-O-p-méthoxybenzyl-6-O-méthyl-α-D-glucopyranoside (126)

Le composé **125** (30,0 g) est dissous dans 460 ml de méthanol/dioxane 1/1 v/v et on ajoute du *tert*-butylate de potassium. Après 15 minutes, le mélange est neutralisé avec une résine Dowex 50WX8H⁺ et concentré sous vide. La purification est réalisée par chromatographie sur colonne de gel de silice pour donner 17,4 g du composé **126**.
CCM : Rf = 0,25, gel de silice, dichlorométhane/méthanol 95/5 v/v

### PRÉPARATION 112

### Méthl O-(4,6-O-isopropylidène-3-O-méthyl-α-L-idopyranosyl)-(1→4)-2-O-benzyl-3-O-p-méthoxybenzyl-6-O-méthyl-α-D-glucopyranoside (127)

Sous atmosphère d'azote, le composé **126** (17,4 g) est dissous dans du diméthylformamide (77 ml). On ajoute du 2,2-diméthoxypropane (26 ml) et de l'acide p-toiuènesulfonique et le mélange est ensuite agité pendant 30 minutes. La dilution du mélange avec de l'hydrogénocarbonate de sodium aqueux puis son extraction avec de l'acétate d'éthyle permet d'obtenir 19,7 g du composé **127** après évaporation du solvant.
CCM : Rf = 0,45, gel de silice, dichlorométhane/méthanol 95/5 v/v

### PRÉPARATION 113

### Méthyl O-(4,6-O-isopropylidène-2,3-di-O-méthyl-α-L-idopyranosyl)-(1→4)-2-O-benzyl-3-O-p-méthoxybenzyl-6-O-méthyl-α-D-glucopyranoside (128)

Le composé **127** (18,5 g) est dissous dans du diméthylformamide (24,4 ml) et refroidi à 0° C. Sous atmosphère d'azote, on ajoute de l'hydrure de sodium (1,47 g ; dispersion dans l'huile 60 %) et de l'iodométhane (2,36 ml). Après une heure, l'excès d'hydrure de sodium est détruit avec du méthanol et le mélange est extrait avec du dichlorométhane et concentré pour donner 20,0 g du composé **128**.
CCM : Rf = 0,85, gel de silice, dichlorométhane/méthanol 95/5 v/v

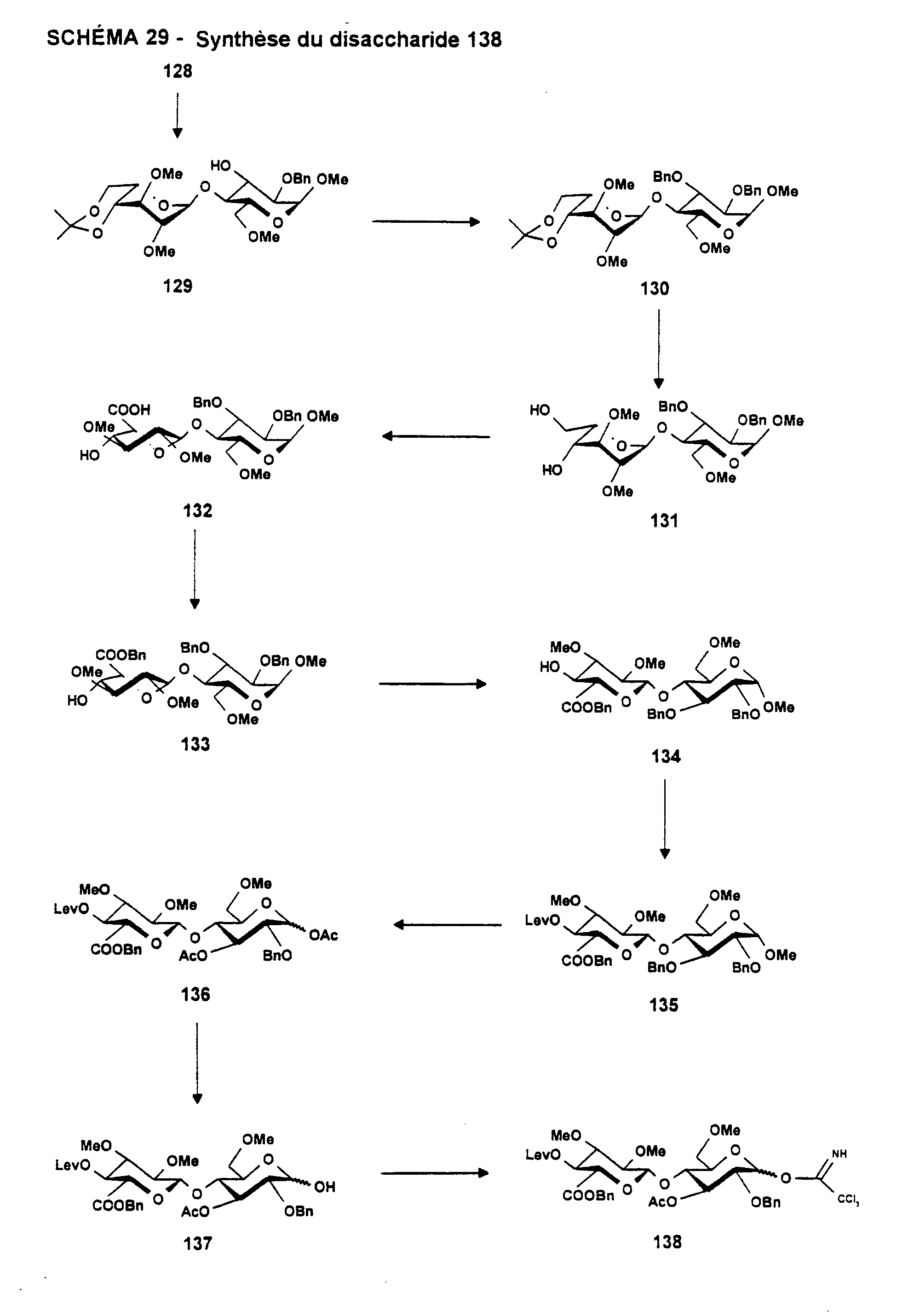

### PRÉPARATION 114

### Méthyl O-(4,6-O-isopropylidène-2,3-di-O-méthyl-α-L-idopyranosyl)-(1→4)-2-O-benzyl-6-O-méthyl-α-D-glucopyranoside (129)

Le composé **128** (18,4 g) est dissous dans du dichlorométhane (838 ml) et de l'eau (168 ml). On ajoute de la 2,3-dichloro-5,6-dicyano-1,4-benzoquinone (7,1 g) et le mélange est agité pendant 18 heures à 4° C. Le mélange est versé dans de l'hydrogénocarbonate de sodium aqueux et extrait au dichlorométhane. La concentration de la phase organique fournit 12,7 g du composé **129.**
CCM : Rf = 0,40, gel de silice, dichlorométhane/méthanol 95/5 v/v

### PRÉPARATION 115

### Méthyl O-(4,6-O-isopropylidène-2,3-di-O-méthyl-α-L-idopyranosyl)-(1→4)-2,3-di-O-benzyl-6-O-méthyl-α-D-glucopyranoside (130)

Le composé **129** (10,5 g) est dissous dans du diméthylformamide sec (178 ml) puis refroidi à 0° C sous atmosphère d'azote. On ajoute de l'hydrure de sodium (1,91 g ; dispersion dans l'huile 60 %) puis goutte à goutte du bromure de benzyle (3,3 ml).

Après 30 minutes, la réaction est terminée et l'excès d'hydrure de sodium est détruit avec du méthanol. On ajoute de l'eau et le mélange est extrait deux fois avec de l'acétate d'éthyle. L'évaporation du solvant permet d'obtenir 13,6 g du composé **130**.
CCM : Rf = 0,50, gel de silice, toluène/acétate d'éthyle 1/1 v/v

### PRÉPARATION 116

### Méthyl O-(2,3-di-O-méthyl-α-L-idopyranosyl)-(1→4)-2,3-di-O-benzyl-6-O-méthyl-α-D-glucopyranoside (131)

Le composé **130** est dissous dans de l'acide acétique/eau 77/33 (v/v) et agité pendant une nuit. Le mélange est co-évaporé deux fois avec du toluène et purifié par chromatographie sur colonne de gel de silice pour obtenir 11,5 g du composé **131**.
CCM : Rf = 0,09, gel de silice, toluène/acétate d'éthyl 1/1 v/v
Rf = 0,68, gel de silice, dichlorométhane/méthanol 9/1 v/v

### PRÉPARATION 117

### Méthyl O-(acide 2,3-di-O-méthyl-α-L-idopyranosyluronique)-(1→4)-2,3-di-O-benzyl-6-O-méthyl-α-D-glucopyranoside (132)

À une solution du composé **131** (11,6 g) dans du dichlomométhane (60 ml), on ajoute du 2,2,6,6-tétraméthyl-1-pipéridinyloxy (33 mg), une solution d'hydrogénocarbonate de sodium (40 ml), du bromure de potassium (218 mg) et du chlorure de tétrabutylammonium (289 mg). Le mélange est refroidi à 0° C et on ajoute pendant 15 minutes un mélange d'une solution saturée de chlorure de sodium (44 ml), d'une solution saturée d'hydrogénocarbonate de sodium (21,8 ml) et d'hypochlorite de sodium (1,3 M, 50 ml). Après agitation pendant 1 heure, le mélange est dilué avec de l'eau et extrait (3 fois) ,avec du dichlorométhane. La phase organique est lavée avec une solution aqueuse de chlorure de sodium, séchée sur sulfate de magnésium, filtrée et évaporée jusqu'à état sec pour donner 13,4 g du composé brut **132.**
CCM : Rf = 0,14, gel de silice, dichlorométhane/méthanol 9/1 v/v.

### PRÉPARATION 118

### Méthyl O-(benzyl 2,3-di-O-méthyl-α-L-idopyranosyluronate)-(1→4)-2,3-di-O-benzyl-6-O-méthyl-α-D-glucopyranoside (133)

Sous atmosphère d'azote, le composé **132** est dissous dans du diméthylformamide (110 ml). On ajoute de l'hydrogénocarbonate de potassium (6,7 g) et du bromure de benzyle (10,7 ml) et le mélange est agité pendant 90 minutes. On ajoute de l'acétate d'éthyle et de l'eau puis après extraction, la phase organique est concentrée. La purification par chromatographie sur colonne de gel de silice permet d'obtenir 9,9 g du composé **133**.
CCM : Rf = 0,43, gel de silice, toluène/acétate d'éthyle 4/6 v/v

### PRÉPARATION 119

### Méthyl O-(benzyl 2,3-di-O-méthyl-β-D-glucopyranosyluronate)-(1→4)-2,3-di-O-benzyl-6-O-méthyl-α-D-glucopyranoside (134)

Le composé **133** (9,9 g) est dissous dans 300 ml de méthanol et chauffé à reflux sous atmosphère d'azote. On ajoute goutte à goutte une solution 1M de méthyiate de sodium dans du méthanol (65,2 ml) et le mélange est agité et chauffé à reflux pendant 3 heures. Le mélange est ensuite refroidi à température ambiante, on ajoute de l'hydroxyde de sodium 1N (22,2 ml) et le mélange réactionnel est agité pendant encore 90 minutes. Après neutralisation avec la résine Dowex 50WX8H⁻ et filtration, le mélange est concentré. Le produit pur est dissous dans du diméthylformamide (192 ml) et un tamis moléculaire est ajouté sous atmosphère d'azote. On ajoute de l'hydrogénocarbonate de potassium (3,2 g) et du bromure de benzyle (4,8 ml) et le mélange est agité pendant 5 heures. Après addition d'acétate d'éthyle et d'eau, extraction et séparation des deux phases, la phase organique est concentrée. Le produit brut est purifié par chromatographie sur colonne de gel de silice pour donner 6,19 g du composé **134** et 1,88 g du composé **133** de départ.
CCM : Rf = 0,55, gel de silice, toluène/acétate d'éthyle 4/6 v/v

### PRÉPARATION 120

### Méthyl O-(benzyl 4-O-lévulinyl-2,3-di-O-méthyl-β-D-glucopyranosyluronate)-(1→4)-2,3-di-O-benzyl-6-O-méthyl-α-D-glucopyranoside (135)

Le composé **134** (6,2 g) est dissous dans 40 ml de dioxane. On ajoute de l'acide lévulinique (2,1 g), du dicyclohexylcarbodiimide (3,75 g) et de la 4-diméthylaminopyridine (0,2 g) et le mélange est agité pendant 2 heures sous atmosphère d'azote. On ajoute de l'éther diéthylique (95 ml) et de précipité est filtré.
Le filtrat est lavé avec de l'hydrogénosulfate de potassium aqueux, séché sur sulfate de magnésium, filtré et concentré. La cristallisation dans éther/heptane permet d'obtenir 6,2 g du composé **135.**
CCM : Rf = 0,26, gel de silice, dichlorométhane/acétone 95/5 v/v

### PRÉPARATION 121

### O-(Benzyl 4-O-lévulinyl-2,3-di-O-méthyl-β-D-glucopyranosyluronate)-(1→4)-1,3-di-O-acétyl-2-O-benzyl-6-O-méthyl-α,β-D-glucopyranose (136)

Le composé **135** (6,1 g) est dissous dans de l'anhydride acétique (256 ml) sous atmosphère d'azote et refroidi à - 20° C. Un mélange d'acide sulfurique (4,9 ml) dans de l'anhydride acétique (49 ml) est ajouté goutte à goutte pendant 30 minutes. Après 60 minutes, on ajoute de l'acétate de sodium jusqu'à l'obtention d'un mélange ayant un pH neutre. On ajoute alors de l'acétate d'éthyle et de l'eau et la phase organique est concentrée. La purification par chromatographie sur colonne de gel de silice permet d'obtenir 4,2 g du composé **136.**
CCM : Rf = 0,24, gel de silice, dichlorométhane/acétate d'éthyle 8/2 v/v

### PRÉPARATION 122

### O-(Benzyl 4-O-lévulinyl-2,3-di-O-méthyl-β-D-glucopyranosyluronate)-(1→4)-3-O-acétyl-2-O-benzyl-6-O-méthyl-α,β-D-glucopyranose (137)

Le composé **136** (4,2 g) est dissous dans du tétrahydrofurane (42 ml) et on ajoute de la pipéridine (4,1 ml). Le mélange est agité pendant la nuit à température ambiante. On ajoute de l'acétate d'éthyle et le mélange est lavé avec de l'acide chlorhydrique 0,5 N. La phase organique est concentrée et le résidu purifié par chromatographie sur une colonne de gel de silice pour donner 3,2 g du composé **137.**
CCM : Rf = 0,33, gel de silice, dichlorométhane/acétate d'éthyle 1/1 v/v

### PRÉPARATION 123

### O-(Benzyl 4-O-lévulinyl-2,3-di-O-méthyl-β-D-glucopyranosyluronate)-(1→4)-3-O-acétyl-2-O-benzyl-6-O-méthyl-α-glucopyranose trichloroacétimidate (138)

Le composé **137** (1,59 g) est dissous dans du dichlorométhane sec sous atmosphère d'azote. On ajoute du trichloroacétonitrite (1,1 ml) et du carbonate de césium (72 mg) et le mélange est agité pendant 1 heure. Le carbonate de césium est filtré et le filtrat est concentré. La purification par chromatographie sur colonne de gel de silice permet d'obtenir 1,57 g du composé **138.**
CCM : Rf = 0,60, gel de silice, toluéne/actétate d'éthyle 3/7 v/v

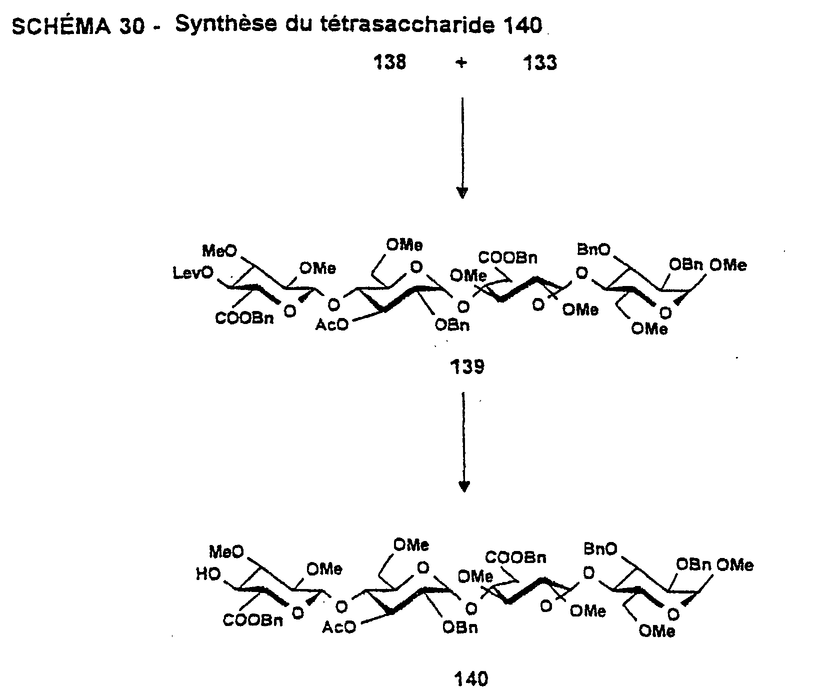

### PRÉPARATION 124

### Méthyl O-(benzyl 4-O-lévulinyl-2,3-di-O-méthyl-β-D-glucopyranosyluronate)-(1→4)-O-(3-O-acétyl-2-O-benzyl-6-O-méthyl-α-D-glucopyranosyl)-(1→4)-O-(benzyl 2,3-di-O-méthyl-α-L-idopyranosyluronate)-(1→4)-2,3-di-O-benzyl-6-O-méthyl-α-D-glucopyranoside (139)

Un mélange du composé **133** (300 mg) et du composé **138** (455,6 mg) est co-évaporé avec du toluène et dissous dans du dichlorométhane (6 ml) sous atmosphère d'azote. Après addition d'un tamis moléculaire 4Å, le mélange est refroidi à - 20° C. Après ' agitation pendant 20 minutes, on ajoute du trifluorométhane sulfonate de triméthylsilyle (15 mol % par rapport au composé **138**). Après 10 minutes, le mélange est arrêté avec de l'hydrogénocarbonate de sodium aqueux. Après filtration du tamis moléculaire, le filtrat est dilué avec du dichlorométhane, lavé avec de l'eau, concentré et purifié par chromatographie sur colonne de gel de silice pour donner 560 mg du composé **139.**
CCM : Rf = 0,50, gel de silice, toluène/acétate d'éthyle 3/7 v/v

### PRÉPARATION 125

### Méthyl O-(benzyl 2,3-di-O-méthyl-β-D-glucopyranosyluronate)-(1→4)-O-(3-O-acétyl-2-O-benzyl-6-O-méthyl-α-D-glucopyranosyl)-(1→4)-O-(benzyl 2,3-di-O-méthyl-α-L-idopyranosyluronate)-(1→4)-2,3-di-O-benzyl-6-O-méthyl-α-D-glucopyranoside (140)

Le composé **139** (532,6 mg) est dissous dans la pyridine (1,9 ml) et on ajoute à température ambiante un mélange d'acide acétique (2,4 ml), d'hydrate d'hydrazine (0,3 ml) dans la pyridine (1,9 ml). Après agitation pendant 9 minutes, on ajoute du dichlorométhane et de l'eau. La phase organique est séparée et lavée successivement avec de l'acide chlorhydrique 0,1 N, de l'hydrogénocarbonate de sodium aqueux et de l'eau. La phase organique est concentrée et purifiée par chromatographie sur colonne de gel de silice pour donner 451 mg du composé **140.**
CCM : Rf = 0,45, gel de silice, toluène/acétate d'éthyle 3/7 v/v

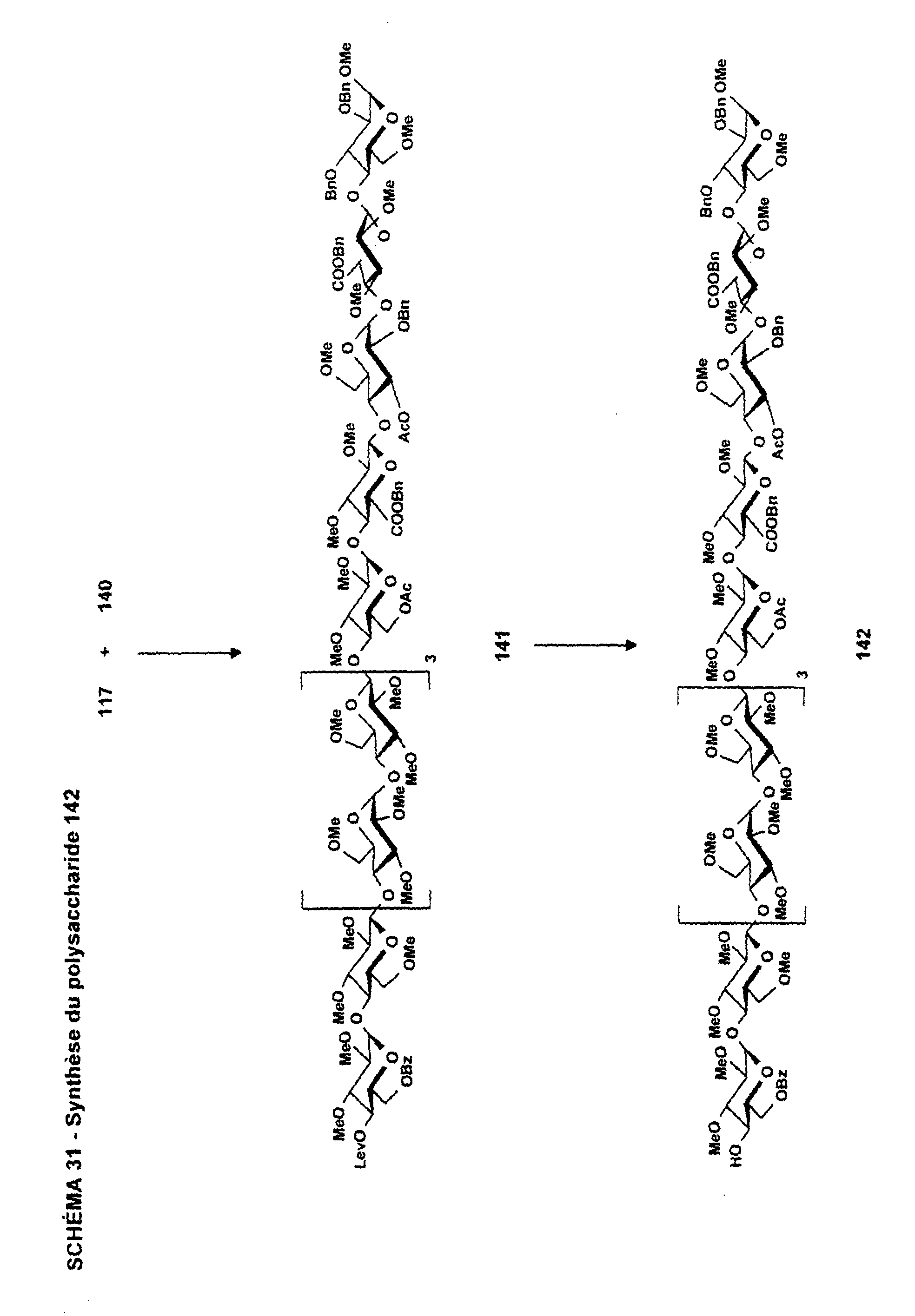

### PRÉPARATION 126

### Méthyl O-(6-O-benzoyl-4-O-lévulinyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(6-O-acétyl-2,3-di-O-méthyl-α-D-glucopyranosyl)-(1→4)-O-(benzyl 2,3-di-O-méthyl-β-D-glucopyranosyluronate)-(1→4)-O-(3-O-acétyl-2-O-benzyl-6-O-méthyl-α-D-glucopyranosyl)-(1→4)-O-(benzyl 2,3-di-O-méthyl-α-L-idopyranosyluronate)-(1→4)-2,3-di-O-benzyl-6-O-méthyl-α-D-glucopyranoside (141)

Un mélange du composé **117** (144 mg, 0,064 mmol) et du composé **140** (76 g, 0,058 mmol) est co-évaporé avec du toluène et dissous dans du dichlorométhane/éther diéthylique ½ v/v (3,0 ml). On ajoute un tamis moléculaire 4Å (140 mg) sous atmosphère d'azote et le mélange est refroidi à 0° C. On ajoute du trifluorométhanesulfonate de *tert*-butyldiméthylsilyle (128 µl d'une solution 0,1 molaire dans le dichlorométhane) et après 15 minutes, le mélange est arrêté avec une solution d'hydrogénocarbonate de sodium. Après extraction avec de l'eau et du dichlorométhane, la phase organique est séchée et concentrée. Le produit est d'abord purifié par une chromatographie sur Sephadex LH 20 (dichlorométhane/méthanol 1/1 v/v) suivie par une chromatographie sur colonne de gel de silice pour donner 124 mg du composé **141** en ratio α/β de 8/2.
CCM : Rf = 0,60, gel de silice, toluène/acétone 1/1 v/v

### PRÉPARATION 127

### Méthyl O-(6-O-benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(6-O-acétyl-2,3-di-O-méthyl-α-D-glucopyranosyl)-(1→4)-O-(benzyl 2,3-di-O-méthyl-β-D-glucopyranosyluronate)-(1→4)-O-(3-O-acétyl-2-O-benzyl-6-O-méthyl-α-D-glucopyranosyl)-(1→4)-O-(benzyl 2,3-di-O-méthyl-α-L-idopyranosyluronate)-(1→4)-2,3-di-O-benzyl-6-O-méthyl-α-D-glucopyranoside (142)

Le composé **141** est transformé en composé **142** selon le mode opératoire décrit pour la préparation du composé **140.**

Le composé **142** est isolé comme un mélange α/β de 8/2.
CCM : Rf = 0,45, gel de silice, toluène/acétone 1/1 v/v

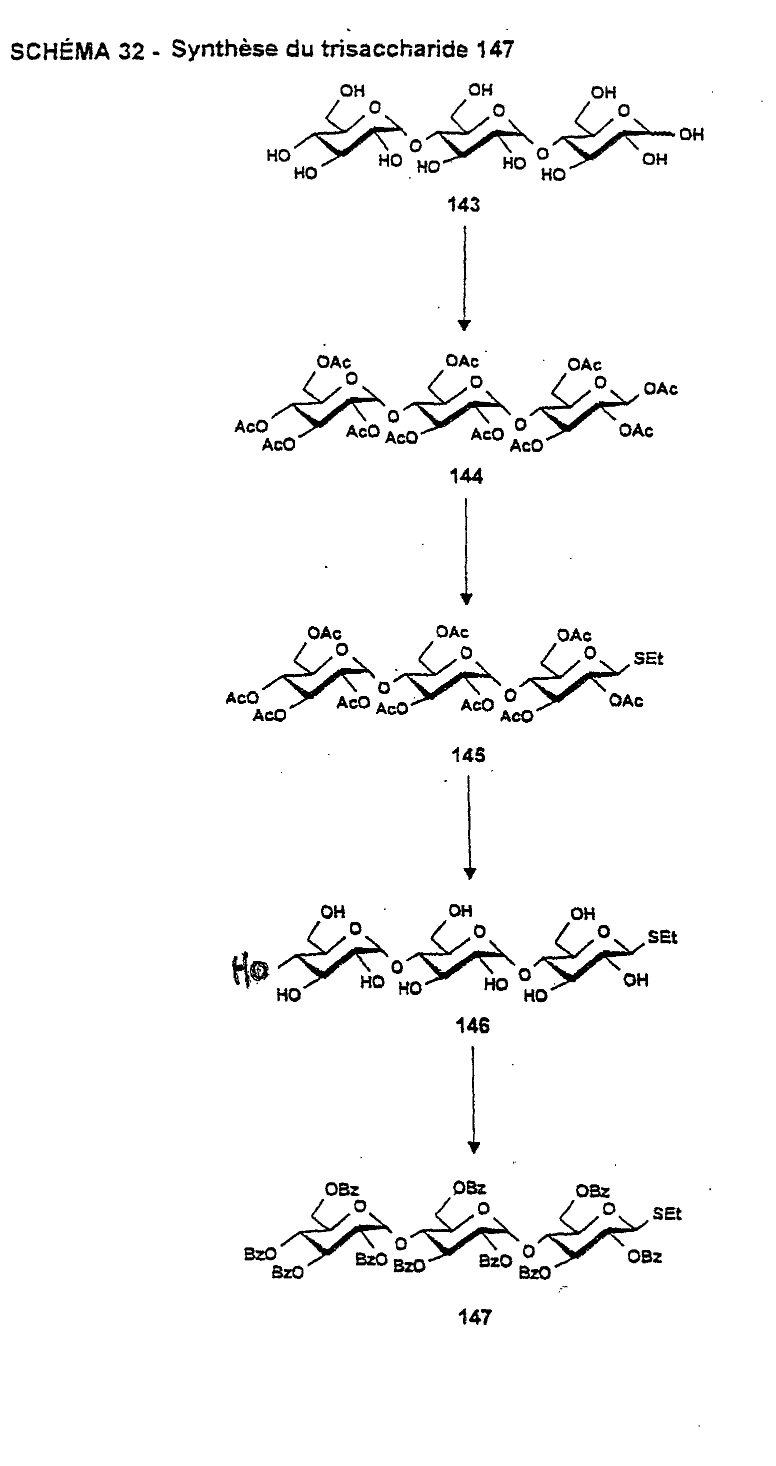

### PRÉPARATION 128

### O-(2,3,4,6-Tétra-O-acétyl-α-D-glucopyranosyl)-(1→4)-O-(2,3,6-tri-O-acétyl-α-D-glucopyranosyl)-(1→4)-1,2,3,6-tétra-O-acétyl-β-D-glucopyranose (144)

On ajoute par petites doses du maltotriose (7 g, 13,9 mmol) (commercialement disponible) à une suspension d'acétate de sodium (7 g, 85 mmol) dans de l'anhydride acétique (70 ml) à 155° C. Après 15 minutes, la solution claire est refroidie et arrêtée avec de l'eau glacée (700 ml). Après extraction avec de l'acétate d'éthyle, la phase organique est lavée à l'eau, séchée sur sulfate de magnésium, filtrée et concentrée pour donner 13,1 g du composé **144**.
CCM : Rf = 0,53, gel de silice, dichlorométhane/acétate d'éthyle 7/3 v/v

### PRÉPARATION 129

### Éthyl O-(2,3,4,6-tétra-O-acétyl-α-D-glucopyranosyl)-(1→4)-O-(2,3,6-tri-O-acétyl-α-D-glucopyranosyl)-(1→4)-2,3,6-tri-O-acétyl-1-thio-β-D-glucopyranoside (145)

Le composé **144** (13 g, 13,5 mmol) est dissous dans du toluène (80 ml). On ajoute sous atmosphère d'azote de l'éthanethiol (1,97 ml, 26,9 mmol) et du diéthylétherate trifluorure de bore (13,7 mi d'une solution d'une mole de toluène). Après 60 heures d'agitation, le mélange est dilué avec de l'eau et du dichlorométhane. Après extraction, la phase organique est lavée avec une solution à 10 % d'hydrogénocarbonate de sodium et de l'eau, séchée, filtrée et concentrée. Le produit brut est purifié par chromatographie sur une colonne de gel de silice pour donner 8,6 g du composé **145**.
CCM : Rf = 0,60, gel de silice, dichlorométhane/acétate d'éthyle 7/3 v/v

### PRÉPARATION 130

### Éthyl O-(α-D-glucopyranosyl)-(1→4)-O-(α-D-glucopyranosyl)-(1→4)-1-thio-β-D-glucopyranoside (146)

Le composé **145** est transformé en composé **146** selon le mode opératoire décrit pour la préparation du composé **95**.
CCM : Rf = 0,80, gel de silice, acétate d'éthyle/pyridine/acide acétique/eau 13/7/1,6/4 v/v/v/v

### PRÉPARATION 131

### Éthyl O-(2,3,4,6-tétra-O-benzoyl-α-D-glucopyranosyl)-(1→4)-O-(2,3,6-tri-O-benzoyl-α-D-glucopyranosyl)-(1→4)-2,3,6-tri-O-benzoyl-1-thio-β-D-glucopyranoside (147)

Le composé **146** est transformé en composé **147** selon le mode opératoire décrit pour la préparation du composé **92**.
CCM : Rf = 0,50, gel de silice, toluène/acétate d'éthyle 9/1 v/v

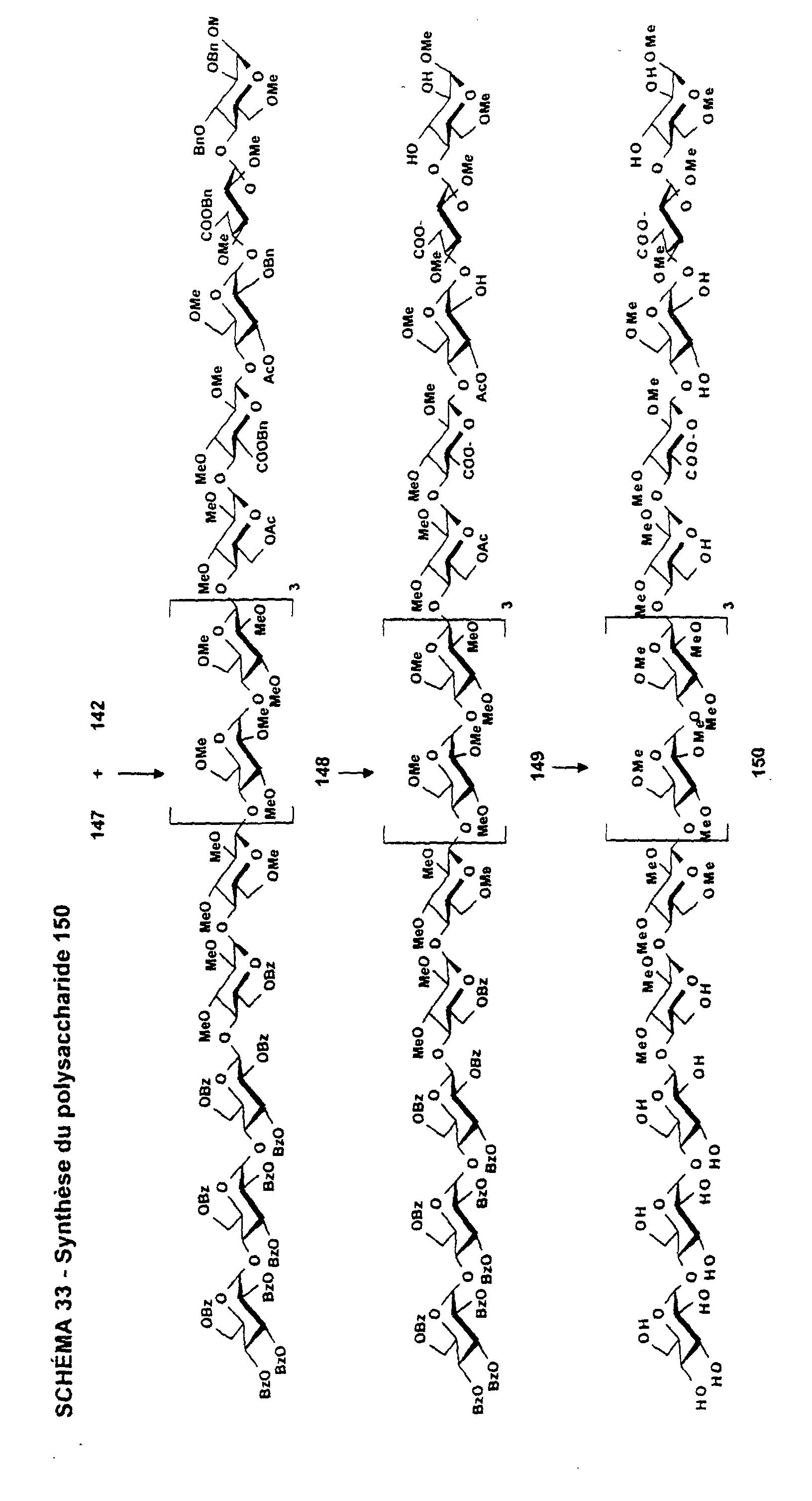

### PRÉPARATION 132

### Méthyl O-(2,3,4,6-tétra-O-benzoyl-α-D-glucopyranosyl)-(1→4)-O-(2,3,6-tri-O-benzoyl-α-D-glucopyranosyl)-(1→4)-O-(2,3,6-tri-O-benzoyl-β-D-glucopyranosyl)-(1→4)-O-(6-O-benzoyl-2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(6-O-acétyl-2,3-di-O-méthyl-α-D-glucopyranosyl)-(1→4)-O-(benzyl 2,3-di-O-méthyl-β-D-glucopyranosyluronate)-(1→4)-O-(3-O-acétyl-2-O-benzyl-6-O-méthyl-α-D glucopyranosyl)-(14)-O-(benzyl 2,3-di-O-méthyl-α-L-idopyranosyluronate)-(1→4)-2,3-di-O-benzyl-6-O-méthyl-α-D-glucopyranoside (148)

Le thioglycoside **147** (105 mg, 0,066 mmol) et l'accepteur **142** (55 mg, 0,017 mmol), (α/β de 8/2) sont couplés selon le mode opératoire décrit pour le composé **109.** Le produit est d'abord purifié par une chromatographie sur Sephadex LH 20 (dichlorométhane/méthanol 1/1) suivie par une chromatographie sur colonne de gel de silice (éther diéthylique/acétate d'éthyle/éthanol 9/0,5/0,5 v/v/v) pour donner 49 mg du composé **148.**
CCM : Rf = 0,30, gel de silice, éther diéthylique/acétate d'éthyle/éthanol 85/7,5/7,5 v/v/v

### PRÉPARATION 133

### Méthyl O-(2,3,4,6-tétra-O-benzoyl-α-D-glucopyranosyl)-(1→4)-O-(2,3,6-tri-O-benzoyl-α-D-glucopyranosyl)-(1→4)-O-(2,3,6-tri-O-benzoyl-β-D-glucopyranosyl)-(1→4)-O-(6-O-benzoyl-2,3-di-O-méthyl-α-D-glucopyranosyl)-(1→4)-O-(2,3,6-tri-O-méthyl-β-D-glucopyranosyl)-(1→4)-fO-(2,3,6-tri-O-méthyl-α-D-glucopyranosyl)-(1→4)-O-(2,3,6-tri-O-méthyl-β-D-glucopyranosyl)-(1→4)]₃-O-(6-O-acétyl-2,3-di-O-méthyl-α-D-glucopyranosyl)-(1→4)-O-(acide 2,3-di-O-méthyl-β-D-glucopyranosyluronique)-(1→4)-O-(3-O-acétyl-6-O-méthyl-α-D-glucopyranosyl)-(1→4)-O-(acide 2,3-di-O-méthyl-α-L-idopyranosyluronique)-(1→4)-6-O-méthyl-α-D-glucopyranoside (149)

Une solution du composé **148** (47 mg, 0,01 mmol) dans de l'acétate d'éthyle (10 ml) est agitée sous atmosphère d'azote en présence de palladium sur charbon 10 % (90 % w/w par rapport au composé **148**) pendant 3 heures et filtrée. Le filtrat est concentré pour donner 42 mg du composé **149.**
CCM : Rf = 0,35, gel de silice, acétate d'éthyle/pyridine/acide acétique/eau 20/7/1,6/4 v/v/v/v

### PRÉPARATION 134

### Méthyl O-(α-D-glucopyranosyl)-(1→4)-O-(α-D-glucopyranosyl)-(1→4)-O-(β-D-glucopyranosyl)-(1→4)-O-(2,3-di-O-méthyl-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(2,3-di-O-méthyl-α-D-glucopyranosyl)-(1→4)-O-(acide 2,3-di-O-méthyl-β-D-glucopyranosyluronique)-(1→4)-O-(6-O-méthyl-α-D-glucopyranosyl)-(1→4)-O-(acide 2,3-di-O-méthyl-α-L-idopyranosyluronique)-(1→4)-6-O-méthyl-α-D-glucopyranoside (150)

Un mélange de méthanol (0,22 ml) et d'une solution de 0,66 N d'hydroxyde de sodium (0,66 ml) est ajouté au composé **149** (41 mg, 0,01 mmol) puis agité pendant 20 heures à température ambiante. Le mélange est dilué avec de l'eau et acidifié avec

une solution d'acide chlorhydrique 0,5 N afin d'obtenir un pH de 6,5. Après concentration, le produit pur est désalé sur une colonne de Sephadex G-25, en utilisant de l'eau/acétonitrile 9/1 v/v. Les fractions hexadécasaccharidiques sont combinées et lyophilisées pour donner 26 mg du composé **150** comme une poudre blanche amorphe.
CCM : Rf = 0,35, gel de silice, acétate d'éthyle/pyridine/acide acétique/eau 8/7/1,6/4 v/v/v/v

### PRÉPARATION 135

### 6-O-Tert-butyldiméthylsilyl-1,2-O-isopropylidène-3-O-méthyl-α-D-glucofuranose (152)

On reprend le diol **151** (10 g, 42,7 mmol) dans du dichlorométhane anhydre (100 ml) et on ajoute du chlorure de tert-butyldiméthylsilyle (7,1 g 47,3 mmol) et de l'imidazole (5,8 g, 85,3 mmol). Le mélange réactionnel est agité à température ambiante. Après 2 heures le mélange est dilué dans du dichlorométhane et lavé à l'eau. La phase organique est séchée sur sulfate de magnésium, concentrée et le résidu est purifié par chromatographie sur une colonne de gel de silice (acétate d'éthyle/cyclohexane 1/9 vlv) afin d'obtenir le produit désiré **152** (11,9 g 80 %) sous forme d'un sirop. [α]_{D} - 34° (c 1,9, CHCl₃).

### PRÉPARATION 136

### 6-O-Tert-butyldiméthylsilyl-1,2-O-isopropylidène-3-O-méthyl-5-C-vinyl-α-D-glucofuranose (154)

On ajoute à - 78° C dans du dichlorométhane anhydre (40 ml) du chlorure d'oxalyle (3,2 ml, 36,8 mmol) et du diméthylsulfoxyde (5,2 ml, 73,4 mmol) et on agite pendant 30 minutes. Ensuite, le composé **152** (6,4 g, 18,4 mmol) est ajouté et on agite le mélange pendant encore 1 heure. Puis, on ajoute de la triéthylamine (15,3 ml, 110,0 mmol) et après 30 minutes, le mélange réactionnel est dilué dans du dichlorométhane. Un traitement classique permet d'obtenir le composé 5-ulose (**153**) qui est directement utilisé pour la réaction suivante. La cétone **153** brut est reprise dans du tétrahydrofurane anhydre (100 ml) et on ajoute une solution 1M de bromure de vinyl magnésium dans le tétrahydrofurane (28 ml, 27,6 mmol) à O° C. Après 1 heure, le mélange réactionnel est dilué non pas avec du chlorure d'ammonium et lavé à l'eau. La phase organique est séchée sur sulfate de magnésium, concentrée et le résidu est purifié par chromatographie sur une colonne de gel de silice (acétate d'éthyle/cyclohexane 1/9 v/v) afin d'obtenir le composé désiré **154** (70 %, 4,8 g) sous forme d'un sirop.
[α]_{D} - 40° (c 1,3, CHCl₃).
Anal. Calculée : C, 57,72, H, 9,15. Trouvé : C, 57,77, H, 9,23.

### PRÉPARATION 137

### 1,2,4,6-Tetra-O-acétyl-3-O-méthyl-5-C-vinyl-β-D-glucopyranose (156)

Le composé **154** (3,5 g, 9,4 mmol) est repris dans l'eau (50 ml) ; on y ajoute de la résine IR-120 (1 g) et on chauffe à 80° C pendant 6 heures. La résine est filtrée et le filtrat est concentré. le produit brut **155** est acétylé en utilisant de l'anhydride acétique (12 ml) et de la pyridine (13 ml). L'excès l'anhydride acétique est détruit avec du méthanol et les solvants sont concentrés. Le résidu est extrait avec de l'eau et du dichlorométhane. La phase organique est séchée sur sulfate de magnésium, concentrée et après purification par chromatographie sur une colonne de silice (acétate d'éthyle/cyclohexane 3/2 v/v), le composé tétra-acétate **156** est obtenu sous forme d'un solide (75 %, 2,7 g). Pf 50° C.
[α]_{D} - 84° (c 1,6, CHCl₃).
Anal. Calculée : C, 52,47, H, 6,19. Trouvé : C, 52,51, H, 6,19.
Cl-MS: 406 (M + NH₄), 389 (M + 1).

### PRÉPARATION 138

### Méthyl 2,3,6-tri-O-benzyl-4-O-(2,4,6-tri-O-acétyl-3-O-méthyl-5-C-vinyl-β-D-glucopyranosyl)-α-D-glucopyranoside (158)

Le composé **156** (1,6 g, 4,1 mmol) et le composé **157** (2,1 g, 4,5 mmol) P.J. Garegg and H. Hultberg, Carbohydr. Res. 1981, 93, C10, sont solubilisés dans du dichlorométhane anhydre (50 ml) et on ajoute un tamis moléculaire (4,0 g). Le mélange réactionnel est agité à température ambiante pendant une heure puis on ajoute du TMSOTf (0,95 ml, 5,2 mmol) à - 78° C. On laisse ensuite le mélange réactionnel revenir doucement à la température ambiante. Après 2 heures, le mélange réactionnel est neutralisé avec de la triéthylamine et filtré sur Célite ; le filtrat est lavé à l'eau. La phase organique est séchée sur sulfate de magnésium, concentrée et le résidu est purifié par chromatographie sur silice (acétate d'éthyle/cyclohexane 4/1 v/v) pour obtenir le composé désiré **158** (2,77 g, 85 %) sous forme d'un solide. Pf 47° C.
[α]_{D} - 36° (c 0,6, CHCl₃).
Anal. Calculée : C, 65,14, H, 6,61. Trouvé : C, 65,09, H, 6,70.

### PRÉPARATION 139

### Méthyl 2,3,6-O-tri-O-benzyl-4-O-(4,6-O-isopropylidène-3-O-méthyl-5-C-vinyl-β-D-glucopyranosyl)-α-D-glucopyranoside (160)

Le composé **158** (2,7 g, 3,4 mmol) est solubilisé dans du méthanol (40 ml). On ajoute du sodium (catalytique) à 0° C et on agite à température ambiante pendant 3 heures. Le solvant est concentré et le résidu **159** est repris dans de l'acétone anhydre (40 ml) et on ajoute du 2,2-diméthoxypropane (2 ml) et de l'acide p-toluène sulfonique (catalytique). Le mélange réactionnel est agité à température ambiante pendant une nuit. Le solvant est évaporé, le résidu est repris dans du chloroforme et lavé à l'eau. La phase organique est séchée sur sulfate de magnésium, concentrée et le résidu est purifié par chromatographie sur une colonne de silice (acétate d'éthyle/cyclohexane 1/1 v/v) pour obtenir le dérivé 4', 6' isopropylidène -O-**160** (1,7 g, 70 %) sous forme d'un solide. Pf 55° C.
[α]_{D} + 13° (c 0,8, CHCl₃)
Anal. Calculée : C, 67,97, H, 7,13. Trouvé : C, 67,87, H, 7,16.
CI-MS : 707 (M + 1), 724 (M + NH₄).

### PRÉPARATION 140

### Méthyl 2,3,6-tri-O-benzyl-4-O-(4,6-O-isopropylidène-3-O-méthyl-5-C-vinyl-β-D-mannopyranosyl)-α-D-glucopyranoside (162)

On agite du chiorure doxalyle (0,35 ml, 4,0 mmol) et du DMSO anhydre (0,57 ml, 8,0 mmol) dans du dichlorométhane anhydre (10 ml) à -78° C pendant 30 minutes. Le composé **160** (1,4 g, 2,0 mmol) dans du dichlorométhane anhydre (10 ml) est ajouté à la solution et on agite pendant encore 45 minutes. Le mélange réactionnel est neutralisé par addition de triéthylamine anhydre (1,7 ml, 12,0 mmol) puis dilué avec du dichiorométhane. Après l'avoir lavée à l'eau, la phase organique est séchée sur sulfate de magnésium, concentrée et le résidu **161** est directement utilisé pour la réaction suivante sans purification. La cétone **161** est reprise dans du tétrahydrofurane anhydre (15 ml) et on ajoute une solution 1N de super hydrure dans le tétrahydrofurane (4 ml, 4,0 mmol) à - 78° C. Le mélange réactionnel est agité à température ambiante pendant 1 heure et on ajoute ensuite de l'hydroxyde de sodium à 5 % (2 ml) et du peroxyde d'hydrogène (1 ml). Le solvant est évaporé et le résidu est repris par de l'acétate d'éthyle et lavé à l'eau. La phase organique est séchée sur sulfate de magnésium, concentrée et le résidu est purifié par chromatographie (acétate d'éthyie/cyclohexane 2/1 v/v) pour obtenir le composé **162** (1,0 g, 70 %).
[α]_{D} - 11° (c 0,5, CHCl₃).
CI-MS: 724 (M + 18), 707 (M + 1).

### PRÉPARATION 141

### Méthyl 2,3,6-tri-O-benzyl-4-O-(2-O-acétyl-3-O-méthyl-5-C-vinyl-β-D-mannopyranosyl)-α-D-glucopyranoside (164)

Le composé **162** (940 mg, 1,3 mmol) est dissous dans la pyridine (3 ml) et on ajoute de l'anhydride acétique (0,3 ml). Le mélange réactionnel est agité à température ambiante pendant 3 heures. L'excès de pyridine et d'anhydride acétique est concentré et le résidu **163** est directement utilisé pour la déprotection de l'ispropylidène en utilisant de l'acide acétique à 80 % (5 ml) à 60° C pendant 2 heures. L'excès d'acide acétique est évaporé et le résidu est purifié par une chromatographie sur une colonne de gel de silice (acétate d'éthyle/cyclohexane 4/1 v/v) pour obtenir le diol **164** (660 mg, 70 %) sous forme solide. Pf 53° C.
[α]_{D} - 10° (c 0,8, CHCl₃).
Cl-MS : 709 (M + 1), 726 (M + 18).

### PRÉPARATION 142

### Méthyl 2,3,6-tri-O-benzyl-4-(2-O-actéyl-3-O-méthyl-6-O-tosyl-5-C-vinyl-β-D-mannopyranosyl)-α-D-glucopyranose (165)

Le composé **164** (600 mg, 0,9 mmol) est dissous dans la pyridine (3 ml) et on ajoute du chlorure de tosyle (240 mg, 1,3 mmol). Le mélange réactionnel est agité à température ambiante pendant 3 heures. Le solvant est évaporé, le résidu est dilué avec du chloroforme et lavé à l'eau. La phase organique est séchée sur sulfate de magnésium, concentrée et le résidu est purifié par chromatographie sur une colonne de gel de silice (acétate d'éthyle/cyclohexane 1/1 v/v) pour obtenir le composé tosylé **165** (297 mg, 80 %) sous forme d'un sirop.
[α]_{D} - 26° (c 0,8, CHCl₃).

### PRÉPARATION 143

### Méthyl 2,3,6-tri-O-benzyl-4-(2,6-anhydro-3-O-méthyl-5-C-vinyl-β-D-mannopyranosyl)-α-D-glucopyranoside (166)

Le composé **165** (550 mg, 0,6 mmol) est repris dans l'éthanol (3 ml) et on ajoute ensuite une solution 0,1N d'hydroxyde de sodium éthanolique (5 ml). Le mélange réactionnel est chauffé à 70° C pendant 3 heures puis neutralisé par une résine IR-120 (forme H⁺) et filtré sur Célite. Le résidu, après concentration, est purifié par chromatographie sur une colonne de gel de silice (acétate d'éthyle/cyclohexane 1/1 v/v) pour obtenir le composé **166** (292 mg, 70 %) sous forme d'un sirop.
[α]_{D}⁺ 13° (c 0,5, CHCl₃).
Cl-MS : 666 (M + 18).

### PRÉPARATION 144

### Méthyl 2,3,6-tri-O-benzyl-4-(benzyl 3-O-méthyl-2-O-5-C-méthylidène-α-L-idopyranuronate)-α-D-glucopyranoside (167)

Le composé **166** (260 mg, 0,4 mmol) est dissous dans du dichlorométhane (20 ml), la solution est agitée à - 78° C puis on fait buller de l'ozone pendant 30 secondes. La couleur de la solution devient jaune pâle. On ajoute du diméthylsulfure à la solution puis le mélange réactionnel est lavé à l'eau. La phase organique est séchée sur sulfate de magnésium, concentrée et on passe directement à la réaction suivante sans purification supplémentaire. L'aldéhyde brut est repris dans du tert-butanol (16 ml) et on ajoute du 2-méthyl-2-butène (5 ml) et de l'eau (16 ml). On ajoute ensuite successivement au mélange du NaH₂PO₄ (700 mg) et du NaClO₂ (700 mg). La suspension est vigoureusement agitée à tempéraure ambiante pendant une nuit, diluée à l'eau et extraite à l'acétate d'étyle. La phase organique est séchée sur sulfate de magnésium, concentrée puis on passe directement à la réaction suivante. L'acide brut est repris dans du diméthylformamide (25 ml) et on ajoute de l'iodure de tétrabutylammonium (0,7 g, 2,0 mmol), du bicarbonate de potassium (0,25 g, 2,5 mmol) et du bromure de benzyle (0,250 ml, 2,1 mmol). Le mélange réactionnel est agité à température ambiante pendant 5 heures. Le mélange réactionnel est extrait avec de l'eau et de l'éther. La phase éthérée est séchée sur sulfate de magnésium, concentrée et le résidu est purifié par chromatographie sur une colonne de gel de silice (acétate d'éthyle/cyclohexane 2/1 v/v) pour obtenir le dérivé **167** (236 mg, 80 %) sous forme d'un sirop.
CI-MS : 774 (M + 18).

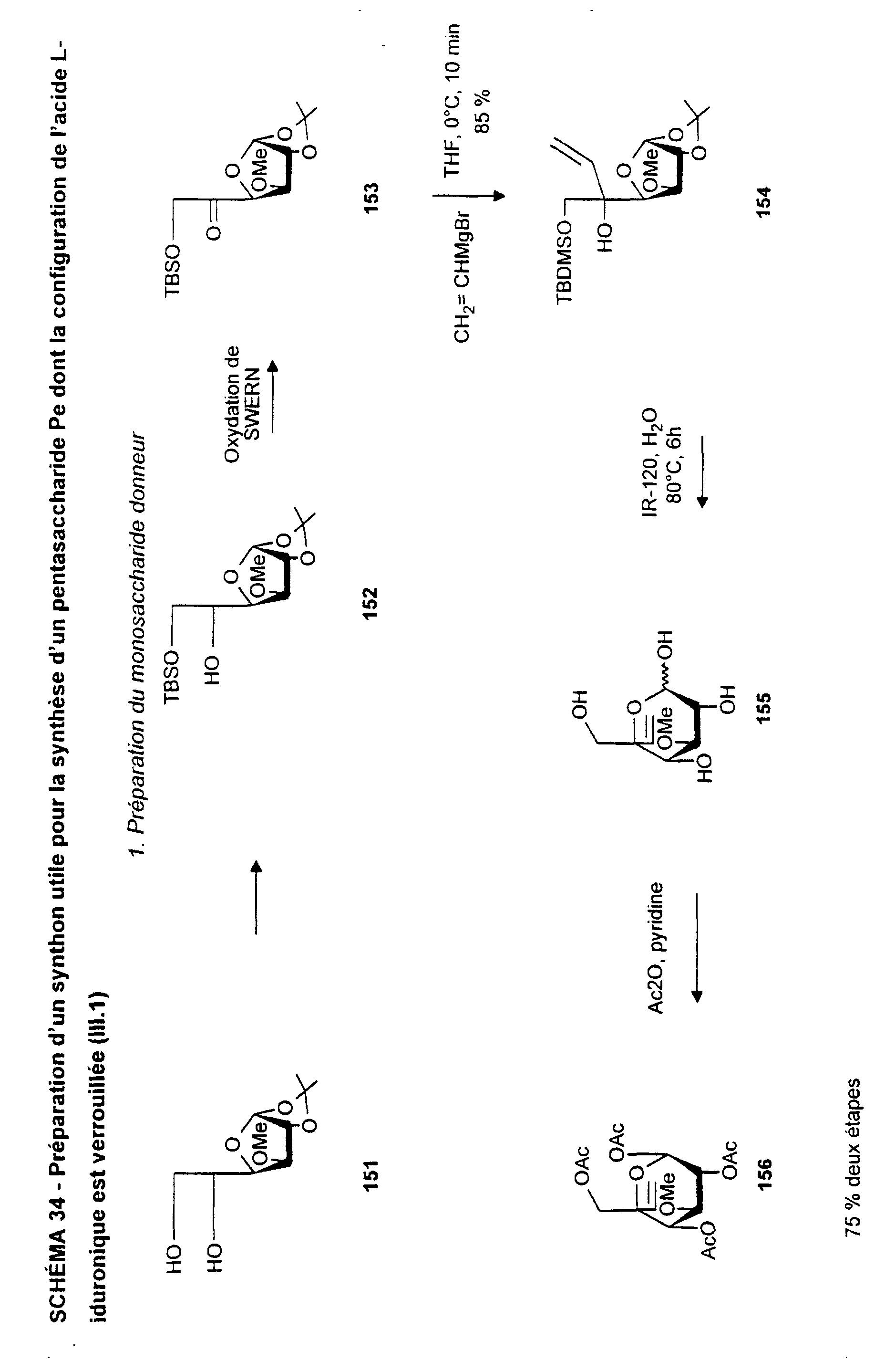

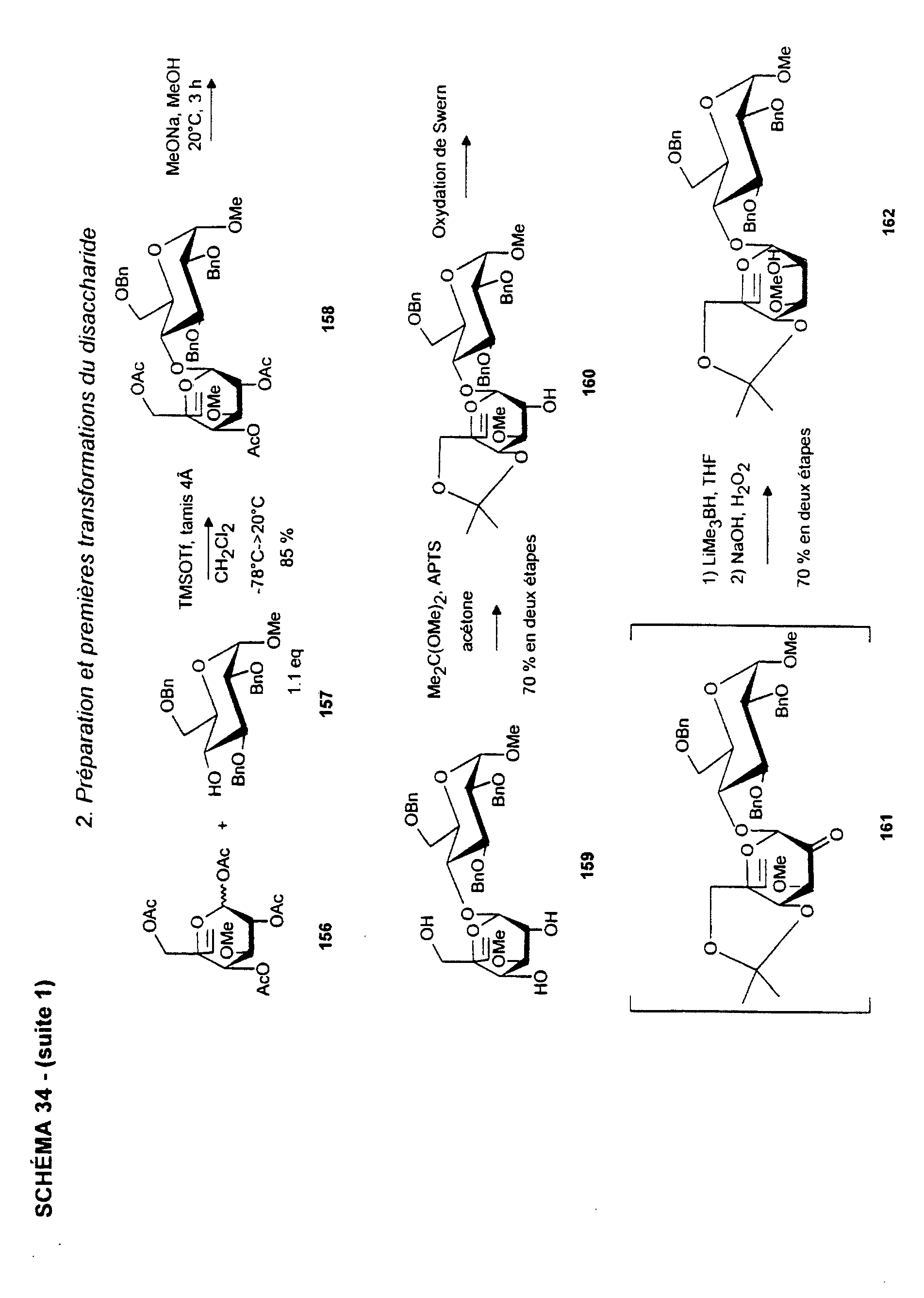

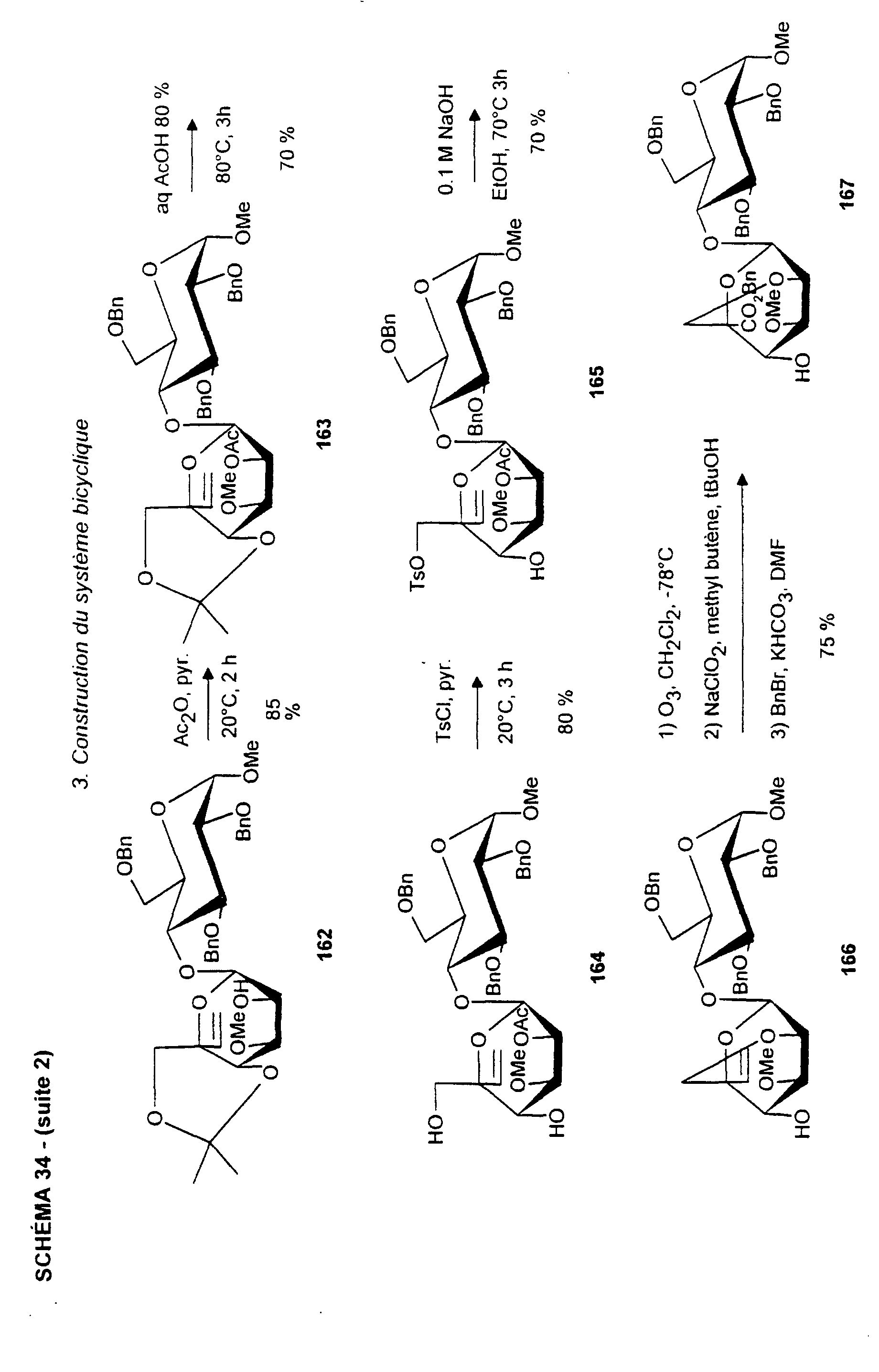

### EXEMPLES

### EXEMPLE 1

### Méthyl O-(3-O-méthyl-2,4,6-tri-O-sulfo-α-D-glucopyranosyl)-(1→4)-O-(3-O-méthyl-2,6-di-O-sulfo-β-D-glucopyranosyl)-(1→4)-[O-(3-O-méthyl-2,6-di-O-sulfo-α-D-glucopyranosyl)-(1→4)-O-(3-O-méthyl-2,6-di-O-sulfo-β-D-glucopyranosyl)-(1→4)]₆-O-(2,3-di-O-méthyl-6-O-sulfo-α-D-glucopyranosyl)-(1→4)-O-(acide 2,3-di-O-méthyl-β-D-glucopyranosyluronique)-(1→4)-O-(2,3,6-tri-O-sulfo-α-D-glucopyranosyl)-(1→4)-O-(acide 2,3-di-O-méthyl-α-L-idopyranosyluronique)-(1→4)-2,3,6-tri-O-sulfo-α-D-glucopyranoside, sel de sodium (168)

Le composé **31** est traité selon la MÉTHODE 5 pour donner **168** (80 % sur les trois étapes). [α]_{D} + 41 (*c* = 0,8, eau). ESIMS, mode négatif : masse monoisotopique = 7133,26 ; masse chimique = 7138,90 ; masse expërimentale = 7137,26 ± 0,0 u.m.a. ¹H RMN (D₂O) δ des principaux protons anomériques : 5,71 ; 5,48 ; 5,46 ; 5,44 ; 5,17 ; 5,08 ; 4,81 ; 4,78 ; 4,67 ppm.
Une procédure identique permet d'obtenir les composés **169** et **170.**

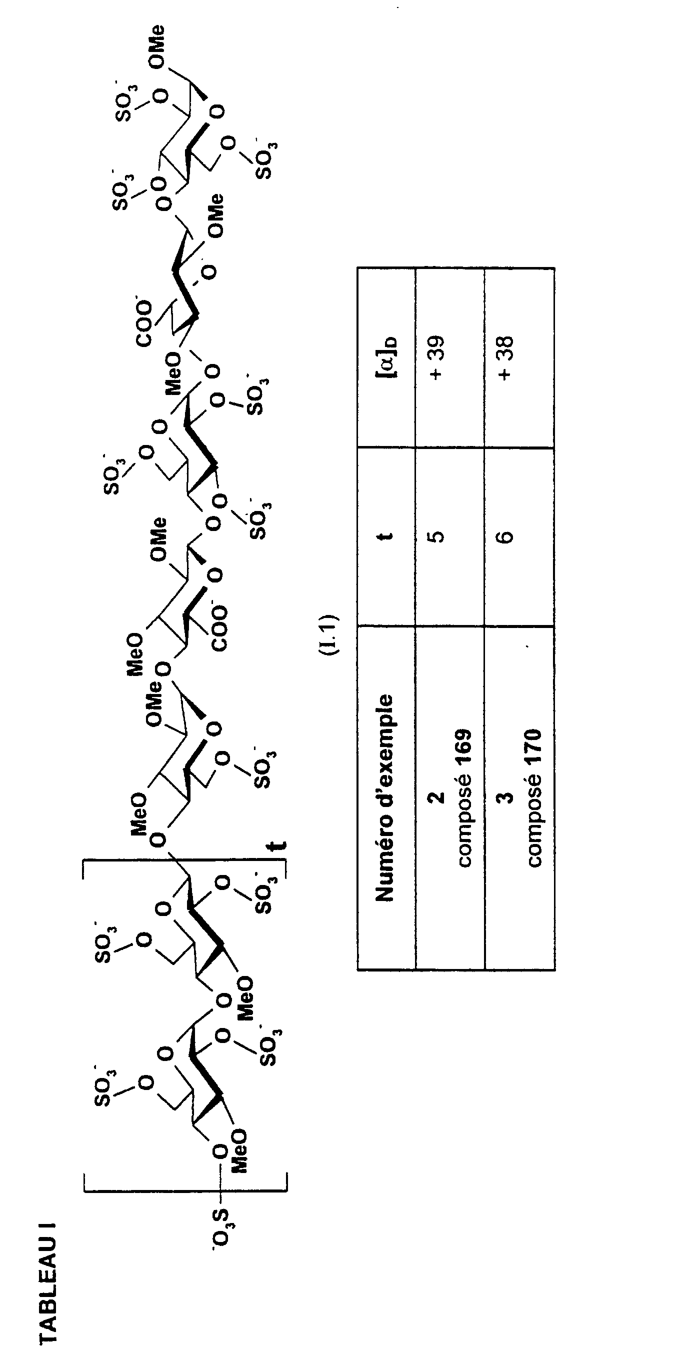

### EXEMPLE 4

### Méthyl O-(2,3-di-O-méthyl-4,6-di-O-sulfo-α-D-glucopyranosyl)-(1→4)-[O-(2,3-di-O-méthyl-6-O-sulfo-α-D-glucopyranosyl)-(1→4)-]₁₅-O-(acide 2,3-di-O-méthyl-β-D-glucopyranosyluronique)-(1→4)-O-(2,3,6-tri-O-sulfo-α-D-glu-copyranosyl)-(1→4)-O-(acide 2,3-di-O-méthyl-α-L-idopyranosyluronique)-(1→4)-2,3,6-tri-O-sulfo-α-D-glucopyranoside, sel de sodium (171)

On traite le composé **51** (55 mg, 10,5 mmol) selon la MÉTHODE 5 afin d'obtenir, après lyophilisation, le produit sulfaté **187** (50 mg, 77 % en trois étapes). [αJ_{D} + 107 (*c* = 0,52, eau). ESIMS, mode positif : masse monoisotopique = 6194,16, masse chimique = 6198,83, masse expérimentale = 6195,33 ± 1,79. ¹H RMN (D₂O) δ des principaux protons anomériques : 5,71 ; 5,67 ; 5,48 ; 5,43 ; 5,17 ; 5,10 ; 4,68 ppm.
Une procédure identique permet d'obtenir les composés **172** et **173.**

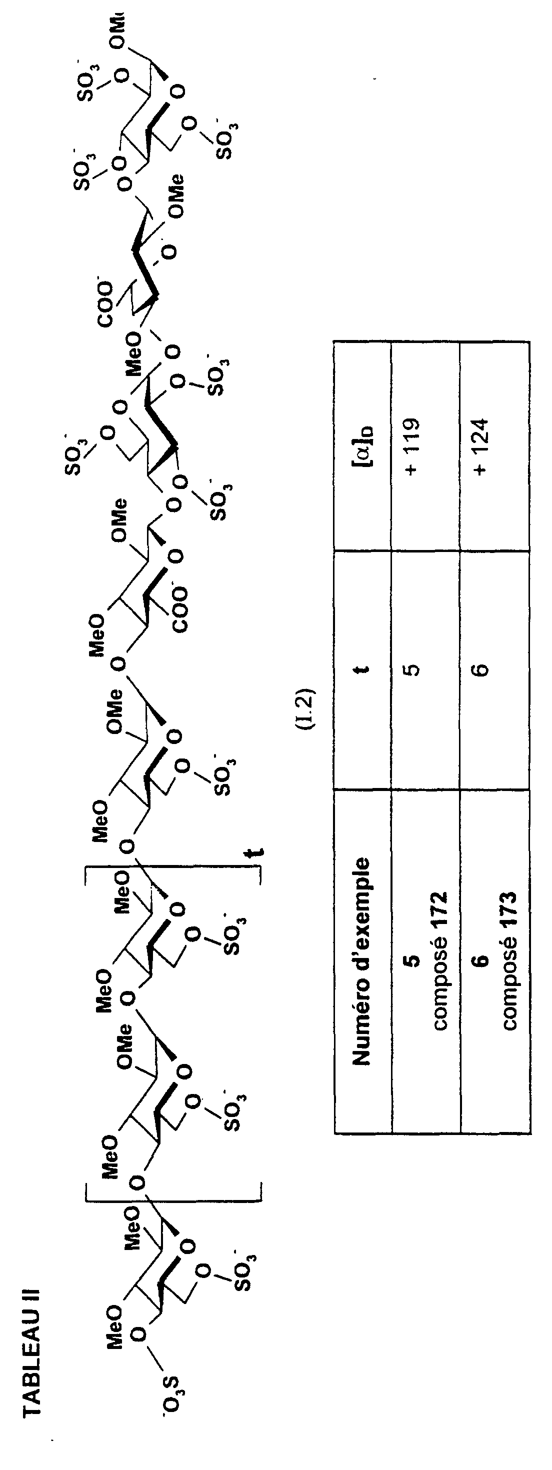

### EXEMPLE 7

### Méthyl O-(3-O-méthyl-2,4,6-tri-O-sulfo-α-D-giucopyranosyl)-(1→4)-O-(3-O-méthyl-2,6-di-O-sulfo-β-D-glucopyranosyl)-(1→4)-O-(3-O-méthyl-2,6-di-O-sulfo-α-D-glucopyranosyl)-(1→4)-O-(3-O-méthyl-2,6-di-O-sulfo-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₄-O-(2,3-di-O-méthyl-6-O-sulfo-α-D-glucopyranosyl)-(1→4)-O-(acide 2,3-di-O-méthyl-β-D-glucopyranosyluronique)-(1→4)-O-(2,3,6-tri-O-sulfo-α-D-glucopyranosyl)-(1→4)-O-(acide 2,3-di-O-méthyl-α-L-idopyranosyluronique)-(1→4)-2,3,6-tri-O-sulfo-α-D-glucopyranoside, sel de sodium (174)

Le composé **75** est traité selon la MÉTHODE 5 pour donner **174** (84 % sur les trois étapes). [α]_{D} + 62 (c = 0,46, eau). ESIMS, mode positif : masse monoisotopique = 4966,39 ; masse chimique = 4970,04 ; masse expérimentale = 4969,63 t 0,78 u.m.a.. ¹H RMN (D₂O) δ des principaux protons anomériques : 5,69 ; 5,63 ; 5,57 ; 5,46 ; 5,44 ; 5,41 ; 5,15 ; 5,06 ; 4,79 ; 4,66 ; 4,62 ; 4,41 ppm.
En procédant selon l'EXEMPLE 7 et en utilisant les intermédiaires adéquats, on prépare les EXEMPLES 8 à 12 décrits dans le TABLEAU III ci-après

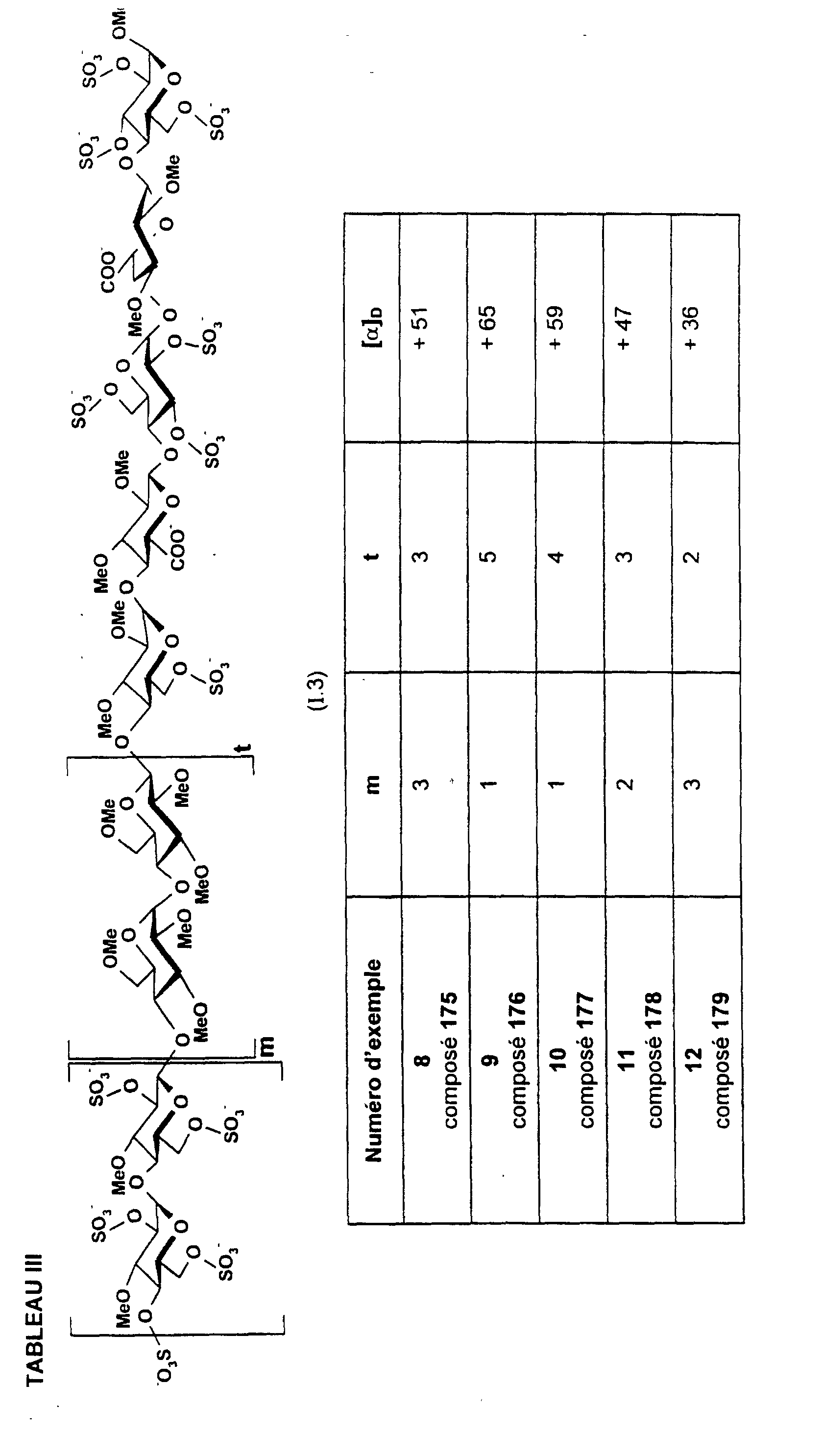

### EXEMPLE 13

### Méthyl O-(2,3,4,6-tétra-O-sulfo-α-D-glucopyranosyl)-(1→4)-O-(2,3,6-tri-O-sulfo-α-D-glucopyranosyl)-(1→4)-O-(2,3,6-tri-O-sulfo-β-D-glucopyranosyl)-(1→4)-O-(2,3-di-O-méthyl-6-O-sutfo-α-D-glucopyranosyl)-(1→4)-O-(2,3,6-tri-O-méthyl-β-D-glucopyranosyl)-(1→4)-[O-(2,3,6-tri-O-méthyl-α-D-glucopyranosyl)-(1→4)-O-(2,3,6-tri-O-méthyl-β-D-glucopyranosyl)-(1→4)]₃-O-(2,3-di-O-méthyl-6-O-sulfo-α-D-glucopyranosyl)-(1→4)-O-(acide 2,3-di-O-méthyfl-β-D-glucopyranosyluronique)-(1→4)-O-(6-O-méthyl-2,3-di-O-sulfo-α-D-glucopyranosyl)-(1→4)-O-(acide 2,3-di-O-méthyl-α-L-idopyranosyluronique)-(1→4)-6-O-méthyl-2,3-di-O-sulfo-α-D-glucopyranoside, sel de sodium (180).

L'hexadécasaccharide complètement déprotégé **150** (26 mg, 0,0084 mmol) est dissous dans du diméthylformamide (0,87 ml). Sous atmosphère d'azote, on ajoute un complexe de trioxyde de sulfure triéthylamine (125 mg, 0,67 mmol, 80 eq) et le mélange est agité pendant 16 heures à 50° C. Le mélange est refroidi à 0° C et on ajoute de l'hydrogénocarbonate de sodium aqueux (227 mg, 2,6 mmol). Le mélange est concentré à un petit volume et appliqué à une colonne de Sepahdex G-25, élué avec de l'eau/acétonitrile 9/1 vlv. Les fractions appropriées sont séparées, concentrées à un petit volume, appliquées sur une colonne Dowex XW4 Na⁺ échangeuse d'ions dans de l'eau et l'éluat est lyophilisé pour donner 37 mg du composé **151** comme poudre blanche. [α]²⁰_{D} = +67,6 (c = 1, eau) MS ESI : poids moléculaire est 4370,6 (H⁺-form) C₁₂₈H₂₂₂O₁₁₃S₁₆ (Th P.M. = 4370,14).
RMN ; déplacement des protons anomériques (ppm) :
unité1 : 5,17 ; unité 2 : 5,03 ; unité 3 : 5,41 ; unité 4 : 4,42 ; unité 5 : 5,49 ; unité 6 : 4,66 ; unités 7, 9 et 11 : 5,67 : unités 8, 10 et 12 : 4,46 ; unité 13 : 5,61 ; unité 14: 4,94 ; unité 15 : 5,59 ppm ; unité16 : 5,69.

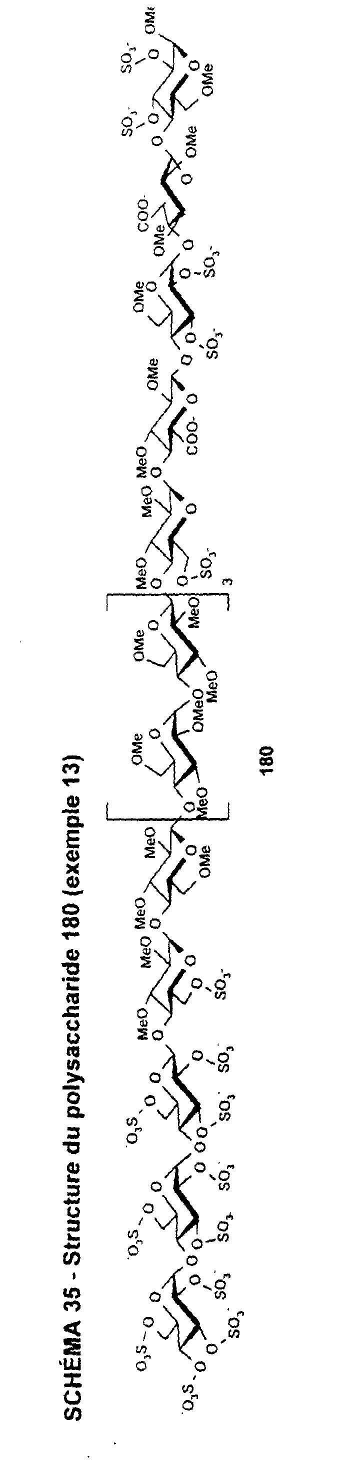

## Revendications

1. Polysaccharide de synthèse comprenant un domaine de liaison à l'antithrombine III constitué par un enchaînement de cinq monosaccharides portant au total deux fonctions acide carboxylique et au moins quatre groupes sulfo, ce domaine étant lié directement à son extrémité non réductrice par un domaine de liaison à la thrombine comprenant un enchaînement de 10 à 25 unités monosaccharidiques choisies parmi des hexoses, des pentoses ou des sucres désoxy dont les groupes hydroxyles sont indépendamment éthérifiés par un groupe (C₁-C₆)alkyle ou estérifiés sous la forme de groupes sulfo ainsi que ses sels, notamment pharmaceutiquement acceptables.

2. Polysaccharide selon la revendication 1, de formule : 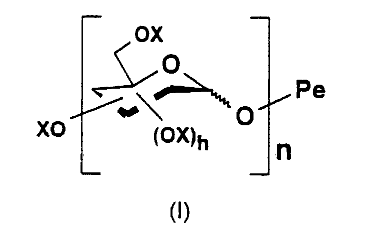 dans laquelle
- le trait ondulé désigne une liaison située soit au-dessous soit au-dessus du plan du cycle pyranosique, 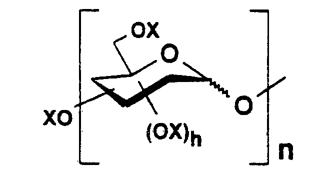 désigne un polysaccharide Po, contenant n unités monosaccharidiques identiques ou différentes, lié par son carbone anomère à Pe, 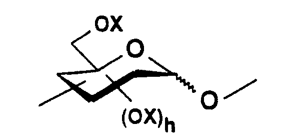 est une représentation schématique d'une unité monosaccharidique à structure pyranosique choisie parmi les hexoses, les pentoses et les sucres desoxy correspondants, cette unité étant liée par son carbone anomère à une autre unité monosaccharidique, et les groupes hydroxy de cette unité étant substitués par des groupes -X identiques ou différents, les groupes X étant choisis parmi les groupes (C₁-C₆)alkyle et les groupes sulfo,
- n est un nombre entier de 10 à 25,
- Pe représente un pentasaccharide de structure : 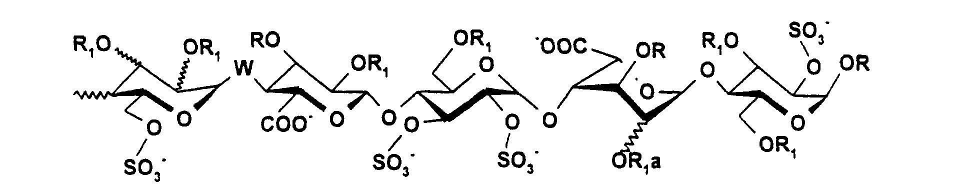
dans lequel
- R₁ représente un groupe (C₁-C₆)alkyle ou un groupe sulfo,
- R₁a représente R₁ ou constitue avec l'atome d'oxygène auquel il est lié et l'atome de carbone porteur de la fonction carboxylique sur le même cycle un groupe
C―CH₂―O,
- R représente un groupe (C₁-C₆)alkyle,
- W représente un atome d'oxygène ou un groupe méthylène,
ou un de leurs sels, notamment pharmaceutiquement acceptable.

3. Sel d'un polysaccharide selon la revendication 2 dans lequel le cation est choisi parmi les cations des métaux alcalins, en particulier le sodium et le potassium.

4. Polysaccharide et sel selon l'une des revendications 2 ou 3 de formule : 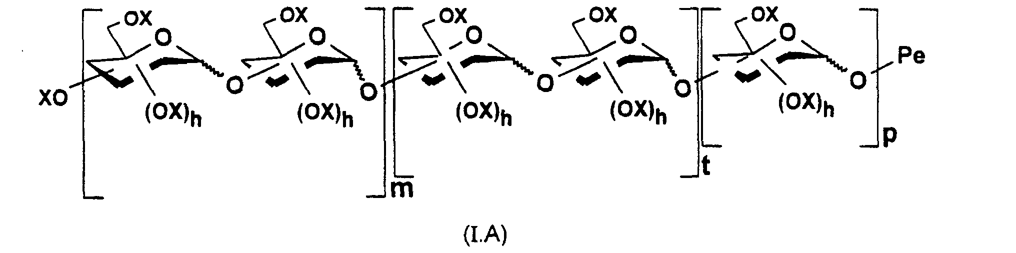 dans lesquels 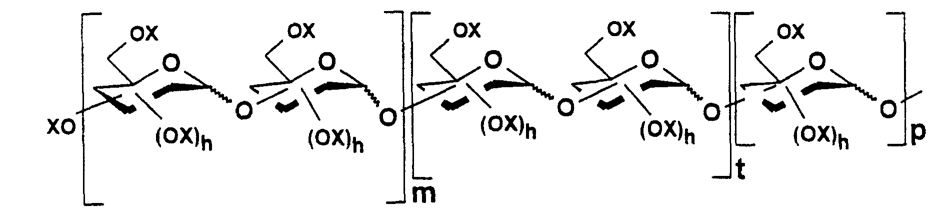 désigne une famille particulière de polysaccharides Po, liés par leur carbone anomère à Pe tel que défini pour (I), 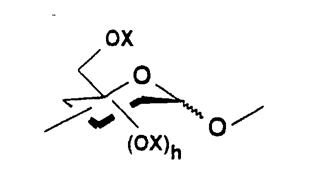 est tel que défini pour (I),
- les OX sont tel que défini pour (I) et, pour un même monosaccharide, peuvent être identiques ou différents,
- les monosaccharides contenus dans [ ]ₘ forment un disaccharide répété m fois, les monosaccharides contenus dans [ ]ₜ forment un disaccharide répété t fois,
- m varie de 1 à 8, t varie de 0 à 5 et p varie de 0 à 1 étant entendu que 5 ≤ m + t ≤ 12.
et leurs sels, notamment pharmaceutiquement acceptables.

5. Polysaccharide et sel selon l'une des revendications 2 ou 3 de formule : 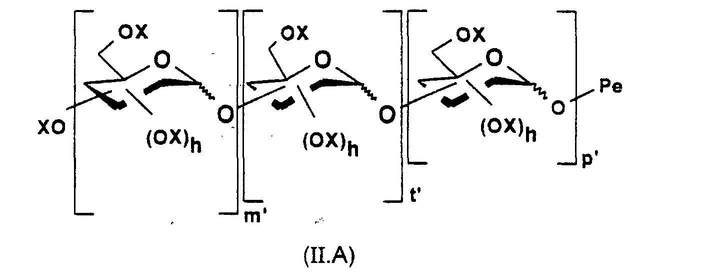 dans lesquels 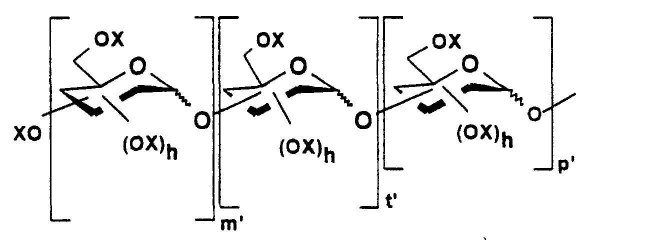 désigne une famille particulière de polysaccharides Po, liés par leur carbone anomère à Pe tel que défini pour (I), 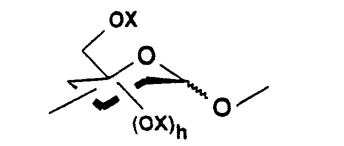 est tel que défini pour (I),
- les OX sont tel que défini pour (I) et. pour un même monosacchande, peuvent être identiques ou différents,
- le monosaccharide contenu dans [ ]ₘ. est répété m' fois, le monosaccharide contenu dans [ ]ᵣ est répété t' fois, le monosaccharide contenu dans [ ]ₚ, est répété p' fois,
- m' varie de 1 à 5, t' varie de 0 à 2 4 et p' varie de 0 à 2 4 étant entendu que 10 ≤ m' + t' + p' ≤ 25
et leurs sets, notamment pharmaceutiquement acceptables.

6. Sel selon l'une des revendications 2 ou 3 dont l'anion a pour formule : 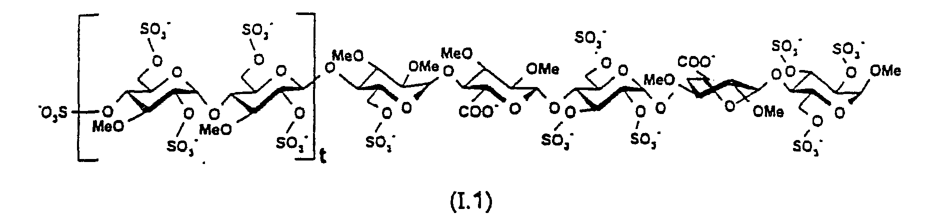 dans laquelle t représente 5, 6 ou 7, et le cation étant un cation monovalent pharmaceutiquement acceptable, ainsi que leurs acides correspondants.

7. Sel selon l'une des revendications 2 ou 3 dont l'anion a pour formule: 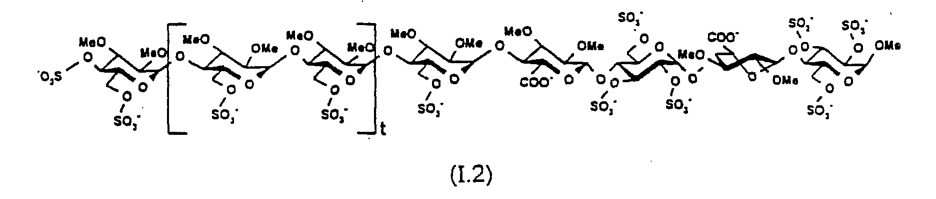 dans laquelle t représente 5, 6 ou 7 et où le cation est un cation monovalent pharmaceutiquement acceptable, ainsi que leurs acides correspondants.

8. Sel selon l'une des revendications 2 ou 3 dont l'anion a pour formule : 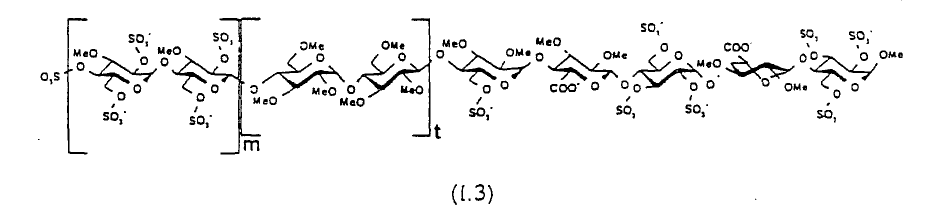 dans laquelle m représente 1, 2 ou 3 et t représente 2, 3, 4 ou 5 et où le cation est un cation monovalent pharmaceutiquement acceptable, ainsi que leurs acides correspondants.

9. Polysaccharides choisis parmi :
• Méthyl *O*-(3-*O*-méthyl-2,4,6-tri-*O*-sulfo-α-D-glucopyranosyl)-(1→4)-*O*-(3-*O*-méthyl-2,6*-*di-*O*-sulfo-β-D-glucopyranosyl)-(1→4)-[*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)]₄-O-(2,3-di-O-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium
• Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)]₅-*O*-(2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium
• Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[*O*-(3-*O*-méthyl-2,6-di-*O*-sulfo-α-D-glucopyranosyl)-(1→4)-*O*-(3-*O*-méthyl-2,6-di-O-sulfo-β-D-glucopyranosyl)-(1→4)]₆-*O*-(2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium
• Méthyl *O*-(2,3-di-*O*-méthyl-4,6-di-*O*-sulfo-α-D-glucopyranosyl)-(1→4)-[*O*-(2,3-di-*O*-méthyl-6-*O*-sulfo-α-D-glucopyranosyl)-(1→4)-]₁₁-*O*-(acide 2,3-di-*O*-méthyl-β-D-glucopyranosyluronique)-(1→4)-*O*-(2,3,6-tri-*O*-sulfo-α-D-glucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium.
• Méthyl *O*-(2,3-di-*O*-méthyl-4,6-di-*O*-sulfo-α-D-glucopyranosyl)-(1→4)-[*O*-(2,3-di-*O*-méthyl-6-*O*-sulfo-α-D-glucopyranosyl)-(1→4)-]₁₃-*O*-(acide 2,3-di-*O*-méthyl-β-D-glucopyranosyluronique)-(1→4)-*O*-(2,3,6-tri-*O*-sulfo-α-D-glucopyranosyl)-(1→4)-*O*-(acide 2,3-di-*O*-méthyl-α-L-idopyranosyluronique)-(1→4)-2,3,6-tri-*O*-sulfo-α-D-glucopyranoside, sel de sodium
• Méthyl *O*-(2,3-di-*O*-méthyl-4,6-di-*O*-sulfo-α-D-glucopyranosyl)-(1→4)-[*O*-(2,3-di-*O*-méthyl-6-*O*-sulfo-α-D-glucopyranosyl)-(1→4)-]₁₅-*O*-(acide 2,3-di-*O*-méthyl-β-D-glucopyranosyluronique)-(1→4)-*O*-(2,3,6-tri-*O*-sulfo-α-D-glucopyranosyl)-(1→4)-O-(acide 2,3-di-*O*-méthyl-α-L-idopyranosyluronique)-(1→4)-2,3,6-tri-*O*-sulfo-α-D-glucopyranoside, sel de sodium
• Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)]₂-[*O*-(2,3,6-tri-*O*-méthyl-α-D-glucopyranosyl)-(1→4)-*O*-(2,3,6-tri-*O*-méthyl-β-D-glucopyranosyl)-(1→4)]₂-*O*-2,3-di-*O*-méthyl-6-*O*-sulfo-α-D-glucopyranosyl)-(1→4)-O-(acide 2,3-di-*O*-méthyl-β-D-glucopyranosyluronique)-(1→4)-O-(2,3,6-tri-O-sulfo-α-D-glucopyranosyl)-(1→4)-(acide 2,3-di-*O*-méthyl-α-L-idopyranosyluronique)-(1→4)-2,3,6-tri-*O*-sulfo-α-D-glucopyranoside, sel de sodium
• Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[*O*-(3-O-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)]₂-[*O*-(2,3,6-tri-*O*-méthyl-α-D-glucopyranosyl)-(1→4)-*O*-(2,3,6-tri-*O*-méthyl-β-D-glucopyranosyl)-(1→4)]₃-*O*-2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-(acide 2,3-di-*O*-méthyl-α-L-idopyranosyluronique)-(1→4)-2,3,6-tri-*O*-sulfo-α-D-glucopyranoside, sel de sodium
• Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)-[*O*-(2,3,6-tri-*O*-méthyl-α-D-glucopyranosyl)-(1→4)-*O*-(2,3,6-tri-*O*-méthyl-β-D-glucopyranosyl)-(1→4)]₄-*O*-2,3-di-*O*-méthyl-6-*O*-sulfo-α-D-glucopyranosyl)-(1→4)-*O*-(acide 2,3-di-*O*-méthyl-β-D-glucopyranosyluronique)-(1→4)-*O*-(2,3,6-tri-*O*-sulfo-α-D-glucopyranosyl)-(1→4)-(acide 2,3-di-*O*-méthyl-α-L-idopyranosyluronique)-(1→4)-2,3,6-tri-*O*-sulfo-α-D-glucopyranoside, sel de sodium
• Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-*O*-(3-*O*-méthyl-2,6-di-*O*-sulfo-α-D-glucopyranosyl)-(1→4)-*O*-(3-*O*-méthyl-2,6-di-*O*-sulfo-β-D-glucopyranosyl)-(1→4)-[*O*-(2,3,6-tri-*O*-méthyl-α-D-glucopyranosyl)-(1→4)-*O*-(2,3,6-tri-*O*-méthyl-β-D-glucopyranosyl)-(1→4)]₃-*O*-2,3-di-*O*-méthyl-6-*O*-sulfo-α-D-glucopyranosyl)-(1→4)-*O*-(acide 2,3-di-O-méthyl-β-D-glucopyranosyluronique)-(1→4)-*O*-(2,3,6-tri-*O*-sulfo-α-D-glucopyranosyl)-(1→4)-(acide 2,3-di-*O*-méthyl-α-L-idopyranosyluromque)-(1→4)-2,3,6-tri-*O*-sulfo-α-D-glucopyranoside, sel de sodium
• Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[*O*-(2,3,6-tri-*O*-méthyl-α-D-glucopyranosyl)-(1→4)-*O*-(2,3,6-tri-*O*-méthyl-β-D-glucopyranosyl)-(1→4)]₄-*O*-2,3-di-O-méthyl-6-O-sulfo-α-D-glucopyranosyl)-(1→4)-O-(acide 2,3-di-O-méthyl-β-D-glucopyranosyluronique)-(1→4)-*O*-(2,3,6-tri-*O*-sulfo-α-D-glucopyranosyl)-(1→4)-(acide 2,3-di-*O*-méthyl-α-L-idopyranosyluronique)-(1→4)-2,3,6-tri-*O*-suffo-α-D-glucopyranoside, sel de sodium
• Méthyl *O*-(3-*O*-méthyl-2,4,6-tri-*O*-sulfo-α-D-glucopyranosyl)-(1→4)-*O*-(3-*O*-méthyl-2,6-di-*O*-sulfo-β-D-glucopyranosyl)-(1→4)-[O-(2,3,6-tri-*O*-méthyl-α-D-glucopyranosyl)-(1→4)-*O*-(2,3,6-tri-*O*-méthyl-β-D-glucopyranosyl)-(1→4)]₅-*O*-2,3-di-O-méthyl-6-O-sulfo-α-D-glucopyranosyl)-(1→4)-O-(acide 2,3-di-O-méthyl-β-D-glucopyranosyluronique)-(1→4)-*O*-(2,3,6-tri-*O*-sulfo-α-D-glucopyranosyl)-(1→4)-(acide 2,3-di-*O*-méthyl-α-L-idopyranosyluronique)-(1→4)-2,3,6-tri-*O*-sulfo-α-D-glucopyranoside, sel de sodium.

10. Procédé pour la préparation des composés de formule (I) **caractérisé en ce que** dans une première étape, un précurseur complètement protégé du polysaccharide (I) désiré, contenant un précurseur protégé du domaine Pe prolongé à son extrémité non réductrice par un précurseur protégé du polysaccharide sulfaté Po est synthétisé puis, dans une seconde étape, les groupes chargés négativement sont introduits et/ou démasqués.

11. Composé de formule : 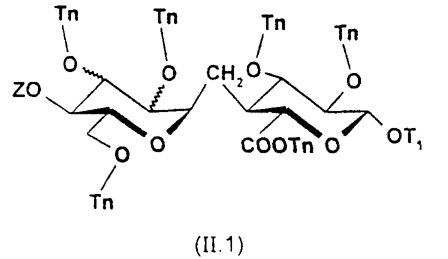 dans laquelle T₁, Tₙ identiques ou différents représentent un substituant temporaire, semi-permanent ou permanent, Z est un groupe protecteur d'une fonction hydroxyle

12. Composé de formule : 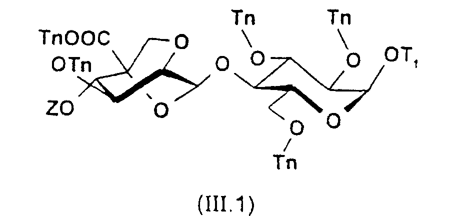 dans laquelle T₁, Tₙ identiques ou différents représentent un substituant temporaire, semi-permanent ou permanent, Z est un groupe protecteur d'une fonction hydroxyle.

13. Compositions pharmaceutiques contenant comme principe actif un polysaccharide ou sel selon l'une quelconque des revendications 1 à 9, sous forme de sel avec une base pharmaceutiquement acceptable ou sous forme acide, en association ou en mélange avec un excipient inerte, non toxique, pharmaceutiquement acceptable.

14. Composition pharmaceutique selon la revendication 13, sous forme d'unités de dosage, dans laquelle le principe actif est mélangé à au moins un excipient pharmaceutique.

15. Composition selon la revendication 14 dans laquelle chaque unité de dosage contient de 0,1 à 100 mg de principe actif.

16. Composition selon la revendication 15 dans laquelle chaque unité de dosage contient de 0,5 à 50 mg de principe actif.

17. Utilisation des polysaccharide ou sel selon les revendications 1 à 9 pour la préparation d'un médicament utile dans les pathologies dépendantes d'un dysfonctionnement de la coagulation.

## Claims

1. Synthetic polysaccharide comprising an antithrombin III binding domain consisting of a chain of five monosaccharides having a total of two carboxylic acid functions and at least four sulpho groups, this domain being bound directly at its non-reducing end by a thrombin binding domain comprising a chain of 10 to 25 monosaccharide units selected from among the hexoses, pentoses or deoxy sugars wherein the hydroxyl groups are independently etherified by a (C_{1-6'} alkyl group or esterified in the form of sulpho groups and the salts thereof, particularly the pharmaceutically acceptable salts thereof.

2. Polysaccharide according to claim 1 of the formula: 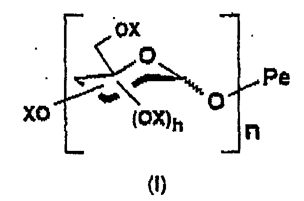 wherein
- the wavy line denotes a bond located either below or above the plane of the pyranose ring, 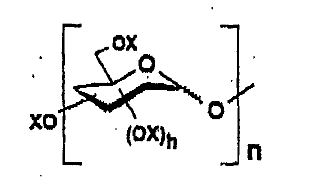 denotes a polysaccharide Po containing n identical or different monosaccharide units linked by its anomeric carbon to Pe, 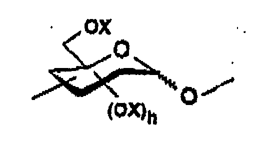 is a diagrammatic representation of a monosaccharide unit with a pyranose structure selected from among the corresponding hexosas, pentoses and deoxy sugars, this unit being linked by its anomeria carbon to another monosaccharide unit, and the hydroxy groups of this unit being substituted by groups -X which may be identical or different, the groups X being selected from among the (C₁₋₆) alkyl groups and the sulpho groups,
- n is an integer from 10 to 25,
- Pe denotes a pentasaccharide of the structure: 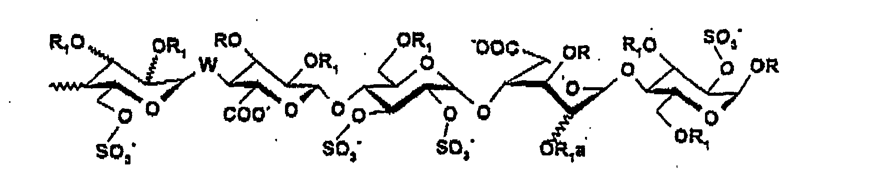
wherein
- R₁ denotes a (C₁₋₆)alkyl group or a sulpho group,
- R₁a denotes R₁ or together with the oxygen atom to which it is linked and the carbon atom carrying the carboxyl function on the same ring it forms a group
C - CH₂ - O,
- R denotes a (C₁₋₆) alkyl group,
- W denotes an oxygen atom or a methylene group, or one of the salts thereof, particularly the pharmaceutically acceptable salts thereof.

3. Salt of a polysaccharide according to claim 2, wherein the cation is selected from among the cations of the alkali metals, particularly sodium and potassium.

4. Polysaccharide and salt according to one of claims 2 or 3 of the formula: 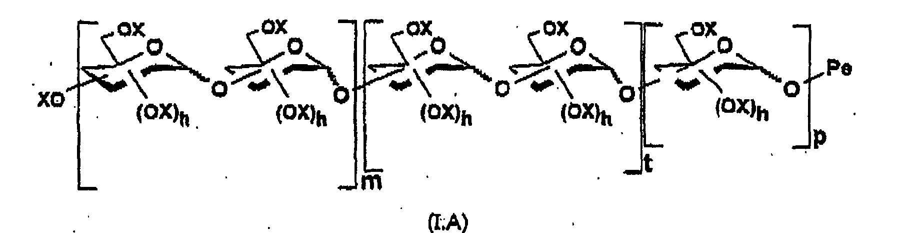 wherein 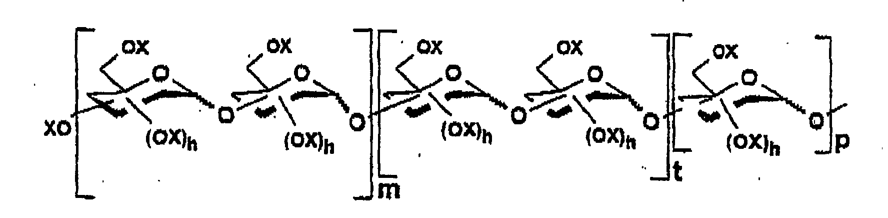 denotes a particular family of polysaccharides Po linked by their anomeric carbon to Pe as defined for (I), 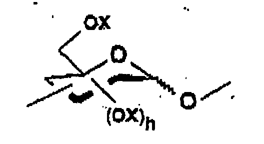 is defined as for (I),
- the OX are as defined for (I) and, for the same monosaccharide, may be identical or different,
- the monosaccharides contained in []ₘ form a disaccharide repeated m times, the monosaccharides contained in [ ]ₜ form a disaccharide repeated t times,
- m varies from 1 to 8, t varies from 0 to 5 and p varies from 0 to 1, given that 5 ≤ m + t ≤ 12,
and the salts thereof, particularly the pharmaceutically acceptable salts thereof.

5. Polysaccharide and salt according to one of claims 2 or 3 of the formula; 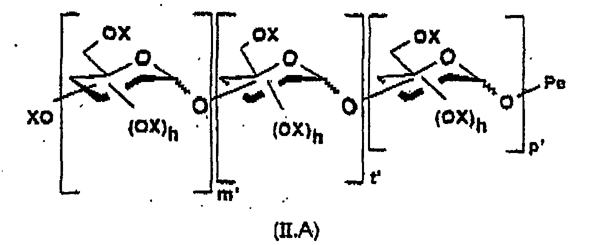 wherein 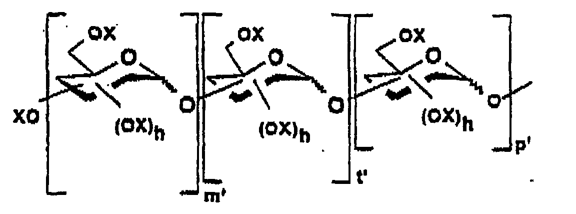 denotes a particular family of polysaccharides Po linked by their anomeric carbon to Pe as defined for (I), 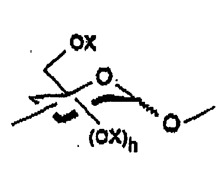 is as defined for (I),
- the OX are as defined for (I) and, for the same monosaccharide, may be identical or different,
- the monosaccharide contained in []ₘ, is repeated m' times, the monosaccharide contained in []ₜ, is repeated t' times, the monosaccharide contained in []ₚ, is repeated p' times,
- m' varies from 1 to 5, t' varies from 0 to 24 and p' varies from 0 to 24, given that 10 s m' + t' + p' ≤ 25, and the salts thereof, particularly the pharmaceutically acceptable salts thereof.

6. Salt according to one of claims 2 or 3 wherein the anion has the formula: 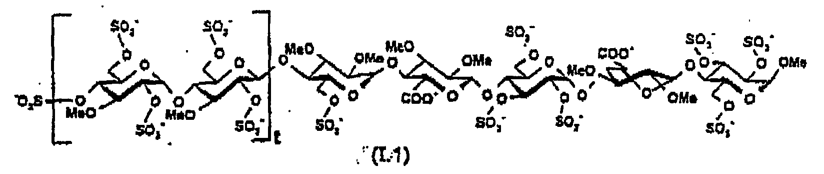 wherein t denotes 5, 6 or 7, and the cation is a pharmaceutically acceptable monovalent cation, and the corresponding acids thereof.

7. Salt according to one of claims 2 or 3 wherein the anion has the formula: 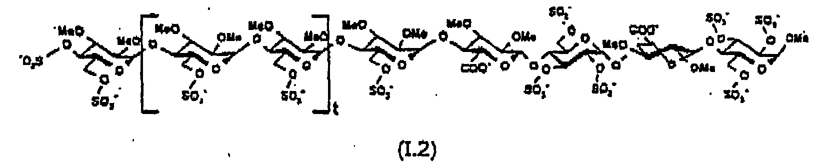 wherein t denotes 5, 6 or 7, and the cation is a pharmaceutically acceptable monovalent cation, and the corresponding acids thereof.

8. Salt according to one of claims 2 or 3 wherein the anion has the formula: 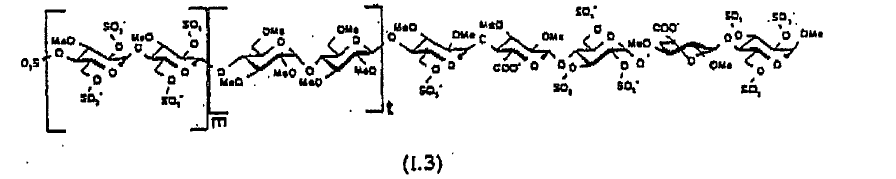 wherein m denotes 1, 2 or 3 and t denotes 2, 3, 4 or 5, and wherein the cation is a pharmaceutically acceptable monovalent cation, and the corresponding acids thereof.

9. Polysaccharides selected from among:
• methyl *O*- (3-*O*-methyl-2,4,6-tri-*O*-sulpho-α-D-glucopyranosyl)-(1→4)-*O*-(3-*O*-methyl-2,6-di-*O*-sulpho-β-D-glucopyranosyl)-(1→4)-[*O*-(3-*O*-methyl-2, 6-di-*O*-sulpho-α-D-glucopyranosyl)-(1→4)-*O*-(3-*O*-methyl-2,6-di-*O*-sulpho-β-D-glucopyranosyl)-(1→4)]₄-*O*-(2,3-di-*O*-methyl-6-*O*-sulpho-α-D-glucopyranosyl)-(1→4)-*O*-(2,3-di-*O*-methyl-β-D-glucopyranosyluronic acid)- (1→4)-*O*-(2,3,6-tri-*O*-sulpho-α-D-glucopyranosyl)-(1→4)-*O*-(2,3-di-*O*-methyl-α-L-idopyranosyluronic acid) - (1→4)-(2,3,6-tri-*O*-sulpho-α-D-glucopyranoside, sodium salt
• methyl *O*-(3-*O-*methyl-2,4,6-tri-*O*-sulpho-α-D-glucopyranosyl)-(1→4)-*O*-(3-*O*-methyl-2,6-di-*O*-sulpho-β-D-glucopyranosyl)-(1→4)-[*O*-(3-*O*-methyl-2,6-di-*O*-sulpho-α-D-glucopyranosyl)-(1→4)-*O*-(3-*O*-methyl-2,6-di-*O*-sulpho-β-D-glucopyranosyl)-(1→4)]₅-*O*-(2,3-di-*O*-methyl-6-*O*-sulpho-α-D-glucopyranosyl)-(1→4)-*O*-(2,3-di-*O*-methyl-β-D-glucopyranosyluronic acid)-(1→4)-*O*-(2,3,6-tri-*O*-sulpho-α-D-glucopyranosyl)-(1→4)-*O*-(2,3-di-*O*-methyl-α-L-idopyranosyluronic acid) - (1→4)-(2,3,6-tri-*O*-sulpho-α-D-glucopyranoside, sodium salt
• methyl *O*-(3-*O*-methyl-2,4,6-tri-*O*-sulpho-α-D-glucopyranosyl)-(1→4)-*O*-(3-*O*-methyl-2,6-di-*O*-sulpho-β-D-glucopyranosyl)-(1→4)-[*O*-(3-*O*-methyl-2,6-di-*O*-sulpho-α-D-glucopyranosyl)-(1→4)-*O*-(3-*O*-methyl-2,6-di-*O*-sulpho-β-D-glucopyranosyl)-(1→4)]₆-*O*-(2,3-di-*O*-methyl-6-*O*-sulpho-α-D-glucopyranosyl)-(1→4)-*O*-(2,3-di-*O*-methyl-β-D-glucopyranosyluronic acid) - (1→4)-*O*-(2,3,6-tri-*O*-sulpho-α-D-glucopyranosyl)-(1→4)-*O*-(2,3-di-*O*-methyl-α-L-idopyranosyluronic acid)-(1→4)-(2,3,6-tri-*O*-sulpho-α-D-glucopyranoside, sodium salt
• methyl *O*-(2,3-di-*O*-methyl-4,6-di-*O*-sulpho-α-D-glucopyranosyl ) - (1→4)-*O*-(2,3-di-*O*-methyl-6-*O*-sulpho-α-D-glucopyranosyl)-(1→4)]₁₁-*O*-(2,3-di-*O*-methyl-β-D-glucopyranosyluronic acid)-(1→4)-*O*-(2,3, 6-tri-*O*-sulpho-α-D-glucopyranosyl) - (1→4)-*O*-(2,3-di-*O*-methyl-α-L-idopyranosyluronic acid)-(1→4)-(2,3,6-tri-*O*-sulpho-α-D-glucopyranoside, sodium salt
• methyl *O*-(2,3-di-*O*-methyl-4,6-di-*O*-sulpho-α-D-glucopyranosyl)-(1-4)-*O*-(2,3-di-*O*-methyl-6-*O*-sulpho-α-D-glucopyranosyl)-(1→4)]₁₃-*O*-(2,3-di-*O*-methyl-β-D-glucopyranosyluronic acid)-(1→4)-*O*-(2,3,6-tri-*O*-sulpho-α-D-glucopyranosyl)-(1→4)-*O*-(2,3-di-*O*-methyl-α-L-idopyranosyluronic acid)-(1→4)-(2,3,6-tri-*O*-sulpho-α-D-glucopyranoside, sodium salt
• methyl *O*-(2,3-di-*O*-methyl-4,6-di-*O*-sulpho-α-D-glucopyranosyl)-(1→4)-*O*-(2,3-di-*O*-methyl-6-*O*-sulpho-α-D-glucopyranosyl)-(1→4)]₁₅-*O*-(2,3-di-*O*-methyl-β-D-glucopyranosyluronic acid)-(1→4)-*O*-(2,3,6-tri-*O*-sulpho-α-D-glucopyranosyl)-(1→4)-*O*-(2,3-di-*O*-methyl-α-L-idopyranosyluronic acid)-(1→4)-(2,3,6-tri-*O*-sulpho-α-D-glucopyranoside, sodium salt
• methyl *O*-(3-*O*-methyl-2,4,6-tri-*O*-sulpho-α-D-glucopyranosyl)-(1→4)-*O*-(3-*O*-methyl-2,6-di-*O*-sulpho-β-D-glucopyranosyl)-(1→4)-[*O*-(3-*O*-methyl-2,6-di-*O*-sulpho-α-D-glucopyranosyl)-(1→4)-*O*-(3-*O*-methyl-2,6-di-*O*-sulpho-β-D-glucopyranosyl)-(1→4)]₂-*O*-(2,3,6-tri-*O*-methyl-α-D-glucopyranosyl)-(1-4)-*O*-(2,3,6-tri-*O*-methyl-β-D-glucopyranosyl) - (1→4)]₂-*O*-2,3-di-*O*-methyl-6-*O*-sulpho-α-D-glucopyranosyl) - (1→4)-*O*-(2,3-di-*O*-methyl-β-D-glucopyranosyluronic acid) - (1→4)-*O*-(2,3,6-tri-*O*-sulpho-α-D-glucopyranosyl) - (1→4) - (2,3-di-*O*-methyl-α-L-idopyranosyluronic acid)-(1→4) - (2,3,6-tri-*O*-sulpho-α-D-glucopyranoside, sodium salt
• methyl *O*-(3-*O*-methyl-2,4,6-tri-*O*-sulpho-α-D-glucopyranosyl) - (1→4)-*O*-(3-*O*-methyl-2,6-di-*O*-sulpho-β-D-glucopyranosyl) - (1→4) - [*O*-(3-*O*-methyl-2,6-di-*O*-sulpho-α-D-glucopyranosyl) - (1→4)-*O*-(3-*O*-methyl-2,6-di-*O*-sulpho-β-D-glucopyranosyl)-(1→4)]₂-*O*-(2,3,6-tri-*O*-methyl-α-D-glucopyranosyl)-(1→4)-*O*-(2,3,6-tri-*O*-methyl-β-D-glucopyranosyl)-(1→4)]₃-*O*-2,3-di-*O*-methyl-6-*O*-sulpho-α-D-glucopyranosyl)-(1-4)-*O*-(2,3-di-*O*-methyl-β-D-glucopyranosyluronic acid)-(1→4)-*O*-(2,3,6-tri-*O*-sulpho-α-D-glucopyranosyl)-(1→4)-(2,3-di-*O*-methyl-α-L-idopyranosyluronic acid)-(1→4)-(2,3,6-tri-*O*-sulpho-α-D-glucopyranoside, sodium salt
• methyl *O*-(3-*O*-methyl-2,4,6-tri-*O*-sulpho-α-D-glucopyranosyl)-(1-4)-*O*-(3-*O*-methyl-2,6-di-*O*-sulpho-β-D-glucopyranosyl)-(1→4)-[*O*-(3-*O*-methyl-2,6-di-*O*-sulpho-α-D-glucopyranosyl)-(1→4)-*O*-(3-*O*-methyl-2,6-di-*O*-sulpho-β-D-glucopyranosyl)-(1→4)-*O*-(2,3,6-tri-*O*-methyl-α-D-glucopyranosyl)-(1→4)-*O*-(2,3,6-tri-*O*-methyl-α-D-glucopyranosyl)-(1→4)]₄-*O*-2,3-di-*O*-methyl-6-*O*-sulpho-α-D-glucopyranosyl)-(1→4)-*O*-(2,3-di-*O*-methyl-β-D-glucopyranosyluronic acid)- (1→4)-*O*-(2,3,6-tri-*O*-sulpho-α-D-glucopyranosyl)-(1→4)-(2,3-di-*O*-methyl-α-L-idopyranosyluronic acid)-(1→4)-(2,3,6-tri-*O*-sulpho-α-D-glucopyranoside, sodium salt
• methyl *O*-(3-*O*-methyl-2,4,6-tri-*O*-sulpho-α-D-glucopyranosyl)-(1→4)-*O*-(3-*O*-methyl-2,6-di-*O*-sulpho-β-D-glucopyranosyl)-(1→4)-[O-(3-*O*-methyl-2,6-di-*O*-sulpho-α-D-glucopyranosyl)-(1→4)-*O*-(3-*O*-methyl-2,6-di-*O*-sulpho-β-D-glucopyranosyl)-(1→4)-*O*-(2,3,6-tri-*O*-methyl-α-D-glucopyranosyl)-(1→4)-*O*-(2,3,6-tri-*O*-methyl-β-D-glucopyranosyl)-(1→4)]₃-*O*-2,3-di-*O*-methyl-6-*O*-sulpho-α-D-glucopyranosyl)-(1→4)-*O*-(2,3-di-*O*-methyl-β-D-glucopyranosyluronic acid) - (1→4)-*O*-(2,3,6-tri-*O*-sulpho-α-D-glucopyranosyl)-(1→4)-(2,3-di-*O*-methyl-α-L-idopyranosyluronic acid)-(1→4)-(2,3,6-tri-*O*-sulpho-α-D-glucopyranoside, sodium salt
• methyl *O*-(3-*O*-methyl-2,4,6-tri-*O*-sulpho-α-D-glucopyranosyl)-(1→4)-*O*-(3-*O*-methyl-2,6-di-*O*-sulpho-β-D-glucopyranosyl)-(1→4)-[*O*-(2,3,6-tri-*O*-methyl-α-D-glucopyranosyl)-(1→4)-*O*-(2,3,6-tri-*O*-methyl-β-D-glucopyranosyl) - (1→4)]₄-*O*-2,3-di-*O*-methyl-6-*O*-sulpho-α-D-glucopyranosyl) - (1→4)-*O*-(2,3-di-*O*-methyl-β-D-glucopyranosyluronic acid) - (1→4)-*O*-(2,3,6-tri-*O*-sulpho-α-D-glucopyranasyl) - (1→4) - (2,3-di-*O*-methyl-α-L-idopyranosyluronic acid) - (1-4) - (2,3,6-tri-*O*-sulpho-α-D-glucopyranoside, sodium salt
• methyl *O*-(3-*O*-methyl-2,4,6-tri-*O*-sulpho-α-D-glucopyranosyl)-(1→4)-*O*-(3-*O*-methyl-2,6-di-*O*-sulpho-β-D-glucopyranosyl)- (1→4)-[*O*-(2,3,6-tri-*O*-methyl-α-D-glucopyranosyl)-(1→4)-*O*-(2,3,6-tri-*O*-methyl-β-D-glucopyranosyl) - (1→4)]₅-*O*-2,3-di-*O*-methyl-6-*O*-sulpho-α-D-glucopyranosyl) - (1→4)-*O*-(2,3-di-*O*-methyl-β-D-glucopyranosyluronic acid) - (1→4)-*O*-(2,3,6-tri-*O*-sulpho-α-D-glucopyranosyl)-(1→4)-(2,3-di-*O*-methyl-α-L-idopyranosyluronic acid)-(1→4)-(2,3,6-tri-*O*-sulpho-α-D-glucopyranoside, sodium salt.

10. Process for preparing the compounds of formula (I), **characterised in that** in a first step a fully protected precursor of the desired polysaccharide (I) containing a protected precursor of the Pe domain extended at its non-reducing end by a protected precursor of the sulphated polysaccharide Po is synthesised, then, in a second step, the negatively charged groups are introduced and/or unmasked.

11. Compound of formula : 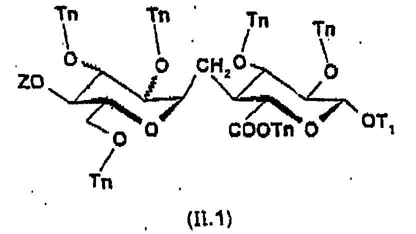 wherein T₁, Tₙ which may be identical or different denote a temporary, semi-permanent or permanent substituent and Z is a protecting group of a hydroxyl function.

12. Compound of formula 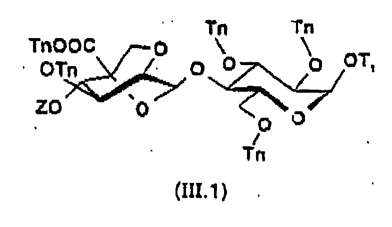 wherein T₁, Tₙ which may be identical or different denote a temporary, semi-permanent or permanent substituent and Z is a protecting group of a hydroxyl function.

13. Pharmaceutical compositions containing as active ingredient a polysaccharide or salt according to any one of claims 1 to 9, in the form of a salt with a pharmaceutically acceptable base cr in acid form, combined or mixed with a pharmaceutically acceptable non-toxic inert excipient.

14. Pharmaceutical composition according to claim 13, in the form of dosage units, wherein the active ingredient is mixed with at least one pharmaceutical excipient.

15. Composition according to claim 14, wherein each dosage unit contains from 0.1 to 100 mg of active ingredient.

16. Composition according to claim 15, wherein each dosage unit contains from 0.5 to 50 mg of active ingredient.

17. Use of the polysaccharides or salts according to claims 1 to 9 for preparing a medidament which may be used in pathological conditions dependent on clotting disorders.

## Patentansprüche

1. Synthetisches Polysaccharid enthaltend eine Domäne zur Bindung an Antithrombin III, die durch eine Kette von fünf Monosacchariden, die insgesamt zwei Carbonsäurefunktionen und mindestens vier Sulfogruppen aufweisen. gebildet ist, welche Domäne direkt an ihrem nicht reduzierenden Ende an eine Domäne zur Bindung an Thrombin gebunden ist, die eine Kette von 10 bis 25 Monosaccharideinheiten aufweist, ausgewählt aus Hexosen, Pentosen oder Desoxyzuckern, deren Hydroxylgruppen unabhängig voneinander durch eine (C₁-C₆)-Alkylgruppe verethert oder in Form von Sulfogruppen verestert sind, sowie deren insbesondere pharmazeutisch annehmbaren Salze.

2. Polysaccharid nach Anspruch 1 der Formel: 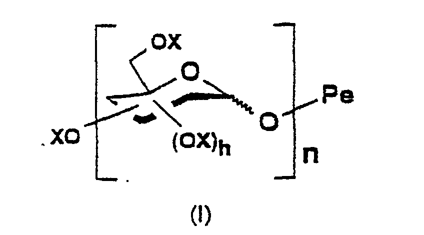 in der
- die Wellenlinie für eine Bindung steht, die entweder unterhalb oder oberhalb der Ebene des Pyranoserings liegt, 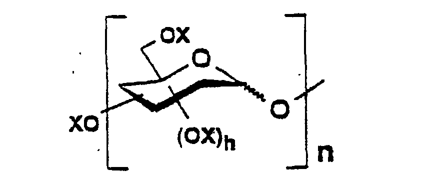 für ein Polysaccharid Po steht, welches n gleichartige oder verschiedene Monosaccharideinheiten aufweist, welches über sein anomeres Kohlenstoffatom an Pe gebunden ist, 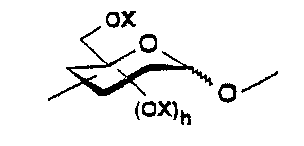 eine schematische Darstellung einer Monosaccharideinheit mit Pyranose-Struktur darstellt, ausgewählt aus Hexosen, Pentosen und den entsprechenden Desoxyzuckern, welche Einheit über ihr anomeres Kohlenstoffatom an eine andere Monosaccharideinheit gebunden ist, und welche Hydroxygruppen dieser Einheit durch gleichartige oder verschiedene Gruppen -X substituiert sind, wobei die Gruppen X ausgewählt sind aus (C₁-C₆)-Alkylgruppen und Sulfogruppen,
- n eine ganze Zahl von 10 bis 25 bedeutet,
- Pe ein Pentasaccharid der Struktur: 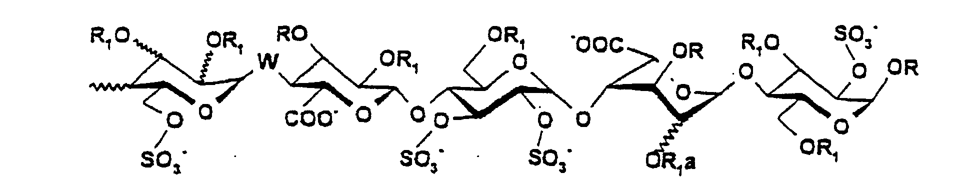 darstellt, in der
- R₁ eine (C₁-C₆)-Alkylgruppe oder eine Sulfogruppe darstellt,
- R₁a R₁ bedeutet oder zusammen mit dem Sauerstoffatom, an das es gebunden ist, und dem an dem gleichen Ring vorliegenden Kohlenstoffatom, welches die Carbonsäurefunktion trägt, eine Gruppe
C-CH₂-O
bildet,
- R eine (C₁-C₆)-Alkylgruppe darstellt, und
- W ein Sauerstoffatom oder eine Methylengruppe bedeutet, oder eines seiner insbesondere pharmazeutisch annehmbaren Salze.

3. Salz eines Polysaccharids nach Anspruch 2, worin das Kation aus Alkalimetallkationen, insbesondere Natrium- und Kaliumkationen ausgewählt ist.

4. Polysaccharid nach einem der Ansprüche 2 oder 3 der Formel: 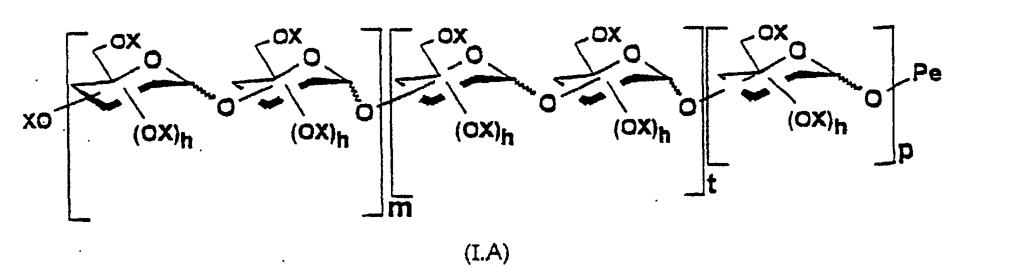 in der 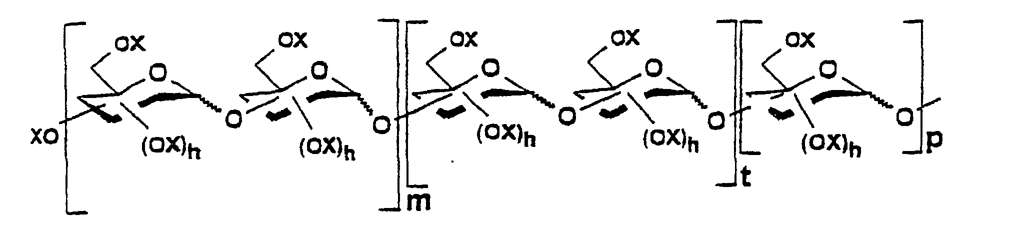 eine besondere Familie von Polysacchariden Po darstellt, die über ihr anomeres Kohlenstoffatom an Pe gebunden sind, wie es bezüglich der Formel (I) definiert worden ist, 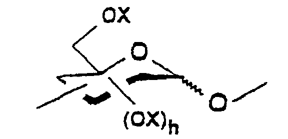 die bezüglich der Formel (I) angegebenen Bedeutungen besitzt,
- die Gruppen OX die bezüglich der Formel (I) angegebenen Bedeutungen besitzen und die im Hinblick auf ein und dasselbe Monosaccharid gleichartig oder verschieden sein können,
- die in der [ ]ₘ enthaltenen Monosaccharide ein m-fach wiederholtes Disaccharid bilden, die in [ ]ₜ enthaltenen Monosaccharide ein t-fach wiederholtes Disaccharid bilden,
- m von 1 bis 8 variiert, t von 0 bis 5 variiert und p von 0 bis 1 variiert, mit der Maßgabe, daß 5 ≤ m + t ≤ 12 bedeutet,
und deren insbesondere pharmazeutisch annehmbaren Salze.

5. Polysaccharid nach einem der Ansprüche 2 oder 3 der Formel: 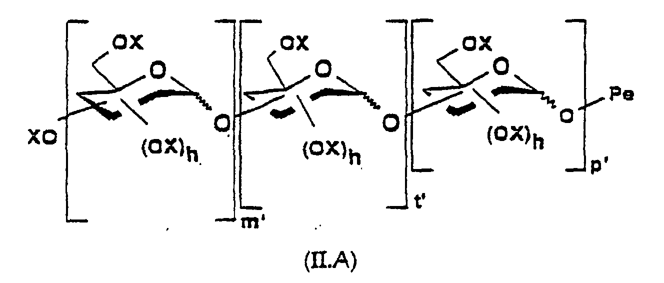 in der 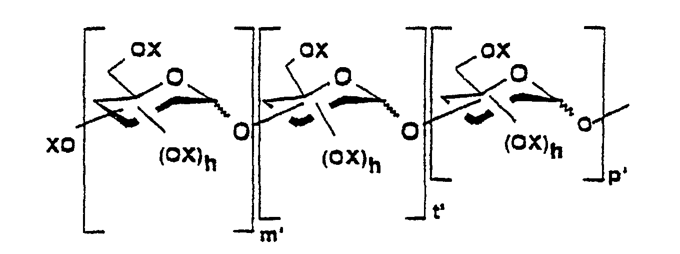 eine besondere Familie von Polysacchariden Po bedeutet, die über ihr anomeres Kohlenstoffatom an Pe gebunden sind, wie es bezüglich der Formel (I) definiert worden ist, 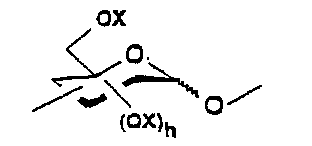 die bezüglich (I) angegebenen Bedeutungen besitzt,
- die Gruppen OX die bezüglich der Formel (I) angegebenen Bedeutungen besitzen und die bezüglich ein und desselben Monosaccharids gleichartig oder verschieden sein können,
- das in [ ]ₘ, enthaltene Monosaccharid m'-fach wiederholt ist, das in [ ]ₜ. enthaltene Monosaccharid t'-fach wiederholt ist, das in [ ]_{p'} enthaltene Monosaccharid p'-fach wiederholt ist,
- m' von 1 bis 5 variiert, t' von 0 bis 24 variiert und p' von 0 bis 24 variiert, mit der Maßgabe, daß 10 ≤ m' + t' + p' ≤ 25 ist,
und deren insbesondere pharmazeutisch annehmbaren Salze.

6. Salz gemäß einem der Ansprüche 2 oder 3, dessen Anion der Formel entspricht: 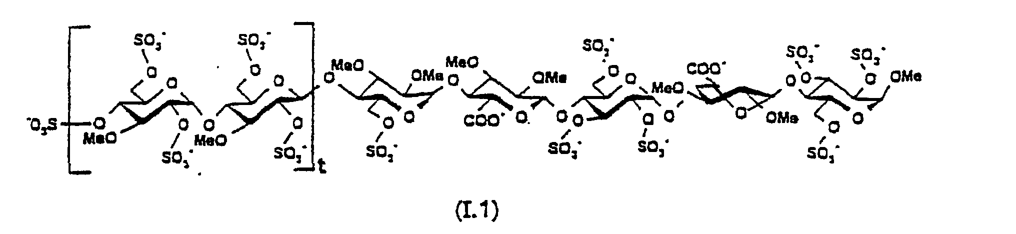 in der t 5, 6 oder 7 bedeutet und das Kation ein einwertiges, pharmazeutisch annehmbares Kation ist. sowie die entspechenden Säuren

7. Salz nach einem der Ansprüche 2 oder 3, dessen Anion der Formel entspricht: 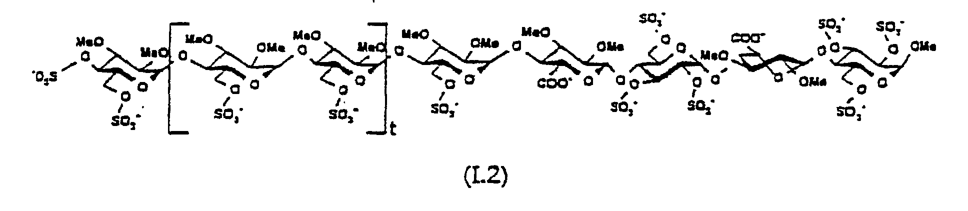 in der t 5,6 oder 7 bedeutet und dessen Kation ein einwertiges, pharmazeutisch annehmbares Kation ist, sowie die entsprechenden Säuren.

8. Salz nach einem der Ansprüche 2 oder 3, dessen Anion der Formel entspricht: 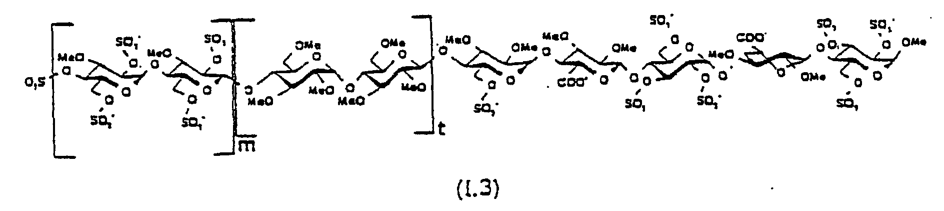 in der m 1, 2 oder 3 und t 2, 3, 4 oder 5 bedeuten und dessen Kation ein einwertiges, pharmazeutisch annehmbares Kation ist, sowie die entsprechenden Säuren.

9. Polysaccharide ausgewählt aus:
• Methyl-*O*-(3-*O*-methyl-2,4,6-tri-*O*-sulfo-α-D-glucopyranosyl)-(1→4)-*O*-(3-*O*-methyl-2,6-di-O-sulfo-β-D-glucopyranosyl)-(1→4)-[*O*-(3-*O*-methyl-2,6-di-*O*-sulfo-α-D-glucopyranosyl)-(1→4)-*O*-(3-*O*-methyl-2,6-di-*O*-sulfo-β-D-glucopyranosyl)-(1→4)]₄-*O-*(2,3-di-*O*-methyl-6-*O*-sulfo-α-D-glucopyranosyl)-(1→4)-*O*-(2,3-di-*O*methyl-β-D-glucopyranosyluronsäure)-(1→4)-*O*-(2,3, 6-tri-*O*-sulfo-α-D-glucopyranosyl)-(1→4)-*O*-(2,3-di-*O*-methyl-α-L-idopyranosyluronsäure)-(1→4)-2,3,6-tri-*O*-sulfo-α-D-glucopyranosid-Natriumsalz,
• Methyl-*O*-(3-*O*-methyl-2,4,6-tri-*O*-sulfo-α-D-glucopyranosyl)-(1→4)-*O*-(3-*O*-methyl-2,6-di-*O*-sulfo-β-D-glucopyranosyl)-(1→4)-[*O*-(3-*O*-methyl-2,6-di-*O*-sulfo-α-D-glucopyranosyl)-(1→4)-*O*-(3-*O*-methyl-2,6-di-*O*-sulfo-β-D-glucopyranosyl)-(1→4)]₅-*O*-(2,3-di-*O*-methyl-6-*O*-sulfo-α-D-glucopyranosyl)-(1→4)-*O*-(2,3-di-*O*methyl-β-D-glucopyranosyluronsäure)-(1→4)-*O*-(2,3,6-tri-*O*-sulfo-α-D-glucopyranosyl)-(1→4)-*O*-(2,3-di-*O*-methyl-α-L-idopyranosyluronsäure)-(1→4)-2,3,6-tri-*O*-sulfo-α-D-glucopyranosid-Natriumsalz,
• Methyl-*O*-(3-*O*-methyl-2,4,6-tri-*O*-sulfo-α-D-glucopyranosyl)-(1→4)-*O*-(3-*O*-methyl-2,6-di-*O*-sulfo-β-D-glucopyranosyl)-(1→4)-[*O*-(3-*O*-methyl-2,6-di-O-sulfo-α-D-glucopyranosyl)-(1→4)-*O*-(3-*O*-methyl-2,6-di-*O*-sulfo-β-D-glucopyranosyl)-(1→4)]₆-*O*-(2,3-di-O-methyl-6-*O*-sulfo-α-D-glucopyranosyl)-(1→4)-*O*-(2,3-di-*O*methyl-β-D-glucopyranosyluronsäure)-(1→4)-*O*-(2,3,6-tri-*O*-sulfo-α-D-glucopyranosyl)-(1→4)-*O*-(2,3-di-*O*-methyl-α-L-idopyranosyluronsäure)-(1→4)-2,3,6-tri-*O*-sulfo-α-D-glucopyranosid-Natriumsalz.
• Methyl-*O*-(2,3-di-*O*-methyl-4,6-di-*O*-sulfo-α-D-glucopyranosyl)-(1→4)-[*O*-(2,3-di-*O*-methyl-6-*O*-sulfo-α-D-glucopyranosyl)-(1→4)-]₁₁-*O*-(2,3-di-*O*-methyl-β-D-glucopyranosyluronsäure)-(1→4)-*O*-(2,3,6-tri-*O*-sulfo-α-D-glucopyranosyl)-(1→4)-*O*-(2,3-di-*O*-methyl-α-L-idopyranosyluronsäure)-(1→4)-2,3,6-tri-*O*-sulfo-α-D-glucopyranosid-Natriumsalz,
• Methyl-*O*-(2,3-di-*O*-methyl-4,6-di-*O*-sulfo-α-D-glucopyranosyl)-(1→4)-[*O*-(2,3-di-*O*-methyl-6-*O*-sulfo-α-D-glucopyranosyl)-(1→4)-]₁₃-*O*-(2,3-di-*O*-methyl-β-D-glucopyranosyluronsäure)-(1→4)-*O*-(2,3,6-tri-*O*-sulfo-α-D-glucopyranosyl)-(1→4)-*O*-(2,3-di-*O*-methyl-α-L-idopyranosyluronsäure)-(1→4)-2,3,6-tri-*O*-sulfo-α-D-glucopyranosid-Natriumsalz,
• Methyl-*O*-(2,3-di-*O*-methyl-4,6-di-*O*-sulfo-α-D-glucopyranosyl)-(1→4)-[*O*-(2,3-di-*O*-methyl-6-*O*-sulfo-α-D-glucopyranosyl)-(1→4)-]₁₅-*O*-(2,3-di-*O*-methyl-β-D-glucopyranosyluronsäure)-(1→4)-*O*-(2,3,6-tri-*O*-sulfo-α-D-glucopyranosyl)-(1→4)-*O*-(2,3-di-*O*-methyl-α-L-idopyranosyluronsäure)-(1→4)-2,3,6-tri-*O*-sulfo-α-D-glucopyranosid-Natriumsalz,
• Methyl-*O*-(3-*O*-methyl-2,4,6-tri-*O*-sulfo-α-D-glucopyranosyl)-(1→4)-*O*-(3-*O*-methyl-2,6-di-*O*-sulfo-β-D-glucopyranosyl)-(1→4)-[*O*-(3-*O*-methyl-2,6-di-*O*-sulfo-α-D-glucopyranosyl)-(1→4)-*O*-(3-*O*-methyl-2,6-di-*O*-sulfo-β-D-glucopyranosyl)-(1→4)]₂-(*O*-(2,3,6-tri-*O*-methyl-α-D-glucopyranosyl)-(1→4)-*O*-(2,3,6-tri-*O*-methyl-β-D-glucopyranosyl)-(1→4)]₂-*O*-(2,3-di-*O*-methyl-6-*O*-sulfo-α-D-glucopyranosyl)-(1→4)-*O*-(2,3-di-*O*-methyl-β-D-glucopyranosyluronsäure)-(1→4)-*O*-(2,3,6-tri-*O*-sulfo-α-D-glucopyranosyl)-(1→4)-(2,3-di-*O*-methyl-α-L-idopyranosyluronsäure)-(1→4)-2,3,6-tri-*O*-sulfo-α-D-glucopyranosid-Natriumsalz,
• Methyl-*O*-(3-*O*-methyl-2,4,6-tri-*O*-sulfo-α-D-glucopyranosyl)-(1→4)-*O*-(3-*O*-methyl-2,6-di-*O*-sulfo-β-D-glucopyranosyl)-(1→4)-[*O*-(3-*O*-methyl-2,6-di-*O*-sulfo-α-D-glucopyranosyl)-(1→4)-*O*-(3-*O*-methyl-2,6-di-*O*-sulfo-β-D-glucopyranosyl)-(1→4)]₂-[*O*-(2,3,6-tri-*O*-methyl-α-D-glucopyranosyl)-(1→4)-*O*-(2,3,6-tri-*O*-methyl-β-D-glucopyranosyl)-(1→4)]₃-*O*-(2,3-di-*O*-methyl-6-*O*-sulfo-α-D-glucopyranosyl)-(1→4)-*O*-(2,3-di-*O*-methyl-β-D-glucopyranosyluronsäure)-(1→4)-*O*-(2,3,6-tri-*O*-sulfo-α-D-glucopyranosyl)-(1→4)-(2,3-di-*O*-methyl-α-L-idopyranosyluronsäure)-(1→4)-2,3,6-tri-*O*-sulfo-α-D-glucopyranosid-Natriumsalz,
• Methyl-*O*-(3-*O*-methyl-2,4,6-tri-*O*-sulfo-α-D-glucopyranosyl)-(1→4)-*O*-(3-*O*-methyl-2,6-di-*O*-sulfo-β-D-glucopyranosyl)-(1→4)-*O*-(3-*O*-methyl-2,6-di-*O*-sulfo-α-D-glucopyranosyl)-(1→4)-*O*-(3-*O*-methyl-2,6-di-*O*-sulfo-β-D-glucopyranosyl)-(1→4)-[*O*-(2,3,6-tri-*O*-methyl-α-D-glucopyranosyl)-(1→4)-*O*-(2,3,6-tri-*O*-methyl-β-D-glucopyranosyl)-(1→4)]₄-*O*-(2.3-di-*O*-methyl-6-*O*-sulfo-α-D-glucopyranosyl)-(1→4)-*O*-(2,3-di-*O*-methyl-β-D-glucopyranosyluronsäure)-(1→4)-*O*-(2,3,6-tri-*O*sulfo-α-D-glucopyranosyl)-(1→4)-(2,3-di-*O*-methyl-α-L-idopyranosyluronsäure)-(1→4)-2,3,6-tri-*O*-sulfo-α-D-glucopyranosid-Natriumsalz,
• Methyl-*O*-(3-*O*-methyl-2,4,6-tri-*O*-sulfo-α-D-glucopyranosyl)-(1→4)-*O*-(3-*O*-methyl-2,6-di-*O*-sulfo-β-D-glucopyranosyl)-(1→4)-*O*-(3-*O*-methyl-2,6-di-*O*-sulfo-α-D-glucopyranosyl)-(1→4)-*O*-(3-*O*-methyl-2,6-di-*O*-sulfo-β-D-glucopyranosyl)-(1→4)-[*O*-(2,3,6-tri-*O*-methyl-α-D-glucopyranosyl)-(1→4)-*O*-(2,3,6-tri-*O*-methyl-β-D-glucopyranosyl)-(1→4)]₃-*O*-(2,3-di-*O*-methyl-6-*O*-sulfo-α-D-glucopyranosyl)-(1→4)-*O*-(2,3-di-*O*-methyl-β-D-glucopyranosyluronsäure)-(1→4)-*O*-(2,3.6-tri-*O*sulfo-α-D-glucopyranosyl)-(1→4)-(2,3-di-*O*-methyl-α-L-idopyranosyluronsäure)-(1→4)-2,3,6-tri-*O*-sulfo-α-D-glucopyranosid-Natriumsalz,
• Methyl-*O*-(3-*O*-methyl-2,4,6-tri-*O*-sulfo-α-D-glucopyranosyl)-(1→4)-*O*-(3-*O*-methyl-2,6-di-*O*-sulfo-β-D-glucopyranosyl)-(1→4)-[*O*-(2,3,6-tri-*O*-methyl-α-D-glucopyranosyl)-(1→4)-*O*-(2,3,6-tri-*O*-methyl-β-D-glucopyranosyl)-(1→4)]₄-*O*-(2,3-di-*O*-methyl-6-*O*-sulfo-α-D-glucopyranosyl)-(1→4)-*O*-(2,3-di-*O*-methyl-β-D-glucopyranosyluronsäure)-(1→4)-*O*-(2,3,6-tri-*O*-sulfo-α-D-glucopyranosyl)-(1→4)-(2,3-di-*O*-methyl-α-L-idopyranosyluronsäure)-(1→4)-2,3,6-tri-*O*-sulfo-α-D-glucopyranosid-Natriumsalz,
• Methyl-*O*-(3-*O*-methyl-2,4,6-tri-*O*-sulfo-α-D-glucopyranosyl)-(1→4)-*O*-(3-*O*-methyl-2,6-di-*O*-sulfo-β-D-glucopyranosyl)-(1→4)-[*O*-(2,3,6-tri-*O*-methyl-α-D-glucopyranosyl)-(1→4)-*O*-(2,3,6-tri-*O*-methyl-β-D-glucopyranosyl)-(1→4)]₅-*O*-(2,3-di-*O*-methyl-6-*O*-sulfo-α-D-glucopyranosyl)-(1→4)-*O*-(2,3-di-*O*-methyl-β-D-glucopyranosyluronsäure)-(1→4)-*O*-(2,3,6-tri-*O*-sulfo-α-D-glucopyranosyl)-(1→4)-(2,3-di-*O*-methyl-α-L-idopyranosyluronsäure)-(1→4)-2,3,6-tri-*O*-sulfo-α-D-glucopyranosid-Natriumsalz.

10. Verfahren zur Herstellung der Verbindungen der Formel (I), **dadurch gekennzeichnet, daß** man in einer ersten Stufe einen vollständig geschützten Vorläufer des gewünschten Polysaccharids (I) herstellt. der einen geschützten Vorläufer der Pe-Domäne enthält, das an seinem nicht reduzierenden Ende durch einen geschützten Vorläufer des sulfatierten Polysaccharids Po verlängert ist, synthetisiert wird und dann in einer zweiten Stufe die negativ geladenen Gruppen eingeführt und/oder von ihren Schutzgruppen befreit werden.

11. Verbindung der Formel: 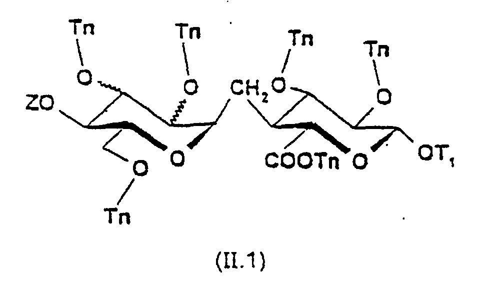 in der T₁ und Tₙ, die gleichartig oder verschieden sind, einen temporären, semipermanenten oder permanenten Substituenten darstellen , und Z eine Schutzgruppe einer Hydroxylfunktion bedeutet.

12. Verbindung der Formel: 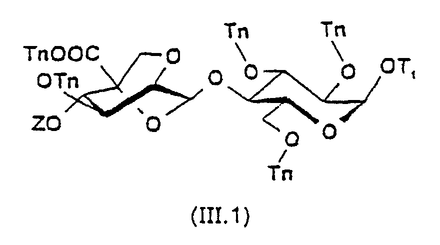 in der T₁ und Tₙ, die gleichartig oder verschieden sind, einen temporären. semipermanenten oder permanenten Substituenten bedeuten, und Z eine Schutzgruppe für eine Hydroxylfunktion darstellt.

13. Pharmazeutische Zubereitungen enthaltend als Wirkstoff ein Polysaccharid nach einem der Ansprüche 1 bis 9 in Form des Salzes mit einer pharmazeutisch annehmbaren Base oder in Form der Säure in Kombination oder in Mischung mit einem inerten, nichttoxischen, pharmazeutisch annehmbaren Trägermaterial.

14. Pharmazeutische Zubereitung nach Anspruch 13 in Form von Dosiseinheiten, in der der Wirkstoff in Form einer Mischung mit mindestens einem pharmazeutischen Trägermaterial vorliegt.

15. Zubereitung nach Anspruch 14, worin jede Dosiseinheit 0,1 bis 100 mg des Wirkstoffs enthält.

16. Zubereitung nach Anspruch 15, worin jede Dosiseinheit 0,5 bis 50 mg des Wirkstoffs enthält.

17. Verwendung der Polysaccharide nach einem der Ansprüche 1 bis 9 für die Herstellung eines Arzneimittels zur Behandlung von pathologischen Zuständen, die von einer Dysfunktion der Blutkoagulation abhängen.
